(19) Europäisches Patentamt
European Patent Office
Office européen des brevets

(11) EP 2 955 235 A2

(12) EUROPEAN PATENT APPLICATION

(43) Date of publication:
16.12.2015 Bulletin 2015/51

(51) Int Cl.:
*C12Q 1/68* (2006.01)

(21) Application number: 15172901.9

(22) Date of filing: 25.01.2010

(84) Designated Contracting States:
AT BE BG CH CY CZ DE DK EE ES FI FR GB GR HR HU IE IS IT LI LT LU LV MC MK MT NL NO PL PT RO SE SI SK SM TR

(30) Priority: 30.01.2009 US 148413 P

(62) Document number(s) of the earlier application(s) in accordance with Art. 76 EPC:
10705647.5 / 2 391 735

(71) Applicant: Koninklijke Philips N.V.
5656 AE Eindhoven (NL)

(72) Inventors:
- KAMALAKARAN, Sitharthan
5600 AE Eindhoven (NL)
- JANEVSKI, Angel
5600 AE Eindhoven (NL)
- HICKS, James, B
5600 AE Eindhoven (NL)

(74) Representative: Kroeze, Johannes Antonius et al
Philips International B.V.
Intellectual Property & Standards
High Tech Campus 5
5656 AE Eindhoven (NL)

Remarks:
This application was filed on 19-06-2015 as a divisional application to the application mentioned under INID code 62.

# (54) METHODS FOR THE SUBCLASSIFICATION OF BREAST TUMOURS

(57) Provided is a method for the analysis of breast cancer disorders, comprising determining the genomic methylation status of more than one CpG dinucleotides. Furthermore, a computer program product stored on a computer-readable medium comprising software code adapted to perform the steps of the method when executed on a data-processing apparatus is provided. A device comprising means for supporting a clinician is also provided.

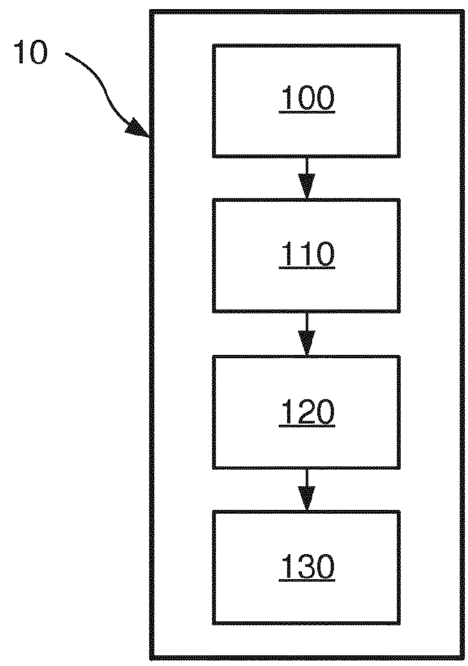


FIG. 1

EP 2 955 235 A2

SEQUENCE LISTING

<110> Koninklijke Philips Electronics N.V.
Cold Spring Habor Laboratory

<120> Methods for the Subclassification of Breast Tumours

<130> PH012398WO1

<150> US 61/148413
<151> 2009-01-30

<160> 600

<170> PatentIn version 3.5

<210> 1
<211> 584
<212> DNA
<213> Homo sapiens

<400> 1
gcaggtgcag ccctcaggga gggggtgcgt gtgtgcaggg aggttgttcc gggaagacaa 60

atttcagttt aatgtaaaga ggtgcttttt cttcccaggc aaagttgggg tgcctaaaga 120

gtaaggagct ctctcagtgg tggtgtgcaa gtggggttgc tcatgtggag ggatgacacc 180

aggaaaggct tgagtcttca aggttcctgg aggacccccc gcctcccgcc aacacatact 240

cacacacatg tacattcaca gagccctgca ggagagggcc tgggagaggg tgggaggccc 300

cttacctctc cagggaccag tgcaaccagc caggttaagc ggccttggtc tctgtgcctc 360

cgccagtcac gcgggcttgt ttcgcggcct gggccgagag cgtgtccatg atgaagcccc 420

gcgcccaagg acgagccccc gccagcgcag ttcgcagcag ctcctggtcc ctgtagcagc 480

gccgcttctg gggaccagtt aggggctggc tggagcgtag cagaacctgc ggaccggact 540

cagttctgga agtgacccct cagccttagg ggtcctctaa gcca 584

<210> 2
<211> 516
<212> DNA
<213> Homo sapiens

<400> 2
cggccctccc gggtggttgc tggctgctcc ctgtggggtg gcaggtggcc ggcttccacc 60

ctgcccgagc cgccgcctac ctagcatgct gggctcgcct cccagcaggg acaggatgta 120

cttgttgagg aagagcgtgc agaagctgaa gaagaaccac agcgtgaggt agagcagcgc 180

ccgcgagctc cacacaccca agtctgactc gatgaccgtg gtctccgtga tggtgacggt 240

cagtacgttc tcatctgtgc cgccgtcgct cttggcaaaa acaatcttct cacttcggtg 300

accaaacaga gagccccagc tgagaggcga cctgcctttc ggcttctctt cggagccagg 360

aaccagctct tccagtgctg ggttttcac cgaggacgac atgctgaagc cacagccacg 420

aacgatttta cctccaggct gggcagcatg ggtcaccgtg accgcccggg ggtggggccg 480

cagcagggac tccgggcgcc aggaacgagg ccacca 516

<210> 3
<211> 470
<212> DNA
<213> Homo sapiens

<400> 3
cccacactga atggattgaa tggatgaagg agtgagtgct gtacttagcc gggggcgcta 60

ggcgctgcgt cgggagcctg ggacatttcc tcctgctgcc tgggaggtaa caccctggac 120

tgctggagtc tgcattccag gagagcgggc agaccacaga aaagggagaa aaggggtgga 180

gggtcaaggc gggcggacac agggaacagg gagcggggac agggcttact cagcaatcag 240

agaagacctc acggaggagg tgaggtgttt aggtggttgg tgaaggcgga agccccagga 300

atgggttagt gcccttaaaa aagagaccta gaaggctccc tcaccccgac cactgtgtca 360

agacacaatg aggagccatc taggaaccag gaagagaccc tcaccagacc ccgaatctac 420

cggcaccttg atcttggacc tccagcctcc agaaatgtga gaaataaatt 470

<210> 4
<211> 669
<212> DNA
<213> Homo sapiens

<400> 4
gtgagcattt gacagcctgg aacagcactc acaccccag gagagcatcc ggcagcctgg 60

agcggaaccc acggccacag gcgagcatct gagagcctgg gtcggcaccc acaccccag 120

gtgagcatct gatggtttgc ggcaacaccg acacccacag gtgagcatct gacagcctgg 180

aacagaaccc acacgcccag gtgagcatct gacagcctgg aacaacagcc tgcaccacca 240

ggtgcgcatg tgacagcctg gaacagcacc aacaccccca ggcgagcatc tgacggcctg 300

gaacagcacc cacaccccca ggtgagcatc agacagcctg gaacaacacc catacccaca 360

ggtgagcatc tgacatcgtg gagcagcacc ccacacccac aggtgagcat ctgacagcct 420

ggagcagcac ccacaccccc aggtgagcat ctgacagcct ggaacagcac ccacaccccc 480

aggtgagaat ctgattgtct ggagcatcac acacaaccac aggtgagcat cggagagtct 540

ggagcagcac caacatccca aggtgagcat ctgacaacct ggagcagcac ccacacctcg 600

aggtgagcat ctgtcctccc ggagcaggac ccatacctcc aggcgagcat ctgaacccat 660

ggagcagca 669

<210> 5
<211> 440
<212> DNA
<213> Homo sapiens

<400> 5
ctgacaacct ggagcagcac ccacaccccg aggtgagcat ctgacctccc ggagcaggac 60

ccatacctcc aggcgagcat ctgaacccat ggagcagcac ccacgccccc aggcgagcat 120

ctgaccgaac agagcagcac ccacaacccc atgcgagcat ctgacagcct ggaacagcac 180

ccacaacccc aggtgagcat ctgacagccc gcagcagcaa ccacacgcac aggtgagaat 240

ctgacagccc gtagcagcac ccacaccccc aggggagcat ctgacatcct ggagcagcac 300

cgacaacccc aggtgagcat atgagagcct ggaacagcac ccacaacccc aggtgagaat 360

ctgacagcct ggaagagcac cccacatcac cgggtgagca tctgacagcc tggaacagca 420

accataccct caggtaagca 440

<210> 6
<211> 438
<212> DNA
<213> Homo sapiens

<400> 6
tatagggcag gggggaagcaa gcaaggggca gaccaagacc tccccccgcc ggtcttggtg 60

gaaaggcctg gacttcgttc tgtccacccc tgccaggtgg ggtgcagtgt cactgcagct 120

cccgccccgt ccctgagtcc tcctcacccc atcccccagg caagaagcgt tgttccttct 180

tggtgacctg ggcacaatgt acagcctttg gcacacaggc atctggcttt gtttttgtgc 240

cctcctctcc ctcaaggctg agtgtggatt tccatcatct ctggacaccc ctcagcccaa 300

agacgctctg tgccatggag aagcccccag gataaagctc gggactgcgc agatccccgc 360

taagagtaga tgagaggccc agtgtggccg gacgtcccct cagacacagt gaagtgagca 420

cctccatgca agcaccgg 438

<210> 7
<211> 443
<212> DNA
<213> Homo sapiens

<400> 7
cccagccgcg cctgcacacg ctcattacac atggcctgca ccggccagcc gggctctaac 60

atgtcctcaa ctttcaggaa accagagcct ccgagtaagt atcagatgag ttgctaatgt 120

tggaaaaaaa gcgagaaaca tgcttggggg ctctgtttta cggctgcact tgaagtcgga 180

atctgcgatc gtaacccaat gctggcctcg ccaacgtgtt ttcggtattg tcgggataat 240

tgaaagtcca gacaaatttg gctatcaggg ctccaggcag cggaatccca tggtggcccc 300

tggtgtgatg agctcgccga ggccgtctca ggtcctgcgc ccaccacggt cttcgggagc 360

agctgtcggc tttgcaaaag ccccaggact ttccgggtgg acatgggggg cttggctttg 420

tcaccgggac ctcttgaaag gca 443

<210> 8
<211> 709
<212> DNA
<213> Homo sapiens

<400> 8
aggagtccac agaagcgatt tcatgttggg acgtgccctg tgcccgctcc ggctcagcct 60

ggctctcagg ctgtggggag aggtccccaa aggctcacaa gccctgctcc tgcccaccag 120

actgagccca agccagggct cagggacctc ttgacagagc tgattggcag gcacagacag 180

atcccctggg actgaggctt ttctctggag cctcccacat tctgggggcg aggaccccag 240

ctgtggctgc atccacaggc accaggggaa gggactggca ccaaggcacc tgtgcagaaa 300

gtgtggggcc ccccaccgag cagggctgcc tccagctggg gttgcgtggc acttccccag 360

caggaacctc tctcatgtac caggtcaagg ctggttgaga aacatagagc cagggcttgg 420

gatgagccta gaaccaggaa gggctgacag cgctcctcca caaggctttg cctgggtgtt 480

ccctccctgt gcaggaatgc gctgcttcct ggtctcccat ggcatcctcc acctgcccac 540

cccgtttaac tcgccctcag ctcagtctgt ctcccgatct tttcattttc ttcatggggc 600

atatcccact tcagcactgt cttgttttga atttgctgac acctgcctca ccccaccccc 660

ggcccctgct ctcgactgca agccaatgat gtgaagggtg tcctgacct 709

<210> 9
<211> 482
<212> DNA
<213> Homo sapiens

<400> 9
agcgaggaag acggccccgt cccagggctg ctgagccctc caaggcttcc ggagcagggg 60

tggatggtga gtgggccctc tccctacgag agccgagcag ggtgggtgtg ctgtggccct 120

catcttcctc agttttccca gtgcctgtct ttccttgtgc caaggacggg agtctgggcg 180

gcttctgccc accaccaggt gagacagctc cgtggtttct aggaagaagc cacccgtgga 240

accatgtagg tgatttcgtg cccatttaaa actacgccga ggctgctctc tgtgcccctt 300

ctctcccagg ctttccagta gagtctcggc tccctctggg agccacctgc tgcagccatg 360

ggtgcatcgg gtgcgtgtgc tcctggggga ccctttttgct ggggctgggg tcagacagcg 420

cttcacccag gccgggattc tagggagagc ggtgccgaga agttgccctc gtagccaagg 480

tc 482

<210> 10
<211> 450
<212> DNA
<213> Homo sapiens

<400> 10
tcaacttcca gaggccgccg cttcatgaat tacacaccat gcaaacgtcc ggccagcggc 60

ttaagtataa agcgtgagaa acacaggaga tcattccgtg ttcgtggttt tgtaaggttt 120

gaaaagcgat ttaaaataat tttaggttgt cctggggttg gcgaacagtg taataggaaa 180

tttgagaagg caaagttttt aaactgaaag gttttgcctc cttgtttcta atttttagaa 240

acatgatgga tgtcagccgt gacgttgggc cgacagcttc tgggaacgat ttcccagctc 300

gctcggaccc aggcggggga ccctgccagg agggcaccga gggccatggc cacgtacaga 360

aagaggacgg cttccagtct tcaaattgga aacctgcacc cggcctccct tggctaacct 420

caatcccata agcaatccga aggcattgct 450

<210> 11
<211> 416
<212> DNA
<213> Homo sapiens

<400> 11
ggcgaggaaa tacaggatgc gggctctgaa atgggaacgc tgctgtcccc ggagcattgc 60

acggataaat aatcgtctta gaataatgca tcggctttag gtagggccag gtggcctgag 120

ctgcccgaca gaggcctccg tggccctgcg tgagggccag attcagctcg gctggtcagg 180

ggagattcca ggggctggga ggggcgttcg tgggcagtgg cttcgtggac cccagtggga 240

ctttgtggtg gtctgtgcag cgcagccagg cccacccgcc tgccccagag gcgtgggctg 300

cgcagaggct ggggctgttt gcccttcccc ttggcagcgg atgtgcttct ctccgagctt 360

cagggccggc ccctggtggg gtgaatggca gtcacacatc ctccaggctg tcatgg 416

<210> 12
<211> 524
<212> DNA
<213> Homo sapiens

<400> 12
tttggatggg aaaagtgctt tgcagcccag ctgctgttct cccggaggcc ggggctcctg 60

gaggctgccg cttgcttttc tccgcctggg tggcaaacgt gtgtacttaa cttgaaaaac 120

ctcagggtca ataagggttt cagtccacat tttcagagcg atcttctcga cattttagaa 180

aagcctagtt ccctctgtta aaccctgttt cccccaggat gcacccttgt cactcagcac 240

ttccccatct gtccccacca cccacttccc aaactcgagg gccctgctcc ttgagggcac 300

ccctcctgga ggctttgctg aagcaccgat tgcctgcaca caccagcccc gccccaggtc 360

agtgggcctg ggtggacctg tgaattacag gtctagcaag tcctcagggg atgcaggggc 420

tgctggcggg gactccactc agactcctga gccacagggg ctgaaaccca cagccgggtc 480

cacccaacct ctaaaacctt ccggtcccac ctcgctccaa acac 524

<210> 13
<211> 427
<212> DNA
<213> Homo sapiens

<400> 13
tcttccaggg aagctattgt gtcaggtaaa gaggcggcca tacatcagcc ggctctgccc 60

ctcaaataga taatcaattt ccctgaaagc atcaataacc agagtggaca tttattgccc 120

cgtggatggt ttatctttgg ggctggggac agcagggccg tgatgaatac caaatctgcc 180

agggaacaca attaaatgct gcgaagataa aggattttat atcgtgcaca aaaaagcaat 240

caaattcata attgaactcc attgcatgaa accagccaga gataaatatc acacccaaaa 300

gtgtataacc tgggtgccgt gttatgggtc aggcgacagt gttgccaata cccagtagtg 360

tctccctctg gaggccccgg tgcgcccctc tcgctggggt accagcttcc aattgccact 420

ctctctc 427

<210> 14
<211> 607
<212> DNA
<213> Homo sapiens

<400> 14
gacggagccg ccggggcagc cagatgtgtg ggcaccgcac aggcaaagcc ggtggcatgc 60

tccaatgcaa ggccatcttc tttacctctg caggaacaaa tcggatcagg tgaggctgat 120

tctcctgcat ccgtgcctca gcccttagtg gtcccagatc attgtggtgg ccacttttag 180

gacagggcca gagccttggc ctcgacctcg cccagcaggc aggcagccct cccaggaccc 240

ttgggtgaaa ctccagcttc ctcctccagg tagctcaggt gccagcaggt aaggctggga 300

gcagctatcc ctgaagccaa cccacttcct tcctgttcag agctaagagc tgccaagaag 360

cacggggtgg ctgctcagat tcatgaggtc tgctctgcac ttttacaacc taataaaatt 420

tgggggcatc tgagaggaac accgaggggc acacggggac gttgacatgt tgtggtgggg 480

tcattcagac aggacctgag tgccgctctt catttggtcc tgaattcggg tgtgcggtga 540

ggctgcgcct ggcaccccgg atccctccac ggcttccccc ttatctagag catctggccc 600

catcttg 607

<210> 15
<211> 391
<212> DNA
<213> Homo sapiens

<400> 15
gcacccggca ccctgctgcc cggcggggcc tgcagtcagc caggacaccc ggcagccaca 60

aggccgccct ctctggagcc tgtggtctgg ttcggctagg gtattgtgat ctgaggaccc 120

ttagaaaact ggccacccga gaacttttcc agtgtgactg ctggcctctc tggcagacgt 180

gtccaaactc agaaaacctc tgctgctctg accacaggga acgtcagagg ggacggggca 240

gcggaacaaa gcacaaagcg tccctgcgtc ctctcaacgt ggcccagctc ctgcagacac 300

tcagcgagcc gtggcctctc ccatcctccc acgcttccct ccggccggcc ctggagatcc 360

gtggagccac acggtgccga gcgcggcgct g 391

<210> 16
<211> 477
<212> DNA
<213> Homo sapiens

<400> 16
catcaggcgg agggaggtcc gacgccgtgt ccacacccaa tctgccctcc ggggagacgg 60

gagtggggga ggcaagcatt tgaaatcctc cctttccaac agagaaacac tgttggggag 120

acggtcagag gaggctgccc ccagctctgc ttttccttgc ctgggtgcag agtatttaaa 180

aataaaagcc gcagctgcca agacagccca tggagcgttt gctgtgtctg gacgctgttc 240

taaggagcgc ttcacataga tgatcttagc ctgccccaga gcggccccag gaggaggcac 300

tgtctctaaa cccactttac agaccagcaa gctgacagca ggagatgtga agtcactggg 360

cagatcgagc agctcgggaa ggtgggcacg ggacagatcc aggctcagag gtgagagtcc 420

tggagaccgg gccagtgtgg cggttcctta tctccagctc tgggatcgat aaactgt 477

<210> 17
<211> 602
<212> DNA
<213> Homo sapiens

<400> 17
cttgggggcc agcccgcttc gggagaaccg agcctctgcc ctgccgtccc ggggctggac 60

acattccctc tgcagagctg cagctctgtc aagaatgcga ggcccatttt cacacaagtg 120

attcagacca cggggaagcc actttcagat gcattcgact ttgtgttttg cttttacact 180

tacaatgaag aggaaagaat tctctttcgt gagaactttt tgctcttggg agaaaataaa 240

gaaagtcact tccattccca tatgcttttc ttttgagaaa gcctcacgat aatttgcttt 300

tcctctctcc aaaagaggtc aagaggaggc agtgcgtgga ggacagagcg catgagccag 360

ccagatcgtg agatcagaga taataagatg ggaaacatta tcagaatagg acatgttctt 420

tcagcatttc tttaaagcag aagtttcttg taccgagaaa gtgaaaatgc cataaactat 480

gcccccgttt ctggcccctc catgagcccc aggggtgtat tcagggccag ccttacctgc 540

ctggaagaca gccgggagac ctgtccatac cactcagtaa gggcaggcca ggctcaggtg 600

tg 602

<210> 18
<211> 660
<212> DNA
<213> Homo sapiens

<400> 18
ccgggttcct ccccgtggct tccgggggcc ctctggttgt gggagacccc gggctcctgt 60

gcaggcctgt gttaggttca gggttccctg tttcagcagc ttgaccccga gcaggagcca 120

ggccccaggg aaagctcctt gccgccaggc aggaagcgaa agggaagacc agagtccccc 180

gagccaggtg gaggggcttg tggaggatgc ctggggaggc ctaaggggga ggtcagagac 240

ccagaaagcc ccctacactt cccagatgct gcgttcccag ctgcattcat aactgcagct 300

gcaaagcctc tgccacccac tgggggtgtg acaggggcgg tgaccacagg cagggccagc 360

gtgcagggag agaggtcccc tggaagcagg aggagaggac agcgtcccct gggcagagga 420

caggcccagt aaccctgggg aggaggggc ttcagaggcc cccaccccgt gtcactgagc 480

ctgaggatga gaggggacca gagggagagg ccgacatcgg ggccctgcct gaggaaggcc 540

ctgctaaggg agcctgcctg gggccctggg ccacagagga cggcaccgag agggcttcag 600

gcctgctgtc cggacacttg tccactaatt aggattcccg cgtggggccg cctgccgcaa 660

<210> 19
<211> 544
<212> DNA
<213> Homo sapiens

<400> 19
ctgcctaaaa cagtgtcctt ggtctataaa atgggctagc tgtgatgacc gggcagggcc 60

gtggagaaga tggaggttca ctgcacacag tggcacctgc ttagggatgc tatattttta 120

tatcgtcttg gctggggatt tcctccttga ttttctagtg agttctcttc aaagtggccc 180

ttgtcacaga actctaggct atggattggc atcgtcattc tcccagttgg cactgcctgc 240

tgtccagccc ctgcaggcca catggaagcc gcctttctct ccacaccagc cagtcctttc 300

cagcacagct ccgaactcag tgcgattggc aaggcgagct ggctgactga cttttgggat 360

agggccacac caagcctacg cccagcaatg cctacacagg tctggcaggc tggctctgtg 420

gctccaggca tgtgggcagg atggagcagg cgggacaggg gttgtctttt cttaatcacc 480

tcccctggtg cagccggcca tgctggactg tggtgacccc agagcctggc tccctggttg 540

gtct 544

<210> 20
<211> 477
<212> DNA
<213> Homo sapiens

<400> 20
gaagcaagga gctcgggcct tggggagttc cctcatgtca ttctcatgcc ggataaggtg 60

ctcctgtgtc caccctccta ccttggtcct tacttacatg agcccaactg ggaatgtgct 120

ccgtgattca ggtccactta atttctagaa tgctcaggta gaaactaatc attaaaaaca 180

tgtaaattaa tttaccccag gagcttaata gattagccat cgactttttt cctccttttg 240

gattctaacc tttcacggaa attaatcaca aagggctcaa aatagccacc gaggattaac 300

atgctctgtt cagtcttggt gagtgggaac tcaggcccag cccctcagag aggaggaagg 360

atccccgcat cttcacattc ccttttcccc tcccagcagt cctctgttgg attcgataca 420

gccaccccgg gagtggctgg aagcccctgg ccacctgact cttccttcac agcagtt 477

<210> 21
<211> 656
<212> DNA
<213> Homo sapiens

<400> 21
aatgaatcat ggccccatct gtagaagctt aaggaaacgc tcagggagcc ggctgttctg 60

ggccaacagt gaaggacagg atacatcttc caggctgcta tatccactct acaaggagca 120

ggtcagagac tcaggagaca gcccagggct cggacaggga gcctggcttg atttaggtca 180

gtcttaaaac atcactggaa ttgaatgaca acgtccagct gcagcctctg ttgctggtta 240

gaaggttatt cacccagggc ggatgggcag cgccactcgg ggtgaggcac tggggcaccc 300

gtgctcctct gtgtcccccc agctggaggt tagcaatgac ctggagatca gctaatgcat 360

cacagtgatg ctgtttgtgg ggctctctgt gccagaccaa gtgtgttttg taagtatctg 420

ctcatcctgt gcttgtcact gactcctaac ccagcaaaag ttgggggaag agcaaacatt 480

tcttgaagca ccccctgagc tctggcagag cagatgggag agggatctac cttcagccca 540

ggtctttgtt tttcatgcac tgtgtttatc cacaggcacc tgaagctgga gtccctctcc 600

ttcacccggg ccctacccca gctagatgaa gggcttctca ctccactccc gggcac 656

<210> 22
<211> 491
<212> DNA
<213> Homo sapiens

<400> 22
tagagactct catttggctc tagtgaggct gctctctgaa gtcagggtcc ggagctagcc 60

ccttgccagc tgtatgactg ctcttctcaa aagtgggggg cccctggggg actgctgggg 120

tcactctccc tgcattcctt agagtggccc cttctccagg ctgacgtcaa tttcactgct 180

ggctctcaca agggcaggtc ccacatccac agagacagag gcagcttggg gctggtgaat 240

cccagagggg attttatcaa gttcatgatt ttataaaagt ggaagtgagc tagtatctac 300

atgagttgag ctttaaggag acaaagcatt tctagtgggt tcccaaatga tacatctact 360

cacaacttca gagtggggcc ttattggaat aaaggtctta gcaggtataa ttaagcaaaa 420

ggtcttgaga tgagatcacc ccggattctc caggtgtgtc ctaaatccta tgacaagtgt 480

ccttataaga g 491

<210> 23
<211> 351
<212> DNA
<213> Homo sapiens

<400> 23
cctgaaacct gcctgcagga cctgggatct ggagagctgc ccgctggccc ggaggatggg 60

cacccatcca atcttggctt aggaaagggg ctgcagaggg gcgggtgagg ggtggcgggg 120

atgcagcccc accctggcca gtgcctcatc tcctgcctcc gcataggcac caagtctttc 180

aacatgatgt ccccgacggg cgacaactcg gagctactgg ctgagattaa ggcaggcaag 240

agcctgaagc cgacgcccca gagcaagggg ctgaccacag tgttctcagg catcgggcag 300

ccggccttcc aggtaggcgg gcccagcagg agcctgcgac ccggcttccc t 351

<210> 24
<211> 407
<212> DNA
<213> Homo sapiens

<400> 24
cagatcaggt ttctcctgaa ggccctacct ggagtgatgt tgctgtgccc ggtccctagc 60

tccgtgcctg gtaagatgtt cacggggctt agtgaacacg tgtctagctg agccccactg 120

agctctgatg gggcatttt gttgcttgtg gagcaagaag ccatttggat ggggcagagg 180

tcagctgcca ggactgtgtg gcctgacctc catcctgcac catgcccagc agagtcaagg 240

ctgtcagagg gattttctcc cgcgggtctt cctgtgcaaa tcaataaccc ttcaggcagc 300

agcagggagg gaagcatgca ctgaggaggc aagggtcagg gctgttcctg cagcttccgg 360

caagtccttt gcagggtttt tcagtatctt ccgctcgagc aggtgct 407

<210> 25
<211> 563
<212> DNA
<213> Homo sapiens

<400> 25
aggatctggt aagaggatga atttggctct aaacaaatgc ccatggcccc ggaagacctg 60

tcggggccac caagttagtt tctctggcaa aatttgattc cttccatctc accactatag 120

taactgttgt tgttatgtta ccaggagcca cacaaaggtt gacccatcaa ggtggagcca 180

cgttaccagg agccacacga aggctgaacc agcaaagtgg aggaccagct gtttaattga 240

ctcgggtctg gtgacaggcc aacatggaga gagacagccc agcttcaaga acagcaaggc 300

agccctttgc acagttccca ctggggtggg gaggctggag atgaggactg cattccacat 360

cagagttgtt ttatttctgg atattcacag acagctagaa gtcagaaaaa taagccaaag 420

tgggaagccc ctggcagacc agctccactg gcttcctctc cagagtcact gttccgaagt 480

gacaggtgga agctctttca gttcctcctt ttccgggtac acttcagaca ccttattcat 540

cttggtgaaa atctggttga tct 563

<210> 26
<211> 330
<212> DNA
<213> Homo sapiens

<400> 26
cttccttctt atctctcaac ctcatctctg tccccgccaa tattcagccc ggctaagaag 60

cttaggagct ggctaaggct gctcctccac ctggatgcac acacagggct agggctgtgg 120

agaggcggga gcaccagcgg cccacgccga ggaagatagg cgctccctcc tggggtgggc 180

agcgcaggtc tggcctgcgg gtggggggtg gagagtgaga ggcacgcggg gcaccagctc 240

agctcccctc tggggatttc gtactcgcag tttaaggaac cggtggagca cgaactccac 300

tcctgagctg gcgcaagcgt ctcccttcca 330

<210> 27
<211> 549
<212> DNA
<213> Homo sapiens

<400> 27
ggtctccttg gccagcagca gggcctgccc ctcggcttcc agctgctccc ggcgggcctc 60

aagctgggcc agctgccatt gcacctcaaa caggctgccc tccagggctt ccttctccaa 120

gctgcagcac atacagtgcg tgaggccctg ggactcagcc tccctggccc caggaaactg 180

tggatctcag ggagtggcca ggcatgggcc aagtgccaat ctacctgcct ggccaggccc 240

acactccctg gggaagcctg agcccacagc taggacgagt tggcaagcca gactccttag 300

taactgcccc tgggggcccg tgcctgggtg aggctgttca tggcgtggcc ccaggaggac 360

cgcccagggc gggttgggca gagcccaagg ccttactgca ggtgtgtggc ctcctctgac 420

acggtcctgc cttcacgctc cgcaggccgc cagctgcaca gccaggccag cgtgctcctt 480

ggctagcgcc tccttctccc gggccacttg ccccagcgtc tccacctgtc actgggcctc 540

ccaccgggc 549

<210> 28
<211> 480
<212> DNA
<213> Homo sapiens

<400> 28

catgacaaat acatagaaac taaagctcta ttattttgct gtaatgggcc gggggaatag 60

ctctgaaata atattcttct gtctgcaata aaggcttttc tctgaagctg tcttaatgct 120

gtgaacaaag cctggagatc tctcaagggc tccttctctt gaattactgc ccctctcaag 180

gccttctgcc atgataagtt caccaaccac ctggcgggtg gatagggacc gagacggtga 240

agcagcgggg ttgagggagc ataagctgcc acgcacaggc tggtaggggt ggagaggatg 300

ggtgagtgtg agaaggcaca cccagaacca acagtgtctg ggtggcagtt gtgccatgct 360

cagtccccac actctggaga tgcaccttat ctgtcaacat ctacacttag gcagcagccc 420

tcaggaacac cggaggtcaa ttcagcttga gagctggact cctcttaagt gaccatgtgg 480

<210> 29
<211> 618
<212> DNA
<213> Homo sapiens

<400> 29
ccccagcccc aaccttcctg aggcccccag agcatgtgag ccagggctcc ggttccatct 60

cgcctctgct gagcagcgga gcctttctgg tctaaccccc atgtcacggc ttccctgatt 120

ccaatttggc ggaagacagt gggtttgcag agtggctggg tctgcactcg gcttcactac 180

acagccgctg tgtggccctg gacaagtcgc ttcacctctc tgggcgcccc cgcttctcca 240

tctgacaaat gcggctgtaa tttacccttc atggcattgt ccagtgggat catctgagca 300

aatgtgcttt gtaaatggac aggtgctcca tgctgccgct ttcaggtaat ctacagcctc 360

ctccacctga ctgcactgcc agccccctg caccaccatc tccttcagat cttcccctac 420

caacagtctg ccccatggca cggagctcag aatacaaagt aaccactttc ctcccgtgac 480

ccctgcctcg gtttataatg acacgtcctc aggccaaatc cttcactatt ggacaactgc 540

cgatccccgt tatggggctg gaagcctccg gtgggggct ttgttggctt ggtcactgca 600

ggcacgttgt aggggctc 618

<210> 30
<211> 583
<212> DNA
<213> Homo sapiens

<400> 30
tcagccaggg agggcgggac atgctagact gagaagctgc ctttcttccc ggaaaaaatg 60

accttgggcc aggactggca atagatcggc tgggatccag acagcctggg tccccagcgg 120

ccctgggtgc tgcagtcagg gcggaaatgg gctctcaccc cctcttgcac tcgcatccga 180

gggagtaacc ctgcatttgg gacaaatcca aattaaacta gactcccgag aactgctcct 240

aggtgatccc tgaaccacat gggtctccag tgtccaagtg accctctatc tccctcccaa 300

caaacctgcc ccagggagtt ctctggccaa ggggacatac gcgggctgga gggttcacat 360

cttagttcgg ttgcttcctg gctgggtgac cttggacagg ttgctcccct gctctgagac 420

tctgggcagc ctaatgatgt gatgccccag cagccttggt gagaggtgag gggaggaggg 480

gaattgcaaa gcacccagct catgttgggc accgcataga tgacagcccc ttccggctgc 540

tgctttccac ctgccctgct ggggatcagc acacagactc ccc 583

<210> 31
<211> 749
<212> DNA
<213> Homo sapiens

<400> 31
atcaccggct ccctaggggc gggaaccctc tttgaaaggg gagcaaggcc gggtgcggtg 60

gctcacacct gtactcccaa cactttggga gtttgaggcg ggcggatcac gaggtcagga 120

gatcgagacc atcgtgacta acatggtgaa accccgcctc tactgaaaaa aaaagggggg 180

agcaaaggaa gctcctttgg ttgggctgag cctggaaccc cagggagaag acccattagg 240

aaagacttcc agaaaagaca ggtagccctt gttggggtca ctatcaggat aaaagaccag 300

agttattttg tattcacaga tttgttacac taattgatca gaacccaggg tcccactgcc 360

agtggtggcc agtgttgcct cttagagatt aggctggagc cacagaacct ggccaaggtg 420

gtccaagcct gcagccgctc ccctgttggt gggttcttca agaacaactc gggtgcttag 480

gcttctcaga gctgaaggag aggcctgctg tcatgagagc ggtgcaggag gaggtatttg 540

ggaaagcctg gagatcccca acctacttct tcccccattg gtccttgact gcaccctggc 600

caggggcgat gggggaaggg aactcttggt tctggaataa tatcttaacc acctcctcta 660

tgccaggccc ctgagcactt tacacccatt aatctgttcc ggttctccca accactctgc 720

aaagtaaggg ttattattcc catttccac 749

<210> 32
<211> 346
<212> DNA
<213> Homo sapiens

<400> 32
gtgaggccgc gccccgccgc gttcagcgaa gggaagcagg caccccgccc ggctctccgc 60

tgcctccaca ctcggggacc ctgggcgctc acgtgttccc ctctgggcct caggattcaa 120

ggatgcgccc tcttgcaaac agtccgtggc ctgcgacaga aagaaaaccc ctgggagcgg 180

tcggggtggg gacccacaaa acgctgtggc taaaccgcat ggcgccaagc ccgatcggag 240

cgtggcgtgg gaatcgagtc aaatttaccc tgctaggtgc ggctccaaca aggtcccggg 300

tactcctcta acgcccagtg agggcagcac tgcctttgtc cctgcc 346

<210> 33

<211> 396
<212> DNA
<213> Homo sapiens

<400> 33
ccggacgtga tgcccccggc ccacaaccat ccccacactg gccgagcccc ggatacctgt 60

atgggagccc gagagggagc tgaggagccg cgagtgctgg ctgtggccgt cgtgaacctc 120

caccacgtcg ttgagggccg tgtgaaagaa ggcgaactgg ccaaacacca ctgtggagga 180

gacacagtgt ggggactggg caggcacgct gggggcagga gaggggatct aggggtctag 240

gggcccaagc caggaggagg gcagccctgg ggaagttgtt ggttcctggg ctgtggcttg 300

ctgcactggt tagtgcagtg gcacagtctg gcagtggcca gacacccggc acagggaggc 360

ggggagctgg gaaggccggg ctggccttca caaggg 396

<210> 34
<211> 807
<212> DNA
<213> Homo sapiens

<400> 34
ccccttggtt ctggcggggc tcacttgtgc ccttctgccc tcttgtgtcc ggaatgtatt 60

ctgcagcccg tttgagaaac gtcagcactc ggccgtcctt ttggggagac tgttctgcga 120

ttcccacaat ggggctgtat ataattggga ggcgtctgag gaattgctca ttttggaatg 180

agggtgagga gtcagaaaac atatgaacaa caacaaccaa aacccatacc atagtccttg 240

ggcacagtaa caaaatacaa gcagatctgt ccactggtgt agttctgggg gtagttgggg 300

gacaagacca ctccgtccga acccacatac tgtcccccac agggggctga aagagaaacc 360

agatagagag tcaggtgacc ttgtgggcct cttaccagtg accatcctct gttctgcccc 420

agcctgagct tctggcccca gtagggctgc cctgaggtgg gcaggcccta gcttggagaa 480

ggcaagtcct gcggaccctg ccctgcccaa gaacgtgcac ccctggcctg gcatgtagcc 540

tgaaacttgc cctgaagcta ccgagggccc tcttgggcag gtgtcagttc ttgccctcta 600

cctattaacc ctggcattgg tccctgaagt cacagcctta gggagcacat taggaacctc 660

ttctgcttac acgtggagcc tagaaatgat ctgctgccct ggctgacatc taacctggac 720

agtgagtgtc agtgtgaata gggagctgaa gcttccccgg actaacccca tggggagtgg 780

ccagcccatg tgacccagcc accttct 807

<210> 35
<211> 573
<212> DNA
<213> Homo sapiens

<400> 35
aggcagagac agaatgaggc cctgctttgt tggggctgtg tggggtggcc ggagtctact 60

ggcctggggt ttcttggatg tgcaagatag gaggctgctt ttgacaaatt cctttgtgag 120

caaagtcatc acgagtgtgt gggctcagag acaggccctc catgccaacc accacctgct 180

tccctcgtcc cacaccagca gccagaagtg gaccatccac agcccctggt gacctccccc 240

caccacgcct atgcttcaga tctggggtgg agactctaga tgaaatgagc cccccacctc 300

agccccagat ggagaaatga ctcaaagaaa gttaaataaa aggtgggtgc atgatcagaa 360

acggtaaaga atgtaagaga aagctgcagt caaagttcaa agctgggact tcagctcaaa 420

cacagacatt tcttctgaga aacctcttcc catcagatac ttctcgagcc agagtaagca 480

gtcaccagag catcccctac ccagtgccac ccatcaaggg caccgggcag agatggcacc 540

tgtgatggtt ctggtaggtg gtgcaatgct ctg 573

<210> 36
<211> 318
<212> DNA
<213> Homo sapiens

<400> 36
gagtctttat tgtttgtggg gcaggaggag tgatcattta ccaccgctcc ggttcgcagg 60

gagaagcatt tccactgaaa catcctccag acctagctct tcactcgctc acccccacct 120

ccccttggga agaggaggaa ctcctctgat tagtgggttt gggtgaaggc actgaacatg 180

cttagctcct gccctgcctc tctctgggtt ctgcctgcct gcagaaaatt ccctgcatct 240

tttttccact agcggagcgg gctctgcccg ggcgaggcag atctgcagtt ccagggagga 300

ggttctgtgt tctcctgt 318

<210> 37
<211> 386
<212> DNA
<213> Homo sapiens

<400> 37
tcaaagcagc cagaccacct ggtgggaggc ggcacacact ctgccagtcc ggacacgggt 60

tagggagtgg catcacattc cgccgagtca ttgcctgcga agactaacgt cacctctgat 120

gtcagggctc caaggggctt cgttccagct ccaggctcca ggaaagggcg tcgcagggcc 180

cagaggttac acaacttccc ccaagatcgc tgtggaaatg actggcagag cagggattca 240

aacccaggtc cctgtgggtg cctctgccca gccgcctgca gggtctgcag gtggccatgt 300

cccacgtgtc acctttacag acccacggct gagagccggc ccagccttgg cctggcttcc 360

ttctttctca tctctaaaaa tgcctt 386

<210> 38
<211> 685
<212> DNA
<213> Homo sapiens

<400> 38
ggacgcggct acggcgggac tcatgcccgg gccagttagc gccacctacc ggccactccc 60

ttcctttcac cgttcacatt gtcctgctgg cccgcgtctt aaacccattt cacagtgccc 120

agaagtagtg ggcattcgta cccaggaagc gattctgcct tcgttatgcc cctgtccttt 180

ccaaatgcct ggaggatcta gctgttagat cctcccagct gccactgcct gttttaacca 240

taagctgctt ctctcgaata ttcagagtgg gacaactcca aagaccagac caggggtcaa 300

ggggttcagg tcagtgccat aaccatttct tgctggaagt atgtgaatag tgactggtac 360

ccaccagact gccaggaata tgtctctttg gccccaaggc ttgagccaca aaatccagct 420

gcagcgaact gccatctctt aagacataaa aacatacaca accgccactg ttaaaaatac 480

atttatcatt aaaatatatt acacatggag acaggatgca tcatatacag tttggaagac 540

ttgctggccc agaaaatccc acttgtttca ccgaacactc attttttcag tgattttaca 600

ttttattttt agagacgggg tctccctctc tcacccgggc tggcgtacag tgatgtggtc 660

ataggtcact gcagcctcaa actcc 685

<210> 39
<211> 423
<212> DNA
<213> Homo sapiens

<400> 39
tgatgcccct cgcgcacccc tgatgccggt cgcccacccc tgaagattcc gggtgggcga 60

gggaatcgtc agagggatca cgatctttca gctactctac tcctatgatc gactgcatgt 120

aacagagatt ctaacgttca acgacaagag gattctcaac gtccactcca ggctcacaga 180

tgccaaactg gcattaactg gcgagccgtg ctgagcggcg gcagcctctg catcggagat 240

tcctggtggt gcgctgcccc aggggagcga ggatagcttt gcagttctcc agcagttcgc 300

cgaggagcag ctgagcgccg accatgtctt catttgcttc cacaagaacc acgaggacag 360

agccgccttg ctccggacct tcagcttttg gggctttgag attgtgagac cccttgtccc 420

caa 423

<210> 40
<211> 876
<212> DNA
<213> Homo sapiens

<400> 40
cgtcctccca gtcatgtcct gggaatccgg atggcgcagg aaggccaccc ggtgacccta 60

agagtggcca cctgtcctct ctgaactgga ctttctcttc tggcccttcc actcttccat 120

gtgtacacat atgccaagta aatttgctgt ggcctcctgg aaccatctcc cccatccctc 180

tcacccctta ggaagagttc tgtgtccctc cattacccat acgcccgcag gagaaacagg 240

cttctatgcc ctatgaaagt ccttcctgag atctaacttc catccctcta gctgcaacca 300

cagtctcttt cctctttctg ctgcctttga gcaggtaatc tggagctgga gttgggaatg 360

atgagacact ccctgcagtg agggctcctc acaaggtggg gaagggacct ttgtagtccc 420

ccagctggga agagagccat gggatccaga ggtgatgctg agcttgaaga ttagatttcc 480

cagaacagac ccagtttcag atattctggg tgttaagggc aacctgaatt caaattccat 540

ttctgctaca gaacaagagc ctcaaaaaga cccccctagaa acaccccccag ggcgcttgca 600

actgctttcc tgtgcacctt tcatgtccta gaccttagtc ccatttccca ggatctatga 660

ttgagaagaa ccagccaggc agttccacag agggaggaca gtgctgcata ttttgggggt 720

gccatcttca gctcatagag agaagatggg ttaactcagc ccaggcacca catccctagt 780

ggggccttgg ggtccagaaa ctgtccccag cttccagtag aggatccggg cataacctgc 840

cctggggcac ccggctctgg ccctgggcac acctgg 876

<210> 41
<211> 567
<212> DNA
<213> Homo sapiens

<400> 41
cccctctgtg gcactccagt gggcaaggag gccttggaag gcgcagggcc ggggccagcg 60

ggaccactca ggctcacctg ggactccaga gggaaggggc atctcaggac gactccacag 120

ggtagggggc tcctagctca acatcttccc tgctttaccc aagagaggtc aagacgacag 180

gactgagagt atgatgtgct cgccacccct tgccctcacc ccacgcccca cccacacagc 240

ccctacacct tcaggaggat gggatgagtc atcattaggt cactgggtca tcgagtaagc 300

agtgggggcc acagggtgca gtttcctccc ttcctccccg cccagcccac ctacggctct 360

cctcctactc tctgcccccg ctactttcct gtgctctgat gtgggaggga tatcgctcat 420

tgacaggttt caaaactaaa agtagagcgc atttagtctt ctcctgtggc tgagctcagc 480

tcccagctcc cagaactttg ccatgctgct gcagggccgg catgccccct cctcaccctc 540

acggggaggg cccccatggt gttagcc 567

<210> 42
<211> 406
<212> DNA
<213> Homo sapiens

<400> 42
ggtgaaccca cagacattca ttctcactgg ccagacgatg aggagacacc ggcacagaca 60

caccgacaca cacgcacaac ttgcttgcgg tgctggctct cacttgcctt cagacatcta 120

atgacactcg gcacttcaag acatcacaaa ccttgcagag gcccgagtga cagacctggc 180

tccgctctct tccttcttcc agctccccct ctactcacct ggggtgctgc ggttcgagct 240

gggggtgtgt aactcggggg cagctctctg aggggcggcg atcgagtcat gctggcgggg 300

atgggggcg cgggcgcccc caacccgcgt tatctgggcg ctcccatagg ctagcccggt 360

ctcccggtac cgctgggccc cgcgtaggga ttcagtgggg ccgcca 406

<210> 43
<211> 271
<212> DNA
<213> Homo sapiens

<400> 43
ttctttcaac ccgagctagg gccccactaa cccggtccct cccagagccc ggattcggcc 60

tcggtctcgg atgcgacggg tatcagaccc aagcggcaac gtcgctcacc cgaagagggg 120

gcgggaaaat cttcccagga ggtgatcccg aatgtcccca cccgaggctc gaggcctggt 180

gaatgtgccc attgtccttt cggggcctct cccttggagc ccggcggccg cacccacgaa 240

actgaccaga aatggatgaa gccggagtgc a 271

<210> 44
<211> 626
<212> DNA
<213> Homo sapiens

<400> 44
tctggccagt ctgcggggtc aaagtaccca gtcacagatt tgaccttacc ggagaggcag 60

aagggaaagg agcccacagg aggcatgcgg gctgggcaaa gcgccctcgg cggcgtctag 120

agcactcgga gctgccccat tgcctccgcc caaggtcgcg gcccaaaggg agccctggaa 180

agttcagcca ggggccgagg agtgccaggg ttggcactac ggggagactt ttgcgacagg 240

agagagacta agagctcgat tccaggtagc agtgggagcc cgcggcaaag aacgcccagt 300

cccgcaggct gcatcccaga ccgcagcgcc tagctcggaa aagctgggcg ggggctgcac 360

cacctgagtc gcaaggacag cacggcctcc ccctcctcac cagtgttctc cacccccaga 420

attggggcgc ggagagccaa tctgggctgg aaagggagcg gcgcccacct ttgggcagcc 480

cgagcagccc cgctcctttt ctgtcgcaga ccccaggctg ggaggcgggg gcgccaggtc 540

ggcccaagct gccgcagcca ctgctttaaa gggagccggg gacgcggcgc tggggactgg 600

gcgaggcggg tggcagaggc aagagg 626

<210> 45
<211> 887
<212> DNA
<213> Homo sapiens

<400> 45
cataccacca ggttgcaatt atttctgtca acctgagcta tgctgagtcc ggtgggctaa 60

tacagatgag gcagggctgt gtctgtgtgg tgtagagcat ggagtcagtc tgtgtcctga 120

gctgtgatga gatcattctg tgagagtaaa taactgagct tgtacttgaa agttgaggac 180

aatgtggaga acaaagagct aagagcacaa gcaaaggtcc agaactagaa catcacaggg 240

tgtgttttgg agactggatg atttgagtaa agaatcatga agagcagaag tagttatcag 300

gctagaaagt taggctggag ccagataagg atgggcctga gtatttggaa tatatactgt 360

tggcaacaga tgactaaata ctcgggttct cttgaaggag tacagggcag aaatcaggag 420

atagaatgag aaatttgaca tttacaccag aactttccag ccacaaagag caaggtagag 480

tcaattaatg cactgagtct agttagaatg attgggttaa ggtgagagtg gccattccta 540

acacttagca aaacagtgtg tatgcatatg cctgtgtgca gttatttgat tatacataca 600

ctttccagct gggctaggct ggcctatctg ggcccagtca actgctgtgg acactgagct 660

ttaacaccaa taagtgcgtg ccgtattcat cctcattctc atttctttct cagtgcctct 720

tgaccctcca aggattattg cactgatcca gaactgctaa aatggagaaa gccaggcctg 780

acctgtgcgc acagcacaca gcattgatat ttgatgtgag attaggtcag agtcagccgg 840

gaagccttct aaagcgtggc atacagccaa caaaatgaaa aggcttt 887

<210> 46
<211> 304
<212> DNA
<213> Homo sapiens

<400> 46
aaccacactc tcccggtcca ggccccgctg gagaacgtcc caccgactcc ggggacagaa 60

aggccgttta tgtaaaacga cgtttttcct atttcgcctc ccaccctcat gcaaattttg 120

attttcaact cttccaaccc ttccgcaccc tgaaacaaac ctcatctcct ttcctccctc 180

ttctggctcc gcgggttact cgccccgccc ccacccttca gtcccttagc aacgcgtcct 240

cggtccgtag aaaccggttg ccgagctctg cctacctctg aaaggcctag agagcagagt 300

tctc 304

<210> 47
<211> 685
<212> DNA
<213> Homo sapiens

<400> 47
tgtagtccca gctactcgga gaggctgagg caggagaatg gcgtgaaccc gggaggcgga 60

gcttgcagtg agccgaggct gcgccactgt actccagcct gggtgacaga gcgagactcc 120

gtctcaaaaa aaaaaaaaaa aaaaaacccc acaatatcag gctacagtta tcatagcaat 180

tctgctttta atgcactcag gacaaacttc tagctctgca tatatgtcat ttcaatgaca 240

catcctgtct cctacccgac tagaatgtga gcagccctgt gatctctatg tgctcataaa 300

cagtgcctaa tgcgaatcct ggctcttgga aggcgctcag ctagtaagtg aggagctcag 360

ctctgaagct gtaaccaaga ccgcgcgggc aggaggaaag gacacttcag cgctctgcgg 420

ttgcaaacac acccctgcgg gtgctgccac ttagcaaagt cttaggttcc cgcttactcg 480

cacacgcatc ctgacttccc ccaaccctgg aactacctac caggagcgga gaccagcaga 540

ccgacagcac gcacatgatc cccataagct gaatggccgt ctcggtcgtg atgcggcccc 600

actgggcact ggactgagat gccgtggcct tggcccggca gcgggacacc agggccttaa 660

tggtggccag gttgcaggaa aaggt 685

<210> 48
<211> 517
<212> DNA
<213> Homo sapiens

<400> 48
tttactatct ttctgcacag tatgttcaaa atgaagctca aatctggccc ggcaatcccc 60

ttaaaatgcc aggaaacctc tcatgggtgt taatggtctg gatttcccaa atttcttcgg 120

ggcagaacag aagaaaggtg aaaggggatg ctggacactt cctcaagagg aataaaaagg 180

taacattccc tggaccttgg aatattcaac tttagagttg aaaggtcaag gagaaaaaca 240

tcctgagaag tactaaatca attctcctcg gagattttgc caaatacact tgccatgtga 300

gctccacctc ttaaaaaatc cagtccccta caagggattg gtccctccga cgacctcggc 360

gaccaggggc gctgagcact cacctaggag cgggaggcgt ttcattggcg gtggccgcag 420

tggtggcggt ggcggagagc gcggcggagt gagtgcggct gtggacccgg gaccgggcgc 480

cgctgggcgg gcgcggcagg gtcccggatc cgtcctc 517

<210> 49
<211> 446
<212> DNA
<213> Homo sapiens

<400> 49
gctaattttt tttgtagttt tactagaggc agggttcctt catgttggcc gggctggttt 60

agaactcctg accttaaggg atcctcccgc ctcggcctcg caaagagcta ggattacagg 120

tatgagccac cacgggaaga gcattcttta gacaaccagg aaaacttgaa tttagtgttt 180

atccttaact gagctttgtg gatcatgact aacaatcgcc aatagcttgg gaagtttaag 240

aaggactttt tgcgtttagg aagtagagag ggcagagata aacaaaggaa ccagaaacgg 300

agcattgggt tccaggaggc acaagaggca gcagaagtga ctgaacattg atatcttgag 360

ctcaagagct ctaaataagc ccaggcagtt caaagccggg ctctaactgt ggctgaaatt 420

gctgacctgc gtatagttta taatct 446

<210> 50
<211> 391
<212> DNA
<213> Homo sapiens

<400> 50
tccgaactgc ggcggctccg ctagacagtt gcagaagctg gcggacggcc gggacccccct 60

gccttgcgca gccaggcgtc gaggaggcgg cggcgagtgc tgcggtgctg gctgggtggc 120

cgagtggtcc ccgcagcctc tggacccagc catgctgctc tggatgcagg gcttcgtgct 180

ggaggcggtg gcctgccagg ataacgatga ctacttacgc tacgggatcc tcttcgaaga 240

cctggattgc aatggggacg gcgtggtgga catcattgag ctccaggagg ggctgagaaa 300

ctggagctcc gcgtttgacc ccaactccga ggaggtgaga ccggaaggtg gcacaggcag 360

cctggggaga cgttgggcag agggagaagc a 391

<210> 51
<211> 391
<212> DNA
<213> Homo sapiens

<400> 51
ttgcttctcc ctctgcccaa cgtctcccca ggctgcctgt gccaccttcc ggtctcacct 60

cctcggagtt ggggtcaaac gcggagctcc agtttctcag cccctcctgg agctcaatga 120

tgtccaccac gccgtcccca ttgcaatcca ggtcttcgaa gaggatcccg tagcgtaagt 180

agtcatcgtt atcctggcag gccaccgcct ccagcacgaa gccctgcatc cagagcagca 240

tggctgggtc cagaggctgc ggggaccact cggccaccca gccagcaccg cagcactcgc 300

cgccgcctcc tcgacgcctg gctgcgcaag gcagggggtc ccggccgtcc gccagcttct 360

gcaactgtct agcggagccg ccgcagttcg g 391

<210> 52
<211> 589
<212> DNA
<213> Homo sapiens

<400> 52
aaggggcaga ggactgggct gccagctgag aggtgctgaa cggggaaccc gggccaaact 60

gttgtatgtg tgtggataag gaggccctct tggttgctca ccctggggct ccttggctgc 120

tccactttgt ccttagttcc tgtgcccgat taggattggg gatggtactt ttctcctgtg 180

gctagattct ttgggttcca cctagctaca ggggcaggaa caatggtacc aagccctcag 240

cctgttctct ttgattctta ggtcttttct cctggcaccc tgtattcatg gccttggcgg 300

tgagtttagg cttctatctg atttgtttgg gtattcctca cctcattctc ccagggaagc 360

acccagcttt cagaaagctc agcctttcta aggctaagac aggtggagga aacatctagg 420

gctcatcttt ccctgcagcc tatgggattg ggaaacctgg agggctgggc cataggcagt 480

tgtcagatgg ttgaaaaggg cgggagggca tggaagataa tttctttctc cattggggcc 540

ggaaaggaga agggggaga ggtgttatcc ggtcatctcc cgacttgtc 589

<210> 53
<211> 762
<212> DNA
<213> Homo sapiens

<400> 53
actctgctcc agcccaggtt tcggggagcc gggatccact tgggcaggcc gggagcctca 60

gactccagac ttttcatggt gcgctccttc ctgcttactc aggaggaagg cgaggcagtc 120

cagcatcctg ggtggagtgg aggtgtttcg agtccatatc taaatctttt tcttagagca 180

ccctaagcag gctgctgtct ttgatcccca tgcctctgct gtttatcttg gtgtgattca 240

tcactgtaat ttaagacgtg gagagagcaa agttcccatc ccaggcaggg gaggcgcagt 300

gcaggttgga ctgtgtaagg aaatggcagc atggcgaggt ttgtgccgcg gcctacaagc 360

agggctgcat gctccccagg cagactgtgg cagagccaag cccctcactt gtagggaagc 420

gtgtctccta ggtgccccag taggggaggt ctgccagcat cgctgtgctg tggggaaggc 480

agccagaggg cttcatccat aactggctca gctcctcagg aggagaccag cagtgtgtct 540

ctgcactcag aactgccact gggtcgtggt gttaagccca ggaggggtgc atatgttgac 600

aaccttgtat tgcttagttg ctgagaccag aagatccagg taattgcatg agctattagc 660

tacttctggt tctcacaaac tcctcccagt gttgatagag aatggtgtcc tccgggcatg 720

ctctgggtat agttttattt gtattattag ggactcatgg ag 762

<210> 54
<211> 310
<212> DNA
<213> Homo sapiens

<400> 54
ccacggctcc ctcggaacgc tttcgggtca tagcatacca cgacccatcc ggatcctgga 60

tccactcggc ggcctcgcag tagaattcgc cctggtcaga aggctgcagg tggaagatgg 120

tgaggcggaa ggtggtcctc cccagcttgt ccagccgcac ctcccccagg ctctgcctct 180

gggcatattc gctgctggag tgaagcatga aatctcggct cagggagatg acctccacgg 240

gcttctcgcc aactttctgc cggagccagg ccacagacag gtggctgtgc tgaatggtct 300

ctgaggccac 310

<210> 55
<211> 196
<212> DNA
<213> Homo sapiens

<400> 55
gcagagcgga ggaaggtccg ggagaaaagg ggcgggacgg aggagaatcc gggatcgcct 60

ggcagaaaaa gagaagggag tttctgaatc ctgggaagag gaggcgtggg tagggatgct 120

tagcccgaga tccgacagca gggaaccgga gcgctccggg ggaggggctt aatgctgggg 180

aagggatgtc ttaaaa 196

<210> 56
<211> 169
<212> DNA
<213> Homo sapiens

<400> 56
aagccagcga gccgctgcag ccctgatcga gttaaggcgc ggcggccccc ggggccgctg 60

gagaaggatg cggacggggc cagtgactcg tagtagatcc ctccgcgcgg agctcgggcc 120

ggcgcttctt cctgcgggaa acccctgggt gcccaaggcg gcggggccg 169

<210> 57
<211> 757
<212> DNA
<213> Homo sapiens

<400> 57
gtctgatgga cctgagatga gaaaccagaa ctcctgcccc tagttccccc ggctttcctc 60

aggctgggga gtgagtgcgg tgagatagaa agggcaagcc ctcctgctgt ttctctctgg 120

acaggggcgg gatgctgggt gaagggtgaa aggaagaggc tggagaaggg agaaaagctc 180

cagctcacac taagtctgaa tttttttaa atgcggactc cgtggcccct cccttacccg 240

ccccaatcct ctctgaagtc ctggttgtga ggggccaagt cccaaagtct gctgctccgc 300

ctctctgtgt gcagagccat ggggccttca caggctgcag tgggtcccga gcccccaggg 360

ctgtgcctgc tggtcctgac caagatcgcg gctgccgagg tcagtccagc gccaagggca 420

cagggccagg gcaggcgggg cagggctacc cgaagcgcat agaggctgct ggtgtcaacg 480

tgacgtcttc tggggcgcct ggcatcccta ggagtggaag ccgctgatga agtcaaagct 540

gcctcctcct tcaggaagac tttgctccca tagctggcga acaggaagcg gagcagcgcc 600

aggaggatct gcgggcgctg ctgagggctt ctttgcaggg acagtgcagc aggcagccag 660

ggacaagact gcacggcagc gccccatggc caggggaagc tcagaaccgg agtcgcccgc 720

tgcccggcga ttctccatcc ctggatcggt acagggg 757

<210> 58
<211> 557
<212> DNA
<213> Homo sapiens

<400> 58

```
ggggctggcc cttaacatgg atcagtcata aattagtggc ttggtactcc gggaagataa      60

aatcttcccc atttgtctag tgattgacaa tgcgtgaatg ctttaaaagt tgcaatcagc     120

gtccctggat gggctcgaac caccaacctt tcggttaaca gccgaacgcg ctaacccatt     180

gcgccacaga gacaagcact accagttcta ataggcaata taggaagggc acactcacta     240

aacttcctcc gtcccttcta ttctcagcgc ccgcctggca ggacgactga gcaaggcttt     300

ggaaaaccag agagattaga gcggtgagtt gcgctggtca cattggatac ctgcgcgtta     360

ggagattctg gagccagacg gagagccgaa tggccttcgc ccgccctgcc cctctcttgc     420

ttcagaagcc cccagaaact ccgcggtcgg cgacccagcc cgagccgcct gaggtcccaa     480

ggggaagctg aacgcctggt gggctcccgg gatggttctt cccgttcttt gtgccgcctt     540

cacccagtga aggagcc                                                    557


<210>  59
<211>  1658
<212>  DNA
<213>  Homo sapiens

<400>  59
ggccgaggtg ggtggatcac tcacttgaag tcaggagttc gagaccagcc gggccaacat      60

ggtgaaagcc cgtctctact aaaaatacaa aaattagctg ggtgtggtgg cgacgcttgt     120

agtcccagct actcaggagg ctgaggcagg agaatcgctt gaacctgaga ggcagaggtg     180

gcagtgagcc gagatcgcgc cactgccctc cagcctgggc gacaaagcga aattctgtct     240

ctcaaaaaaa atgcataaat aaataaaaga ggggtgggga agcaaaacga cgggcagtag     300

gtgtggggcg cattgggatt ctatagtggt tagtaccctg ccttgtgcct gcagcaacct     360

ctgttctaat ctgaatcctg gtacagtcag actctatctt ggacccactg gggcgaaccc     420

acgagtcttt tggtttgctt ttaattcctg caccagctgc ggcctttatc tgcagccaga     480

aagcagggtt taccgctggc cccacagcgc catacggtct ggggaaaaga aggaaaccca     540

atagtacaca aacaaaggcc caaagagaaa ccttccaagt gctctatgcc tcacggttta     600

gcagaaaata tcaagcaact ctcaacctag ctggtctgta gcttccacaa atgaaatact     660

gtattcattg cagcctttct ggttgagata tttcaaatat ttggtggagc ttttaatgag     720

acggagagac actctcgagt gtggaagaaa aacatgaggg ggtgtgagga taaggcgact     780

ttaggacaga aaaaacaaag agacaaggaa gccacgtaaa cgttttcggg taggcgtgag     840

gcgatgtcag ttttgaaccc cgtttatgtt aggtagagag cgcagccctc ttctagcaca     900

aacaccgttt cccacattga agaaatcaca gagatcagca actctagagt gcgatgaaga     960

agcttcactc tgggagaacc cccttcgtga ccacggtctc tttcctgcca ggtaagtggg    1020

aatgagcgca tgccctgcag ggacagcaca gcgtcctcgc cctggtcgga cgctcagggt    1080
```

caccacccta cccactgccc ccttcgccat tcttccaaac cactctctgc caaagattcc 1140

accgacagtc accccacacg acaacccagg ccgcctttca gcagtggctc ccgccccgca 1200

accacgcgcc ctctcacccc cgcggttctg cccgccgcct ctgtccagtc tgtgcacttc 1260

acctccctgg ctcccgctct cccctgagct tacagtggac gcggggttct tccaaacccc 1320

tcttgggaat actgaatgga aaagggggag cgtgcgcaag tgcttggtag agtgtagaca 1380

ttgtgggatt tgactgtggt accatcgctt tgacgtccta gtgctgattt ttacacctgc 1440

attctgctta gggcactggc aacagttttc cgtttgtgcc tactccacct gctgtctttg 1500

ttgggtcagc gaacatcgcc tccctctacc gctcaatcag caaaagggac cgcccttgag 1560

gacctcaccc gccgctcact cccctcccaa cttcgcgggc atcgcctccg gtcgcctctt 1620

ccgaaggcct aacgagcatg ttagctgcga acggaggt 1658

<210> 60
<211> 356
<212> DNA
<213> Homo sapiens

<400> 60
acgacgactc ttctcctgcg tggcaaactg ctcctccgcc cggtactgcc ggtctcgctc 60

ctgccgcagc ctctgctctt gttcctcaag ttggagctgc tcttcttccc agcgatactt 120

tccgtcacgc tgttgggggc gcagctgctg ttcttccctc tcctggcgta gctgttcctc 180

ctcgcggaat tttctgtcag actcttggct gcgcagctgc tgttcctccc tctcctggcg 240

gagctgttcc tcctcgcgga attttctgtc gcgctcctgg cggcgcagct gctgttcttc 300

cctttccgga cggagctgct cttcctctag gatttttctg tagcgttctt ggcggc 356

<210> 61
<211> 725
<212> DNA
<213> Homo sapiens

<400> 61
gagccgcggc agggagtgga tcctcccagt cgccctggag tacggctccc ggccttacct 60

tgggccagta aactgggacg gggttgggac gagggagtgt taccatggta actgggcggc 120

gcttcgtgcc ctagcctctc ccgccaggct ggctgcccac aatggttggc ccctttgcag 180

gtagtcggct gaccttacac tcccgcaatt gacccctcac tcccgcaatg ccgatacccg 240

tcacccctca ctgtatttgc tccacaatat aaacatccta caagtctctg cttccactca 300

ccccacgaca aactccaaaa ccaagttata agaaaagtct aaaagacttt ctgggcctca 360

ggttcaaaag agccccactc agtctccttt caaagtcggc tcaggtaggt aggttctctc 420

tcccgtctcc tggaagtcaa tgaatggaac tgtcggagtg gaagggatgg gcaagatggc 480

acaaattctc attctatttg gcatggtatg aacttaagtg ttctatttag aacagacttt 540

tctccttttt tttttttttt tttttttttt taagagacaa gatctcgctc tgttgctgga 600

gtgcggcagt ggcgagatca caactcacta cagcttcgaa ctcctgggct caagcgagcc 660

tcctacctca gcctccggtg tagctgggac tacaggtgca cgccaccccg tcaggctccc 720

tgtta 725

<210> 62
<211> 380
<212> DNA
<213> Homo sapiens

<400> 62
tcaggaacca tgtcctcctc atctctccgc ccttgttctg ggcttcgccc ggaagaggca 60

cagatggacg tccgtggaag aaacgaccca cccatagtcc agcccaaacc cagggtgagc 120

gcagagtcta cacgctgggg actcctggga cgggctggca aaaacgcgtg gccaggggaa 180

ggtgtgatcg gtctgagggc tgatggggga gccaaggaga agggaatgtg cccagcgcac 240

ggatgtggtc agggcgggag gcgcgtccgc accgattttc tggaccacta gggagatgag 300

cccgtcgtca atgtagatgc ggcccccac cggcacgacc cggacaatat tggggtagtc 360

cacccacacg gtgttcgcgt 380

<210> 63
<211> 301
<212> DNA
<213> Homo sapiens

<400> 63
ctgtgccccg ccgggcgggg accgtgggag ccgcggacaa gcccaaggcc ggagcggttc 60

caggaggacc ctggtgagga gggctcggcc catgggtgta gaccgatgga cctgggcacg 120

aggggcgggg caggacaagg gagcaggaga aatgaataga gaaagggtgc ggtggtggct 180

gaacgatgga gacgaatgcg gagagaatgg acataccgct gttccgaggg gcgagggcgc 240

aggctggagc ccggccgagg cagccagcag atgcggagtt gacaagctga cggctgctcc 300

a 301

<210> 64
<211> 712
<212> DNA
<213> Homo sapiens

<400> 64
cccgagaggc agacatgcca cccgtggctc tttgctcacc cccacctgcc ggtgccaggg 60

tgtccctgcc atttccagtc ctggaaacct ttgctttctg actcgacagt ccccagcagg 120

aaagagcaag tgtgaatcac taagtcctga ggtgggggtt gtgggggcag tccttggagg 180

caagagagga agcagttatt gcttgtttgc acattgactc cacccactct gtcctgcagc 240

tgtgacgtgt cctgttccca gggcacttct ggcttcttct gccccagcac ccattcttgc 300

caaaatggag gtgtcttcca aaccccacag ggctcctgca gctgcccccc tggctggatg 360

gtatggaggg tggggcctgt gggcatgggg tgtgggtctg gggagaattc tgtgggtggt 420

gctaagcagg gctccaaggt gagtgagggg ggtgagctct gtggttatgg gggtgtcaag 480

gggtcctgtg gccttgggga ggaggctggg tgcctggggc cctgttgacc tcttatcccc 540

acagggcacc atctgctccc tgccctgccc agagggcttt cacggaccca actgctccca 600

ggaatgtcgc tgccacaacg gcggcctctg tgaccgattc actgggcagt gccgctgcgc 660

tccgggttac actggggatc ggtgagtggc gtggggcggg cgggagacgg ga 712

<210> 65
<211> 396
<212> DNA
<213> Homo sapiens

<400> 65
gcgctccagc ctccctacca gcccaggggg ccggacccca agtgcgagcc ggtggcgtgg 60

gtcagagcgc aggagcgagg cgcccacgga cctggtctgc gtttctgagc cgcacgccac 120

ggctgcgaga cccgttcccc atcgccgccc ccgctcgctg acacacccat cccgcctctc 180

acctgctggt gacacaagtg agaaggctgg ccccacggtg gtgaaaaaaa aaaacaccac 240

acgaaagaaa gaaagaaaga aagaaagaaa gaaagaaaga aagaaagaaa gaaagaaaga 300

cagaaagaaa caacaaaaac aaaacacaaa aactctgggt ctgtgccggg gatccgcgct 360

cagcaaggcc cgccacagca aatctgccca cacggg 396

<210> 66
<211> 345
<212> DNA
<213> Homo sapiens

<400> 66
ccaaaacgcc gccgcttctc aggcacgtcc gttcttcctg cccacccgcc ggctgtcgca 60

gaaacagccc aggaccatgc gccagcgccc gcgaccctct accaattgcc cttcggacag 120

acgccctccc caccacctca cacgccctct tccctggccc cacacacagc gagcgaccgc 180

gaccaccttc cacgctcttc cctgcctatc tcctccgccc gccttctcct cactcgccca 240

aacagacaca gcccagattc ttcccctatt cctcctttc cctccttcct cccaccggcc 300

tgcgcccacc gcccaccgcc ttgaatcgcc gctgcgctgc ccaga 345

<210> 67
<211> 351
<212> DNA
<213> Homo sapiens

<400> 67
gcgctccagc ctccctacca gcccaggggg ccggacccca agtgcgagcc ggtggcgtgg 60

gtcagagcgc aggagcgagg cgcccacgga cctggtctgc gtttctgagc cgcacgccac 120

ggctgcgaga cccgttcccc atcgccgccc ccgctcgctg acacacccat cccgcctctc 180

acctgctggt gacacaagtg agaaggctgg ccccacggtg gtgaaaaaaa aaacacctca 240

cgaaagaaag aaagacagaa agaaacaaca aaaacaaaac acaaaaactc tgggtctgtg 300

ccggggatcc gcgctcagca aggcccgcca cagcaaatct gcccacacgg g 351

<210> 68
<211> 345
<212> DNA
<213> Homo sapiens

<400> 68
ccaaaacgcc gccgcttctc aggcacgtcc gttcttcctg cccacccgcc ggctgtcgca 60

gaaacagccc aggaccatgc gccagcgccc gcgaccctct accaattgcc cttcggacag 120

acgccctccc caccacctca cacgccctct tccctggccc cacacacagc gagcgaccgc 180

gaccaccttc cacgctcttc cctgcctatc tcctccgccc gccttctcct cactcgccca 240

aacagacaca gcccagattc ttcccctatt cctccttttc cctccttcct cccaccggcc 300

tgcgcccacc gcccaccgcc ttgaatcgcc gctgcgctgc ccaga 345

<210> 69
<211> 351
<212> DNA
<213> Homo sapiens

<400> 69
gcgctccagc ctccctacca gcccaggggg ccggacccca agtgcgagcc ggtggcgtgg 60

gtcagagcgc aggagcgagg cgcccacgga cctggtctgc gtttctgagc cgcacgccac 120

ggctgcgaga cccgttcccc atcgccgccc ccgctcgctg acacacccat cccgcctctc 180

acctgctggt gacacaagtg agaaggctgg ccccacggtg gtgaaaaaaa aaacacctca 240

cgaaagaaag aaagacagaa agaaacaaca aaaacaaaac acaaaaactc tgggtctgtg 300

ccggggatcc gcgctcagca aggcccgcca cagcaaatct gcccacacgg g 351

<210> 70
<211> 381
<212> DNA
<213> Homo sapiens

<400> 70
caagttcgcg gacaccacgg tgcagtcgga catgaagcac tggcccttcc gggtggtgag 60

cgagggcggc aagcccaagg tgcgcgtatg ctaccgcggg gaggacaaga cgttctaccc 120

cgaggagatc tcgtccatgg tgctgagcaa gatgaaggag acggccgagg cgtacctggg 180

ccagcccgtg aagcacgcag tgatcaccgt gcccgcctat ttcaatgact cgcagcgcca 240

ggccaccaag gacgcggggg ccatcgcggg gctcaacgtg ttgcggatca tcaatgagcc 300

cacggcagct gccatcgcct atgggctgga ccggcggggc gcgggagagc gcaacgtgct 360

catttttgac ctgggtgggg g 381

<210> 71
<211> 669
<212> DNA
<213> Homo sapiens

<400> 71
gagataactg gcaaagaaga ggccaaattt tttttgagac tgcagtctcc ggtgtaaaat 60

gagggtgcaa ggtgcatccc cgctcctgat ttttgaatca tcccttccac caccttggaa 120

tgacttctta cgtagaacag ccagcaagtg gagaaacttg cctcaataat gcaaaactgt 180

ctccctaact tacttccttt caatgttcaa gcaaaacaaa gccctctcta catccaacat 240

gacagaaacc caacatgaca gaaagtatga gaatggttgg aaattgctga aacagcaaca 300

gtaaagattt aatgagctct caaagtacct ctagatgtgc aaatatgaca acccaataaa 360

aagaaacaca cacataacct agagccttca aaaatctaga tttctgcctt tccacacctt 420

aacttggaat cattcaggtg ggttcctaag cattctctcg gaggtgagga atcctccaaa 480

gaggagactc taggaggggc ccaggtgtgg caatcttcaa aaggatgagg gatgtccaga 540

ggagaggtgg cgggagggtg cacttaaaac acgcgaaaat tggaaacgtg ccttctgcag 600

tcagttcctt cggattttcc ggcatttaga agccagttct actccttcag cccgaccgta 660

tctgcaggt 669

<210> 72
<211> 259
<212> DNA
<213> Homo sapiens

<400> 72
cgcctcccct cccctcagcc tcagtgcatc ctgaggacgc ccgccgagcc ggaggtgggg 60

ctgaggaaca ataaagttgc gttttttttt aaggcagcca cacaacagcg tcccacggga 120

gggggcggag gaaactaaaa aagcggagta gaaggcacta ccgctgtcga ggccgccgcc 180

gccaccgcgg tccctgtagc agccaacccc ggcgcgccgt ggccggccgt gcgcgcgcgc 240

gcgagcggcg gaagaggcg 259

<210> 73
<211> 469
<212> DNA
<213> Homo sapiens

<400> 73
aggaaagtca ggtgtagggg agattaaaag gtaaactggt gggactgacc gggctggtgg 60

gactagttag cgaagccaag tgtttgggta gaaacagaga tcttgtagga ggtgctccga 120

gctacatttc gggatttggg atgaaaagag aaagccaggg ggttcgccgc tcctggtttg 180

ttccttaccg accctaaact gtatggcgga gcttgtgggt caggcgtgga ggcggcatcc 240

actcccacgt gccccacatg cagcagcaat cccacgcggt tttcgcaagc ggctgggagg 300

caagcgggcg tccccccaca cccccccccc actgcgccct ggacccacgc cacgcaccgc 360

caaggagtag ctatggtggc acgtggagga ggggcgggta ggcagggcgg caccatcacc 420

gggaccagca tcttggaacg gtcaatctca ctgctcatac tctcctccc 469

<210> 74
<211> 416
<212> DNA
<213> Homo sapiens

<400> 74
cccgcccgct gcaccgcacc tcgcctcgcc tctctgctct cctaggcccc ggccgcgcgc 60

cacccgcctc ccgccaccat gaaccactcg ccgctcaaga ccgccttggc gtacgaatgc 120

ttccaggacc aggacaactc cacgttggct ttgccgtcgg accaaaagat gaaaacaggc 180

acgtctggca ggcagcgcgt gcaggagcag gtgatgatga ccgtcaagcg gcagaagtcc 240

aagtcttccc agtcgtccac cctgagccac tccaatcgag gtaaaggctc ggcccccgcg 300

tggtccgtgc gcccctttcc agagcaccct ggcccagtgg cggggaccaa gagacgcacg 360

catccccggg gatgagggga ggcgaggggc tgccggcccc aggcagggtc tacgca 416

<210> 75
<211> 560
<212> DNA
<213> Homo sapiens

<400> 75
atgatttggt gtaaaatgaa aaatcagccc aaattgcaag ccaagccgcc ggtggttatg 60

aatggctgct gtgactccgc cccaaggttc cccaggagaa tggatttatg tacttctctt 120

ccagtggggc acacagtgga gagagtttga gacaacactg ctgagagaat tgccacatgg 180

gcgctggctg cacagaccca aacctcacag agtgaaaggg gactttcctc acagagtgaa 240

agtcgcagct ttcccgagcc cagggaagcc ccccagagag gggctgtagt gggggctggg 300

cagttctagg agcctccact gccctgggcc acccctccgc cgccagagcc acgatccctg 360

cagccgcctg tggcaggtcg tcattggtgc cctggtggag cagccctagg aagggcggcc 420

gcccctgcct gatgccgacc ctaccccttg ctcctcgcag gggcgtgggc tgcatccact 480

acgagatggc cacagggagg cccctcttcc cgggctccac agtcaaggag gagctgcacc 540

tcatctttcg cctcctcggt 560

<210> 76
<211> 662
<212> DNA
<213> Homo sapiens

<400> 76
tctccgggct gcacctgcga cccctccgcc cccgctccca ttccctcgcc ggagggaagc 60

cgcgagttgc gagtcgtgat agaaacttaa atcgggggtt gcgctgggtg gtccctgctg 120

tgcctgctat tgcgctcctg gcttcttaag aacattctca aggggctcct accgcggtgg 180

tttttatgta agatcaggaa tcagtttcct gattacagat gggaatactg aagcgttagc 240

gcggcaggtg acttggagaa ggtggcccag ttggaaagaa agaaattgca acccagtgta 300

tttgttttcc agtaacatcc cctccaggat atcccactag ttgagaaatg tttgggttac 360

tctcccaaat attcccaagg ttatttcccg agcctgggag cgcaacaaat acataaatag 420

gcgctcgttg aatttttctc gtctagccaa ctatcagtgt actctggggc cttcaaaaca 480

ttctgccgtt tgtctcgggg agatggggtt aggaagaatg aatatgggag aaaatgacag 540

tttccttcgc ggaaatcaaa gcgcagatgc agttcccagg tgtctctgca tggaaacagt 600

cgaagctgct gccggaagct ctgctggcct ggacaaggcc acccagaata aagtcacaga 660

ag 662

<210> 77
<211> 277
<212> DNA
<213> Homo sapiens

<400> 77
catcacctaa agctcctcgc aggccaccgc gagggcagcc gaccggctcc ggaatctggc 60

cgcaggttga agccgctggt gcgggaccct cgggcaggag gtgaggaccc cctcccttct 120

ctcgcccctc aatcatctta gggcggtggc tacaggacag agagaggggc gtgcccctcg 180

gctgtgaagt gggcatgccc gtgtgatgcc cccgcccgtc gtctcaccgg ggcgcaccgc 240

gctggtcctc ctccgccagt ctcccgagct ccggcca 277

<210> 78
<211> 802
<212> DNA
<213> Homo sapiens

<400> 78
agccctccgc ctcgaggcca ctggtgcttg ggggtgggaa aagagtgacc gggggcaact 60

gagcaaaaca tagaacagaa actccctctc gaatagagac ccacgcacgg aaggatgata 120

cactgtgatt ccccgacgtg ggcctgcagc aagagacggg tgctaacagc gtccgagcca 180

atccgtaagc atcagaggga caaagaaatc ataaacaaga ggcaggatgc aactgcttca 240

acgcagaaag aatgagtcat ccaggaaggc gaggtggtgg cgggttggct gtggttttgc 300

agaggcgggc aggaggggca gggggccagg gagagggcag ggttgggagg gtctctttta 360

agggaatcct atgcatgaaa gtctgtgtaa gaaagggtgt gcctcctaca gaaggatatt 420

tgttttgtcc taaacttgcc gtgtgttctt ggaggcgcgg gcctccctgc tgtcatcaca 480

cctcggatca ccccaggcaa tctcgcccct gtggcttcac ctgggtccag ccagacctct 540

gctcagctct gcacgtacat gccccctcga tgtcacctcc tgacttggca agtcatacac 600

cctctctcta cttgcccagc ttcattcaca aaaccaggtc acattacccc tcgtccatcc 660

atgctcagtc tggaaaccct gggatcccag acaccagcct ctccctggcc ctctgtttcc 720

aatcagacgg ccaccccgac tgacgctggg gccggggcac ccttgggcgt tatccagtct 780

ccctgtcctc cctctcctgc cc 802

<210> 79
<211> 717
<212> DNA
<213> Homo sapiens

<400> 79
gctcatcccg aatcccgctc tattccacgt gctagaagca gtggtgttcc gggagcctgt 60

cgcgagtggc cgttaagagt tctagaacgt gttctccacg gatcttcggg gtagtcctgt 120

ttggataata tcaacactga gaggaagaat tcctaaatga ttttcctaaa gccatgtcag 180

gacaaaactg caccagagtt aaaggcgaag aaggcaagaa gaccttattc aagtctatta 240

tgataggaga gagagagaga ttcagagatt caactccact gaaacaaagg atgagagggt 300

ttctacaagt tgggatggga aggagattag ggccatcatt gtctgttaat tggcctcctg 360

caaagaaaca gagagcttcc cccatcttca tgacaggagg tggttgcaca gcttagagcc 420

aagcacccac caaggctggg ctccatcctt ccacagggac aggagatgac gccatcttgc 480

cagtgatcac attttgaagg gacagctccc agctccttga gaaagacagt cctgggttgt 540

acagccagca agagacttct agaaagattc aggtatcaaa tggcagagaa agaacataca 600

ataatacatt ttctaaagca aatgctctaa agatagggag ttccaggtcc cagaggtggg 660

aagaagccgg gtcaagtctc agtccagacg aggaaaccca aaggctcagt ccagacg 717

<210> 80
<211> 464
<212> DNA
<213> Homo sapiens

<400> 80
ggaaggagcc tgtgagctcc tcctttccag gagcgaaacg ctggcccacc ggccccattg 60

cctgggccag cactcaccag gggcatttat ctgtacctgt gttgctgtgg gtaatgtact 120

cacaggccct tcccagccag cgcctctcac ccatgctcgg ggctcagcat agtgtccaga 180

ggtcacgtgt ctctctgttg tgattctgct agagggtctc agcacaggaa gatcaactga 240

gtctgcaaaa atgaaaagaa catccatcac cacattcagc caagccctga gccccgtacc 300

tgcaggggct gattttcacc tggagtggcc atggggttcc ttgggtggga gtgatgtttg 360

tgagtttcca ggccattgct caacatgaag acacatttcc taccttcatt tccccggtga 420

gtttcctttc ttcctgggct gccctgtgcc tttcctcctc aagg 464

<210> 81
<211> 872
<212> DNA
<213> Homo sapiens

<400> 81
ccaccacagg cgatttggaa gggacgatta tgagaggagc caccacgtcc ggccacacag 60

ctctgagaag ggcagaaaga gagtcacgcc tgcgcccatg gagccccaac ttgtacctgg 120

accacgctgc agcctgtccc aacccagaga agctgacgag atccttgtcc tccatcaccc 180

agacaagagc tagatggtaa aattaggaca taattttaag catatatact ggatatgtat 240

gcctcatgag gaagggttat tctcaaatct gcaaatattg agtacagttg cagtgcaata 300

tcaatggacc ataaagacaa taatcatata cctaaatcac ttcactgtga ggaccatttt 360

gaattcttta aacaataaac aggactttta catagatatt tttgcacttt ttgtatataa 420

tgttttaata gcagtttaca ataccatgca aaatattaat ttaggttatt cccaggaccc 480

tatcctaata ggggtagacc actactgcgc tctttcttca aaatcaaatt tgaaccaaag 540

actcttactg ctgctcacag agtcgtttgt tgcttatttt gtgagaaatt aggcaaaacc 600

tcaatagcca agactgcctg gcttctcctg atggatgagc acatacctat gatcacaggt 660

ccctcctaca aatgatggat aataggtcct tttaaaggca gatgagactt acctgaatct 720

aaggggctga agtcacagcc ttcacttaac agctttcaga gaaggtgctt tggcgctgcc 780

taggcaaggt ggaattggtg tggagtgtgg gaagaggctc tccggaggca ggtgcagtgg 840

gtggagcagg gcaggagtgg ctcgggcagc tc 872

<210> 82
<211> 369
<212> DNA
<213> Homo sapiens

<400> 82
ggcagggcgc tgggctggct gctctgtgag ctcctcccag tgcggggccc ggtgtgacta 60

tgggtctggt aagaacagct gactgtgagg cagagatcaa acaagacaca gacaagccac 120

acggtgcgaa ctgtcagaac ctgggataaa gacagagctg gcaaataaac atacatgaca 180

ggcacaaacg ctagatggtt tggctctcag ggcataccga aaatcaaacc atttgcagaa 240

aaaacaaatt gtaagaatca aaatgcagtc actcagcaaa gaaacatgag cacttacaca 300

ggctgtattt ccaccatccc ggggtcagag acggtgcctt cagcccctga ccctcccttc 360

agccctccc 369

<210> 83
<211> 865
<212> DNA
<213> Homo sapiens

<400> 83
cctgggggag ggtgctcttg tcactaagct tcagccactc tgggtctccc gggaggcttt 60

gtccctgaga ttcttagagg gtctcccatt cggcttggtt tcctgaaagt caggtgaggg 120

ttttttacaa aatgatgtct aggatattcc agtattagga aaacctacac atttcaaagt 180

gtgaagtaga cttgcctata tttgtggcat ttaggtggaa tgcttgcttt ctgatatatg 240

taacctgtaa acaaaaaaca cctttctttc ttttggttcc cagcactttt ctttttaaat 300

tacactagaa aataacaaaa aatacatggg aaataatttt aaaagtaccc ttatcataat 360

tttttaaaaa tttctttttc atgacgcaga tgagattttg gaatgggcta ggccccaggt 420

tgctcagaac gtgcaagggt catcatgcaa atgaaacacc aaagttctgg ttgtagggcc 480

ttgtggctga gagtgtgcaa gaactgtgaa gttatggctg gcaaaatagc aactgggcga 540

gtgaagaagc attattagtt gtatgatgcc caataactgt agtaaccaga acacacctaa 600

gtttatctgg atgacatgca gatggtatca cccgctgaca gcaggaatat tggccagggg 660

atttacaccg tggcttgcgg ctccagtgcc tatattagca ctgggtcagc ctctcacctg 720

caccacgtcc catccagccg ccctgccccc tggccctggt gccctgatac cctggaggac 780

gcacgcatgc tgccccagcg ttggggacag cgcgccggat gagaggggct ctgggaaagg 840

ggacccgggc cgcgctggtg acagg 865

<210> 84
<211> 710
<212> DNA
<213> Homo sapiens

<400> 84
ggtgggcggt gggggaagac aggatgcaaa tcctgcagcc tgctgaatcc ggctgcactc 60

agcctctgct gctcgctctg tgactggggg cagttttctg ggcagtggga ttagtgacgg 120

tgctcccagg cgtgcacgca ggtgcccatc ccatctctcc atgcagtcat ctggctgccc 180

ctgggagtgg cgtggcctgt cctcctccct ctctgctctc tgagccctgt cctgtgcgct 240

agcagcagag tcaggggctc tgggtgggct tccccgctgt gagcgccttg aaggcagggg 300

ctttgtcgta ttcatccttc accaaacctg caggatgcta acagcacatc tgtccttgca 360

atttggaaaa cagaggcggc ataccttctt catttccttg ccaatttctc atgattaact 420

gggctgatat gagtattgtg tttctctatg aaggaagagg acccgtatta tagtttggct 480

atttcagagt acaccaggca cattcggcaa ggcttctact gaaaggcctg gtcagccact 540

ctgtaggagc tggtgcctgc tcatgcagcc aagcttggtt ccctgcagcc tcccaagcac 600

agtctggcca ggttggttat ggctgttcct caggctgctg tggaaggctc ttgttggctc 660

cggccccaat ccctctttca agtcacagtg gtcagggtca caaaactggt 710

<210> 85
<211> 620
<212> DNA
<213> Homo sapiens

<400> 85
gccggggtac agtgggaggg gcactaggat gcactgctgg ataggacccc gggcaggtga 60

tgggcagtca tcaagaagga cttctgggta cctggtttag acaatgggtg ggcagtggag 120

acttctcaag acaggggatg aagcagtcat caggtttggg gtaaaagtca ggacaaactg 180

gcttcacgtt ggttggtttt gaagaacctg tgagtcttcc aagtagatag agccaaacac 240

aggcctggaa ctctgcagag aaggcaggag ctagaaatag gcgtggagcc cttgtgaggg 300

tgcagcacca ccagggactg tgcgcaggtg caaagaggcc aagaatggaa ctccagggag 360

ctgagagaga gagagaggga aagaaagaaa gagagagaga gagaagaggt cggggaagac 420

agggccgaac tggatgacag tgatcatggg gaggccacaa ggaccaactg ccccacgagg 480

cgtgtggaca gtgataccta atactacaga aaccgtccta ggatgcaaca cccagtggtg 540

tggggactgg gtgacttttg cactcctttc cggcctgggc actctttcat cctctgagtg 600

cgaaaaacac accttttcta 620

<210> 86
<211> 629
<212> DNA
<213> Homo sapiens

<400> 86
gggtttcacc ctcctctcct cttcctccca gctgtccctc cctcccctcc gggacactgc 60

ataaggaatt gccccaagac tcaggagtgt acacaggccc tgggcaggtt cacagagcag 120

ctctgcttcc agctgcgggt agttccctca gcgccccccg ccccacctgc ttgaaccagg 180

ggtcagccat gatgggtcga gaatgttccc caaaaggata tgtcaaagcc ctacccccaa 240

gtacctgtga acctgacttt atttggaaat acgatctttg caggtgtcat caagttagga 300

ggtcactagg gtgggccctg gtccaaaacg attgatgtcc tcagaagagg agagagactg 360

acactcggga aggagagcac tctgtaagga cgaaggcgga gacgggactg acgccagaag 420

gcagggaccc agagaagggt gcccccgtca gtgagtgagc aggccctgca gatgccttga 480

tttgaacttc ttgtgtccag gactgggagg gagcacactc ctgctgtttt aagccacgca 540

gttaacagtg ctttgttcca cagggcaagg cagaggctcc ggagaacaga gatgcaggag 600

acagcaggct gaagcaagct cagagcacg 629

<210> 87
<211> 529
<212> DNA
<213> Homo sapiens

<400> 87
ctggggactt gaatcagctc tgcctggcct tggctgcttc agtacctgcc ggatgtgtgg 60

cttcgggaac actccttaaa acctgattgc ctcagtttcc tcctgtattc aaagtttgtc 120

tgtccaattg tttataacaa aatcaacctc caggagagaa tatgtgtaaa gtctgcttag 180

cgttactact tttattctta ttgttagaaa tagctgactt gcaaaaatat tcctgaccat 240

attatggggg aggggaacac gtgtgagctg cgtcttgcct atgcacagag cctctggtgt 300

ggatgtgtcc caccgagcac tgagcctccc atgcagatgt gtcccgcttg tgcacagggc 360

ctctcgtgtg ggtgtgtccc gcctgtgcac ggaacctctg atgtggatgt ttcccgcccc 420

cgccctccac gtgcagtgaa cctctcgtgc agatgtgtcc cgcctgtgca cggagcctcc 480

ggtgtgcgtg tgtcccgcct gtgcacggag cctccggtgc gggtgtgtc 529

<210> 88
<211> 315
<212> DNA
<213> Homo sapiens

<400> 88
ggacactccc aggggtgatc ttttcattat atgtgatggg ctgggctgcc gggccatctt 60

tgtggtgaga ctctttgccc ctgttctaga caacagtgtg ggctgcctag gtgtccgtct 120

ctggactcca gcagggtggg gctgctgacc aggctccaca agtgctctgg gcagcagctg 180

cagactcgac acttctgcca aggcggcctg tgtgggcaga accagggcat ggaaatagct 240

gaaatccaat gggcgtgtat gcttccggct gctgccctgc tcatgctctc atctagcagc 300

cccatctgct gcgtg 315

<210> 89
<211> 593
<212> DNA
<213> Homo sapiens

<400> 89
caatccaggg tcattctaat gatgaatgag atgctgcctg caggagctcc gggcactggg 60

accagggcag aagaggtgtg gacagaggtc aggtccctgc cttgttgttc tggccaacac 120

```
aggctcatgt atggaaaaca gggaaggtga ggagactgca gctggagcaa gtctagtatt      180

catgcatgcg aagtaaatga tgaagcactc cacaaacatt cattgaaccc ttgtattggc      240

ttatgtagct ggttgactga aaaatcagtc ctccttttt ggtaagaaac tctctgatga       300

cagcatgtaa tcttcatcag gtcttcataa ttccagaaaa tgtcttaaaa attgctttta      360

tttgagctga tgggccttgt cctgggttgg ggcactagga atcctccagc acacaggaag      420

gctgcctgca caggtctcct tgtatctgca cagctggcca cccaggagcc gatgcaattc      480

caggtgagtg aagaagcctg tggatataaa ggatggagac agactccaat cctttccatt      540

ttccggttgg atgttattag tagcgtttga tagaagagga ggccctgaca aag             593


<210>  90
<211>  585
<212>  DNA
<213>  Homo sapiens

<400>  90
atggcttagt ggatgcgggg tcctcggggt tccaaagggg acaaatgccc gggtccctct       60

tcttttcacc tcatttctga ctgagccaca atctgctact tcctttcttt tccatcaatt      120

ttccaggaag gtgaatgcat gcctgggtca ccctcatgtc ctcacggagc acagctcttg      180

tctctgtgct ctggaatacc tctgggggaa gctcatagga ggggcccacc cagcagcaca      240

gcttgaggta tatcttctga tgcagaactt catcctgagc catcgcgtgc cctgattctc      300

ctcatctctc tgggtatttc ctggttaccc tgcaaggttc aggtctccat cactcctctg      360

cctcagtcac caagtgctga ttcccttggc ctaagcccct gtcctgattt tgtttggatc      420

tctgagggag atgagggaca agggcatttg ttccttttgc cccaagaagg ggagaggccc      480

tgcctggaaa caccaacaga actgcagcac aaagagagac ctgaagacca ccatccggtg      540

tttcccagct cttaatcaca gagcacatca gttatctaca gacag                      585


<210>  91
<211>  378
<212>  DNA
<213>  Homo sapiens

<400>  91
acttttctga aaaatgaagt ttctatgcaa aaatggcact tttttaggcc ggttaggatg       60

ttgtgtgact acaaggtctt tggtgatgtt tagtgtctga gctctgccag cctgttttcc      120

atgtggcaga ggagggatca cagagatgtt gtcaggatgt catctgctgt tctgatgagg      180

gctctgccta gcggtgatgc tgaaaaacaa gaggtgctgt ggtatgacag ataatctcca      240

catagctcgt gtgtgtttta tacgggaagg aatacatgac ctgtcatagg ttggtttgcc      300

tttatcttga ttatttctct taatgctccg gtccaaaatg aaatgtacct ttactctgat      360

ctgcagtaat tgaattta                                                   378
```

<210> 92
<211> 290
<212> DNA
<213> Homo sapiens

<400> 92
ggtgccctgg acagcgggct agatgtctcc tgcaggctcg aggacttccc ggaggggggcg 60

gcgttttctc ggggcctggg aagaatgggc gggtttcagc gggcgccttc tgggtgactg 120

cccgtttcgg cttcccctga ccccgtaaca gagcggaaga ggcccagaga cgccagagga 180

ggggcgtcag cgggacactc aggccaataa gctctgactc ccgctgtctg caaaaatgac 240

cggatgcagg aatgtcacct agaaccggcc agctttccga cctctccggt 290

<210> 93
<211> 484
<212> DNA
<213> Homo sapiens

<400> 93
ctggatggag gtggagatgg gcaaccttgg gaagaggact gtgccaggcc ggggcaggac 60

agtgggtcct gtcctgcaag gtcctggtga attcttcctc aactgcagag gaggggaggc 120

atttggggac ctcgaggtct cttcctggaa cctggcccct gagagaaggc tgcccatctg 180

ggatttgggg agtggaagtc tcttctctcg gtgggcattt ctagttattc tacctcatgg 240

tggtgcagag caagccagga cggggcctca ccaggctgga tcctgaacaa accactgctg 300

tatggtcgca ggctcacagc gccccgagtc tcagtttccc caagtgtaaa tgggagttgc 360

ccctgtcctg cccgttctct ggcatgtggt gagagtgaca gtgcatgtgg agtggcccag 420

tgtcctcata gggccgggtt cccaccctgg ctccatcgca gggggagtgt gcaagtgatt 480

gcac 484

<210> 94
<211> 480
<212> DNA
<213> Homo sapiens

<400> 94
ggtgaagacc cctgagcact gtgggtgtga gcgctctctg gggaggggcc ggccctggct 60

ggtcagcctt gtgtggcact gtactggaga ggcacatctg agggacagct aagctccccc 120

tggcgggcag ggattgtgcc ccatttcatg cacaaagcat gcttaagttc cccctgcgag 180

aagccctgct gtgtgtctgg attcttaggc ctgaaaggcc ttacctttca catcaagcga 240

ccagtctttt ctaatgagtt caagtggtag cacatctgat agggcaaaac agcctcattt 300

acaaagagaa aaataaataa aaatgttata tagccttgag aataaggcca gacagtcagc 360

ccaaggtccc tggagcaaca catagagaaa aagagttggt aaaagtcttt cttcttctgt 420

ggactctgtc cgggcagcca tgagctggaa ctcagcccag ccctcgtgcc acgggatatt 480

<210> 95
<211> 424
<212> DNA
<213> Homo sapiens

<400> 95
tccaaaagct gtctgttgag ggcagccagg ctggagaggg cagggtcccc gggggaaggg 60

aagcctgtgg gtagcctcag agccactctt ccccttgagg catctgctga ttcatgagca 120

gcatgggggc caagaggcta aagccaagca gaaagtgtgg ataagaagct gagaacttga 180

gtaggagact ttggcaattt cgaagggctg ggagataaat ttggagttca aactacaaga 240

caagcagacc tgggggccaa gatgtctggg gaagagaagc acgtggaggt tagcttctaa 300

ggcccctttc tctggaaggc attcacggat ccgtaagcag cacccagcca aggagtgaag 360

catggctaag agaccgggaa gctgagcaga gtttttggca gtctcgtggg gcttggaaga 420

caga 424

<210> 96
<211> 431
<212> DNA
<213> Homo sapiens

<400> 96
atttctggct gcaggagggg gcgaggggca gccgcgacgc ccgccaggcc ggcctaccag 60

cagctcgctc tgggctctcc tatacacgcg caggacacac acgagcacac gcacacactc 120

acatgggggc tcgccacatg ctacagatgg gaccaagtcg ggggccacgg cagcagaaaa 180

aaaaggacac gatgcgtacg attaggtttt ggcctttagt ctgaaaaagt gttgattgaa 240

agtgtacaac agagagcggg tgcaagcggc cgagggccat ggagccgcca ataaaaaaga 300

atgtccttaa ataaagttca cagagtaaaa accagaaccg ccagtccttc cctccaacac 360

aacagagcac aggcacagaa ccggtgatga gcccgaggag cagaggcggc tgggaaggac 420

agcagaggct c 431

<210> 97
<211> 746
<212> DNA
<213> Homo sapiens

<400> 97
cttagtgttt agcaagccca aaggaagctc acagttattg aggggaggcc ggtggaaagg 60

aaaggggaac agaggagtgc tagaaaccag gtatcaagca ggtggattat ggaaaagcaa 120

tctcagatag ccacagcagc agagaataac aacagcagag cctaaacaaa caagaagagc 180

ccttgggatc cactttttca tgaggttctt cagtggagca ctcctgaggt agtggccaag 240

ctctcctaat caccagccac cacgttgccc tgggcaaggc ttgtcacaaa gaagagcagg 300

gccacagaca aaggcagcca gcctcacctc gcctcagcac actgcacagc agccatctaa 360

tagcaccctg caaaactgac agattgttcc tctcaacata aactctgctt cactcctaaa 420

aggattttat tacctctcgc ataggagttt gatattggcg agattaaatt aacacagtta 480

aaaaagggag gaaagaaaaa gaagagaaaa gaaagagaaa ccctatgggc aggtgagggg 540

actgctatta gcactcagag ctttgtggga cagcacctgt cagctcttac ttcttcgagt 600

gactctccca gtgtgtcaag ctgtggaagg tctccaggag atgctccgta tgagaggtta 660

aggctggttg agtgacaaac ttacagggac aaaagccggg acaggatgcc atgtgaagtt 720

tggagccagg gccacccacc cagggt 746

<210> 98
<211> 625
<212> DNA
<213> Homo sapiens

<400> 98
tgtgtccctt tctccctccc tccaagcctg ggcagagcag tgggcaggcc gggagacgtc 60

ttgaagatgt ttgcttccct tcttcagctc tccaggctgc tgacatcctg gctccctgaa 120

gtctaacagg ggagaaaaga gtggattgaa aataagaaaa tgctcgcgtg aaggagtaaa 180

aaggcagctg gaagtacaac attcatttgg agatggaata aataaatgag ataaaatcct 240

aaataaatat cttacacggt tctaattgca ttttaaaagc tgccaccaga gaacagataa 300

aatgatgctg tatttcatta gtggctcctg taattctatg gtattcgcca gcaagccctg 360

ttaaagtact ctgtaattaa agaccagttc taggtcctgt aatttagagc attatcaaaa 420

tacggcaagt gacaggaggt aaagaaatgg aaacaaagcc tgctttcatg aggtcctggt 480

agaccacata gaatgtgaat ttttacagct cagcctttgt attgccctca ccaccagggc 540

cagtccacat caatgctttc catttgacag gtgaccggtt atgtttttgt tttgttttgt 600

ttttagatga agtctcactc tgtca 625

<210> 99
<211> 889
<212> DNA
<213> Homo sapiens

<400> 99
tgtcatcacc aaacctatca ggtgagcacg accaaatttc aagcatgtcc gggactacta 60

acaagattgc caggtggaat tgagactgct taaagattta atcaattgtc tgcagtgcag 120

cttctatttc caaatcaaag ctggaatttg tttgtgtggt tcctccacag ctgtctgcac 180

ctgttcctta gggaaaggaa gaatcagttt ccatgatttc tgtcttttcc tccacctcct 240

cagagttctt gaacttggcc aggccatgct tccaaactac atttggccca aatcttacag 300

tatctagtct ccctgctttt gctcttttct gaaatgataa cccagattga gctgggtttg 360

gtttcattcc cacatttatt gattttcttt ccataaataa atagctatgg ggtctattat 420

gtgtccaaag agtcaataaa gcacataggg aagcatgtca aaaatgacga agaaagttga 480

atccagcaag aatctttcca gtgccaccca tggagcgaaa tcatccacat ttgccgttca 540

ccttcactgc ctcccaaata ccaaacatca gtcgctggca tgtgattagt gagggagctc 600

tgagttgatt aagtgcggtg acattttgaa aaaagcagga cattcctcat tatccgcaaa 660

tccgtgggta tgagagagct ctctcgttca catattagca tttccacatc cgtctcctct 720

tctctgcaac ctctagcaag gcagcctgcc ttattagtaa catgtttggg attaacagct 780

ttgctattgt gttagcatct ctgtcattac ccagggaggg aaattggcaa gtgctaagcc 840

ggttactaac agacctaaac agtgcccagg atggaattca cacatggaa 889

<210> 100
<211> 735
<212> DNA
<213> Homo sapiens

<400> 100
tctaactgaa gaggctaaga acgtttcagc ggttcctgga ccaggtgacc ggaagactgg 60

gataccaact ttaccctcta ctttctactc acacacagag aagcctggta gtttctacca 120

acaggtcttg ccacatagtc atctacctga agaggctttg gaagtttcag ttgctcctgg 180

accagttgac cagacgattg gcacaccaac tgtaacctcc ccttccagct catttggaga 240

gaagcccatt gttatctaca aacaggcctt tccagagggt catctacctg aagagtctct 300

gaaagtttca gttgctcctg gaccagttgg ccagacaact ggcgcaccaa ctataacctc 360

tccttcctac tcacaacata gagcaaagtc tggcagtttc taccaactgg cattgctagg 420

tagtcaaata cctgaagagg ctctcagagt ttcttctgct cctggaccag ctgaccagac 480

aactggcata ccaaccataa cctctacttc ctactcattt ggagagaagc cgattgttaa 540

ctacaaacag gcctttccag atggtcatct acctgaagag gctctgaaag tttccattgt 600

ttctggacct actgaaaaaa agactgacat accagcagga cctttaggtt ccagtgcact 660

tggagagaag cccattactt tctaccggca ggctctgcta gacagtcctc taaataaaga 720

ggttgtgaaa gtttc 735

<210> 101
<211> 380
<212> DNA
<213> Homo sapiens

<400> 101
gcgcagaaac tcctggagaa cagccccggc cgccaggtac tcgcccgccc ggaccgcaaa 60

gcccacagag cttcaattcg caggaagggg agtggggagc cctgagctgg ggtgttcctg 120

gaagaggtcg gggagcgggg ggagaggcat tccaggctgg aggcctggaa acgtttggaa 180

gctttgctaa ccttggagac catgtcagga aagaagcgta cccaggccgt ctgctgtgtg 240

cccaccccga tccccatcag tgccccacgc actcgagctc ttcttcggcg tctgcagcct 300

cttcatccgc gcggagaggc ggaggtctcc cggaaacagt gtccgactgt ggcaccccag 360

agcgtccaat gcgagctcag 380

<210> 102
<211> 350
<212> DNA
<213> Homo sapiens

<400> 102
ccacctccct cgccgtcggc cccgaggtcc ccggagtccc cgggcccccc ggaggggcgg 60

tgtgcgcgcc agcgtcccac agaagctggc cgagatgccg agcagccagt atgggctgat 120

cgtgttcgtg gcggggctgc tgctgctgct ggcctgggcc gtgcacgccg cgggcgtgag 180

caagagcgac ctgctgtgct tcctgacggc gctcatgctg ctgcagatgc tgtggatgct 240

gtggtacgtg ggccgcagct ccgcgcaccg ccgcctcttc cgcctcaagg acacgcacgc 300

cggcgccggc tggctgcacc ggctggtgag tccaggcacc gggcaagcgg 350

<210> 103
<211> 388
<212> DNA
<213> Homo sapiens

<400> 103
ctccctttct gccgctgcaa aagcgttctt tctgaccgag acctgcggcc ggaacaagtc 60

ccgctgggcc tccatccaga aaacaggttc gctttttgag aaagaaaaaa aatcccaaac 120

ttggaaaaag gcaaaaaacc aaaccaaaac aacaacaaag tcacacacac acacaaaacc 180

accttggaaa aatacaaaaa atagataagt aaatttctta aatccccaac ttggaaaaag 240

gccacttgct tgcgttaagg gtaacttcta gcattttcta ataagcctac ttagtaaaag 300

ggggacgttc tattctttaa agaaccccct tggctgtccg ggttggggat gtaactgttg 360

tctaggtaca gtggattaac gaaggaca 388

<210> 104
<211> 323
<212> DNA
<213> Homo sapiens

<400> 104
cggggagccc tgcttgtcag gggcgggctc ctcatcactg tcgacgctcc ggctcagcag 60

ctgctcattc tcggatgagg catcgttctc gctgcaaaaa caagagcgat ggtcattagc 120

```
agtgcctggg ggggctgcag ccctgacaag ttctcctgtg gtgaacttgt ccattgccca      180

gctgtggaca gtgggggca atgacttgtt tttcaggtgt ttactgggca ccttgtgggc      240

acgggaccag gggacatgag acgagacata caccggtggc ttcctgcttc tttccaaaca      300

aacaagtccg tggtgggtga cct                                              323


<210>  105
<211>  849
<212>  DNA
<213>  Homo sapiens

<400>  105
agaaacataa aaccccggat cagccatttc ttccccctct ggacaagccc ggacgacacg       60

gggccactgt tctgtgactc tgacctttgc cgtacattaa acaagaggag ggcgggtcac      120

atggacacct cattaattcg ccctccctgt ggcaggagga tggagggaca acagagggca      180

gaactctcca cactgcagcc ttatcaagga taacgagtga ccgtgttggg ggctcagggg      240

aggggcgagt atccctaggg gcggggcctc agtgaaggga taaattagtg catcctttgt      300

ttgcctgcca ataggtgctt ccaggggagc cctgggggct agggaagttt gtttgcccaa      360

agcgtttaaa caatccccct gcaatcctgt ttgcactctt catttgctca gcaaacctgc      420

ccctcggata tagctttgga gtaaactcag gcccagctgc tgctgagctc agggatgttg      480

tttggccctc ccaagctggg gcaggggctc caaatgtaca caatgccccc ccaactacca      540

ccaccacgca aataacaccc catctgacct catcctgctg ggcaagtgag agttaaacat      600

cctagttcaa gttcattctg tctcttatag ctaagtacct gacttttcaa aggtcagagc      660

tgctgcaggg ctgtctctgc tctcaggagg acacccttcc tcccactccc tctttgccag      720

gctgcagtgg aggggcctgg tggccctggg gtgttgggtc gggccatctt gcttgcaaca      780

cgccccgcag tcctggggcc ggcggggttg gggcggggag tggggggggtc ctgaggtctg      840

tttactaca                                                              849


<210>  106
<211>  506
<212>  DNA
<213>  Homo sapiens

<400>  106
agttggagca ggagaccaaa ccaggagatg ccagggccac acaccagccc ggttctccct       60

ggagcctcga gaagcagagc taccaccctg gatctgcagt cagcactccc acccctgccc      120

tgcccctgcc ccaagcccag caccacccat ggagagggtg acctcaggcc ttggagattg      180

ctggctcgtg gctgcaggtg gcttggatcc atgcacttgg atcctggtat gctcccctca      240

catgtcaaaa ttcctgtctc catccaatca tggcataaca ctgtactccc tgggggtcca      300
```

gcctgcctat ctcacaccca gagttgcact gccagtctgg gctgggatag cgatgtggtt 360

aactgcaacc aactgctgtg gttataaacc aggtaagggg cccagtgaca gccccgctgc 420

atcattggca aaggactgct tgggggctga gctgcccggg ggctggatga gatgacccct 480

ggaggccttt ctgggtgcca gactct 506

<210> 107
<211> 368
<212> DNA
<213> Homo sapiens

<400> 107
agaggaattt caaccctggt gagagaggag gcctccttgg tggtttcacc ggtcccccag 60

tgggaaatgc cacatgagca cttgcatctg tgactgctcc ctgttgccta ttatttccct 120

aaagttgccc tcacacagct atttttagta caaaggcttt attaccaagc caaagatgtt 180

ggctctgagt gatccaagaa tggacaacca agtgagtggg tagtcaggga ggcctgttca 240

ggagccaatt aggcatcgtg agctccacca gcatgccacg gaagcacttc tgggatagga 300

catgaagaag agctgtgccg ggggatggtt tggggtgagg gatgccaaga ctttaggtct 360

ctctttta 368

<210> 108
<211> 454
<212> DNA
<213> Homo sapiens

<400> 108
ccggtgctgg cgccgccggt acagccccga cgcggccagc gcacccagcc gggccgccat 60

ggcgcggggc agccgccttg aggtcgcggg cgcgggccgc ctggtgcagg ggctatggga 120

ccacctcgga ggggaggcgc ggccgcctgg ggccagacac caagacggga cgggcgtgtg 180

ggcgcctccc ccgcgctggt cgcgagctca cgttacgcgc tgggatctcc aaagggcagc 240

agagtcaact ccaaatagaa aggatattgt tcgcgccgcg ggggcagctc ctgaaagctg 300

aggctggcgg gggttgggtc ccgctgggag gggactggtc tgggaaggcc ccagacagca 360

ctgaggtgag gtccacagtg gcggcaggac acccaggagg tctccggctg ccacctgcca 420

ggaggaaagc ctgtgcccgt ggacacactg agga 454

<210> 109
<211> 642
<212> DNA
<213> Homo sapiens

<400> 109
agccagcgac tgggaagtga ttccgccctc aggggagtgg gggaccaacc ggcagtgcgg 60

agccgaggcg tgggaggctc tcctggcggg ggccgcggtg agggggcgcg gcggctggcg 120

cttctggagc ctgggtccgt accccctcct ccccaagtcc cagccgccca cgtccccgca 180

gcccgcgcgg gaggaaaggc cttgaccttg cacgctgggg cgctgaagtt agacgctagc 240

ctactgggct gcacttccag ggaccagggc ggcgggggcc gcccaccacc aggaccgaga 300

gggaaagcga ggctttcctt tcctgctcga ccccctcacc cctgcggctg gctttgggac 360

agtgccccgc agccccgcat tgaagagatg ttgggctgtc tgcagaggcc tgcggagcgg 420

ctttatggct tcctccttgt ttgaaaagaa tatcagacga ggtgcatttt cccccatcca 480

tgtttattat actccaatga aagagttata aaacattgag ccaattctta agaatcgtga 540

gtgcccacca aatattctgt ctttcgtggg ttggttggag agtccagcgg cccgggccca 600

acctggctgc ggagggctcg ctccacgttg gctcgtgtgt gg 642

<210> 110
<211> 516
<212> DNA
<213> Homo sapiens

<400> 110
gagccccgag aggccgggtg cctcctccca cccccgccca gcagaaggcc ggtccgagag 60

gctccactgt gccaagcgcc ctgccaactg actggctctc gctttggctg gggagattgg 120

ggatgagggg aagaaaaggt tctccctggg tggctgcaag aaagagtgcg cccgcctgga 180

gagcgcccag agagtttagg aggggaaggc gattgcagag tcctggggcg gggcgtgggg 240

agctgcgaac ggaaaaggct ggggcgggga ggtgaggctg gagcccagga cgccctggga 300

gagtgcccta ctctgccgca gcgccaagct cgcagagccg cgggtaaggg gtacaaagtg 360

cgcctggtgc ggctggaagg atttctgggg ttggggagta atggggacct cgagggacca 420

ggagcagaac tgtgagaaac ggagctaggt gatgtctggt ttactccggt ttggggatgg 480

tatagacagc taggtgtcct gaagacagac aaagtt 516

<210> 111
<211> 585
<212> DNA
<213> Homo sapiens

<400> 111
gcagcttgtg cccaagaaga agcggcagcg gttcgtggac aagaacggcc ggtgcaatgt 60

acagcacggc aacctgggca gcgagacaag ccgctacctc tcggacctct tcaccacgct 120

ggtggacctc aagtggcgct ggaacctctt catcttcatt ctcacctaca ccgtggcctg 180

gcttttcatg gcgtccatgt ggtgggtgat cgcctacact cggggcgacc tgaacaaagc 240

ccacgtcggt aactacacgc cttgcgtggc caatgtctat aacttccctt ctgccttcct 300

cttcttcatc gagacggagg ccaccatcgg ctatggctac cgatacatca cagacaagtg 360

ccccgagggc atcatcctct tcctcttcca gtccatcctg ggctccatcg tggacgcctt 420

cctcatcggc tgcatgttca tcaagatgtc ccagcccaag aagcgcgccg agaccctcat 480

gttcagcgag cacgcggtga tctccatgag ggacggaaaa ctcacgctta tgttccgggt 540

gggcaacctg cgcaacagcc acatggtctc cgcgcagatt cgctg 585

<210> 112
<211> 469
<212> DNA
<213> Homo sapiens

<400> 112
tcccgcgctg ggaaacccgg aggggagggg ctgtggaggg cgggagcccc gggcagtagg 60

tctgtgcttc ttccttggtc ccattacacg ggaagggcaa ggtgacgcca ctcagccacg 120

caggcctcgc attggtgcct tcccttccct agccaggacg cggctattaa acgctgcttt 180

tagccgattg tatttcctgt ctttgaatcc gttttctttc tcctctctgg ttggttggtt 240

ctagctctaa gcccatcaaa gactttaagg atcctacaca gagtgctgca ggctcccttt 300

gtcagaattg gggcccctag gagctgagtg cggcaggttt cgcctgttcc tggagctcgt 360

agtcagggtc gctagtgcac tgccactctg aggactggac ggagttccag acctgcctcc 420

ggggacctcc tgcgtgaggg cggaggccct ttcatgaccg tgagatgct 469

<210> 113
<211> 625
<212> DNA
<213> Homo sapiens

<400> 113
ccagagcgct gtgcaggggg gcagacggca ggcgcgggct taggcgagcc gggtgctgcg 60

aattttccag ggcctcgagc acagcattgc cagccgagtt ggacaaattg gagaatctct 120

tgcccgccgt agggctgtgc agccctctct ccatcagaat catctctttt cttattcttt 180

ccatcatctc agctttctta aaaatgtcac agtggccctg ctgtcccgtc ctaatgatag 240

gctgcgccta gggctaattc tcattcagcc ccagcgaacg cctctaaata ttattatcgc 300

tttcggaggg aggcggaaaa attgtgggat gccagagcga gggaatgact ggtcaaaatg 360

acccatacat ccttccgatc ccgagatgtc attcatcaaa aagtagcgcc cgctcgtcat 420

taaggtacga atgacgctgt tcgaataatc atttattgta acaggtttat aagcaaataa 480

atacaccctc ctgtcagtgg gtaatgaagg cagcttcaga gcagacaaat agatccaggt 540

aggaggcgag aagagacaag tgaggaggaa ggctccggtc ctttgcccgc tccgagccag 600

ttctgctcgc ccgggggttt ggcct 625

<210> 114
<211> 306
<212> DNA

<213> Homo sapiens

<400> 114
gacgcgagtg ctcggaagag ccgccccacg tgctttcggg gaaactgccc gggagcgaga 60

aggactggga ttgagaggaa accgaaaagc gaaagttaga gcctgcggga gcagtacgca 120

ggcgcagcac gcaggcgcag cggggctttc ccgcagcccg cttcctccca acggtttctc 180

cctggcggcg ggcgcactgc tcgcacgctc gccctggggc tgcagatcgg cttgcgggac 240

ggggtcccag agaggccggt ccacgcggcc cgcacgcgcg agtgttagct ctgcacacga 300

cggaag 306

<210> 115
<211> 298
<212> DNA
<213> Homo sapiens

<400> 115
ggggagagcc cactacaggc aaggcaggaa ggttaatcat tttgttaacc ggaaagggtg 60

ctgccccgcc tggccgtcgt caccgagcgt gtcagtcacc cgctctcgat ttcgccgcag 120

aattacctgc tcaaagagcc accccaagaa gccctcagct ccgaggtctt cccgcccgtc 180

gtctgcggga agtaaacggc gtcctccctc caaagaagtg gatgcccctt cgactctcct 240

ccgccccccg gcggttccta aatctaggaa agccagcccc ggcatcgctc actactcc 298

<210> 116
<211> 579
<212> DNA
<213> Homo sapiens

<400> 116
taaaaacaat atttccttca tctccctgcc tcgcactcaa tcctccaccc ggttgtttat 60

aaacaatccc ctcgactgtg gatgccaaat tgttcctttg ggctgtgcca gccccaggac 120

ctcactcacc taaaggatac cgttgcagct ccaccaaacg tctgtgaaac ggggccttac 180

gcctctctcc tggcccgtgt gtgtgtgtgt gtgtgtgtgt gtgtgtgtgt gtgtgtgtgt 240

gtgtgtgtgt gtggtcaggg cgtgtgtatt cgcctggccc gtgtgtgtgt gtggtcaggg 300

cgtgtgtgtt cgtgtgcgcg ccgcttcgct aacgcagagt cctcgccaga cgctctggga 360

aacaggtgtt gcaggggcgc caagatccag cgacacctac agactggggg tgacgccccc 420

gacttggccg tgtggggaac agccgaagca gaagcaggga ccctcttcct ggagcccaag 480

agaagcttcc agactcacca atttggcggg cacggggcaa acttgtagcc ggaagcgagg 540

cgcagctggg gcagcccggg ccgggtcctg ccgcactgg 579

<210> 117
<211> 486
<212> DNA

<213> Homo sapiens

<400> 117
ccaggactgg tgggaaggag tggtgacccg ctcgggctcg gcgtggaccc ggagcgcgcc 60

tggcgctgtc cgtggtgctg agaggtgggg aggccagcgt ggcggcggcg ctgggcggaa 120

tgtgggcgag acatctccgc aaggcttggc ggtccttccc cacggccctc atcctctcat 180

tgccaccagg cctcctccca cagctgttcc tgaatccgct gcattggcct ctaaaaccaa 240

aaatcaggga gagggggcga cagactagga gtttgcgctc tcttccccat gcgctctgcg 300

caggagttct cgagtgtgcg cagtctggca cgtgagtcag cgcggggtct acgcggattg 360

gtgcggggga aggggagaag gatggtccca ttccccctcc atcctgcttt ccctcagcgg 420

tccccttatt ggaaaccgga tcacagcacg aggaagccct ggctcgtctc ctggcccagc 480

cttctc 486

<210> 118
<211> 392
<212> DNA
<213> Homo sapiens

<400> 118
cgggaaggac agaggcaggc taatgagacg ccgcagcccg acggggttcc ggggcaccgc 60

gaggagagac acaggcctgg gtgcagaggc cccagccgcg agcctcattc actggggaaa 120

ccagggacca ggagggctcg gcggggccac cacccccgcg tgcacagtgg agtcttctcc 180

cctgtccccc tccctgcaca cacgtgcggg tccctgggtt gggagggccc tgatgggaag 240

ggggaggagc caggcacggg gcctggcacg tagtgggcct tcattgaaag gctgtccctc 300

ttcccttcgc ctttctggtc caggacctgc cccagccaag gccgggcaga atgggggtgg 360

ggggtggaga agcggaggct ggagtgagga gg 392

<210> 119
<211> 532
<212> DNA
<213> Homo sapiens

<400> 119
agccttccaa gcgctgctgc agtgcggtcc cagctccgcg gggggtcccc ggggatgacc 60

taaatcgggg attgcggtgg gacagacggt ccaggggcta aggaaccccg tggagcaggc 120

agcggcgctc aggaggcagc agcttgagat ccacggctcg atttttaaaa attgtaaggg 180

agaaggatgc ctcgaaagcc tctgtggtat tttgtcgcat ctcgcacccc tgtctaaact 240

tcagtccact cgtggagtgt ggacccccac gccgcatggg ctcaggaggc gcttcgcctt 300

tggaaagaaa tcgcatgtat taagggaaag cacgtgtgct actttgagaa aggaggcaga 360

aagttaccaa acgcgggaag gggaggctcg ccaaccaagc gaggccgtgg gacactggcg 420

agtcgcagag gctcctggag ccagccgctt ggaaacaaac aacaacgcgg cgggcgaact 480

cccgggcagc ctcccccgtg ccggggcgcg ggggccgccg cgagaacccg gg 532

<210> 120
<211> 514
<212> DNA
<213> Homo sapiens

<400> 120
tcaggggagg gaagggagat gcggatggga gtggctctcc tgccgagtcc ggaggcagcg 60

gctgaggctc cagcccctcc ctatgtctgc agcgtccgtg tgcctggccc tgcgcccctg 120

cctcaacggc ggcaagtgca tcgacgactg cgtcacgggc aacccctcct acacctgctc 180

ctgcctctcg ggcttcacgg ggcggaggtg ccacctgggt gagtgactgg cccagggcgg 240

gaccacccgc tggctgcgct gggctcagga ggagcactgt aggctccgcc agtggccctg 300

ggcgcccagg gtcccaggtc aggagtctct gtccccaggg tgtgggtgac ttgcttaggg 360

gaccactggg gaccaaaggc catggccccc tggagtgagc accatgggcg ggtgcagttg 420

ctgccctctc tgagccaatc tcgggcttgc tcctcctccg tccccggccc aacccgagcc 480

cttagagaaa actcctcatc cgggttcccg ccgc 514

<210> 121
<211> 718
<212> DNA
<213> Homo sapiens

<400> 121
gacacctggg gagctgaatg cacatggctt gctgccagcg gctccctccc ggggaggagg 60

ctgcacagag ccagaacgca gtctgcagca gcaatgacct tccgaccccc agggacagga 120

caaagactct gtccctctca catcctcacc ctccttactt ctctggctcc tcagcaccac 180

tcagtaaggt ggtgactgtc attgccccca gtttacaaat gaggaactga ggcttggtag 240

gagggggcac actcggtcca ggacttcaaa gcctgagctc ttcacctctg ctctctccca 300

gaagggagac acgggccaag tagggcaagg atggaggaaa ggtcccattc ctgctctgct 360

gcccgcaagc tgtgtgccct cagtcaggaa agtaacctct ctgggctccg tgtactcctc 420

cctaaggcct gcacgtggta cacactcgat tagtgttaat tcttcctctt ccccgaggag 480

gagctgctca cagtctagag ggaggatcta aatatttaat taaggaaact tacaatgcaa 540

tctgagatgg gactgaatag agcaatgagc aaagtgatct ggtgcaccaa gggaggaggg 600

ccgagcaacg acgggctggc caggtagaga ggcaggacgc caggcctgct tggctcccac 660

ctgcacaccg gtaccctcag aggcacagcc taggcatcct tcaccatgac agagaggg 718

<210> 122
<211> 500

<212> DNA
<213> Homo sapiens

<400> 122
caatgtcacc tcctcaggga ggccttcctt gatcacccag tctctactcc ggtggttctc 60

aaacctggag ggtttgataa aacacggggt ttctgattca gtgaggctgg ggagggtccc 120

aagaatgtgc ggcgccatct gagagccatc gctcagccct attgccctgg tttccctccc 180

tcgctttgaa atggtgtctc cagcgacttc ctcccgtcta cgctccatga gggcaagaat 240

ctggggctgt cttgttccgt gctcagcgca gaggctggcg cggcactgaa cgaataccca 300

gagttaaaaa taaagggata gatggtccgc tgggcgaagg agccaggaac accccagaaa 360

cccaactcgc tgagttctcg tccttcgaac cccagcgcc tcccgcggtg cccatggcca 420

cggccccttt aaatgtgcgc agggacgccc cggccctcgc ctccctgaat ctctgaccga 480

gcaggggaaa aaaaaatcct 500

<210> 123
<211> 689
<212> DNA
<213> Homo sapiens

<400> 123
ggagcactct gggacttgta ggcggccttc gacccggcgc ggcgagaacc ggtgtgtgca 60

gcttcgcttt aggacgcctt ccccttggaa ggtttaggtt gtcgcgtcct gtaaattagt 120

catttcccca tccacccacg ggaaaatatt tttacatagg ctaaaagtca gacattgcaa 180

taagaaataa tcaggaaagc tcagtttgga taccaagtca gctgataacg tcctattgaa 240

cgtattgtcc atttttctcc ccaaacatct ccagttttat catgaagagt tcaaactgca 300

ctttctgacg ttgacttaaa cttcttagca aaagaaaaaa agatggtttg ggatcaattc 360

cttcctaaat tcagccactt aactcaggac ctcaattttt tcgtaaacaa actatgggtg 420

agatgtattt ctagggccag gcgcggtggc tcccgcctgt aaacccagcc tttggggaag 480

ccgaggcgga aggatcgctt gaggtcagga attcgagacc agcctgccca acatggcgaa 540

accccgtctc tactgaaaat acaaaaatta gcctggtgtg gtggcagcac ctgtggtccc 600

agctactcag aagactgagg tgggaggatc gctcgaaccc gggaagttgc agtgagccga 660

gatcgcgcca ctgcactcca gcctgggcg 689

<210> 124
<211> 584
<212> DNA
<213> Homo sapiens

<400> 124
atctaattcc ccatgtcaga ctgcgtacta ccttattgct tttgtgtccc ggtgagtgaa 60

gtttttccta ttttcttgtt atggacagaa aaaatccttg gagtccctag gctttatgga 120

```
acattattag cacagctctc taaccagtgt caccctcaaa acatgtcact tatctttgcc      180

tagacatgaa aagcccagct caaactcctt tggcccctta tcctggtaca aatttctttt      240

gggccctcac tatgtcagtt tccacctata tattaatagc tttgcatttc atgtgcttct      300

ggcatttgat tttttttct ttatctaaag gttatatatg tgtgtacaac tttaacgtgt       360

ttttattact atttttatgt gtttgtgaag ttatgtaagg gagagtggat aatatgggag      420

gtaggcatcc aaatcaaatc agtttagcac tcctgaaaga tgagctgatc catgcaattg      480

ccgtgactat gcagtggttg actagacgta aagatgttgt ttagggttat cagccggggt      540

gtttcttctg accaaattac ttgaactgca tgggacccat tatt                      584


<210>  125
<211>  656
<212>  DNA
<213>  Homo sapiens

<400>  125
tctggtcagt accctacctc aaccacgcaa cagtaccggc ccatggcccc ggttcagtac       60

aacgctcaga ggagtcaaca gatgccacag gcagcacagc aagcaggtac ttggaatctg      120

tttcccattt gcttctcaac ccagttattt ttgctggtga atcttgtact ctttggatga      180

gtgtccaagc ttggcaattt aaaccagcac attttctcta cccagagaag ataacaaagc      240

atttgattga ctctgtgtgg aaaagtagta ggtacaagaa ggtgaagcat atgtcagact      300

agcatccctg gcatgcatgt taaacagtct ccataaatta ttttttgatc cttcttctgt      360

taaagcagaa agtcaaccat gtccgtacct ttctagttca taccttcttt taattttttt      420

tttcttttca atttgaagag agtgcttcct ctgttcttaa ggctagggaa ccaaattagg      480

ttgtttcaat atcgtgctaa aagatactgc ctttagaaga aggctattga caatccagcg      540

tgtctcggtg gaactctgac tccatggttc actttcatga tggccacatg cctcctgccc      600

agagcccggc agccactgtg cagtgggaag gggggccgat acactgtacg agagtg          656


<210>  126
<211>  722
<212>  DNA
<213>  Homo sapiens

<400>  126
caacgcgcag cggcccaggg cgcgcgcagc ctgcgcggcc tcccgaggcc ggagttgttc       60

cccgaaagtt gcatggggcg gcggcggctt tcatttctgc accagagctg gggcgatcg       120

gctttcctgc cgctcttagt ggggcggacc gtaataaggg ggcttttatt actaatctgc      180

actccttgac ctaaccgtaa cccactcgca actctgcaat cttcatgcaa tccacaaggc      240

agggagaggg gagcgttccc tggggaaccc acggcagcct ctgatgagat gcttttgaag      300
```

tcattttaat actctccaaa gttcatgtga attttatact atcttctcta atttagctat 360

gtattgatat taaaaataat gtcctaacgt ttattaacat ttaataacta aattaacatt 420

gagttaaatt ggctctagta aggttccaat ccgccttcct ctacctcccc gacaggagga 480

ctgttgggca gatgtggaag cactggtggg tctgggatgt cacgatgaaa ccaggatctt 540

gtggacgtgg cccttccatt gatcaacttc acagacacta acaggcagaa aagaaaaacc 600

catcgagaat gtggcccagg cggcttagct aaaggacaca tgcaacccca ggaaaggggc 660

gtccctcagg cccggccgct ggttagttgc tctgcttctc ccgcagcaga aaacccctgc 720

aa 722

<210> 127
<211> 501
<212> DNA
<213> Homo sapiens

<400> 127
cggaggtcga cacggaagtc tggaccgccg aggcgtgtgg ctcgtctacc ggcaggtgag 60

cgtgcaccgc gggcgtggga tgctgtgggg acagtgcggg ggacgtgtgg ggtcttcgga 120

tgcgaacgcg agtccgtgcg atgcccaggt caagtttgca gagagagtct gcggtgtctg 180

tctccttggc ctcctcttga tgctcctgcg ggttgggggc tcggaccaaa gcttctattt 240

gggggaaggg cttcaggaca ttcttgcctt gagatacttg acaaaggttg aaaagacaca 300

tcaggtggga gcttgtagct gctgctgatg gggtgagaca ggtgcagtcg agtgtgcacg 360

catatgtgtg tggctcctgg gtaagggtca gctcgagatg gggagaagcc tctgcgacgg 420

gtcattctgt ccccaagagc cacttagatt ccggagagtg aaattgagcc gctctgaggt 480

ggtcactaga tgttatggca g 501

<210> 128
<211> 593
<212> DNA
<213> Homo sapiens

<400> 128
tgttgctggt ctgcaggctc acctgccagg gccacttcct gtctggagcc ggcaatcctc 60

cagttatcct tgaagtccgc tggccacagg ctggggcagg tcaatgaaag gaagggacta 120

agagtaacgc tgctgcagca ccttcaccag agccaaatgc gcctcggaac cctcaaagca 180

aggcctgcat cccctgaggc tcttcggaaa atggctgtgt cctcccaggt ctcagggagc 240

cgccccgccc ctggacagat cgtgtcccct gagcccctcc tcctctaggg caggtcccct 300

ccccctcaaa ccaggccagt tctctgcggc tgggcatggc ccagaagcgt cacctgacgt 360

ttaatgctac tgtccctttg gtccatgttg atgatagtgt gtgtgggatt gccagagcct 420

ggtaagaatc agactcacct gagggaagta acaccgaagc ggggcgcact gcgttccccc 480

tggactgggg ggccgctggg gatcctggag cccctacagg tggcggctcc catccgctcg 540

ctccggggtc ctggccgcct ggccctgagg ggagcgcgga ggggaagagc agt 593

<210> 129
<211> 460
<212> DNA
<213> Homo sapiens

<400> 129
tcctctgggt cctcccgccc ttcacagcca tgctcggact gcagggcccc ggccccgcct 60

tggcgcgtct gaaggatgca gctctgccgc ggagctgagg agacgtagcc ttctggggta 120

gggatggagg gggtcgtctc aggggacata cccctgccca ggggtctgag gaaacacgac 180

cctgaattaa gagggatggg gctgagggct tcacagcccc gccctgggga ggtttcagag 240

accgccttaa cctctgcggg tcacattcac gggaatggct gggctaggca caccaggctg 300

ctcccaagtc agaggacaga ggctgagacc cctcagcagc cacctgctct gggtgagcct 360

ccacccgcat gatatttcat ttcctttctt ataatgaggc cagtgagtcc cggtgggtgg 420

cagccagggc ccagggtgtg caggttggag ggaagggaaa 460

<210> 130
<211> 511
<212> DNA
<213> Homo sapiens

<400> 130
attaatagga gccagccctc tagcagcctc agattgactt cccagcaccc ggaagggcac 60

ctcaacctgt cccacactgc tggtgcttag agttacctgt caaagctgct caaggctctc 120

ccatgtgtgc tgtggaccac ggcatctgtt gtcaacacca gcgtggcctt tcctatgaac 180

atgaagctga gggttcctgt gaatgtgagg gtctgagcca gctcctcaga gatggaggct 240

ccctcctctg gcctcacctg ttaccagcat acctagccag gtgactgcag ccttcaccct 300

gcatctaaat tgatggcccc agtccttgag gtttgacagg ctgttatctt gggcccaggt 360

atcctgcaag accatatttg ggtgatcctt ctggtttgga agcactagga tcccaagcat 420

cctcctgact ggcccatcag ggtttactcc aatcccaagc ccggttaaat gtgctgtgtg 480

ctgcccatag ggccaccatt cctcttagtc c 511

<210> 131
<211> 468
<212> DNA
<213> Homo sapiens

<400> 131
tcagatccgt attccgagcc cgcacgcata ccaggctgtg ctcagggtcc ggaggaggcc 60

gacccagtag ccgtgggccc tgtcggaggg gagccagctg ggcgcgcgtt gttttctgag 120

gtggccgcgc tgtctttcag ggtgtgtgta cattcgaaac tccactgaac cttgattgaa 180

gacctacagt gtggcagtcc atggcctggg gactggtaag gattgaggcc gtaggatggg 240

ctcgagtgtg gcggtgtccc cagggcccca gtgagcaagt gggtgtgctg ggaacctggc 300

cacaagtgaa cctcctccag gcgaggggat gccctgcagc atccagccgt caaaacctcc 360

tgtagaaacg ggagcgagca tcaagcctgt gtgtgtgcat gcttgggcgg agtctacccg 420

gaggagaaag aggcttccgg gctggccctc caaagccggt tgtacagg 468

<210> 132
<211> 694
<212> DNA
<213> Homo sapiens

<400> 132
gaaaggagag cctcacgagt cgcatctcca cttaaccccg ccgcttgccc ggaaacacct 60

tggccctctt ccctcttcaa tcccagtgca accgagaaaa ggccccacaa gtttgagaca 120

ctcaaagaac tcacagcgaa ctcacacata catcggctct gtagggtaaa acatccccgc 180

ctgtttacca agagatcaga tccgaaaaga cagtatgaaa tgagacctct ggggagactg 240

tcaaccccag gggtaaaaca aaaattctga tcagaaactg agtttcccaa agaaggggct 300

aaatgttttc caacactttc ggggctcagg gaagatgact ctgtaaggac actgagaatc 360

ttcctcgcgt gccacgggga ggaggactgg gggcgtttga ggggctcagc gcaccagagg 420

agtgaggtgg aggagggcgt tcccgcgtcc tcctcttcaa tccagagcag ctcaacgacg 480

tggctccctt tctatgtatc cctcaaagcc ttcgcgtcgg attaaaggtg ttcttgatcc 540

ttctttacca accacggtgc gggccaggcg tgatgaggga tgagggagag gaaacctcag 600

tagcaaaatt gttcagagga cgttcggagg gcgcggggag cagccggatg cacacctaaa 660

gtctcgcaac acccaactcc tcctccgcag gcag 694

<210> 133
<211> 558
<212> DNA
<213> Homo sapiens

<400> 133
cactcagtta tgacttacct gtcccagttc cccaaagcca agctcaagcc gggggctcct 60

ctcaaaccca aactcaaccc gaagaaagcc agggcctatg gcagaggtga gtgctggtcc 120

tctggtgttg tattggagac atgtcctctg gtgttggaga tgatttcatg gcttcaagag 180

tgatgttctt agaatcaaaa atagataggt gtaatcctca aagagacccc aagcctcctt 240

tgtaacacat tttatgactg ttttattctg ccttgttttt ctaaggcttt aagaaatgtt 300

tctgcttaga tggaaagggc aagtttgctg cttggtgatt ttagtgcagt agcccattgc 360

tcccattttt cagaagagga atcgcggggt agggagtcgg gggagtttgg tcttgcccca 420

gatcaccaca gtcagtgatg gggtgggcc atctggctgc tgattcattt ctccttctgt 480

tacactaagc ctgcctcaga tttccagccg gagtgggagc tattgttaac ccctggcaga 540

tacttccttg ctaagaca 558

<210> 134
<211> 157
<212> DNA
<213> Homo sapiens

<400> 134
cggtgagaaa agagtctcaa attaaccccc ctgagccctt ccctggaccc gggcctctgc 60

cacgcgtgct gggagctgga cagtgaaggg gcaaagttaa agaggaccgg agagccacct 120

cgcatttacc tctttccccc accaccaagg agatgcg 157

<210> 135
<211> 223
<212> DNA
<213> Homo sapiens

<400> 135
cagagttcgg tcgacagacg gaagaacaga agaacgttga cgtcacatcc gggagcgcag 60

ggttgccgtt agttaccgag gaacgcggaa gggtttctat tgatgtcgat ttcgtgctta 120

cagcagttct tagcgaaggg gaagctatgg ctaccgatag aggtcgattt ccccggaagg 180

aatattcaag ttttcgtgtg tgtgctttcc aggaaaatag ttc 223

<210> 136
<211> 327
<212> DNA
<213> Homo sapiens

<400> 136
gctgggggga ataaaatcat acaggtggag cctgcttccc acgagtgccc ggcatcctgc 60

tggggccctc gggtgccgcg cgctccagct cacctggtac acgtcctgca gcccgcacca 120

cgtccgcagc acctggtggc aagcccgcag cctctccgtg taccatctcc gcagcgtgga 180

cacggtcaag ttccatacga agcggctgcc gctgaacagc aggtcctcca cgccacacat 240

gaacaccgaa gccatgcctc ctccttcgct ctgaacccgg gcgtcccggc tctcgtacgt 300

gcagccccga ccccagcggc ctgcccg 327

<210> 137
<211> 347
<212> DNA
<213> Homo sapiens

<400> 137
ggcgggccgt ggggcgcatg cgcggccggc gtccagctct ccgggaaccc ggtacctatc 60

cgcccctttgg tcgggccttc tccgcctcat gacactggtt caaagccaaa cagaaaagcc 120

cgacgagttt attatcccct aaaggacgtc atgtagataa ttaaatgaca tgaataccgt 180

cgaagatacc tgcctgatat tccaaaatgg cccaacggag ccctgatcgc ggcgttccta 240

tgttgaggtt ttaacttcga ttttaagagg ggtcctggga gatagtaggc agcttgccgg 300

caacatcaac aacaaagata catcgtggga tttttgttat ttttaaa 347

<210> 138
<211> 361
<212> DNA
<213> Homo sapiens

<400> 138
agtctgtgtc cctcaacacc tcctgaggca gctgatcgca ttagaggccc ggatccttcc 60

cctcgatgta agctgccccc atgtcacgtg acccactcag gctctgggtg aaggggggtgt 120

tagtaatgag tgcctctgcg actgccatga gaacatgcca ggtcagcttg ccagagtagg 180

agaaacatat gcacagagct gaggcacccc aggcctccag gggcagccca cagcactcga 240

cctgccgatg tgagcgagcc caggacgggt gggctcagtc tcctaagcct cgaagtgctg 300

ggcaacttct ccgggtgatt ctaatcttgc tactgctgag gcgcactagc ccaaaatggc 360

c 361

<210> 139
<211> 650
<212> DNA
<213> Homo sapiens

<400> 139
ccgcacccgg gcaggagaga acctcctcgc cgtacccctg agagcccccc gggggggacc 60

gtgtctcgct gccccgctgc gtctgtcttc cccactctac ccctcttttg aatccagaaa 120

atgacagaaa ttgtcagggg acatttgttt ctggtctttt aaacctagat gccagtccct 180

tcctccacct cgcgagacac cgatgcacag cgcagtcgtc cgaccgacag ggcggaaagg 240

aacgaaggtg gggagagcc agggtgtggc gaggcccgtc agggcggtca gacctaggga 300

aacctggcgt acttggcgca gatggggctc gactcttgcc ccccgtggct tctagcccgc 360

ccttccctct cgaggcctag accctttatt tgggcggcct cttgtggagt ctgtgggtgc 420

ccccgcagac ctggccagcg agagaggagc tgccaatatc aggcgcgcct acctcaaacc 480

agctgctcat ttggatcatt ttatgccaaa tcctaacctg ccccatggga gagaaggaac 540

ggtgacgctc tgaatgccag tgccactatc tccaggacag tgccctgcgt ttcttgatcc 600

cggggtcgcc aggctgacaa tgccactccc caccctggca attaggtgta 650

<210> 140

<211> 366
<212> DNA
<213> Homo sapiens

<400> 140
ggtccacttc ctactctcga agctagggaa tggggaggga tccgtggccc gggagtcctg 60

cgaagccttc ctcgccatcc tgtgccatcg ccctgacctt tatagcggga gtctgggtgg 120

agggcgcgga gatgctgggg tgggggctcc actcgcatag gtgtgacatc gccctctact 180

gggtccgcag ggagggcaag atcgtggtcg tttagcgcca aggcgggagg ggccctggaa 240

accacacgat caggggaact cttggactgt gcgccccagg tcgaggatct cgagggatgt 300

cttgagccca gaggtccggg ggaagtaagg tactctgtcg gctgtgcctt gtgcagccac 360

cacgca 366

<210> 141
<211> 592
<212> DNA
<213> Homo sapiens

<400> 141
ccgttcacca cggttggcgg ccctggtcac tcccacggtc tttaccagcc gggtgccagt 60

ggctgggacc aggttctgac cactgtgccc actcactgtg gaatcgcgtg agcaggccag 120

accctgcgtc gtaaggacag agcaggacac gtcctgtcat ggggcacccg ctgtctgcga 180

ctttgtttcc gtttgtttcc gcctcagctt gggggagaca gttggcaggc accagaggcg 240

ctcctgtgag gtcacctggc cagtctcagg attagctgca ggctttggct gattttcttg 300

ggtcttgtac acagatgctt gtcagcaaat accagagact tatctcttcc cttccaacat 360

gcaaaccttc cttcatgttc ttgtcttaag cgttttggca caacctccag cctaatacca 420

gcctctaggg agataaacag tgctgccgat ttcacaggcc aggaaaccga ggctgagggc 480

cccccaccca cctcctgcta ccccgcaccc ctcctggggc cttgctgggc ggggtgactc 540

cccggaggcg gcaccaaggg gaggttcgtt tcgaggctca gagcactttt ct 592

<210> 142
<211> 429
<212> DNA
<213> Homo sapiens

<400> 142
gggcttctgc ccagggcgtg tggtgaccca cccttgcccc ctgcatgccc ggtggctgag 60

gccacacccc acctagagca tcctgacagc aggagccccg cttggtgcca ggcagtcctg 120

gctggggtgg ggcaggtgcc atacagttgg ccctgggtag accctcaccc cgaccccccct 180

gtgcccacag tgaagcccag tgtctccatc ggtcaaggta gaggtagaac cctgagattc 240

gaaggcttct ccacacacac tcattcattc acatgtcaaa tgtgtactga gcaccatctc 300

tgttccctgc actgttccag gctctgggca tggcaaggac aggacagtca caacaggcct 360

gtggtgagaa tcttctggcc ggggcggggt ggggagcagc agcgtgaagg aaccagtaaa 420

cacacaggt 429

<210> 143
<211> 1541
<212> DNA
<213> Homo sapiens

<400> 143
gccaggttgt cccggggacg cagggccgcg cagacgtccc cctacgtccc ggggcccctg 60

ccccaacccg cgtcccaagc gcctgctctc gcgcctgctg caaggcagga cttcgcgcgc 120

ttttccccaa cccatgacac tctaggaaat tcacagagcc aaagttagca tagaccgctc 180

gttgggggct tggccagtga gttgggaagt gccctggagg tagcgcggga aacaagggga 240

aggagggaga cagacagcag ctgggcccct ttgggctcag agaagggaca aggggagctc 300

atctggcaag aatgatctga aaaggcctcc aagggtgggc gggattttgc gggggaggg 360

aggaatttcc aactggaggg acaatgacaa agggggtgaa tagagcctgg gcatcttgag 420

aggaattaat gttaattcat ttcctttttt tgcgtggggg gatagggtct ctgtcatcga 480

ggctggagtg cagtggcatg atcatggctc actgcagcct cgacctcctg ggctaaagcg 540

atcctcctgc ctcagcctcc caagtagctg gaactatagg tgcgtgtcac cacgcccagc 600

taatttttga aatttttttgg tagaaattag gtctccctat gttgtccagg ctgttctcca 660

actcctgggc tcaagcaacc ctcccgtctt ggtcccccaa agtgctggga ttacaggcgt 720

gggccactgg gcctacccta atttacattt aaattgtatc ttgtgttgtt ccagagaaga 780

tttcaggtcc ctggaatcaa ggtgatctgc ttctgtcctg tgctgctttt ctagtatttt 840

cagagccatc gtttcatttc tttttttcca gctgttgagt tgtttcaggc aagagggtaa 900

attcagtctg ttactgcatc ttagctggaa gcacaagttc ccaatatggg atgtatgggg 960

gagaggaggt aaaggaatcc cccaggaggg tggtggaggg agaatgacct catgcgaagt 1020

gactccagag acagatcaaa tgctgtcatt gccatcattg catgttttta agcctcagga 1080

cagaatggcc aggtgagcct ggcctagatt ctcacctgtg cctgccttcc ccttgtgttc 1140

cctcgttcct gctcttctct ccatctcctg gtgcctagaa tcagaggtca cctgttttac 1200

cccacataca tgttttgccc tgaccccact cctgagagct acaggtaggt cttggtgggc 1260

ttagaagggg atagtccaaa ttggcctatc ccccccaagc atgtccctgt tggtcatctg 1320

gaatgtggtc ctaactgcaa atgttccagc tctgtgaatc ctgcccccag tgtctctaga 1380

aaggcccgtt ataaacccac aggaattgga agctggactg ggagcagagt cttctgggat 1440

cctgcatctg acagtggtgt ggggttctaa gagccctttt cctatacagc ccggtttccc 1500

agataactaa gcaatatttt attcgtggct gcatacacag g 1541

<210> 144
<211> 466
<212> DNA
<213> Homo sapiens

<400> 144
ccagcccctc ctctcacacc tgcctttggc tgtccctggg ctctgagtcc ggagcccacc 60

ctgcttcacc cctgcggcca ctcgtggaga cgccaggctg tgggtttctc ttccgttcac 120

cgagagtcca ctcccattcc tttccatggt ccagaaactg gttggcgtct ctcactgctt 180

ggcacctgta tccctcccaa gctccttcct ggcatgaacc agttctcact tgttcttctg 240

tggggtctca ggaggggtga gatgggcaca gagcctggta attccactgg ggtttggata 300

agccaggaag ggatacgatg agacaggcat tttagggggg cctttggagg ccgctcagag 360

gatgggctgg ataggggagg cgctcacagt aaggagggca ggtaggaggt caccaccggc 420

cccagggcag tgaggatgag gctgactgcg gcttgagagg gctgtc 466

<210> 145
<211> 821
<212> DNA
<213> Homo sapiens

<400> 145
cacagtgagt ggaagcatgc cggtgcctct gctgctggaa cccggctgcc ggaggatgga 60

aaggacagct ctgccacaga gacacaacag atcccactag atagggtggg agacaaagag 120

actcaagcca gctgcaaccc tgaaccagct tctctgccct atgcagaccc tggaaagtcc 180

tctgaattcc agggcaataa aacaaaacaa acaaaaaact ctttactcct aagtaagagc 240

tcatgtgacc ccagagctct cagaaccctt cagggtgacc acagcacatg aaacgcactg 300

cagaactctt ggaaccctgc ggggtaactc ttggaatcct gcggggtcac cacagcacgt 360

gacgtgcact gcagggctct cagaaccctt tgggatgacc acagcctgtg aagtgcattg 420

cagagctctg agaacctttc agggtggcca cagcacgtga cgggctctgc agagctctgg 480

gaaccctgcg gggtgaccac agcacgtgac gggctctgca gagctctggg aaccctgcgg 540

ggtgaccaca gcacgtgacg ggctctgcag agctctggga accctgcggg gtgaccacag 600

cacgtgacgg gctctgcaga gctctgggaa ccctgcgggg tgaccacagc acgtgacggg 660

ctctgcagag ctctgggaac cctgcggggt gaccacagca tgtgaagcgc actgcagagc 720

tctccaaacc cgtcagggtg acaaaagcac gtgaagtgca ctgcagagct ccggccagac 780

aagggtgtga gagcaggcat cccagggctg caccttagaa c 821

<210> 146
<211> 381

<212> DNA
<213> Homo sapiens

<400> 146
ggtgagtcgg ggtgcgggcc ggtgcctctc cctgcgcccc aggcctgccc ggtcccgtgg 60

gcctgtcctg tcctgggcct gccagccctg cggccccgcg caggtggagg gtgcgagtgt 120

gcatgtgtgc ggttgtgcgg gtgtgcgagc atcacgtgta tctgtcctca acagccgaag 180

cttgctgtgc atgctgctga tgcgctctcc tccctggtac tctttgtgtc tgtgtttcca 240

cgtattttct tctggttcac gtttactagg acgacgagag acagcgtgag cgagcacgtc 300

ctttgagtcc tccttaccct gttttcagag ccgggccagg cccagcagcc cccttagagg 360

tcagaagggc ctcgcctgcg a 381

<210> 147
<211> 553
<212> DNA
<213> Homo sapiens

<400> 147
atctgggctc tggagagaag gctggggtgg ttcagcctcc accgcccacc ggggctgtgt 60

tctggggctt ggggacccac tgaggatagg ctctggctcc tgtctcagga ggggccctga 120

catagggggt tgggggtggc ccctttgccc cagctgtcca aatcacaagc tccacaggca 180

gctgcttcag gggcacgaaa ttacatctct ctcaaaatac ccctttcttt cttctaattg 240

ccatgagtag aataatccca ctctgtcttc tctctcaccc ttgacggtga atttaattta 300

atagactgta actgtgctgc ctctgatctt taaatgccat gtttatttgc ctcgttagaa 360

tggactcgat gattggagaa agtgtggcga tgtcttgtga agtgggtttt gacctgcgcg 420

ggtttcggca ttgctgcctc ggcagtccca gattgaaacc gatgtggatg caaacgcctt 480

cccttcccgt cgggtggagc ggccggcggg gctgaggctc cctccgctga gtgtccgggt 540

gggtcctgaa ctg 553

<210> 148
<211> 403
<212> DNA
<213> Homo sapiens

<400> 148
tgggaaaatc cctagagcca ggatcttcat tcctgctaag ccagacagcc ggaagacaca 60

cccaaattct gtccctctta cttcagggaa catgtccact ttcggcagca ttacaatttt 120

ggcaccaaat gtgctaactg caattccacc atacaatgcg taactggaaa tggaggcaac 180

atctccgatc ctgaacgatc gatgcgagaa tccaggatat gcacggctta ttttggcctt 240

ttcccactga aacaagggcc agtattaaaa atggcacgct atcctctgtt tcactccctg 300

cttttaaacg tctccgatgt ttctccctga gacagggcct cacttccgtc agccgggctt 360

ttctacggta taattttcct tgtttgcttt tgtccaaatt aga 403

<210> 149
<211> 377
<212> DNA
<213> Homo sapiens

<400> 149
ggatgcgcca agcacccttc ggttttcccg gggagaattt tccccggccc ggggactagg 60

gtctggcgct ggggcgcccc tcggacctgc gggatcgccc ctacactctg gcgcgctgag 120

ggcggtgagc gagggcgcca aggcacaggt ggggcgggag tcgagcgcgg aggctcgggg 180

ggcgggacgc ggggcctggg agcggccagg gaccgcggca gcgcctcagt gccagcctgg 240

cgcccgcgac tgcctgcccc agcccctcag tggcggcttg ctctcttctc tcgctccgaa 300

ccagacacag ccgctgccgc tgccgtccgg cgcgctacag actcccgaga acagccctgg 360

ctgtcagcga gcaccag 377

<210> 150
<211> 279
<212> DNA
<213> Homo sapiens

<400> 150
gccagaggcg cttcaggagg caagagaagt ccccgcgcgc tccgcagccc ggcgcagctc 60

atggtgagcg ccctctgggg ctcgagggtc ccttggctga gggggcgcat cctcggggtg 120

cccgatgggg ctgcctgggg gtcgcagggc tgaagttggg accgcgcaca gaccgcccct 180

gcagtccagc ccgaaatgct gccgccaggc agcaacggca ccgcgtaccc ggggcagttc 240

gctctatacc agcagctggc gcaggggaac gccgtgggg 279

<210> 151
<211> 467
<212> DNA
<213> Homo sapiens

<400> 151
cacccagcgg ttggtgaacc agagctgcgg gctctgagag cttcggtccc gggcggcagc 60

ccacagcggg cgcggggcct gggtgcgagg ggcggggcag agaccgcgag agagagttct 120

cgtgaaattc gggagactca acgtggggga aaaacagtat ctggggatcc aggtgcggaa 180

atgcacctca agtgcaggaa cgcgggtcag ggataggccc gacgggcctt gggggtgggg 240

agaagggtga ataatggaag gttggtgcgg gaaagaacct tggtacaaat cccgccattt 300

taagcggagg ttcggagaga aaacaaggtc actaacccag gaaaatgtct gcctctttcc 360

cataaatcgc ctggccctgg ccacagacct cctggagact ctgttctctg ctcacgccgg 420

tcagtcttag attggttggc tggagtactg ctggacagat agagaaa 467

<210> 152
<211> 388
<212> DNA
<213> Homo sapiens

<400> 152
taggaaaacc ataattatta ttctttaatg ctctcagagt aggaaaagcc gggcatgtga 60

cagagcctgt cctgtacctt ggtaaacatg ggcttcccgt gctgggtgac catggtacac 120

tggtcgcagt ccaccgtgat ggatgagttg tggtatgact ctttcgagtg tttgagaatg 180

taatacaggt cggtcacccc tccttcaaac acagtgctaa agtaacgggg gatgagggtc 240

ctgccgatag ctgggagaga aacacagaga aacaaaccat taacaaaaat atcccaccag 300

ccccacacac ccaacaccat tgccaaacct cctaaaaccg gatactgatc atctcaagaa 360

accataccag gaggcaaatt tctcttta 388

<210> 153
<211> 279
<212> DNA
<213> Homo sapiens

<400> 153
accctccgca acgcgcacac gcacacccct cgggcggtcg aacaggagcc gggccttgcc 60

gcagctcagc tccaggcacc caggcgagcg acggaccaga tctgcggctc cgcgcttccc 120

tgttggccta acatcttaaa accagaggcg ggcttcctgg tgccgagacg tcactccgcc 180

gcggccctcc ccagccctct ccgcctccgc ctcctcccag acccttctcc gggtgcgact 240

gacgtggctc cgcaccaatc aggacgcccc gagccgcgg 279

<210> 154
<211> 437
<212> DNA
<213> Homo sapiens

<400> 154
cagcggcagg gcgcgggccg agccgattcc ccgcctcctt gcggcgtccc ggcggcctct 60

taatccctgg cctttcccac tcccactccc gctctcttcc cccttcctct ttgccttccc 120

cgccccgtag agctccaccg cggatcgggc gggttcgggg ctggcctcgc ctcctcaggg 180

aggggttatc ggccccctaag gccgccccac agccacacac gctggaaaca ccagctcgac 240

ccaaacaaac gtgtgcaaaa taaataaacg ggataggaag aggggaaaga ggaggtggcc 300

tggcctcttc ctgccttttc acgatattcc agtgtcaaca ggaaacccat ttgtttgaca 360

cttaaacaaa aagaggagga acccgaccgg ggccgaactg ggagagatgg gacagctggg 420

agctgctgaa ctttgga 437

<210> 155
<211> 341
<212> DNA
<213> Homo sapiens

<400> 155
tgtccgtcac catcaccacc aaggacaaag acggtgagct gtgcaaaacc ggcaacgcct 60

acctcaccgc cgaactgagc acccccgacg ggagcgtggc agacggggag atcctggaca 120

acaagaacgg cacctatgag tttttgtaca ctgtccagaa ggaaggggac tttaccctgt 180

ctctgagact ctatgaccag cacatccgag gcagcccgtt taagctgaaa gtgatccgat 240

ccgctgatgt gtctcccacc acagaaggcg tgaagaggcg cgttaagtcc ccggggagcg 300

gccacgtcaa gcagaaagct gtgaaaagac ccgcaagcat g 341

<210> 156
<211> 650
<212> DNA
<213> Homo sapiens

<400> 156
tagagatggt gcaccagtag ggactgtcac tggattgggg aaaaaaatcc ggggctgtcc 60

ttatctgctc ccttaggcaa aatcgttctg tgttagtctg gagggtgaac ctattttaat 120

gcaggagggc atagactttt gggtggaagc ggaccctgga gatgtactgg caggtgaata 180

gagtgtggaa aagtggggaa aggagaagca cgattcatga gcctgagttc gggcttccat 240

ggatggaaaa gcatcaggag tggaatttaa agacggtgtg tttcaggtga tgtgaggtcc 300

tggatgcccg aagcttcaag aaaatactgg ttgggcactt agctttccct gtggttgcca 360

atcccacgca ggccgcctct agcgtctcga ttcgcttttc tctgacctgg aacctccgcc 420

aagcccccag ctctcttctt cgacccagcc ttgcacgggg cagctgtcgg ggcaggaccc 480

gcctctggcc tgtcgcagcc tagctcacgg tcaagggcgg gagcctgctg ggaagcacgg 540

ccgcgggggc tcgctcgggg ccacctgcct ggaactcagc gcgaggtcca gagttccctc 600

cggctgcgca gcggcgcacg tgccccgtgg aaggggcggt tcccgcaccc 650

<210> 157
<211> 499
<212> DNA
<213> Homo sapiens

<400> 157
aggtgtttaa acaggtgtgc gtaggtcttt ctacttcagc tctcagagcc ggtcttgttt 60

gcaggttagt gggtggcgca cctcgcggtt gctcccaccc caccettagt aacagggttt 120

gagtaggacc ctggggtggg ggagtggatg ctgctgtttc aagctcgttc gcccaaatac 180

atctttctac ccaccttccc tatcgtcaga gaggtggcct gaccccagca cgtgcaagct 240

tgtcaggctg ggtaagttcc agaaccttct ggagccagga ttctctttcc cagccctggg 300

ccctgagctc ggcggatggg atctgcgagg ctcggggcgc aggggaaatg ggggcgtgtt 360

cctttaaggc ggaaccaggc ggggagtggg acgtcccccg cgggcacagc ccgagccgca 420

cctggggccc aattagcctg gagcgcggcc gggtgtgttg aacgtagcaa ccgcgggcgg 480

cgggcggcgg gcggcgggc 499

<210> 158
<211> 567
<212> DNA
<213> Homo sapiens

<400> 158
cggggctgag aactggcgca tggctgccac ctgctgtggt ggtgaccacc gggagtctcc 60

ctcgcaggct gtctggctca aaaggtgaca aagttccagc cacggggccc ctgctcagcc 120

ctgaggacac ccacactggc acaggggaca ctgcggcagt cagggaaggg cacctcccca 180

cgctggcaca gggtctcagc acaggggaca ctgcggcact cagggaaggg cacctcccca 240

cgctggcaca gggtctcagc acaggggaca ctgcggcact cagggaaggg cacctcccca 300

cgctggcaca gggtctcagc gagagactac ggcatttggg gaagggccat tttgtcattg 360

actggaactt tctcaagaaa gcattttcca cttgcgtttc catggaggtc ggctttgctg 420

gaaaccacag gaccctgccc tccaccagac acacagtgtg cagaggcgtg tacccgaaaa 480

gcaaagtgcg gcctcacacg gttctttaat ctggagccgg actcctggga aacaaaccaa 540

acagacgcag gtacgtgctc agctctg 567

<210> 159
<211> 476
<212> DNA
<213> Homo sapiens

<400> 159
gcggagtgcc caacaccagg ctccacaaac ctgggtgacc tgcactcgcc gggggccctg 60

gtcaaagctg gatccagggt gaagaggctc catcccttgc ccgtcagtgt gaggactccg 120

ccagcggcct cggccattgg agctggatgg gcacaggagc cctgctgccc cagcacacac 180

atttcatcat gaccccaggt gaccagcacg cagggaggct ggcagcccca ctgtctctgt 240

tcctttctcc ctctcggcag caactccacc tgagctgtcc cacaggatcc ccaggggtgg 300

gctgagagtc actctgagaa ctgtccctgt attcatttcc ctgtactgct attccatttg 360

ctgctgtgac gaatcactgc agcttagtgg ctgaacacaa acgcatcatc ttacagctct 420

ggaggccgga aagctgaaac aggtcccact ggcctgaaat cgaggcgtgg gcaggg 476

<210> 160
<211> 286
<212> DNA

<213> Homo sapiens

<400> 160
cactgggagc ccggcctggc cccgacagcg cagctgctcc gggcggaccc gggggtctgg 60

gccgcgcttc cccgcccgcg cgccgctcgc gctcccaggg tgcagggacg ccagcgaggg 120

ccccagcgga gagaggtcga atcggcctag gctgtggggt aacccgaggg aggggccatg 180

atgtggaggc cctgggaaca ggtgcgtgcg gcgacccttt ggccgctggc ctgatccgga 240

gacccagggc tgcctccagg tccggacgcg gggcgtcggg ctccgg 286

<210> 161
<211> 601
<212> DNA
<213> Homo sapiens

<400> 161
tggtccccag aaatcctgaa tccaggaaac ccctcagtcc ggggcagacc gggatggccg 60

accaccctga ctaggggagg gccgtcagaa gtctttgacc ctgatctaag ttctctctgc 120

tttgctgagt ctctttctgt ctctctcctc ctctccctgt ctgaatgtct gcctgcctct 180

ctcccacaca catctgagac acaggtctca cagaaccgcc cttgcccttc tgtctgtctg 240

tctgcctgcc tctctctcac acacacacct gagacacagg tctcacagaa ccgcccttgg 300

ccctctctgc caggagggtg gttctcggag gttcccatcc tctgtgggga tgctgacagc 360

tgtgggtcat ggctggtgcc gcggttctca ctcttggctg cagagagcag ctgggggctg 420

caggtcaggc cacacctagc tccccatggc cctggttcac ttaagtgaca ggctggggtg 480

aggggaggag gaggccaggg tgtgaccgtc ggttccattt ctgctttccc ccagctcccc 540

aagtcaggca ccggcacagt ggcagttggt ttgtgggaca cccatctgtc agtagggcat 600

g 601

<210> 162
<211> 489
<212> DNA
<213> Homo sapiens

<400> 162
gcacctcagc ctcctgagca gctgagacta caggtgtgca ccacagcacc ggctaagtta 60

tttttgtgtg tgttttttttg ttgttgttgt tgtttttttg tagagacagg gtctgactat 120

tgttgccaag gctggttttg aactcctggt ctcaagcgat cctcctgcct cagcctccca 180

aagtgctgag atgacaggca tgagccacca agcctgggct tgtattgttt atactcagta 240

ataaagagca aaatttatat aagcaaaatc agccactgta aagagagttt agacataacc 300

atcacaactg cttcctcaaa aagtgtctgt acaagccaca tccacctagg gcaggtggca 360

ccaccccgcg tgagagagct gggcggagga tggacagggc ttcatcgcgg gaactagctc 420

ctgggccagc agggtaaccc gggaaatcct gtccaaacgg ctacataaac ccccctgcaa 480

cgctgagca 489

<210> 163
<211> 857
<212> DNA
<213> Homo sapiens

<400> 163
aagactgcct gtttctgcac cagtctgctc tggtgttgac agagcttgcc ggctagcaca 60

gccccatgct gggtcacagc gagggttcca gtgagtgaag aaggtgcctg ggggataatc 120

atgtccctgg ttctagactt actttatatt cctgtctgtc ctttcctgca aaactgaagt 180

ctttggaatc agcccttgcc cacacctctg ggatcgtgag tgggctgggg tgtgcgctgc 240

agctgggaga atccacttag catcctgtgg gctgtgaatg ggatgagctc agctctacaa 300

gggtggagac tttacagcta tgaaccaagg tcattttttt tttaaggaaa attccatatt 360

agtgaattgg ctttttgcta aactccatac gtatagaaag atatcagtat ctgttgcaaa 420

tggcagatac aattttatgc acctaataat ttctatagat actgagatga actatgcaaa 480

tctagattta agtgtataat taaactgtgg cacttggttt ctcctcttgc agttataact 540

ctcaaaattg caacataatt gatcattgaa gattttgcct tttcaggata aaagccaaag 600

tgattgctcc aaatggacat ccatgccagt catctgtgta aggatcgctg agtgtctcac 660

aaaagcacgt gggtatgaag gtggggttga cattgatgat taaaccaagc agtacagaag 720

catcggtgct ggtcacttat ccccgtcctt cccatcattc gagatcccct tccagcgaat 780

ggtgagtgag aaggagtcaa tactccccgg acctggagtg tgagattgct ggtgggtggg 840

tcgagtggga ggtctca 857

<210> 164
<211> 474
<212> DNA
<213> Homo sapiens

<400> 164
gcgggcgtca ctccttacct ctgagcttgc ttccttccca cgtcagcccc ggcaggtgta 60

gccgccacac ccagcccctg gctccagtgc caggcattgg ccagggtggg tcagggcaca 120

cggcgttcat ccaaccgcag aagacgctcc ttggcaaccg caccgtaggg taatgatgca 180

ggaggaacgt tgtccaaaac gtcagatttt gaacaaaatg accgtgaata atgtattccc 240

aggcactgct tcctgggcag cactgccaag gcgttctgtg gcggctgcat ctgtcatcgg 300

ggaacacaaa acacacaggg aagtcacccc cacgcacggc ccacacgtgt ttgcaaacag 360

aagctgcgct gttttgaggt tcaaacgcga gaggcggcct ccgacgtgac tcgcctgcca 420

gctccgggag gaaagggtgt gtggtattaa aaattcatag agaacactgc cttt 474

<210> 165
<211> 459
<212> DNA
<213> Homo sapiens

<400> 165
taacgagtgg atcattgctg cagaggaacg ggagaaccct ccagtccccc ggcggctgca 60

tgccacggtc ggctgtgcag taaccaggca gagcacaatg acatcccagc ctcgctcccc 120

agataaaaat ttctctccct ccacttttgc aaaggtaatg aggaagtaat gaggcttgac 180

ttgaattttt tcagccctgg ctttccctcc ttccccagca ggtcccccag gatggcataa 240

gacgctgggt ttctataaaa gctgtgctgt ggctgggtgg aggagcggag tcgggctctg 300

tggcctcaga ggccgcttgc aagagttcag ataaaccgag gcccctttca gcgcgtccgt 360

ctgctctgat cggctccact gaataagacg atttgaggat gtgcaggccc ggccgcgcag 420

ctccctgttt attttaccac ttgatggcta gaaagccct 459

<210> 166
<211> 325
<212> DNA
<213> Homo sapiens

<400> 166
aaaatcagat ccggccaccg tggctaggat gctcgtggcc gggatgagcc ggagctgcag 60

tgaaaggaag cccgttgagt gtcttcagct gatgttatcg gccgctggag aaatgagata 120

ttggcagggc ctgctgagtc cctcgcagct gagcaggtta ataaggtggg gagggggcct 180

gcaggctaat tggatttggc ctggcttgat caacagtgtc aggcctgctg ccagggaagc 240

atacacagtg gctttctctt tgtttaaatt taagccgggc gaggaaaagt cgcactgctc 300

ttgtgaagct gagggtgtgc tcccg 325

<210> 167
<211> 536
<212> DNA
<213> Homo sapiens

<400> 167
tctaagcttc ccttcacaga aaggctcttg tcagatggaa ggatggagcc ggtggggaaa 60

tggcaaactt ttgcttggta gacattgcaa aagaaagact tccatccatc tatctgatca 120

gagaggaaat tagaagggtt ctgggctcaa ataaaatttc cgagaccaac tgctaatgag 180

aggtgaggat ggctggctgt gggattcagc ctcctaagat ttgaggagga acttcgcact 240

tgggaagcca atttaaaaaa aaaatactcc tcatttatgc cagcctcagg aagctaagtc 300

ttcctctgct gccttgaggg gctggaatcg acctgcatgt ggctcccgtt tctgtggcca 360

gaccctggtg gtgaacacat ttcaggcagc cagaaacagg cttgttgtgg ctctgcagcc 420

agacctgcca gccttctcgt caggacgtgc gagcttaatt attccactgc cctctggaaa 480

tgatgccggc ttgtccttga aatccctgac cgggcgccag gccctgctgg atttgt 536

<210> 168
<211> 482
<212> DNA
<213> Homo sapiens

<400> 168
aagaaataaa gaagagtcac taaccttgcc tcctgtccgg gaagcgaacc gggcctggct 60

gaggctgtct cccatagcaa agcgccccaa agttaaggag ctgagccacc acggaaaggt 120

ttgcaagcca gctcctctgg gcacatggca tgattcagat gttgaggaga agaaaagtca 180

aaaaagtttt agccctcagc tccagaaata aagcgctcgc ctgtggccca gcaggaagat 240

gcaggggtgc atggtcaggc gcgagaaggc agcgactgca aagctgcccg cgatctccct 300

gtgctttttt aagcagaaag gagctagaca ccttttttccc ctccctcctc ctaaaagggg 360

agtgattcag ctgacagtgt ccgctttcgg cggggtgtgg aatgggccag agcgggaggg 420

cgcagcccac cccggtcccc acccctccgc ctcccgcatg ccccgcggcc tcgcagcccg 480

cc 482

<210> 169
<211> 723
<212> DNA
<213> Homo sapiens

<400> 169
tggccgtgca agtgccaccg taactggtga gagccgctgg caacccaccc ggagttgaca 60

accgcggaga gacgcagaca cccactgacc tccaggaagc tgagcgtggt ggatggaact 120

ctacgatctc tttctctcca aggacggaaa cctcatccaa gcagtcccag aggaaacgga 180

taaaggtatt tgaaagggag cgagcggccc caaatcgcac aattgagcgg ctgggggagt 240

tatgcgccag tgccccagtg accgcgggac acggagaggg gaagtctgcg ttgtacataa 300

ggacctaggg actccgagct tggcctgaga acccttggac gccgagtgct tgccttacgg 360

gctgcactcc tcaactctgc tccaaagcag ccgctgagct caactcctgc gtccagggcg 420

ttcgctgcgc gccaggacgc gcttagtacc cagttcctgg gctctctctt cagtagctgc 480

tttgaaagct cccacgcacg tcccgcaggc tagcctggca acaaaactgg ggtaaaccgt 540

gttatcttag gtcttgtccc ccagaacatg acctagaggt acctgcgcat gcagatggcc 600

gatgcagcca cgatagccac catgaataag gcagcaggcg gggacaagct agcagaactc 660

ttcagtctgg tcccggacct tctggaggcg gccaacacga gtggtaacgc gtcgctgcag 720

ctt 723

<210> 170
<211> 513
<212> DNA
<213> Homo sapiens

<400> 170
tccctttag gagaaagcaa cttctccagc agcctcctca ttccgatccc ggaggctttt 60

ggccgacgct gactttccct gcgagaggtc gctaggagta cggaggcatg gagctggagc 120

ttagaatata aagggaaaag cagacagtga ttttgttgtt gttgttactg tagtaaatca 180

gaggcaagtt atctgatccc atatttcatt gtgccaagga atcacacatg gaacttgttc 240

aatatagttt ccaagggcct ggcgccgtgg cttacgcctg taatcccagc actttgggag 300

gctgaggcgg gcggatctct tgagcccagg cgttggagac cagcctgggc aacataggga 360

gacactgtcg ctacaaaaaa aatacaaaaa attatccagg cgtggtggcg cgcgcctgcc 420

aacccagcta ctcagggggt ggaggtggag ggaggcttga acccggaagg tggaggttgc 480

agtgagccga gattgcgcca ttgcactcca gct 513

<210> 171
<211> 811
<212> DNA
<213> Homo sapiens

<400> 171
gcttcgggca ggggcgggcg cctcctcctg gccgcccgcc aagcatgccc ggatcccctt 60

agccgcgtcc caacacatgg gcttgcttaa cactctctga gagaaggagg agccaccccg 120

cagtgctgtc tggtcgcttt ttcccatttt tgctccagga tccagagatt tgagcagggg 180

tggggtgagg tggggtggca gggttcctcc ccttccttag gcccagccag agaggggccc 240

tgtctctctg ctcctttcct cgccgtcgcc ctctctctgc caggctctcc tgttcgtcct 300

ccaggggccc acgctagccc ctcacctccc ctgaagcctt ttctaaccgc tgcggcctcc 360

gtggtccccc acgcctgtgt tcctgtagga cgtttacagt tcacacgatg tctcatctct 420

cggtatatgt ctcccaggta aattgtgaag cataaagagc catatgcttt ttttgcttta 480

agcaattttt ttttctgtag tttctgttgt aagcaataat cctgcccagc cactaattgt 540

gtgacctcag gaaaatcctt tctgagtttt ctcatatgca aaatgaaatt tgtaaggtct 600

cttccagctt ttaaattcag ggactttcct gaactagaga actggaagtt ggatagagct 660

attctagaac agtggtttgg ggtctaaata cattgtttct gggggccacg ggtaggtggc 720

atctgtgagc tgccctcagg aagaggggcc aacagtgacc ccgggtttct tctctcctgg 780

acactcacca cctgcctcag ttcttctaaa a 811

<210> 172
<211> 511

<212> DNA
<213> Homo sapiens

<400> 172
acgtggcccg gcgcggcgcg gcccccgggg accgcgagca acaaaagccc ggcccgcagc 60

ccctgctcct cgccttcctt ttcaattctc ccctgatctc aggtggaatt aggaggatta 120

aagcagcaag taaatgaggc gagtgtgcaa aatgactcct ttctgaacca aacggcatgc 180

tccgtgctgg ggaaatgaga tgcacattta aatctcatcc atccaccgcc tgcggccacc 240

gccatgtacc tgcctactta gcccaagggt taattaattg aggcttcaat gcactattgc 300

aagagcatta ttgcatttga tactctacat ctgtgattta aaactctttc agtcccttgt 360

cagcaaggga gggtttttaa ctaggaaatt agcattcaga taagggaacg ctctatttgc 420

ttctgaaagg aaggaaatat taaggatgct ggtgcacaaa ccgggctgtc atatcctgtc 480

ccatcctgga gggagcgcga gggcccggct c 511

<210> 173
<211> 438
<212> DNA
<213> Homo sapiens

<400> 173
aagttctgct tgtgggtcta agctttggag ggctgcagca gaggcggccc gggagccctc 60

tgcgcgggtg cccttaggcg cgcacccctt gctcacccgc acgcgcggct cggtgaggtt 120

ggtccacacg gcgatctcct ccctcatgct catgtcgggg tagcggttcc tctggaacgt 180

ggcctctagc tcttgcaact gctggcttgt gaagtgcgta cgttgccgcc gctgcttctt 240

cttcttggct gggtcgtctg cgccgccgca gcccgtgcct cccgcaccac tgtcctcggg 300

ccccttgggt tccccgccgc gctccttctc tgcagtaggc aggacgggga gcgggtcacc 360

tgggcttacg ccccgttgca ccccgtcccg gctccaccga accgctcgcc accagcgctg 420

gcggtcttcc ctccctcc 438

<210> 174
<211> 361
<212> DNA
<213> Homo sapiens

<400> 174
gctccactac tccgtacccg aggaggccaa acacggcacc ttcgtgggcc ggatcgcgca 60

ggacctgggg ctggagctgg cggagctggt gccgcgcctg ttcaggatgg cctccaaaga 120

ccgcgaggac cttctggagg taaatctgca gaatggcatt ttgtttgtga attctcggat 180

cgaccgcgag gagctgtgcg ggcggagcgc ggagtgcagc atccacctgg aggtgatcgt 240

ggacaggccg ctgcaggttt tccatgtgga cgtggaggtg agggacatta acgacaaccc 300

gcccttgttc ccggtagagg aacaaagagt gctgatttac gaatctaggc tgccagattc 360

t 361

<210> 175
<211> 575
<212> DNA
<213> Homo sapiens

<400> 175
cgcgcaggac ctggggctgg agctggcgga gctggtgcag cgcctgttcc gggtggcgtc 60

caaaagacac ggggaccttc tggaggtaaa tctgcagaat ggcattttgt ttgtgaattc 120

tcggattgac cgcgaggagc tgtgcgggcg gagcgtggag tgcagcatcc acctggaggt 180

gatcgtggac aggccgctgc aggttttcca tgtggacgtg gaagtgaagg acattaacga 240

caacccgccc aggttctccg taacagaaca aaagctctca atacctgaat ccagactgct 300

tgactctcga tttccactag aaggcgcatc tgatgcggat gttggagaga acgcattgct 360

tacttacaaa ctcagtccaa atgagtattt tgttcttgat attataaaca aaaaagacaa 420

agacaaattc ccagtgcttg ttctgcggaa gctgctggat cgtgaagaaa atcctcagct 480

aaagttgttg ttgacagcaa ctgatggagg caaacctgaa tttaccggat ctgtttctct 540

gctgatcctg gtgttagatg ccaatgataa cgccc 575

<210> 176
<211> 313
<212> DNA
<213> Homo sapiens

<400> 176
ctgtaaagag tgtcagccgg ggagcggggc cgccgcgcct gcccggcccc ggcgcacaaa 60

agcttcatat attctgaatg aaggcgaatc gcagcctcgg cctccttgaa aagagccgtg 120

agggagactt aattctctcc ctttcagctg tgctcagact gtgaaatcca cacaatcgcg 180

cccttctctt taaagggagg gagaagaggg gccgcggccg cgcgccccgc catcagctgc 240

gcgcacagag ggagagttca ctccggggac aagggcggcg cgaccggccc ggggctcttg 300

ggcggccgcg ttt 313

<210> 177
<211> 354
<212> DNA
<213> Homo sapiens

<400> 177
gggagtagag ctgagggctg tgaggggaca gtggggcgtc agggctggcc gggttcgtgg 60

cctcgtggcc acgagggagc ttggtagaag gggttcttct gtatttgtga tgggaggggc 120

tggaaagacg gggacgcaac agccctagag gctgcacaac ccctcggagg cctgtggcac 180

gcagagggga ccccttctag gggcctgtga tcaagaaggg agcctgagaa ggtcggaaac 240

tcgggcgtac aaaatggggg cagatcactc ggccgtgggt cccgcccgcg agcagcaccg 300

cggccggcgc acactctgca gcagcgcgcg gaacaggccg ggcggacgct tggc 354

<210> 178
<211> 319
<212> DNA
<213> Homo sapiens

<400> 178
gcagggaaca ctgaagcctg gggagggagc agtgttaaaa attggggacc ggaacttgtc 60

tcatgtcttt ggcactcagc cccaggcacc cccattagac cactcagcct cacccagccc 120

ttccaccctg aggttgtccc aggcctcaca gcaaaatcca gcccccagc tgtccttcac 180

tgctgcaggg gggcaggcac ccactcacca ttgaagcgga cagctcgaat gtactgcaga 240

agcatccgcc catcagtggg ttccatcgcc gggcacaggc ctgtgtgccc agggcagagc 300

gcctggtgca tgctgtgga 319

<210> 179
<211> 311
<212> DNA
<213> Homo sapiens

<400> 179
gcgctccgcc agcctcggcc ccaccccaaa ccccttcctc cttcaccgcc ggctcttaca 60

gggaaatcgg cgcccgagac acagccagcg ggtgttcaga aaggaaaacg ctcgctcaac 120

agccttttcg gttgattctt acattcaagg tgtgttacta tttgtggcaa gcatcttttc 180

atttaaatct cgtgacaacc ccgtgaggac catcgtcccc attttcctct tgaggatgag 240

ggctcagaga caggaggaaa ccggtgctct gatcaaagcc atacagctgg tgacaggtgg 300

gcttggctcc c 311

<210> 180
<211> 534
<212> DNA
<213> Homo sapiens

<400> 180
ctgaagaagt ccaggatttt tctctgctgg ttttagtctt cttaaatacc gggagcctta 60

aaataccttc actgggggca acagagaaaa acagaaatag cagcattgtg tcggccacag 120

taggccatcc cattcacaca gcagcctgca aacaatctct cgtgccacat ttagggaaaa 180

acacaatttt aggaaatcca aacaacccgt aaacccactg acctacagat aacctattcg 240

gcaacaatga aacttggaaa aagtaaaagg gaaaagaaac tcgcaaagga aaaagaaggc 300

tttcctatct caggaactaa gtcaaaagcc caggtatttc aaatccctgc cttcatgggt 360

ctgctctggt tctgaagtcc tgttgggtaa tttacctgtg ctatgtaagt cactatggaa 420

gacagctcag caccgtggct ctgaagtgca gggaatgcca cacacacagc taacgcagag 480

caaccggtct ccaggacaca gacgaacctc gagttaaatt tcacctcaaa agca 534

<210> 181
<211> 802
<212> DNA
<213> Homo sapiens

<400> 181
gcgtgtccca ctggccctcc cacatggctt tgcttgtgga gacattgccc ggcttccccc 60

aggggcccct ggaactctgt ctcggtaagc tcatacaatc acctttccag ataattcata 120

aacaaatacg gaggtgagaa gatcaacagc acaagagtgt tggctgtcat tcaacagaga 180

gggaattaga attgctccac tgggtcttgt ccaaaaaacc aactctaagc acctctcctc 240

ccctctgagc caccccagtg cccgaaggcc acatgtacgg actccatttg ttctagttct 300

tttcataaga gagaagaacc gagtgtttca catgcctgta acacgggcag gaaatgctag 360

gatccatgtg aaacccacca ccctccccca gaagctcctg gctttagcat ggaatactct 420

gcagaacgta gctgccgtga agatgatggc cctgaactgc cacactcccc agcgaggtga 480

caggctaggg actgaggctc gggcaatccc gccaagtatc cccccactct ccccacattc 540

tgacacccta ctgggggctt tcatgcaggt gaaatctggt taaaagaaac agttgggaag 600

agactaaaac actcaccaga aagcccctgc agccaggagg aaagtgtctg tagaaggagg 660

tggtgcattt ccttggccac cgtgggttcc ccgactagct caaggagaaa gggtctttca 720

tggatgcagc agcaaaggta actctgccga cccggcaggg gaccatggct actgctcagc 780

gcccacaggg cagggcctct cg 802

<210> 182
<211> 423
<212> DNA
<213> Homo sapiens

<400> 182
agaggaaatt ctactgccta agaaaatagg catccagata ttcaaagacc gggcctcaga 60

ggtgaacttc acttatatga ggcatttctc tgtgctacca tgaattcttg atgtgcgccc 120

ttcatccacc tgcttcctcg tggcaccttc caaaccatcc agacatgact ctaagtcatc 180

aggttaatga acaggacagg cagagagctg agtggcagta aataaacagg gcctgacccc 240

tatcagaatt taaatgtttt gggatcctga ccccaactat cagcatttgc caacattgct 300

agttctcctc ttatacctct gccttccaaa gtaccacacc aatgagggtg caggccaggt 360

gttagttctg atccggcacc gtgacctgac ttctcacacg gaaagcagcg tggaccttct 420

ggc 423

<210> 183
<211> 131
<212> DNA
<213> Homo sapiens

<400> 183
gctgccgccg aggagggccc gcggctcgag agagagcgct cggcggctcc gggtgcgtca 60

ggccgccccc atgtcacccg ccggaccccg cgccccgggc gctgcggccc cgctcgggcg 120

ccgggcagag g 131

<210> 184
<211> 395
<212> DNA
<213> Homo sapiens

<400> 184
cgcggcgtcc cctgccggcc gggcggcgat ttgcaggtcc agccggcgcc ggtttcgcgc 60

ggcggctcaa cgtccacgga gccccaggaa tacccacccg ctgcccagat cggcagccgc 120

tgctgcgggg agaagcagta tcgtgcaggg cgggcacgct ggtcttgctt acagttgggc 180

ttcggtgggt ttgaagcaca cattaggggg aaatggctct gttcctgcag gtttgcgcag 240

tctgggtttc ttaggtttag ggggttgggt gggtttctct gggggtgcgg tgggaagcgg 300

atcagttcgg ataacggccc tgagcaagag tctctgtccc gctcccggcc tgacgcgggg 360

cttccagcgg acacgaccaa cggtgagcgc accct 395

<210> 185
<211> 348
<212> DNA
<213> Homo sapiens

<400> 185
ctcggcgccc gcaagctgcc acctcccggg gcggccaggg aaactcagcc ggctctggga 60

ggcgggggcc gcggtcgagg ggcgccgcta ttggctgatc gggcggggga ggaggggcag 120

ggaggccgtg attggcgggg acccgagctg aagggggcgg ggccgctata tcagcagagt 180

ccctgtcgca gcgcaggcag ttgggctgct ggagtgcggc gccaccgcgg aggacagggg 240

cagctggcgg gcagcgggtg agggggtggc ggggacgcga gtggcggccg cggggccccg 300

gacaagggtc cgcagagctg cagccttcga gggccagccc tctccgag 348

<210> 186
<211> 697
<212> DNA
<213> Homo sapiens

<400> 186
agttttctgg agtgaatgaa acagctctgc atcctgtggt agtagttacc ggaatctaaa 60

catgttctag aacagtatgc cccaccatgc caattttatg tatgataatt ttaaaattaa 120

ctaaaccagg ctgtttccat tgtactatga tagataaaag gctggagtgg agaaaggggt 180

acgtacattt gaagtgaccc agagaggggt tttggtcgtc ttcatacagc ccctactaca 240

caagcgtgaa atttcgcttt cttcccactt tctcacacga aaactcgtgt gtgtgtgtgt 300

gtgtgtgtgt gtgtgtgtgt gtgttttcct cagccttcag gtgaggaaag ggaaagctct 360

cctttctcaa atcagttccc agaacacact cgctcttgag ctggtctatt tcttatctct 420

cttcaggcaa agtcgtatcc tccaccttac ctgctccagc cgctgtgaca tcacgcaggt 480

tccgttccct gcactaccgc tggttatgtt tccttagtga ccctaggcct cttgcaggag 540

agtccctggt cccagccagc gggagcggct tctccagttt gtggctctcg gagagttcgc 600

ctcgtccctc cctctcagat aaccacctat ttctgacctc tggaggccgg gctgagcccg 660

gaattttatg accagcgccc gctggccgtc gtcaccc 697

<210> 187
<211> 565
<212> DNA
<213> Homo sapiens

<400> 187
ccaaactcta cttccctgtg ccaagaccta tgcctccccc cagcctcacc ggtgaagaga 60

acgtcagtgc catccagcgt cgcgttctcg tctcctattt ccacaattcg gagccccagg 120

tcttgcaggg ctttgcggac tccatcgacc ttaggatagg agaagagggc acggagctgt 180

gacaccccca tcctcaattc ttccccaaag ccccgacatc cagttccttc tgcctttccc 240

cataccacac ccgcgccacg gcgctcacct ctggcctacg agcggggctc cagggccgcg 300

tgattagggc cgtgtcccct tggatcacgg ccgtgtcgcc aagcagcggt cccagcggca 360

atgactcctc aggtggcagt tctagcagct gtagccccag tcgttgcctc agtttacctc 420

ccagcacccc gtgctccctt tgagctttgg ccagatccag agcgggaagg ccagcccccg 480

caccttcccc cgacgccagg ctctctggga ctccccggat cagggcatgg gagcagcggc 540

ccagcccctc ccccggcgtc cccat 565

<210> 188
<211> 483
<212> DNA
<213> Homo sapiens

<400> 188
gaaccagaga aacgccattc gggcttcgtg gggcgggctg cgcgaggccc gggggctcag 60

ggtacagacg ctattcttgc tgggagagcc gaacgcacag caccccgtgt ggggttccca 120

ggggagtgac ctggcctcgg agtcagcagc ccagggggat atcttgcagg ccgccttcca 180

ggactcctac cgcaacctca ccctaaagac cctcagcggg ctgaactggg ctgagaaaca 240

ctgccccatg gcccgatacg tcctcaagac ggacgatgat gtgtatgtca acgtccctga 300

actggtatca gagctggtct tgcgaggggg ccgttggggg caatgggaga gaagcacgga 360

accccagaga gaggctgagc aggaaggagg ccaggttttg cacagcgagg aagtgcctct 420

tctgtacttg ggccgggtgc actggcgcgt gaacccctct cggacaccgg ggggcaggca 480

ccg 483

<210> 189
<211> 352
<212> DNA
<213> Homo sapiens

<400> 189
tttgtgatct tggctggggt accaccggcc tctaggtgag gggcgatgcc ggtggtgggc 60

gtagcggggc gtgggcaggg aaccacacgg gagcctcagc ttggccttga gatggcagga 120

gtgggatggg gcgtcatcct agtagaggct ggacagtgag agatcggtgg gctggtaaag 180

ggtgggaaca ggtcttttct ggggcccgtg ggtggaggga ccttgtgggg aggcgttggc 240

gagagggggg tgaagcgaag cggccactta cccctgtaga gcgcggctct ggtttcagcg 300

cccggtgcgc actcagcagg gcacctgtgg tccggctagt ttgataaaca ca 352

<210> 190
<211> 878
<212> DNA
<213> Homo sapiens

<400> 190
ccgctgcgcg ccaggcacgc ggcttactcg cgcgcagggt ggccaggccc ggcctcggga 60

cggttccgtg gaagcggccg ctttgcgggt agtgggactg tggccttgcg ctatgccctg 120

tgcttccgtg ggacgtgtct aggaacgggt gggctttctg cgggaggcgg gggtcgggca 180

gggctgctgg tctggagtgc ttgtgctctc ttggtagctt ttgtgtggca atggataggg 240

agctggtcca ggaaaagcgt ctgtggaaga agaggagggt ggacgcacct tctccaaggc 300

acctgtagaa gtcactggaa ctcccgtgag gcctcctgtt gaccttaggc cctgcgggag 360

ggagggcacc ttccaccttt cccccagctg aaggtctggt accagacgca ggaaggttcc 420

aggccaacac agcgctcctt cacacagtcc atgccctgtg ccaaactctc ttccagccct 480

ggcgcgacaa gatgtggttg ggccccaga tctcctggag ggcgagaggg acagaccaga 540

gggacggcta gtaaaaaaaa agaaattggc cttcaggaag ggagaggcag ccagggtggc 600

ggggctttca aagggtagct ttgacttcag ctctgccgag gactactagt tggttaccct 660

ggatgaacat ctcgaagctc tgttaactca tcaataaatc aggggtgcca aaactcatct 720

tggcgctcgc ggaggagctt gaacctgggc ctgtggtgag cactgctaaa caggctactg 780

ccccagtggc caggatgctg ctttgcaagc agtgaggggc caggcacccg ggattgggtc 840

ccttcaagcc ctcagcagtg ccttaccagc aaatcatc 878

<210> 191
<211> 514
<212> DNA
<213> Homo sapiens

<400> 191
cactcgaggg tcaaaatggc attccccgtg gcccgagcct tgtctaaccc ggccgtgccg 60

ccacccgcac tctcgcagag agcagggtcg cctggggtgg gtgaaggggt ctcagtcttg 120

cactcacgtg aggacatagt tgacgctgac gatacagtgt ggcctggagg agcgactgtt 180

gtgcacgatg gtcgcgtagc tctgcaccca tacccagccg ccgtgtttcg ccaggaacct 240

gtagtacttg gtggtcacct gtcccttcac cagcactgac ggagagacag gaggcagatg 300

gtggtgaacc agcctgcctg ggactggccc caaatgcaat ccctggtggt aagaattgag 360

ggctccctgt gggccctaat aactgcacca ggcaggagag agagagagac gacaggcaaa 420

ttcccaaagg aactgattgt cccgtggtat tgcatcgagc cctccggtca cccttctagg 480

gatgatacag ggtggccag atgtcttcac catt 514

<210> 192
<211> 321
<212> DNA
<213> Homo sapiens

<400> 192
cagcgccgcg cagcagccgc cccgctgcgc ccacctcccc ggctgctccc ggagggctca 60

caaaggcggt ggccgcccga gtggccttct ccatccaggc gttcgcgtcc tcctccccac 120

cttctctccc gaaggcgaaa atggcagggc caggcgagaa cctgggacag cggtggccct 180

agccctgcga tcctcacccc tcctgctagg agaggctgcg ggctgcccgc ggacgatgtg 240

gccgcggctg ctcccgagcg catcttcggc ccgggtcccc gccgccaccc ctcttctctg 300

ctccttccat cccgcccaga g 321

<210> 193
<211> 487
<212> DNA
<213> Homo sapiens

<400> 193
ggacgtgagc cagtgctggc cgggaagccg cgagcacggc gactgggccc ggagcgggca 60

gttacaagtt accactattc gcagggccag gtacctggcg ctcgctgttg ctgtctgcgg 120

tggctgccct gccatgacct cttgctacca aaggctctgc cattttaaag ttgcctactg 180

gatctggcac agtcccgaaa taccagctgt caaaccagga atagcatgtg aaacgccttt 240

tccttggtct tgggcagggg tttgttttgc aggcaaaacc acccccactt gccctaaagt 300

gagctctagt ctcaactcct tggtttgctt tgttgcaaca gccccaaacc gagagaaggg 360

aaacacattc tctctcctgg agtctggctg ggaaagtttg ctcagtgccc acacttataa 420

aaagcattta ctggggccgg gcgcagtggc tcacgcctgt aatcccagca ctttgggagg 480

ccaaggc 487

<210> 194
<211> 318
<212> DNA
<213> Homo sapiens

<400> 194
gaaatgggag atctgcgggg accaggccgg gactccctgg ctccgaggcc gggacgtggc 60

gcgccctccg ctcctcggtc accctctgct ttttcgaagg tccgtaatgc gtgagtgtgg 120

gtggtccaag gttcagggcc tccttccagt aagtactttt agaagctagt tcccccactt 180

cccatccgtt gcggggcttg acaactagac gaggggagtg agtaaaggtg ccacaacttt 240

gcttaccgta aaatgggtta atactcgccg gggcccaaaa ggctgagtcg ccccgcacgt 300

cttgcttcgt ggagtctc 318

<210> 195
<211> 410
<212> DNA
<213> Homo sapiens

<400> 195
ggcagaacca gaaccggctc cccggggctg cccctgcttc ccctgacacc ggctgcgtct 60

gtcatgggct gcggcacggt catcttcgtt ttgactttca ggtggcaacc gcacggtgcc 120

acaggctgtc gctgtcaccc aagcacaagt gcagtgacaa tcctcgctac gaatttcctc 180

gcggctccat acctgaaata agtatcactg tattatcaca gctctgtgga aatccccacc 240

tttcaaggct cctcttgaaa cacaggttat tgtaagcgga gaaacaactg cttttcaaga 300

tgttaatatc tgttgggata aaggtatttc tcactgagga ttcaccctca ccaagcttcc 360

cggcatgtaa aaaacatcac tgtcactgca ggcagatgta aggaataaga 410

<210> 196
<211> 488
<212> DNA
<213> Homo sapiens

<400> 196
cctgtgaaga aacggctgca gtgagagcag ccgaggcggg acgcagcccc ggacgtgggg 60

tcagagagga gccgtgcgtg gggtcagaga ggagccgtgc gtggggtcag agaggagccg 120

tgcgtggggt cagagaggag ccgtgcgtgg ggtcagagag gagctgagtt caggacgggc 180

atggcagccg caggaggagg gcaaggcgga gcccaggagg ggcaaagtgg acaaagccag 240

ggctggagag gggagaccca ggatcagcac cacgttccac ggggatggca cagaagcctg 300

agaggccacg gccaaatgtg gaaattggct ttgccaaatc ttgcatttca gagagaaaag 360

gcaatatggc attaatgctt gccttgttta attttcgtaa ttttcccttt cttcctgcca 420

atagcttgct ttgttatccg gagggtagcc acggttttag ctcacagtga tgtcaacttg 480

aagcttct 488

<210> 197
<211> 449
<212> DNA
<213> Homo sapiens

<400> 197
tatccttgca gcgagcagga gctggcgctg ggagggcaga cacgtggtcc gggcggaagt 60

gtccgtgcag ccagcgggag ctcgcgctgg gagcggagac aggtgatctg ggcggaagtg 120

tccatgctgc gagtgctcgc ggctgggagc gcagacacgt ggtccaggcg gaagtgtcca 180

tgctgcagca ggagctcgca ctgggagggg agacacgtga tcagggcgga agtgtccgtg 240

ctgcgaggag gagctagcgc tgggagggga gacacgtggt ctgggcggaa gtgtccgtgc 300

tgcgagcagg agctctcgct gggagggcag acacgtgatc ctggcggaag tgtccgtgct 360

gcgagccgca gctcacactg ggagggaaga cacgtggtcc gggcggaagt gtctgtgctg 420

cgagtaggcg ctccggctgg gagggcaga 449

<210> 198
<211> 609
<212> DNA
<213> Homo sapiens

<400> 198
taagcattaa cggcagcgta gaccccgtc cagccgtccc tgaagtgtcc ggcatctcct 60

tcgagggagg ctgtagggtc ctctcgtgca aggcccaggc gcatcgtgac agcttgcacg 120

cgctgtggtt cggggaggtc ttcctgcagg cactgagtca caggtcaaag gctggctcag 180

gtcaggaagc caagcaaagc accgagacgt tgccttgcag tggcttcccg ccgccctctg 240

gtatccattg ttgacttctc ttggttcgat ctttaagtaa tattcttgtt ggctgtctgt 300

ctgtccccta ggaataccac gttttatgaa ccatcttcat agccgtctgc agctcccgct 360

tccccgtcac cactcagcct gctgccctcg caaggattcc acccacctgg cttctgatga 420

gtcagcacta ccacctgacc ccaattattc tggggtccca tgattttcat tgctactagg 480

gaaacctttc tagaaccacg tcttgtatcg tctgctctgg cttacagagg acagacactc 540

ctgagcccct tgacagcacc ggtgcccccg gccagcccat tccttgttga taccaggttg 600

cattcccct 609

<210> 199
<211> 643
<212> DNA
<213> Homo sapiens

<400> 199
ggtggagact gggccctgct gtctgtcctt gcagctgttt tagggccgcc ggccagtctt 60

cctcacctcc agatttcaga ttttcccctc tgcagccaaa cattgctcag taagtgaaag 120

gagaactcag ccctctcaac aggtcctggg tacagccaga gggatacccg tgggcctggc 180

agtgaagaga gatgtgacca cagtcctctg agccagggac cagggccagg gccaggcgga 240

gggaggaaat cattgccctc tcgctgagtc tgaagagaac tgggggcctg gattcagggt 300

aaggggacac agcatggcca cagcgggagg ttggcagttc ctcaacgtgg tggcagtgga 360

ggggcttagg atcaggagcc tgagggggca ccatggcggc gagcgagcca gccctactgt 420

gtactgggag cagatgcccc aagaggagtc agttcttttc aacataactc cctcctgtgc 480

ctgagcacca cagccaggcc tgggttgggg tcatacagga gggcgacagc catgcttggc 540

caaagtgatc cagtgggaac ccagacagag gaagccagag acagtggagg aaccggtgag 600

gggcccccaa catgggcagg aggcagagag tggcagccag gat 643

<210> 200
<211> 460
<212> DNA
<213> Homo sapiens

<400> 200
accgccaaca tctgcgggga caatgacaga ggaatgccta atgagggccc ggcgtggccc 60

cgccacggag cgcaggctgg gacagatggt ctatcattac ccatttttat taattactgc 120

tccccgtgct gcgattggct ccaacggctc aggcacgttg gacgcgctgt ggccgcagct 180

attctggaag gaagaaacgg cccttcattt agcacagcgt gcctttcctc ccagcttcct 240

cagaggccac aggcattttt ggctcagcga ggtccctggg gaactaggct gtgcggaggg 300

aggagtgtgc agctgccacc tgactgatga gaaggggcgg aggggctggt gcggggagag 360

gcagagagcc cctgtgactc cagcctggag cgaggccctg cggggagacc cgggacctct 420

gcagacctgg cccagaccac atctgcatcc cacaggcttg 460

<210> 201
<211> 469
<212> DNA
<213> Homo sapiens

<400> 201
tattccccgt atgttcaaga agaatggaga atgaacgaga gtgagggacc ggaaacgact 60

gtgttccatg ctgagggtgg cagaggctgg cctggatgct ggatgaggct caactcgggg 120

gcctgggctg ttgcactggg gctggactgg ggatcacgga agagggccag gcccgcgtgg 180

gtctccaggg ctgtgctcag gggcgtggtc ttctgcagtg ggggcttggc tggtgagtgg 240

agactctgcg tgactctggg ggcgtggccg agaatgggtg tagctctgta ctagggcgtg 300

gtcatgcgta ggggtttggg tgaggcgtta acagtgggcg ggcgtggctc gggctcgagg 360

gcgtggctca ggctcgtgga ggcgtccgag ctccagggca gctctggggc agaactgtcc 420

ggatctaaca atccctgggg agccggagac cggtgactgg tccgtgggc 469

<210> 202
<211> 872
<212> DNA
<213> Homo sapiens

<400> 202
tcctgctgac agctgggttc gttctgcctc attgaacagg gctttgtacc ggcattagct 60

ctgtgaccac agctttttct gcaggactgg ggatcccagt gactgtgaaa gaggaagacc 120

taaaagactg tgtcacatct ctcacctggg tgtcctggtt caacaagcac tgggctggga 180

ttgtcaaggt accagggctt ttgccacata aggcactgtc cactcccagc atgagaggtc 240

tatctggaaa catggctctg aatgtagagg gggccacaat ttaaaacaat taaagcattt 300

gcaacatatc aatcctctgt actacttaag tgggtttttt tgtttttgtt tttgtttttt 360

ggttttgggt tcttttcgta aggtagctga aaacaactct gctctcccac tttccttcaa 420

aaaggtagag atttattgga aagatatttg agaagctctc agtatctaca gaaaggatga 480

acgaataatc ctggggtcag aacagggcct tctcccagga cgtcacatga taaagcacgt 540

agacttgctc tgtggagccc tgacgttgat gagatttggc tccaacccct cttgcctgca 600

agagtcttag atgatgccca cattccaggc tcagctgcag tgaggtgtcc acctctccat 660

tggtcagcgg tggcctggga cacagcacag gctgaccatg gccctctcct gtgtgttaga 720

gcattccaga agaagagcac tcactgcagc tataaatcac ctcgtcattg tcttctacaa 780

tgttcttctg ctctgcactc cactctcgtt acaagctgtc cccggacccc agggctgccc 840

agttgccagt taagacctga ggtcaagaac tg 872

<210> 203
<211> 538
<212> DNA
<213> Homo sapiens

<400> 203
ccgggcccat tgttagcagg ctattccacg gcagctttgc atctggctcc ggcgggaagc 60

ggaaaacggg aggcggctct cgaagcttcc cgaccttcct gcgccatcaa cttctcagga 120

gtggctggag aaagatgcat gtgccaagcg agacaacaac cccaggtcca tgtgtccaaa 180

tccccgtagc cagggcggcg gccagccaaa gaaatgccgc cccgagcagg cgcgtgcggc 240

tcctggcatt ctgggtttca tacccgtagg gctcgggtgc ggtgagtatt tccgatttcc 300

aggaagtctg ttggaagtta ccagcaggga aagagaagat gcttgctcct ctctttccct 360

ccctctctct ttttttcta cccttcttct tgtccttgtc gctctggttg ggggctggtt 420

ggtttagata cagcgagtgc tccctggcag cctgaactgc ctcccacacc ttcctgatcc 480

ttgggcaccg gggatgtttg cttccgggtt ctgttgtggg aaacagcact gcgggga 538

<210> 204
<211> 558
<212> DNA
<213> Homo sapiens

<400> 204
ataaacagca gaaggtgaat ggcggctgcg agggaaggga gttggaggcc gggagtggga 60

agcgaggctc atgcgggcgc caaggtggcc ctgggggtga cgcgaacatc agggagtcac 120

cagggcactc gtccgtgtgg cgcactcatt gctcgaaatg ttccagtcta caaatggtcc 180

gaagatggtg atttattttt gtaaaataaa tacatactcc atctccacac ccatactttc 240

tcctttttct ttcttttttt tggcggggga gtggggaagg aaaggacatg aagcaaacag 300

aggaagctga gaaggtggaa gtggctttgc tggtggtgtg ggctgctttc agagagctct 360

ctcctcaggc accacaactt ggagaatgtg aatgcttgct ttattttcct ttcctaacaa 420

aaggaagcct tgtctgggag cattcaatca cggactctcc tgaaacctca ctaatatgtg 480

ttcatttctg taggcgcgaa atcagacccg ggttcattcc actggccaga ggcagtcttg 540

ggaggatttg tgttctgg 558

<210> 205
<211> 449
<212> DNA
<213> Homo sapiens

<400> 205
tacttgacgc cgttgaagta ctcggggcac ggcctctcca cgagggctcc ggcagcgctg 60

cggggccagc acgttccgat ctggtccaag gtcgtgttgc agtaggagta gggacctggc 120

ggcgggagag agcgcagtag ggctcagagg ggcccgcagg gacgcggggc tctcggagcg 180

cggggtcagg ggcgcaccca gcgcgcgaga gaaggagccc gcgcagcctc cgaccgctcg 240

cctcccgcct accctcgggg tccaggggtg gcccccagcc gtccaagagc agctcttcag 300

ccagcgccag gctgcagttg gcctccagca ggctgtggag cagtgccgcg tccatcgcgt 360

cccgcagccg cgtgcggaga gggagtggga gtgcgcgccc ggcgtgactg cgagggagtg 420

gacgcgagag tgagcggccg agagggcgc 449

<210> 206

<211> 423
<212> DNA
<213> Homo sapiens

<400> 206
gccagcaacc tctccccgcg ccgcccgcca ggccccgtcg ggcctgcgcc ggctctggcg 60

gtgcccacaa agtccccgag gggcagcgtt ggccaggcct acccaagacc tgccccgtcc 120

cgccaccact gcacccactt tctgctgccc tcatcggtct gcgcagctgt ccccgctcct 180

ccccctgagg caaaactgca ccagaaggca tcttggcgag gtcattgcca ccccgcccat 240

gattgtagaa gagactcaat cctgtttaag aggtttagct ccccaagcgc tattaaaaat 300

aagtttttgg tggcctttgt aaggaaactt ttaatcacac agaatttagc ctctgatcgg 360

ccagacattt aaccggcgca gagtaaaacc ttccattttt aacatccact ctggtgtttg 420

ttg 423

<210> 207
<211> 517
<212> DNA
<213> Homo sapiens

<400> 207
aagtggtccc ggagaagcgc tccctcgcag ccaagctgca agaagtggcc gggaacctac 60

aggcctcggg ccgacccagg aagcctccgc accagaaagc tcgaggagcc cttacccaag 120

tcttgctcga agggcaaagc aaagagccag cacccctgag tgtcactgaa gttcctggat 180

ggggtgtgag tgcgcgcgtt ccgtccgaga cctcagtctc gcccagctat agagccgata 240

aagggatgtc ttgtgggcgt aaggcgcttc gcgcccatct ccaaggccga tgtggtcagg 300

aggtgagggg aaatgtcctt ctggcagaag cccgcggtgc tgcgacgttg acccgcctgg 360

cctcaggctc agggcggcgg gcagcccagg gcacatgtag tttcagcagc cgcgctacgt 420

gggcggggga ccccaggcca ccccacgtgt ccgccctggg cctcctccgg gtcccaaggc 480

gcggcgcctc caggccttgt agcgtcttcc ccgggtc 517

<210> 208
<211> 386
<212> DNA
<213> Homo sapiens

<400> 208
aataaaactg tcctggtgag tcgctcacag cctgtcccag taactgttcc ggagcgtcag 60

cctgacgcag cacccctgaa ggaccacgcc gtgcgggctg cgagcctcgc ccttaaccat 120

gcccagcagg accagagctg ccagcacaaa tgaaggtcct ggccattgaa caacttctga 180

gcctgcactt taagaaggcg ctcttgtttt gaatacgtag tctgtcctgt tctttcaaag 240

taagtgcttt tatcaagggc ctggactgtc ttttaaacat atttttttct cacttgtaca 300

ttaaagggaa atgtatttct gtctagaaaa ggagaccggg agaggaatga gactgtgcgg 360

tattcaggct tggtttaaac tagggc 386

<210> 209
<211> 615
<212> DNA
<213> Homo sapiens

<400> 209
agggcccggg caatggtgtc cttgaggctc tcgcaggtgg ggagttcccc gggcttcagg 60

cctgcgtacc gacgctcctg ggggacacag gcacgctgct ttccctccca agccagtggg 120

cccccacccg actccccact ctgcagcttc ccagaggcct tcccagcaaa ggcagcccac 180

cttgctaatg gagttgtagt aggggtgctt ctcgtccatc gggggcggcg ggatgtcgaa 240

ggagcgcctc cagatcttca cctgctcctc cccgtgcttg gcggccgttt ctgccttgtt 300

gaggcctgtg aggcccccgt aatgccgctc attgaggcgc caagtgcgca ccacaggcag 360

ccacatctgg tccgtgccgt ccaggatggc ccagagggtg cggatggccc gcttcagcac 420

tgacgtgtag cagatgtcaa actccatctt ggcatccttg atggccttgg ctccccgctt 480

ggcctcctcg gtccccttttt cactcagctc tgcatcgaac cagccacaga aacggttctc 540

ctggttccat gtgctctcgc cgtgccggac catcacgagg cggtgagtgg ccatggtggc 600

agcagggacc acaga 615

<210> 210
<211> 552
<212> DNA
<213> Homo sapiens

<400> 210
agggtcaggt cccgcagggc gcaggcgggc gcttctcgca gcccaaaccc gggcggggag 60

tgcgggaacc cctagaacta tgctgggagc cccagataag aggaaggaga tggtgactta 120

agtgacgaag tgatgttttg tccccaccgc cttttaaagg gagaaggggg taaaatttca 180

tttggaggat gattgttacg cgagctttca gagttcagat ggcgtcaagt ggccatcgag 240

tcaacattta acaaaatgtt accgcataaa atattgtcat cgagttatct ggcagctccg 300

cagagaaact tcacaggcat atgcgttata tgacataatt tgcatgggga agggggtccc 360

ttcacgattc ttttatatgg caacctgttc ttggacctgg cagggcccag agaagcgagg 420

aactccgcag ctcgtcgaca cgtctcgtct cctgtcccaa ttcagggctt ggtgaggtga 480

ctcgcggtcg cgggtgactc gccggcagga cactgcctgg aacgcctgga gcgcctccca 540

ctgcagacgt ct 552

<210> 211
<211> 736

<212> DNA
<213> Homo sapiens

<400> 211
ccgcgagaaa gactgtgcct gacctgcacg gaggatgacg ctgaccggcc ggtgatgagg 60

aagccaccaa gggaagcaat tagacacagg acgtgctgga cctgcaggct gcacaggcca 120

agggagggcc ccaggcgcag cgggcggatc tgcgggtccg cgggctgcgt ccctgccctg 180

tggccgagac tgtgacagtc tccgcagttg gcagccgagt gcactgacac ttaggctgag 240

tccgcaaaca gaaaagcacc aaatctcagt ggctccacgc aacaggtgtg tttctcactc 300

tcatatccag gttaggtcac gggacacgcc aacaagtgat ttagggacca ggctctttcc 360

ctctctgtac caccttaaaa atgcagcctc caagttcact gtctaaagga aaaagtaatg 420

atcaagggtg agagattttg ccgcggcctg tgacacagct ccacctaatg taaaggtgtt 480

agaaagctcg gttttcccgc gtggccagga agaggagaaa aagaagtgtc gctggatcac 540

taagacttgg ccagaccctt ctgtgcctcc taaacattct cccgtccatg cagagagcac 600

cctcacgcct tcccccagag gggcaaccac agtcccacca gtcactgccc aatgcaaagg 660

gcccccaggc aatctcactc catggccggg actcctcttg ggctgccacc aatcacctag 720

agggagagtg gttgct 736

<210> 212
<211> 564
<212> DNA
<213> Homo sapiens

<400> 212
tattaaacct gtcttacaca cacacacaca cacacacaca cacacacacc ggattgctgt 60

ccctggttca agtgtgccaa gtgtgcagac agaacatgag cgagtctggc ttcgtgacta 120

ccgaccataa acccacttga caggggaaac atgccttgga aggtttaatt gcacaattcc 180

aaccttgagc tgcgcgggtt ccaagagcca ggcccgtact tgctgttgat gtcattggct 240

tggggagttg gggtttggtg cccagcgcgg tcgttggggg aggggcaagg catagaacag 300

tggttcccag accttgctgc acattggaat tacctgggat taaaaaaaaa aaaatcaaaa 360

caaaaaccag tgtctggctc ccgcccccag acattctgat ttaattggca tggggcaaga 420

cctggacttg ggattttttt taatgctctt catgtgatct gttgggcagc cagatttggg 480

gatcactaga cggaagaagg attgttaaag tctccggaga tgttacttgc caatgctaag 540

agctctttga ggacatctgg aatt 564

<210> 213
<211> 768
<212> DNA
<213> Homo sapiens

<400> 213
atcttccgct gacaccctcc tctggggtcg caggtatgaa tccatcaccc ggagacccct 60

caacaactca ccagactgca ttcccaaccc cctgggaccc gattcctgca cacagccttt 120

ttcgggaagg gagtcccaag agcagctttc agcgcccacc tcacggtctc caatcgcctc 180

ctcctccagg gggagccgac cacggagacg ccccaggagg ccctgtggtc gagactttta 240

tagtcccgca atggtagtcg caggcagccc ttttcctgac caggccgtct ctgcctgcac 300

cattgtcctt gcttaggcgg gctgacagcc ccgacagcct ggcgcccgag gcagcctcac 360

gaatgcgcat gtgccagtgg cggggcacaa ggcgccagcg gcgagcactg gcttgcttca 420

cagtgaacgc cttcgttgcc tggcgactag tccctgcggc ttagcggaga gggggcgtcg 480

gcgttgggct ctcgcctgtc ttctctgggg ggatcctcgg gatactccca cgatctagga 540

cagggcaggg gcgagccagc ctgaggaaac gtcaagggaa ggcccaggga tacaccgcga 600

aatccctaag aatccaaaag gatccgcagg aggcgtcagg cctgcctaga cggagtgggg 660

tgagtctcct taaacgttgc ccccctgtga tttctgacta cagcaggcct gtgttccccg 720

gggctgccct ctcccgggag gggcttcccg gcggagcaga accgcgct 768

<210> 214
<211> 338
<212> DNA
<213> Homo sapiens

<400> 214
ggtctcagtt gcacagacca ggaaactgag gcccacagag gcaggtgtcc ggttgtttgc 60

aacctctcag cctgtgctaa ccccaattgt tcagagagag ccctgaaacc ctctcctctg 120

ggcgccccca ggtgactgcc ccagcctcaa gggctgcctc tgttgcagga aagacgacgt 180

cacaggctac ttcccgtcca tgtacctgca aaagtcaggg caagacgtgt cccaggccca 240

acgccagatc aagcgggggg cgccgccccg caggtaagcg ggggtccccg gggctgggcg 300

gggtcgagcg gggcgcacca cgggttcgct ctgtctag 338

<210> 215
<211> 416
<212> DNA
<213> Homo sapiens

<400> 215
gactcaggtg cccgcccatc ctagactccg tggccgcacc tgtgtcttcc ggatcctcag 60

gtcagcggag gagcggttca ggccttcctc ttgctcgatg gactgctcga tgcctggggg 120

cacaggtggc tgctaagtca taaccaggcc ccttcaaccc cgtcccctac ccgcacaccc 180

agcatctatc caccttccca catcccctag ggtatgtgtg tggcctgtgg ctggtgggat 240

cagtgccagg caccgatgga ggagggggcct acagaagccc ttccagctgt gacgctgtgt 300

ggtgtgtgca tgtgtgtaaa tgcacaccca ctccaagctc tgcgtgtgtg tgaggtctgg 360

tagctccggc caccctatgc ctgggtacca ctccttttct gctcagaaag gctgtg 416

<210> 216
<211> 338
<212> DNA
<213> Homo sapiens

<400> 216
ggtctcagtt gcacagacca ggaaactgag gcccacagag gcaggtgtcc ggttgtttgc 60

aacctctcag cctgtgctaa ccccaattgt tcagagagag ccctgaaacc ctctcctctg 120

ggcgccccca ggtgactgcc ccagcctcaa gggctgcctc tgttgcagga aagacgacgt 180

cacaggctac ttcccgtcca tgtacctgca aaagtcaggg caagacgtgt cccaggccca 240

acgccagatc aagcgggggg cgccgccccg caggtaagcg gggtccccg gggctgggcg 300

gggtcgagcg gggcgcacca cgggttcgct ctgtctag 338

<210> 217
<211> 338
<212> DNA
<213> Homo sapiens

<400> 217
cctagacaga gcgaacccgt ggtgcgcccc gctcgacccc gcccagcccc ggggaccccc 60

gcttacctgc ggggcggcgc cccccgcttg atctggcgtt gggcctggga cacgtcttgc 120

cccgactttt gcaggtacat ggacggaaag tagcctgtga cgtcgtcttt cctgcaacag 180

aggcagccct tgaggctggg gcagtcacct gggggcgccc agaggagagg gtttcagggc 240

tctctctgaa caattggggt tagcacaggc tgagaggttg caaacaaccg gacacctgcc 300

tctgtgggcc tcagtttcct ggtctgtgca actgagac 338

<210> 218
<211> 301
<212> DNA
<213> Homo sapiens

<400> 218
gctgactctc ctgcgcgcgc ccttgcctgc gcgcgggggg cgagtgcacc gggcgctcgc 60

tccctgccga gctcttgtcg cccgcccgag cctttgccag gggatgaggt gggaggcgct 120

agaaaccact tagctacagc caaccgccca atgcagcact cgctcgctcc ccccgccagc 180

ggaagcgtcc gcgaagcaca atgcagcact gagccgcggt ggaggttgca gcgccacggc 240

cgccgcagca ccggccgggg ctgggtggag gtggccgcgg ggaccccggg ggcgcggagc 300

g 301

<210> 219

<211> 570
<212> DNA
<213> Homo sapiens

<400> 219
ggagactaca gtctgagacc cggggctcag cccatgccca ggcctcggcc ggggcgcagc 60

gatcccccaa agccagcgcc gtggagttcg tgccaatcct gacatctcga ggtttcctca 120

ctagacaact ctctttcgca ggctcctttg aacaactcag ctcctgcaaa agcttccgtc 180

cctgaggcaa aaggacacga gggcccgact gccagaggga ggatggacag acctcttgcc 240

ctcacactct ggtaccagga ctgttcgctt ttatgattgt aaatagcctg tgtaagattt 300

ttgtaagtat atttgtattt aaatgacgac cgatcacgcg tttttctttt tcaaaagttt 360

ttaattattt agggcggttt aaccatttga ggcttttcct tcttgccctt ttcggagtat 420

tgcaaaggag ctaaaactgg tgtgcaaccg cacagcgctc ctggtcgtcc tcgcgcgcct 480

ctccctacca cgggtgctcg ggacggctgg gcgccagctc cggggcgagt tcagcactgc 540

ggggtgcgac tagggctgcg ctgccagggt 570

<210> 220
<211> 541
<212> DNA
<213> Homo sapiens

<400> 220
tcgcagcgtc ctcttctggc accccagggt ggccttgttg tctgtttccc ggccgagttt 60

gtgttttgct ggctcgcgga gttttctcct ggctacgggc gagggttgcg cgtttcagga 120

aaaactgagt ggggcactgt ttgtttgttt gtttgttttt tgataggatc tcagtttgtc 180

atccagtctg gagtgtagcg gcgggatctc agctcactgc agcctcaacc tctgggactc 240

aagccatccc cctgcctcag cctcccaagt agctaggatt acagggcgcg ccatcaaggc 300

cagttttatt ttttgtagag gtggggtctg tgttgcccag gctgctctcg aactcctggc 360

ttcacatggt ctcccatttc gacctcccaa agtgctccga ttataggcgt gagacaccac 420

cgcccaacct gttgttgttt tggtggtatt gtttttaatt taatttagag acaggatctc 480

gctttgtcac ccgggttgga gtgcagtggt gccatcatag ctcactgcag cctcaactcc 540

c 541

<210> 221
<211> 468
<212> DNA
<213> Homo sapiens

<400> 221
aggaggttgg tgaaataggg gcgtggtctc cctcgggggc gtggcgatcc ggctcaaaag 60

gcggggcctg ggtggaacct agcctatggc aattgagaag ccagattaaa ggggttacta 120

gcagtctggt ccaaaacccg cccaatggag taggagattg agggtagcgg gggtgggacc 180

tgggtggggg cggggctaaa gcgggccgcc cgcgtgccca agcccacgtg agagggcagg 240

acgcctgaga gcttgaggcc acacgaggct gtggagcggc gtgactcaaa cgtggcgcgc 300

atcagctcgc acacttccaa acctcgcgat agctactggc cctgggcgag ccgttgggat 360

tgcgcttgcg cacagcgtgt cttgtacgtt cccgcgcttt tctggaatct ttcgcccccg 420

gaagggcagc ggcgggcgcc tgtgtggaag gtggggtggc cagagaca 468

<210> 222
<211> 395
<212> DNA
<213> Homo sapiens

<400> 222
cacccgtcca cagcagcact ggatcgccac acacaacact gtcccctgcc ggctctacaa 60

cccgtcaggg agaatctacc accttccaga gctggccaaa ctcgaaggac actacccctg 120

cacctcctac taccacatca gcctttgttg agctatctac aacctcccac ggcagcccga 180

gctcaactcc aacaacccac ttttctgcca gctccacaac attgggccgt agtgaggaat 240

cgacaacagt ccacagcagc ccagttgcaa ctgcaacaac accctcgcct gcccgctcca 300

caacctcagg cctcgttgaa gaatctacga cctaccacag cagcccgggc tcaactcaaa 360

caatgcactt ccctgaaagc aacacaactt caggc 395

<210> 223
<211> 483
<212> DNA
<213> Homo sapiens

<400> 223
gcagccgagg ggtgcggccc ctacctgggc actgcgggca gcactctccc ggcagcagga 60

caggctctgg acagggcagc ggcacgcagc ggcgggccag gcactgcacg ttgccgctct 120

acggaccgac agacgcacca cgggtcagtg ggttagttgg gggaggccga ggagacccct 180

cccacccagt gccaccaggt cccacccctc ccacctgggc gggcctctcc tggcctctgc 240

aagtcccgcc tccactcccc tgggcgcctc ccacccaggt gcagactcac cagacagcga 300

cacagacggc aggggtcaga ggggtggggg aagtccgctc cgctggggta ctctttcccg 360

ccaaaggcac agcctggggg agggaggggc tatagcctgg gagagggcga ctgccccagg 420

gcgtgctgcc ttccgggggc catgccatcc catcccagaa ggccgccctg acccgcccac 480

ctc 483

<210> 224
<211> 339
<212> DNA
<213> Homo sapiens

<400> 224
gagtggccgc gccctgggtc ctgcaccgtg aggctggggg gaccaggacc ggcgggcgcc 60

caccttggtc ctgagcccag ctgccgacgc tgggccagca ccctctccac cgccgccctc 120

cagtgagcag agctgggctc aggcagcagc ccctgggcgc gggaaggtag cgggagctgc 180

atttagggga gggggaggt agcaggagct ccgccagtgg gaaccttgga gtgaattcgt 240

cggcgggtgc caagtcgaag cgccgacctc ctgtctctgg actggggccc ggacccctgc 300

gtccccaaac tgcgtccccg ccttgctcgt cgcccggtg 339

<210> 225
<211> 383
<212> DNA
<213> Homo sapiens

<400> 225
gcacacggac ctggagatga agccccttcc tgacttgcgc acacggaccc ggagatgagg 60

ccccttcctg cctagtgcac acgatcacgg ggatctatag gcacacgatc acggggagag 120

aagcagaacc actcggagag cacattgcag gggttcaacc tcacgcagtg atgggagcgg 180

gctggacact gtgtgtgaag actttctttc tgtgtctggt gctgggctct gaaatccatg 240

gagcacacag ccaggaaggg gaaaatgaaa atgaagtgag gaaaacaggc acaaccatga 300

cacggaggct gagctggacc cgccagggca gcccggaact gcatcctctt tctcccctgg 360

caaggcccat gcttccttac cgg 383

<210> 226
<211> 457
<212> DNA
<213> Homo sapiens

<400> 226
gcagggttgt agctgtagtg tccctgaacc actggggtta ggaggccacc ggaagcctgg 60

cctgctaggg gccttgtgat ttcgcaggcc ccgagaacgt ggtgtgcagg tgcgggccgc 120

ctgggggagc catgtttaaa gtgcggtgag gctcctggaa cggcacccac cgcaggcccg 180

aggccacacc tgtatgtcag aactgggcgg gtcttctcct cccaggcctt cagcgcctgt 240

ggctatggct gtgggcggga ctgatacggg caggcaggcg ctgttctccc cacgtattct 300

cggcttcccc gccatacacc gtttctgagt ggggagaact ggttgactgc tgaatcccaa 360

cacaaatcct tagagcaaag aggccagtga tggcttttgt ttctgcccgg atcctctttg 420

tggatcttcc gttttgcggc ttttcctctc atttgtt 457

<210> 227
<211> 337
<212> DNA
<213> Homo sapiens

<400> 227
aggacccatc ctcggccgct tcccgtcccc ctcacccgcc aggacctgcc ggcccagcct 60

cttcacaccc cgcggcctct caccgcgtgg ctgagcctgg gccgccgttc ggaccgcgcg 120

gcagccttcg attctgtgac tctcctcgcc tcgaaccccc cacggttctc agcataaagc 180

ccagactccg tggctggtgg aagcagcagc tccctggatc ggtcctgggc atatttacag 240

cttcgctttt taccctggga tccactgttc gactcgcaac acctttccgg agcccggcgc 300

ttcactcgca gcctcgcagc ctcgcaaacg cccgtcc 337

<210> 228
<211> 594
<212> DNA
<213> Homo sapiens

<400> 228
gcagctgtcc tcagctacca gtcaggagaa cacttgctcc tgggcgtccc ggtgccagac 60

aggatccaga tttcccttct tatgatgtgc ctttcacaaa acagcgctag cctgattctt 120

tccattttct attttctgtg tctaattttt tccatatcca ttttccacct ggttcattta 180

gtgataccga tttttaagcc ttattttttt aaggatttgt gaatattgta caacttgtta 240

ggataacagt ggaatactgg ctccaaggat gtgtgcaatt ttgatttatt ttcctaatat 300

gtgtgtgtat agataaatgt cagtgaagtg aatatgtgtg tgtgtcaata aaagaaatat 360

acttaatcac ccagttattt agtcaaacat ttaaaaagtg acatgtcctt ctgacctggc 420

cctgcaagct gcaggaggct ccctggcctg tgagtttagg gaactcgtgt cagtctggtt 480

aagtgggtgg tttgggtgcc gtgctggctg tacgtggttt ctcgtcagtg tgctggacat 540

tggccggcca gcgctgcctg ggccttgaag gcagaaatgg ctgcagacac cact 594

<210> 229
<211> 534
<212> DNA
<213> Homo sapiens

<400> 229
tccggtgctc gtccagccct gcgctgcgct gaggagaagg cgggactccc gggaggcacc 60

ctgcaggtct ggatggcctg cctctggcca gagcctggga ggtggaggga gatgggattt 120

tgaggccatt cacctccctc gtaaatggag ccgctgatgc actctcacag aagggaagag 180

gtaatttttc catttgtgag gagagtgcag accctggaag gctgcttagc cactcagcct 240

tttccagaca gctcatttct cagactgcaa aggtcagcag cagaattggg ggagaggggt 300

tttactgcat tcttcagggc agaggccatt ttaatattca tgaaattgca agtgaagcca 360

ctggaatgtt ccatgggagc aggccagcgt gtccttgaaa ggaactgaga gctgacagct 420

tttgtgtcca cgctggactc ttcctgtagg agccctgagc atgggctatg gagacacagg 480

acaccggcca ccgtggcccc acacccaggt tggcaggtgc agctggccct gatg 534

<210> 230
<211> 879
<212> DNA
<213> Homo sapiens

<400> 230
ccaagcgttc atttcattaa gctctttctg gttgcaaagg gcaggcaccc ggagccacct 60

tctgtagggc aaggtcctgt ggagccacgg gaggaatcga gggaggcagg ccctgtctca 120

gcaagtgctg gccgctcccc gacagatgcc cagaagtccc cccaggggta gtggccgcct 180

ctgtgaagac actcagaggg ctgggctgta gccctggtca tcacagcctc agccctggac 240

agctgaattg agcctggcag ctgaattgtt ctccatgttg ctgtgagccc actgacaaac 300

tcctgccctc agtgtctgga tttcacacgc cctgagaaag aggatgtgat ttatcctctc 360

aatcacccac aaacatgaaa tgcgcttgcc tggcctcacc ccacgatggg tcaggaacga 420

tggccaagag ggcaggattt tacggggctg agtgtgtcga cacacatgtt gttcatgctg 480

tcagatgggc atgcctagca cagacgtgtt caagcggtca gatgggcatg cctggcacac 540

acgttgttgt gaactcttgc attgttggta agatgtgtga tggatttggt tttaccaaaa 600

atggcaaatg tggagttatt tttaaggcat acttcgtgtg ctatttcttg atgtggtttt 660

atcacacagt gttgttttcg tccaccttgg tgcaacggcc ctggtgttgc aagaggaggt 720

gagctctgag cagaattctc acatatctcc acttaggaca cattgttgag catcgatgtg 780

tgccagagac ttgcagggca ttgaaggcag acagtgggct gccctgtgcc ggctctcagg 840

tcccaagcct ggatgactag ggcatggacg gtgctgtgg 879

<210> 231
<211> 152
<212> DNA
<213> Homo sapiens

<400> 231
cccctcccga agtgagcgcg gcccggggtc cgcggggcag gagatgatcc gggggcggga 60

gcctctgccg cgcgccccct cccacgcgtg cgcagcctgg gccgggcgtt gggcagcggg 120

agggggcgcg ggcggccacg tggcctcgaa gg 152

<210> 232
<211> 559
<212> DNA
<213> Homo sapiens

<400> 232
agcacgtgct gagtcagtgg ggatttgctt gcaaagacgt catcacaccc ggccaggagg 60

tggtggccca ggctgggtga gggacactgg ggaagttcta acataggaag tcccccaagg 120

agtgaagaga ttgcggaggg gacacacatt cagggtgggt gaggctggac gtgcagtcct 180

gctggttttc tgggttgggc atgtgattga taaaagtgga gttgagggca cattggcact 240

gggagtcaaa tgcgcagaag acttgagctt gctactctcc cgagccagga gctctaaggt 300

gttttcattg ttgaaatcca agggttctga tttaggcttg gctggcacgt tgcaagtgac 360

cccatacttg tggaatcaac tgaggctgat ggattctgca aagcctctga cacctcctga 420

ttttggtggt cctgagcatg aagtgagcag ggaatacatg tggggataaa cgtggcaaag 480

tgccctgtta cccccatcca aagacttccc ggggagactt ccacagtaag gtaccattgg 540

agtgaggatt gcaggaagt 559

<210> 233
<211> 681
<212> DNA
<213> Homo sapiens

<400> 233
gtcagggatc tctgccttac agattttcaa taaccctcct gtctgcagcc ggcccagatg 60

cagggaccct ccaagtcctg agctcactcg gttccctgcg cactttttgg ctttcttagc 120

cccgcacagt tccctagacc tcatgcccct ccctccacct ccagaagctc tggggatggg 180

ctcaaattct gtcatttcct cccctgttgt ccctcaggct cctgggtggt ttcctccttt 240

gcagtggact agcggggttt agggaggtgt ggaagtccag gcaggcagta cctcggcgtg 300

acgcggtgac gcagccgcag gcaggcagta cctcggcgtg acgcggtgac gcagccgcag 360

gcaggcagta cctcggcgtg acgcggtgac gcagccgcag gcaggcagta cctcggcgtg 420

acgcggtgac gcagccgcag gcaggcagta cctcggcgtg acgcggtgac gcagccgcag 480

gcaggcagta cctcggcgtg acgcggtgac gcagccgcag gcaggcagta cctcggcgtg 540

acgcggtgac gcagccgcag gcagtacctc ggcgtgacgc ggtgacgcag ccgcaggcag 600

gcagtacctc ggcgtgacgc catgacgcag ccggagagag cttcacgcac accatctctt 660

cgttcttcct gaactctgtc a 681

<210> 234
<211> 694
<212> DNA
<213> Homo sapiens

<400> 234
aggactcctc gaaacgccca gagaccctat agaccctgca gcctctgccc ggcatatcca 60

ttggattcaa ttaggcacaa cccaactcgg ctccagtcag caccatttct tgggatgtgc 120

agcatttctg tgcctcttgt gaagacaaag ataaattaga cacagacggc attcgagaca 180

cttgccatgt tgaagaggag aggagcacag aaacagctag caatggtgcc agacagaaag 240

ttgtgtgcct gacagggatg agaaactggg gggatttgaa gaggagacac gattaattct 300

gaccagagta acagagaagg cttcatggat gacacagaac ttcagaggga ggattaaagc 360

acaagtaaag caggattttt tattttagca aaagaaacag atgtgaattg gaggcagtgg 420

ctgagttgtt cccatcgatg tctggaagtc cgagaagctc cactggggct gctagctgac 480

ggctgaccca cctgttatca ggggcatttg gacggcgcat gtgccacacc agggacgtag 540

cgtgtggcac ctgccccctc tgtgttttcc tctcacatgg gccaggggct ctgtcccatt 600

aaataaccag aagattaaat gcagacttta caaagttcct catccggatc actctatgcc 660

cacatcctag aaacatctat ggaacttaat tttc 694

<210> 235
<211> 727
<212> DNA
<213> Homo sapiens

<400> 235
acaactttct taagacttac tgattgtgtg ggttttttcc tgagccaacc ggcttacagc 60

atatgtataa aagcaatgat agaagtataa tttggtgtag taattttacc ctaattcaca 120

ctccccttcc atgtgtcctg ccgatgattt tggtaacctt ctgctttcac aaatatgaag 180

tcaacaacat tttctttcat ttttattgtc tgacaaaagg gaaagaaaca ttcattaaga 240

acaagtcacc tgttttcact gaggagttat agacaataac agaatgaagc caccgcacct 300

ttcccacacg ctgctaaagg ctgaccattc caaatactgt caatgcgatg ctggcatcct 360

gtgagatgag cgggaaaaat accttggaag cactggttac ccacaacact ttgaaatact 420

taattgggtt taaatttcat cagagaaaag gttctttcat gaaaactcaa ttaaataact 480

acataaaggg cgtgggggga agtgttcatt actaattacg cagaaagcaa aaaatacgta 540

tattaaatat tacatgtctc ccctcctcaa acacccaagt acttcaaaat aaatggacga 600

cgaatatgtg ggtttcattc ctaaggaacg tcgggctact cagcaaagca aaataccctc 660

ttcgctcttc acctccccgg acggcgttgc gtgtgttgaa agtcacaggc gtaattcaag 720

caggagg 727

<210> 236
<211> 358
<212> DNA
<213> Homo sapiens

<400> 236
ttccttctct cccttatcct tgttcaaggg ccccgggttg gcttcacccc ggggcttcca 60

tggtttcagg ttttccttcc cttccttttt ccccaaggtc gctggaacca gggctgcctt 120

ccagcacttc atggggcacc tggtacttct ggccgtgtgg ccaaaggccc cgcagttttt 180

gcacttgagc tgtgggtgga aaggaagtga tgtcagtgag tgagctgaag ccacaggcag 240

cgatcccacg tcaacattgg gacggattgt gaattcagag ctgaataagg attccaaaga 300

ggggacaccg gcatgggggc cgttaagtgc cgggagagtt cggatacgat gttccctc 358

<210> 237
<211> 437
<212> DNA
<213> Homo sapiens

<400> 237
gaggagagga aatggaaaag cagggcaccc tccacccgag gaagcatccc ggctccgctg 60

cgggggtggt tccagtgcag acacaaacag acagattcaa acacacacag acagattcag 120

actcacacag aaacccacac gcccaacggg cgcttccctt cccctcaggc acccacctcc 180

caccctcggc tcccctgcct ccctgcggaa gccaaccttg gcctgcctgg gacagaagct 240

gcactccctc tttcccatcc cttgcaagcg atgggttctg gatgcctcta gcttggtccc 300

aagtcacacc accatccctg cagaggggag ccctgaatag ccaggagcct tcctcctaag 360

ggatgagcaa ggcctagagc cttctaccgg ttgagaagag tggggtcct ggggagcggg 420

ggtctaaagg agcgggc 437

<210> 238
<211> 654
<212> DNA
<213> Homo sapiens

<400> 238
cccaaacgtt aaaaacgcca tatcccactt tttatacaaa taactacccc ggcctgttct 60

ctttgtcccc ttcccgaagc cttcaggcag accgttccgc aaagtctttt tttttgtttt 120

gttttgtctt ctagacccag ctcctcggcg ctcatctcca gctgctttaa gacaaggggt 180

gggggaaggg gagggaggca agaaaagatg agggtggggg aggggaaaag agggaatgca 240

aggggaagga gggaggagac ggggagaagg aaagattgga agaaaaggat ctccgaggaa 300

ggggctgaga gaagggcagg gtgaactgga ctaaaggcca gagtaggaag gagaagaggg 360

gccaaaaaag aaggggatga aattaagcac agaagatggg taaagaaaaa agtatcaggg 420

aaagggcaaa ataagagaaa gccttgagga taagagggta gaaggctaaa gaacaagggg 480

accactgggt cggggaagcg ctgcctgaac ggcgggacag tgacaaagaa agggcgctgg 540

cgatattcgc accaagggtg cgaaacgcaa tcgggaggtg agaaatggaa agaaggcgaa 600

tgcccggcta caagtagcct gggactgaaa ggggacctgg gggaggggct gggc 654

<210> 239
<211> 604
<212> DNA
<213> Homo sapiens

<400> 239
cgctctgggc cagcttttgt ctaccctgtt ctggggtcgc cttccccgcc ggacccgcgc 60

ctggcgctca cgtggccagt aaataacagt caaatccagg gggaggaaaa tgcccataga 120

acaaagaggc ttgctttctg cacctgacag taatcgctgg tggagaagcc ctaaatcccg 180

caggcgattc acacggaaag gggtgtggga gggcgcgtgt gagtgtgtgt gtccacgtgt 240

gcgtgcccgc gcctacgcgg ctgttcactg gaagggagtc acagcttggg acttaatgtg 300

tttaagccga gagccacttg aattgcgaaa gcgaaggcct gatttttaaa aaagactaat 360

ttttaaaatc cacaccccat tgccaactca ggaattggcc ttcagttgtg tgaatttcag 420

caaaggagat ttaagttacc atattgtatt tatcgggata ctgtgtaaac accctagata 480

tatatttttt aattgtatgt ttgtgaacca ttggtccaca gttaaatgag tacaacgcga 540

gggctactcc atgccggcta tagataaaac ctgtctccaa ttcgtctgca gtctgtggac 600

gaaa 604

<210> 240
<211> 514
<212> DNA
<213> Homo sapiens

<400> 240
gcaccgcagc cgtgcgcgtg tcgtcgttgc gggccgcgat gtgcagggcc gggaggcgca 60

ccttcccctt ggtgccgtag ttgatgaggt gcgcgacgac gttctcatgg ccctgctgca 120

gggctaccgc cagaggcgtg aagccgtcct ggccagagga ggaaaatgct ttgctctgac 180

tcgtctgacg ccagccgccc ttcacacggg acacaccctt gcccctcggg cttatgctcc 240

cagcagcccc agcctgaggg acctacctcc accaaagccc tcttcatccg ccaagtttag 300

cctaaatgcc atctcctccc tgaagccctt cctgcctcca cttcctttct ctgcaatctc 360

acagagcctg ccgaattttt ctttagcatt aatttagttt tcctttataa tttgcttcca 420

gtttgacttg ttttaagaaa cttttgactg ctcagattct ttgccggctc tctttctcac 480

cccatctgct ggtaccagga tagtgccttg acaa 514

<210> 241
<211> 376
<212> DNA
<213> Homo sapiens

<400> 241
agttggacat caggccacac gctgagaggg tagtaaatga aagggagtcc ggtttagtcg 60

cgggcagggc atctcctctt ctgcttgtct aaaagtcgct gggtgcaggg gaagcaatac 120

taagcgggaa tttcttttta gtggcatcta cacaatttgc ctttcctggt tttgttttg 180

ttttgttttg tttcctaagg agggggaggc tctgtagagg aagcttcggc cctgggtgcc 240

cctgggttgg gagcagtcgg ggagcgctga gccagggcgc acgccctgtc ggcgctgcgg 300

gtgtggggcg caggagtcct tctggccggc cctccgcccc cccggggagg ctggagctct 360

ccaaggccgg ggagcc 376

<210> 242
<211> 429
<212> DNA
<213> Homo sapiens

<400> 242
tgcctcggcc tcccaaaatg ctgggattac aggtgtgagc caccgcgccc ggccacctgg 60

ctaattttta agttttagta gagatggggt cttgccatct tgctcaggct gatttcgcac 120

tcctgggctc aagtgatcct cttgcctcag cttcccaaag tgttaggatt acaagcatga 180

gccactgtgc tgggccacag agaatattga gactagtctg gtgtgtggca tgtgctgttc 240

aagtgtttgt gatgatgctc tttattacta tggtcattct ctatttcaat ttctccttgg 300

cacttatcac cagctaacat actatagata cttccttact taattgtgtt tatcgcctgc 360

tccattagaa ggtaaattcc gggcagggcc cggtggctca cgcctgtaat cccagcactt 420

tgggaggcc 429

<210> 243
<211> 568
<212> DNA
<213> Homo sapiens

<400> 243
caaggagctg gcaccccggg attccctctc tgacttctgc ttaaggggcc ggggtggagg 60

ggcggtaggt agcccacaga gtcccagcag gcgggaagca ggcacaacag cggaccttag 120

cacggggtgg gacccaggct cggtccagga gcctcggcct gtccagcact acattcttac 180

ttacaatgct tgcaattttt ggttaaggtg tccgtcagct tctgcagttt cttgcggttc 240

atgtctggcg ctcagagaat cgggccgcgg cgggggtctc tgggcgcgcg gctaccgaga 300

ccctcgcggg acccccgcga gccctggtgc tgggtcaacg cggtgaggtc tcagttactg 360

atctcgtgcg cggccctaac agacggtgta gccaaggcaa cggctcagcc cgccgcggat 420

tggctcccag gctgcccgcg acgtagccaa agctacagct gggccccgcc catcggctca 480

gcgactgctc gcgctgggtt ctcggtgcgc ggtgccgccg gcagggtccc agctggccgc 540

cttgagggct gtattctgac tgttgacg 568

<210> 244
<211> 566
<212> DNA
<213> Homo sapiens

<400> 244

ggcacagcgt tgtgctgccg cgcgtcggcg caggcggggg cgcaaggccc gggtcgtccc 60

cctcgcgcgg tcgggaaaac agctactgag ctgcggccca cgaccctcgg ccgcgcaggg 120

gcggggcggg ggcgctgcgg ggtttctctg ttgctagagc cgtgatgtca cccgcccctc 180

ctaacccgcg gttggttgtt ttgtgtttca gctctgcctg cagttgaaca caaagacctg 240

gaggagactc ccacctcctt cagggagaag aaaggaggca gcgaagcaga ttaaaggaac 300

ttgtggcttg tggccttttt gtgcaagggc cgcctggaag gggagaaaag tgtatgaaag 360

ccaccgtggc ggttaatgag ctgtgagatg aggcgctgct gcggccgctg ctgctgacac 420

tcgctcccag gctgcacccg ccgcccttgg cgcttcatgt acatgtgtct attcaggcct 480

tgcggaggcg cccagaagag catccaacac ggctgccggg gaaagaccgc ccaccatgcc 540

cacggagacc ctacagacag gtagca 566

<210> 245
<211> 355
<212> DNA
<213> Homo sapiens

<400> 245
ctcggcccca accgccaggc ctgccgtttg cgcccctcag ggccagcccc ggagccagag 60

aagcctcgcc ctgggccagg cgcagggccc agctcggaac agccccattt tctcggttgg 120

gtatcgctct ccacaaagac aggcgtctat tcccgaccct cgaggtcagg caaaggccac 180

gagtctttag agtttggatc gatgctaaaa gctgctgcgc tcccctgcag gccccagcct 240

ctcctctcgc agtccccaac ctcttctgac gcagacttta gctgctccac ttcttcgtct 300

gctcccggga tcctctccct ctcgcggcct gcccccgcc tccactgcat cccca 355

<210> 246
<211> 477
<212> DNA
<213> Homo sapiens

<400> 246
cgacgtcacg aacgtacaac tgtaccgtcg cgagaggacg tgatgcgccc ggtgattggc 60

gccgccgctg cggctgcgca ggagacgacc cccgcgggcg ctcccacccc catctcgcgc 120

ggactcgctt taggtctcgg cgagtttctc tgatatgcgc tcgcgggggt gctgccattt 180

catctcttcc gcgcgggctc atcgtgctct cagggtctcg ttgaacaagg caacgctaag 240

agccatcacc acctgtcccc catcacctgt tttctaaact aaacatcttc agctcctcca 300

gttatctctt gtggcatctg tcatgaagaa agtgctcggg cgtgtgtgtc tctctttgaa 360

cacctttgct gagggatttg ctctggtacc ttaactccag agaacgtaaa ggtggatgct 420

ataatgccgg gcgtcaaagc gatttggtac gcagtaagat gatagtagaa attataa 477

<210> 247
<211> 368
<212> DNA
<213> Homo sapiens

<400> 247
ccccgccctg tggccatccg atgccacatg tcctctagag ttcctgggcc ggctgctcca 60

cagcctgctc ctccacaggc tgcatggcct cctgcacgta gacgatgaac tccgaggcct 120

ccccgccctg cgtgtagaca gtcaccgtct caatgccctc cacgtcgtcg gaggacacca 180

ccaggtggtg ctgctcggca agctccacgg acgcttgctg gaccgtctcc tggatcatga 240

ccgtgtggtt ggagctgggc tcctcctcgg tgacctgcgg gaggagggca agagaaaggt 300

cacccagcgc ctacttcccg gtccagcgta acaacaaagt cacaactaca gtttaggagg 360

caccagtg 368

<210> 248
<211> 342
<212> DNA
<213> Homo sapiens

<400> 248
gtgacctgcg ggaggagggc aagagaaagg tcacccagcg cctacttccc ggtccagcgt 60

aacaacaaag tcacaactac agtttaggag gcaccagtgc gctgcgggag cacgtccatt 120

ctccacggat cctctctacc aggctgtgct tgctctcatg cccattttac agctgaacag 180

aatgaggttc agcaatgtag ggcaacgtgg tcagggtccc cagccgtggc aaatggtgga 240

acctaagttc aaacccagag tccgaggttg tttctgatgc cgagctgctt cccggggctt 300

ccaggaggtg tggggttacc tagagaatga gggctggaag gc 342

<210> 249
<211> 303
<212> DNA
<213> Homo sapiens

<400> 249
gacagccgtc gtcctcggga ttcctgtttg aggagacagc cgtcctcccc ggggtccctg 60

tttgaggaga cagccgtcgt cctcgggatt cctgtttgag gagacagccg tcgtcctcgt 120

ggtccctgtt tgaggagaca gccgtcgtcc tcggggttcc tgtttgagga gacagccgtc 180

gtcctcgggg ttcctgtttg aggagacagc cgtcgtcctc ggggtccctg tttgaggaga 240

cagccgtcgt ccccggggtc cctgtttgag gagacagtcg tcgtcctcgg gattcctgtt 300

tga 303

<210> 250
<211> 442
<212> DNA
<213> Homo sapiens

<400> 250
caatgaacgc ggttccaggc acgagatgtg gaggcccaga ggcagatgcc gggtggcggc 60

ggcgcgggac tgtcggaggc gcgggcaggg acctgggctc ccaaggctgt ggccctcccg 120

ccccagtcca gggtccagtt ccaggggcgc gtctggccct ctgttgctgt cttcttaggg 180

ttctctcatg ggaatcggtt cttcagcctt tcttttctcc ccagagaggg tctgtctact 240

cgttgtaaga gggaacaagt ccacattgca gctgtacact ttgcacccat gatgaaaagc 300

gtcacttttg cagcattcca gctggagatg cgtgaccttc ggtctgcggc aggcggaagg 360

gcgagagact tggacaggag gtgcatcttg cccggggaag gctgaacgaa tgagagctcg 420

gtccctggtg agaaagagcc ca 442

<210> 251
<211> 328
<212> DNA
<213> Homo sapiens

<400> 251
cgttttccct ccttccgttt cccggagccg cctgcgtgaa tgttcttccc ggctctgctg 60

ccccctggag gctagtggag gtaaatgcgt gaagtcggcg ttttccctcc gcctgttagt 120

cctcctcatt catctctcgg ccaaaccaga aagatgtgag ggtatttcaa agatatcttt 180

gctatatttt atttcactca aattcaagga tccctctccc aggacagctg tgactacaga 240

ccatgggatt cacatcctcc caccccacgg cgcaggaccg gcaggggatc tccaagaagc 300

ccctagccta tgggtcgctg agaccctc 328

<210> 252
<211> 804
<212> DNA
<213> Homo sapiens

<400> 252
ctggggtttt tacgtctccg aatgcccctg ccctttggtt gccactgtcc ggagcgtgtg 60

aactgggact ggcctttccg ccctctgcga ttggttagcg gtgcgatcac gtttaggttg 120

gtcacatggt tccgcggact cttaagtggg cagcatgggc agtgttattt tcaatggaaa 180

gtaggagact ccacaggctc ttacgggctg ttctctcaaa tgccttcccc tgcctggact 240

ctttgaagag gacgaatgtg tagtgaacat ggaatctgat gtgtaacaag cctgagagcc 300

tagaaaagct ctgcttacaa gagtttccaa acgctagcat taatcattaa tagatgagta 360

atactaactt attaaacgat ttctcagtat agtgccatga cttgcattat gccttttgta 420

acatttcaaa acgctaaggt ataccagaag gagggacaat tatccaggaa ggtgagagaa 480

aagtttcgtt tttgctgcat gtcccttcca ctagaggtta actttacctt atagaatgct 540

aggaatgcac ctaaactact gacatgagtt agtcaaagcc agatgttcac cttgtattgg 600

gcaggtgagg atccttttca ctttgtcaca tgtgaaatat ttgacatgcc atgaacaatg 660

gaaaaacaaa ttcttcaaag gagaggaagc agattcttcc agaactggag gaaatttggg 720

ctgaggcagt tattgaccct aaaccaatcc caaccggcat ctgtcttaag ttcaaagtct 780

cagggcatgt gggctgactt tcta 804

<210> 253
<211> 401
<212> DNA
<213> Homo sapiens

<400> 253
cagcccagca gggcgccatc gcggctgcag gagtcaaggt ccagcgcgcc ggagcggaag 60

gccaggcgga agacctgctc tcccttctga ttgcggatgg agaagccgcc agctttcagg 120

tccagcagct ccgcgcgaag tcgctccgcc ttgcgtaggg agacgctgta gtagcaccag 180

gccaccaccg cggccagcac aagcagcagc cccagaaccg cggagcccag cagcggcttc 240

aggtctttgg aaggcttggg cttggcaggt gagaagttgt cgggcaggaa ggtgtacata 300

gctgcggctg cgatggcctc ggggttctga cgctatgcgt agcagccagg ccggcggcgg 360

cgggggtagg cctggctctt ctcctgaggg ttctggagca t 401

<210> 254
<211> 339
<212> DNA
<213> Homo sapiens

<400> 254
caatttccag aatgccattc ggagcggagt tacgaagcgg aagcgaggcc gggggcgggg 60

gggatccgat gcgcgccgct gccgctgcgt gggggtgagg ggatcaggta cgctgtccgc 120

tggttaaggg ggctggctgg gggctgcggg gtctgtcctg ggacgtccgc ctccctcgcg 180

ggtggggcgg acgttgagaa agcagcgttt agcagtcatg tggcctgtgg tactccacct 240

tcctgatact ttcccttcct gcttccactc gcacgctcag cctgtgggcc gggagagacc 300

cgcaggcggg gtgctctcag gctgaattcg atgtgccat 339

<210> 255
<211> 429
<212> DNA
<213> Homo sapiens

<400> 255
tttcaaactt cagcaagagg acaaatattc agccagagtt atcagttccc ggtttctgct 60

tcgggtcctc tctggtctcc cacagcccct cctgcatcac ccaggtctaa ggggccacct 120

ggcctggcct gtgttccctc gtccctcccc ttccccaccc agatcctgca gcgccttcct 180

ggggcagggg cggcgaaccg tccagagcgg gaggctccat cgcccagggc agcaccgctc 240

cgcccctctc cacacctgcc cagcccctgg caagggagtc gcctgggcct ggcccgctgc 300

ccgcccccca atacctgccc tgtgcctgca acggcgacgc tgccaacaag aggtgccaga 360

gaccgcggcg caacccgccc ggagagcagg gtcccactca cctgggagcg gggcacgtc 420

ccttccggt 429

<210> 256
<211> 375
<212> DNA
<213> Homo sapiens

<400> 256
cccggagctg tgccccctcc cggcgaacag cctgcgccac ccgccgcacc gggaggtggc 60

tgggggctgc cgagcgagtg gtgaacgcga tgggtggaga gcgcccgaaa cgaggtggaa 120

aagggggcgg gggcgaggca cggcgccttc ttgcccccttc ttcagctaca gacgcggcgg 180

cggcggctga gtccctcagc ctcggtcatg tgatagtctc gaagcgctag ctgcgggggt 240

ctcggcgtct cggggaccat tacaaaacgc agccaggaga gcgcgtcgct gccactgccg 300

tcgcctctcc tcctcaagac aagcccggga gaaaggcggc agctgcggcg cggacggctg 360

agcctccccg gccag 375

<210> 257
<211> 591
<212> DNA
<213> Homo sapiens

<400> 257
acgtagcctt ctgccctgtc ccttgccaga tggcgccctc ggaaccgccc ggagaaacca 60

ggtgtggttt ggcctccgag tcccccccac cgcctgctgt gggcacagca ctcagccgcg 120

agggggacag cgggtgggca gcaagactgc ttcctttgcg gcttcagacc ctgttcgctc 180

ctaggttcct gtggtccacg cgccgtctcc acacccactt cctatacttg agttgatggt 240

tagaaccttg tcgtcaccct gcagaagtac agtgccttga atgccagctt ttccgttccc 300

tgatgaaaag atatgttaaa aaaaattatc ggaaaaggtt tcatttgcaa ttggcttgtg 360

cattgataat ctttatttac tgttttaagt tgcagagatg tgaatggttt acaaatctga 420

agctgaagtt caatctttgg ttttctgttg taaatgcctt ttacaaacat tgaattagct 480

accttaagta ttgaagagct tccattgcta ggtgagccct gctttgtcct cagtagagtg 540

ccggttccct gggctcatcc aggggctgag agacggcggg acgctggggc a 591

<210> 258
<211> 273
<212> DNA
<213> Homo sapiens

<400> 258
cgctgaagac gcccacgggt gctcgaacgc ccgttggtgc caggctgccc ggcctttagg 60

gccttggctg aggacacccc tttcctcccc accagacgga gcgaccgccc tttgccaacc 120

ccaagcaaag ctggggtccc aaccttacgt gcgctaccgc cccctccagg catttgacct 180

ccccgcgtcc ccattcttga tgggccccgc cctagaagtc tcccggggtg ggagcactat 240

cctcaggcca gggcggggcc gcgcgcccct gga 273

<210> 259
<211> 457
<212> DNA
<213> Homo sapiens

<400> 259
ggagccgtcg gggcttctcc accgtttgtg cctggacgca gaggcctccc ggctgtgccc 60

gctggaagct gcgggggaag aggaggtcga cagtgagtgg gccatgaccc gtccgtagac 120

ggctgcggga gggttgcaag ggccaagctc ccagagtgac ttcaaggcag gaatggctct 180

gctcccaggt gtcccccacc ccctcctctt tctccagctg cccaagacct ccaggcccca 240

ctcccctggt cccttcctgt ctcctcactc tggctgttcc ccagctgcct ggcaccctcc 300

agcaaaccct cctccgcggg cgcacccaga ggatggccca gctggcggag ccacccagca 360

cacaaaaagt ctgcctttct ttgaccttag gtttccccac cacctgccgg atcagggccc 420

gtggggcggg ggccgctgcc tttgtcaccc cagctct 457

<210> 260
<211> 457
<212> DNA
<213> Homo sapiens

<400> 260
ggagccgtcg gggcttctcc accgtttgtg cctggacgca gaggcctccc ggctgtgccc 60

gctggaagct gcgggggaag aggaggtcga cagtgagtgg gccatgaccc gtccgtagac 120

ggctgcggga gggttgcaag ggccaagctc ccagagtgac ttcaaggcag gaatggctct 180

gctcccaggt gtcccccacc ccctcctctt tctccagctg cccaagacct ccaggcccca 240

ctcccctggt cccttcctgt ctcctcactc tggctgttcc ccagctgcct ggcaccctcc 300

agcaaaccct cctccgcggg cgcacccaga ggatggccca gctggcggag ccacccagca 360

cacaaaaagt ctgcctttct ttgaccttag gtttccccac cacctgccgg atcagggccc 420

gtggggcggg ggccgctgcc tttgtcaccc cagctct 457

<210> 261
<211> 644
<212> DNA
<213> Homo sapiens

<400> 261
cgggttggag gggttccgct ttctccctgg gtccctgccg ctctgtctcc ggcaagtgat 60

gagcaatttt gcggagtggc agaacagaga caaaggtagc tgtgggcttc acggtaattt 120

caaaggaagt gccctacttc gttgcccatt tcgaaggcct ctctacctct gcgttggacg 180

atttctccac ttcctggtgt gcacctgcgt gtgtgcgtgc ctgtgtgtgt gtgtgtgtgt 240

gtgtgcgcgc gcgcgcgtgc tggggtgtgt gcacactatg ccgagagtcg ggcaaatttc 300

gagtgaaggg agaccgagtc ccgcaggttc tcttgctcct gcagtctgcg gaggaaaaac 360

tcctttctgg cacggcggca ggaatccatc taccgacgtt aggaaaggac agggtgtgct 420

ttcgtcccag cttagttttc tatggagaaa caggcagcag aggagagtgg gccagagggc 480

cacattttgc ccacgaccag ccaggtttga gttggttcct cctaggcctc acggtctgat 540

tttctcctcc tctttgtgta cccgacgggt gttgctgtga gctcaggacc atcccggcgg 600

gctgtggatg ggatcatcgt gtgtgtatgg ccacagccct ccgc 644

<210> 262
<211> 457
<212> DNA
<213> Homo sapiens

<400> 262
ggagccgtcg gggcttctcc accgtttgtg cctggacgca gaggcctccc ggctgtgccc 60

gctggaagct gcgggggaag aggaggtcga cagtgagtgg gccatgaccc gtccgtagac 120

ggctgcggga gggttgcaag ggccaagctc ccagagtgac ttcaaggcag gaatggctct 180

gctcccaggt gtcccccacc ccctcctctt tctccagctg cccaagacct ccaggcccca 240

ctcccctggt cccttcctgt ctcctcactc tggctgttcc ccagctgcct ggcaccctcc 300

agcaaaccct cctccgcggg cgcacccaga ggatggccca gctggcggag ccacccagca 360

cacaaaaagt ctgcctttct ttgaccttag gtttccccac cacctgccgg atcagggccc 420

gtggggcggg ggccgctgcc tttgtcaccc cagctct 457

<210> 263
<211> 644
<212> DNA
<213> Homo sapiens

<400> 263
cgggttggag gggttccgct ttctccctgg gtccctgccg ctctgtctcc ggcaagtgat 60

gagcaatttt gcggagtggc agaacagaga caaaggtagc tgtgggcttc acggtaattt 120

caaaggaagt gccctacttc gttgcccatt tcgaagtcct ctctacctct gcgttggacg 180

atttctccac ttcctggtgt gcacctgcgt gtgtgcgtgc ctgtgtgtgt gtgtgtgtgt 240

gtgtgcgcgc gcgcgcgtgc tggggtgtgt gcacactatg ccgagagtcg ggcaaatttc 300

gagtgaaggg agaccgagtc ccgcaggttc tcttgctcct gcagtctgcg gaggaaaaac 360

tcctttctgg cacggcggca ggaatccatc taccgacgtt aggaaaggac agggtgtgct 420

ttcgtcccag cttagttttc tatggagaaa caggcagcag aggagagtgg gccagagggc 480

cacattttgc ccacgaccag ccaggtttga gttggttcct cctaggcctc acggtctgat 540

tttctcctcc tctttgtgta cccgacgggt gttgctgtga gctcaggacc atcccggcgg 600

gctgcggatg ggatcatcgt gtgtgtatgg ccacagccct ccgc 644

<210> 264
<211> 457
<212> DNA
<213> Homo sapiens

<400> 264
ggagccgtcg gggcttctcc accgtttgtg cctggacgca gaggcctccc ggctgtgccc 60

gctggaagct gcgggggaag aggaggtcga cagtgagtgg gccatgaccc gtccgtagac 120

ggctgcggga gggttgcaag ggccaagctc ccagagtgac ttcaaggcag gaatggctct 180

gctcccaggt gtcccccacc ccctcctctt tctccagctg cccaagacct ccaggcccca 240

ctcccctggt cccttcttgt ctcctcactc tggctgttcc ccagctgcct ggcaccctcc 300

agcaaaccct cctccgcggg cgcacccaga ggatggccca gctggcggag ccacccagca 360

cacaaaaagt ctgcctttct ttgaccttag gtttccccac cacctgccgg atcagggccc 420

gtggggcggg gaccgctgcc tttgtcaccc cagctct 457

<210> 265
<211> 642
<212> DNA
<213> Homo sapiens

<400> 265
cgggttggag ggggttccgct ttctccctgg gtccctgccg ctctgtctcc ggcaagtgat 60

gagcaatttt gcggagtggc agaacagaga caaaggtagc tgtgggcttc acggtaattt 120

caaaggaagt gccctacttc gttgcccatt tcgaagtcct ctctacctct gcgttggacg 180

atttctccac ttcctggtgt gcacctgcgt gtgtgcgtgc ctgtgtgtgt gtgtgtgtgt 240

gtgcgcgcgc gcgcgtgctg ggtgtgtgc acactatgcc gagagtcggg caaatttcga 300

gtgaagggag accgagtccc gcaggttctc ttgctcctgc agtctgcgga ggaaaaactc 360

ctttctggca cggcggcagg aatccatcta ccgacgttag gaaaggacag ggtgtgcttt 420

cgtcccagct tagttttcta tggagaaaca ggcagcagag gagagtgggc cagagggcca 480

cattttgccc acgaccagcc aggtttgagt tggttcctcc taggcctcac ggtctgattt 540

tctcctcccc tttgtgtacc cgacgggtgt tgctgtgagc tcaggaccat cccggcgggc 600

tgcggatggg atcatcgtgt gtgtatggcc acagccctcc gc 642

<210> 266
<211> 437
<212> DNA
<213> Homo sapiens

<400> 266
actctctcca aaccacagcc ttaccttgac ggcctccgcg tgggtcaccc gggccaggga 60

tttgtcgttg acgcgcagaa tctggtcccc gacccgcagt ccttccttct cagctagaga 120

gcctggttcc accagagaca cgtagatgcc cacgccgtgc tccgagcccc cacggatgct 180

gaagcccaag ccctcgtggg ccttggcacg ccgcaaactc accaggcgca cctcccctgg 240

ccccgcgctg tcggggccgc cccaggcggg ctgcctgtag gggtggtgg cgggcaggta 300

gaggccctcg gccgtgtatt ggtcgaagag cagctggtcg gagcgcggga tgaccagacg 360

aagcatgggc agcaggcgcc gcttgaccgg actgtccagc agcacgcgca gggtgcgcac 420

caggtcgaag acgttgc 437

<210> 267
<211> 528
<212> DNA
<213> Homo sapiens

<400> 267
agacctcaca ggtgtgggca cacctgcccc cgaccctcct gcctgcatcc gggcccctgc 60

tgcaggtaac gggtggcaga ggttgctggg tgcgggagca ggcagctgaa tacccgtcct 120

ggggaggcag ctgagactct gtgctgccag catgtgctcc attattccac cagactccac 180

ttaacacaga ttcaaagatg acatgaagca tttcaggaca gtgaaagcac agcatgaagc 240

cctacgcttg gggctccctt cttagggcag ggccaggtca cctggacatg aagccagcct 300

gatgggaggg gccatagcac ccacacacac atttcacaca gagcaagggc cttatgttgg 360

ttctctccac agagctgggg ccagatctga tttgaaggga aagttctgtg ttaagcactg 420

tctgcagact gctggcccag caggttagtc ggccagccag ggctctccc tcttcatccg 480

gagcctctgg tgccaaagac acaccatggt cagccctccc tgacttaa 528

<210> 268
<211> 137
<212> DNA
<213> Homo sapiens

<400> 268
cttccgcccg ccgcttctcc tcagcctcgg cggcggcgga agccggagcc gggcgcccgt 60

cctcatcgcc tcagccgcgg gccctgccgg ccgggccgcc ccctccccgc gcgggcgggg 120

agcgcgcggc cgcctcc 137

<210> 269
<211> 505
<212> DNA
<213> Homo sapiens

<400> 269
atccaacccc aggcggtggc aacaggacgg cttgggggca gccaatggcc gggggggcggg 60

gaacctgtgg gtagagtcag cgctggaggg cacagggggt tacgtgcagt cagaggctgg 120

gtctgagtcc ctgctcccca catgttagct gtgtggcctt ctccttcctc ggtttctcat 180

ctaggaagga ggataagagc tgcccctcag cgtccttctg ggaattaaaa agcatgcgaa 240

gggcccaaca cggcgcctaa cgcacactga gtgctccaga gatgctgggt gtgggcgtca 300

ctgtctctga cccaccagat cttgggaacg ccccttctcc ttcctgggcc tcggtttcac 360

cgtctggcta gtgagtgtgg gtgcttcggg ccgcgagttt cggctgggat gggggcccaa 420

acctgcttag gccactcacc tctcggagat gcggccggcg gggtagaaga cgctctgcat 480

gacgatgaag tacgtctggg ggccg 505

<210> 270
<211> 542
<212> DNA
<213> Homo sapiens

<400> 270
gacatgggga ccttaggggt ggcagctgct gccctggaaa ggtttctccc ggcgggtgac 60

ctgccccagg ccttacaact agaaacgtag gctggggttg gaagcagatg cccgcggcaa 120

ggctaagcgc agtctccacc tccaaccccg cccccgcagg gcaaatccag actgggtgac 180

ttggcccagg ggcacatcgc tcccctgtgt ctgctgggct tccctggggc atggatcccg 240

aaagtccgct ggagggacca tgaaagggca ggcctctggc ttctctgggc ttcagtttgt 300

tgaactctaa aacgggctag cgtgggagcc tgcagagaag gcgtgtgggg ctctgcggga 360

gaaggacggg cagcgcgtgg gcggaactgc aggggctggc agggaggtct gagttacgcg 420

gatgggccac gctccgaccg cacccccgag tccgcctgtc cgtgtgtctg tgccgcccgc 480

aggacgtgct tccggcgctg ctcgcaggac tgcggcacgg gcggcccctc tacgctgcag 540

ta 542

<210> 271
<211> 393
<212> DNA
<213> Homo sapiens

<400> 271
gccctgtctc tgagatgatg gactgtgcac gtggccaccc ccacagcccc ggagcctccc 60

ttgaactctt ctctgcaccc tgctgaagaa gcatctcctg gggacggtgg cagccagggg 120

agtggcagag gaagagagga cgggtaggcc ccatctccct gtcccaggtc tggaaatgac 180

gcccgcgccc caagcccttc tctcctgccc agcttcgtct gtgtcgtctg tgtcgtagaa 240

aggcctgctc agaattcaca gcggctcagg ggctcatgaa ttatgtacgg ctccagatca 300

atccgtcagc gctctgtcag ggaggaatca aaggccccgc ggccggcaga cagctctttc 360

tgcgtggggc cgccgcggag agcccggcgc cgg 393

<210> 272
<211> 475
<212> DNA
<213> Homo sapiens

<400> 272
gccggcagac agctctttct gcgtggggcc gccgcggaga gcccggcgcc ggcgagttgg 60

tggagccccg tgggcgctcg gctccccgtc ccttccgaat gccccgtctc tctgtttctc 120

tgccagacgc agatttgaca aatgtcttta aaggcggggt gggggtaaca acaaaaagcc 180

ctggcccgtg tgttttgagc gcgttccaag gtgctcggcg tgaataattg atgccagcgc 240

cacttttgtc atgggaaggt caccctgggt gcggtacagt ctccgccacc acaacagcac 300

aagcttcctg cagcactgat gagacggccc ctcctcggcc aggccaccat tctggggctg 360

ggcaagctgg ccatgaaggg atggcgacct tgctcctgag aaggggcgtc ctgctctgct 420

ctgtccgggc ctccaggccc agaagcgagg gttgaggggg gagccgcggg gagcc 475

<210> 273
<211> 736
<212> DNA
<213> Homo sapiens

<400> 273
gctgccccgg ggagcgggga ccaggcaggc ggcagagcgc aggcagcccc gggccgtggg 60

aaccagacag ggctggactg ggtggcgggg caggcctgag gacgcctgga ggggctcccc 120

acggtccgcg gctccctttg gacccgagct gggagctgct atagcagtct taacaataag 180

acagcagtaa tagaagccat cggttactga gcaacctcct gggccaggta ccatgaaaca 240

ctctacatac agcgtcggga ggttttgcag gggaggaaag agaccagatg aggtgaggga 300

cttacccaag gtcacacggg aagtggcaga gctgggagtc cgactcagct ctgagtgaat 360

tagtatgcca gctacccaat tctcctccat gacccctcgc cccccagcca ccagccgtca 420

tcttagtccc catgataaac cagagcagag ttgcaatgac acagaagaga ctttttcata 480

gataaaatag aaacatataa cttaactcta ttgtgtggct ttttaaatta taaaagtaaa 540

acctcaatat cgaaaaaagg gcccccatct ataaccacac atcctcacac atcacagtta 600

tggggctggg tttccttcca gtatcttttt caatgcattt tttttttaac gtggttgtaa 660

cagtagtcca tgtagaattt tgaatccggc tgtggttttt tttttttttt tcacttgaca 720

ttactttata cgcctt 736

<210> 274
<211> 320
<212> DNA
<213> Homo sapiens

<400> 274
caggcccggg acacagttcg gatctgcccg cggtcctggg ccccaacgcc gggggctgac 60

cctagaaagg cgctggactg gagcccgtga cggggtggga agggcgaggg ggcgcatgat 120

tcacaatgaa aaggccacgt ttccagagtt gccttgaaaa tgcatccctg gacgagctct 180

ggctctgcgt ggaataagaa gccgttagca ggcgtctaca ctcgcactcg gaagctcttg 240

cgtgggtcct gttcagaggt acaacagcac cggccccccca agaggtgccc atgggagagg 300

ccataagtgg ggggtgggcg 320

<210> 275
<211> 574
<212> DNA
<213> Homo sapiens

<400> 275
atcaggagac agtgtgggca gccagtgccc gccgtgccag cgccccttcc ggcgcacttg 60

tgtgcaggtc gctcgcggtt acctgccgct tgctggtctg cgggaggctc ttttgaaagt 120

ggcgcccttt ggctgctcag cctggcgttg catgggggac atggtggcca cagggccttc 180

gtgtattggg ggtgaatcca ggacctggga ccacagagag aggagttctg gctagaatct 240

cctggaataa agagcccccct tacaattcct gggactcatt ggattgaggg actgccccca 300

ggcaggctga agctggggac cccgaggcct gccagtgggt agtggagaag cctgctgggg 360

ggatgctaga gccctgggct ccctgcaggg gctctgagcc cgatgcaggg cgagggttcc 420

gttggccctc agaattgcgg gtgactcctg tcttttcccc tggcttgggg ttcagtaatc 480

caggaggcct ggagagcttt tggtgtgctg cccacctcgg agcccggatg tggcgagtgc 540

tgcctcaccc cggccctgcc aggcatctta tctg 574

<210> 276
<211> 426
<212> DNA
<213> Homo sapiens

<400> 276
ggagctcggc cttgtggggg agccccacac gtccccccag gcaggcaccc gggtcctgat 60

ccgtctgagc tttaggtgaa acccccccttc cctctgtgca tcctgctcca tctcctaggc 120

tcaccttctg tcgaggaggg ctgagcgtcc cccgcggagt cgagcacagc agggccagga 180

tcttctcagg gacccccccgt gccagctgca cgcacggctc tccgcagggc tggtgaacct 240

gttccctgga ggaatttaca caccacgggc tcacacggcc agtgccgatg tgcactgctc 300

cccttacgtg acatgggacg gtgacaggtg ttcggccgcc tgctgtcttt gcacgtccat 360

gtgccctgag tgtctccggc cagggccacc tggcaccagc tgctgtctgt gccacagcac 420

cggttc 426

<210> 277
<211> 1432
<212> DNA
<213> Homo sapiens

<400> 277
tgcctcagtc tcccaagtag ctgggattac aaatgcctgc cgccacgccc ggctaatttt 60

tgtattttta gtagagacag ggtttcacca tgttggccag gctggtctcg aactcctgac 120

cttaggtgat ccacctgccc ttgcctccca aagtattgag attacaggca tgagccactg 180

tgccctgccg aggtggaagt ttggagatgg gccacaaact cctggagata gggccagctc 240

agtcccccctg agcacccaca cacaccctcc tgagagccaa cagaaggagt gagggccccg 300

aggcggatga cccgtgtgcc tcaacccgaa cggggagagg cggggtctgc agcagggtac 360

gggcaggtga tcccccaagg aaagattttc ctgtattgag agagaagggg ccaagaggag 420

gagcttgtca aacaccacag cccctccccc tcctctcagc tccagggggt ccctggtgcc 480

agtgttcggc tgatggagag aacggcaagc gggagagaga gtgtgacccc tgtgggcaca 540

tgacttccct tgctgcactg ctgcacatag cagaggtgtg gtgacgaccc tgttttgtcc 600

cattgggggc gtttgctgtt aggtctgcag aatcctcagt tgctattgga aatggtgaca 660

tcactggcag gggcggagct tcagccatcc ttcaagttag ggaggggcac gcacactcca 720

ggggtggagg gggacaaaga cagggtggtg tggaccagag ggatgggtaa ggctctggaa 780

aagggggcgc tgggagcgca ttgcgagggg gctggagagg gagagaggag cggaagctga 840

gggtgtgaaa cggctggccc cgaacacacc tcgcggcgct ccagtgattc ctggtgtccg 900

acctcagccc cagtcagtgc gggtccagtt tccaggctct cgcggaaggc ctggctgagc 960

acatgcggca gccacggtca ccctccctat tcctcttagc ccgaggaggg gggtcccaag 1020

ttacatggcc acgcagatgg ggcctctccc tcatttctga accttgtggg gaggggaacc 1080

ttgaagggag cgcccccccag agccatggct tagggcctcc cccacccctc tggagctcca 1140

gtctgcaaga gtcaggagcc gaaatatcgc tgactgtggg tgacgactct tgcgcgcaca 1200

cacacataca agcgggcacg acgcgttcgg tcctattaaa aggcacgcaa gggtgcgggc 1260

tgcacgcggt gacacggacc cctctaacgt ttccaaactg agctccctgc aggtccccga 1320

cagcacaggc ccctgtccca ggacccctcc aggcacgcgc tcacacgcac acgcgcgctc 1380

cccggctcac gcgcgctccg acacacacgc tcacgcgaac gcaggcgcac gc 1432

<210> 278
<211> 625
<212> DNA
<213> Homo sapiens

<400> 278
agcacacaga ccaccaaggg gaccacaggg cacgaccgcc ctctgcgccc ggctaaggcc 60

agggccaccg cgggaggcac ggtgcgctgg agaaccgtcc tggcaagcga ggaaccgctt 120

ctgtttcact tttgtttggc aaggcaagaa agttaaaacc aacagcgtcc gtggcctaca 180

gtcagacccc accttctgca gcacatgagg gcacctgcag aggggaactc acagggacag 240

ggcgggggca gctgtcgccc catggctctg cgagaagaga atgagggagg gggcgcaggc 300

catcagggga tctacccagc actgccctga agcctctcgc cccagcattc cagcccagcc 360

ctctgctccc cttctctcgg gttcctcggc gtctatccct gacttaagcc tctgctaagc 420

aaggtccaca gaaacatgtt cactgttaca tcactgtctc aaaaatcaag gcaagaaaaa 480

cacgaaatga aaacaaaact accaatagca gaatctccac accgtttcct gcagcctgag 540

cctcaggaaa ctttccttac tgagtcaaca ggacccggac gggcctcggg aagaacagaa 600

tcggggtttg tgcaggtgtg gacat 625

<210> 279
<211> 854
<212> DNA
<213> Homo sapiens

<400> 279
gaattcagac ctttgtctga ttggtcctct gcttgttggg ggaccagccc ggtccacaca 60

cagcagagtc agcactggtc agtgggagag gttgcagggg gctctaaaaa gagtttctgg 120

cctccatttt ttctcatcat gaaaaatact gtattcagag ttgttaacat tctacaatgt 180

gttaaagcct aagacccagg tcccttcagg aaaatgacac ggtagcttgt gctcacatga 240

gggtggaaat gacaggtatg caatgctgga aggcagaggc aagccgcagc tctgtctggg 300

gctctgctcc gtgcgggctg ccttctgtcc cttggggttc cagtttctcc atagcagaga 360

ttctagttta actcgtatct tttgatgcct gttcaaagtg gaagattata ggaactcagg 420

ggagttttct atgttgcagg agaggtgaga agatcaaagg ggatgaagaa aagggaccct 480

gggctcccaa cgccagggtg cctacctgca gtttaaccag ggcagacccc acaccaagcc 540

ttggtctgag aagcccaatc tgctcctcaa aggaaagaat gcatttactt gggaaaatct 600

gccctgacta aatatgtatt aattttcaat ggaaagggaa gtatgaatat gtttgcctcc 660

ttcctggtca gaatctttca gccagggtct gtgctggcag acaacaagac acaaggccct 720

ttagtcaaga cgccaatcaa gaatttgcag gaaaagctac aagaagaaga atgttcaagg 780

agaggcatgg gctttggggg cagccggggt cctgggagac attcaggttc aagtcctagt 840

tttgctaaat tcaa 854

<210> 280
<211> 625
<212> DNA
<213> Homo sapiens

<400> 280
tacacataaa atgatgtgct cagggtccac ggcgctgtga gcactaggcc gggccgcagg 60

tgaatcgcag cagccacacc taaagacagg gcagctgaac tggcctctgc tggcctggcc 120

tctcaccttc tcccagcagg tgtccactta gctccctggg ttccctgggg aactgtaggc 180

aaggtctgac cagaaagtac agctcttcca gcagagctct ctgcactgac aagtcccaga 240

acaggccttt ccccttcaga gacactgtcc actctagcgg catctcccct cacaggctca 300

cacaccacac agctccagga agccaccaac tcatcccacg ctaggaaaag gcaacgattc 360

ctcactcaga tccacccctt cctacctgta ttgtatttag gcatctcttc cccaaaagaa 420

aagcctagct gtccagtttg gacccaaggc aggaatgaca aggtcacctg tagatctgtc 480

tgtaacaagc ctgtaggttc caggcccttc caaggcaaag cggtgctcaa cagcaacctg 540

aatgggtata tgaccacctc agggagagca gaggccggac ttctgaaaca agggcaggca 600

cacggatgca gccaggcaga gagca 625

<210> 281
<211> 411
<212> DNA
<213> Homo sapiens

<400> 281
gcccagcagc agcgggtgca cagggagcgc caggactcca gcgtcttgcc ggaccagacc 60

cacacgcccg aggtgatgcc caccactagg cacatgaagt acttgagcat gaagacggcg 120

tagtcgggcc tgcgtgcctg gtcgggctgc aggtcccgca ggcacgggca gttgtgcgtg 180

gcctcccagc gcgggcggtt gtgctgctcg tagaagaggc aggcgaccac caccgcggcg 240

ggcacggtgt agagcacggt gaacaggccc aggcggatca tcagcttctc cagcttgtgc 300

gtcttggtgg ggccgtcctg ttgcttgatg accgagcgga tgcggaagag ggacacgaag 360

ccggccagca ggaacatggt gccgatgaag aggtagatga ccagcggcgc c 411

<210> 282
<211> 705
<212> DNA
<213> Homo sapiens

<400> 282
actggtaatc cggagactca cagcggtgac ctgtcagcgg acgcggagcc gggacggagc 60

cacggccgct cagattagca ggatctgatt gacagcatcg ggtagcaga cgctgagcca 120

gggactcagg aatacagggt tcttgcaggg cgtcgcggaa ggaaacgaag tttttgggag 180

aaatccgttt gataaagcct tggcggcttc gaggtcatgt gttgagcctc tggctgcatg 240

aacaaaagcg aagcccgctt tcatggagct tctgtgaaga gcaacaggaa cacaggcagt 300

ccagtcgtcc tgagatactg ggaggagcat ggttgctttt gaacacgtag gagataaagc 360

ctctctaata atgcctgttt tttttttcc ttcactctgt ctcccaggct tgagtgcagt 420

ggcacggtgt cgcctcactg caacctccgc cttctgggct caagtgattc tcctgcccca 480

gcctcccaag tagctgggac tacaggtctg tgccaccatg cctggctaat ttttttgtat 540

ttttagtaga gatgaaggtt ttaccatgtt ggccaggctg tctgatatgc cttaagcctc 600

ttttaaacgc acttttccag gatcggggcc acatcctgga gctgttctgc ctggccgggt 660

gtatctctgg gtccctgcct ctggagcccc tctccgtaga tgtgt 705

<210> 283
<211> 458
<212> DNA
<213> Homo sapiens

<400> 283
accagccgag cctcagtttc ctcgcctgta caaacaaagc agaaggcgcc ggcggctctc 60

agtaaaagcg aatgtagcct ttgtacttcc gacctctcaa tggtgaaatg agctaatcac 120

aggcccaccc cgcggagtgg gacgggagat tcaatgagac ccgccccacg ccgcgaaatt 180

ctctgcatcg tgtctgacaa gcagacagta ctcaataaat ggtagctgta agtaagtcct 240

ttaaacctaa gtacttactg ccttacccaa gaaaagctcc gtgcgaccca aagagctgag 300

aactccccca ccccgacctc cgactcctag gacagtacta ctggcgtctg ccttctgggt 360

ccagaaaact cttcccacgg agccgtccct cggcggtgct tagccctccg gatacccccg 420

agacatccca gatggcccca gtccggtggc gaagggag 458

<210> 284
<211> 791
<212> DNA
<213> Homo sapiens

<400> 284
cagggccatc tgagacactc cagctggcct tggatgctgt gatacaatcc ggtctatgtc 60

aggacatcta atcacacccc tgctccaatc tcagcttcca cccctggatc tggcctgaac 120

ccaccaagaa tgaagtctgc cagcagggaa tagtgaagcc atgcagtgct ccgtcatgct 180

gggccttcct attcacttca ccttgaagcc tcgcatcagt ctcacttccc agatgagtca 240

gggaggttga gtaatgaagc catactgcac catcaaggca ggaagtgaac acatgcctac 300

ctcactcaaa gcccatgctc cttctgcagc cctaagtgaa aatgccaacc ttccccaccc 360

agatgccaag tctctgagca gtacccatgg gagcaaacat gggcccagtc tctggaccag 420

acgcccccag gtgccttagt cctgtggggc caaactccac ttgtcacatg ggaattcaaa 480

atgcagggtt cctgctgata agtgtgagag agagggcact ctgaggccag cagccaccat 540

cccatggtgc ttctgtggag ctgagaggct ggatttttgg tggaggtgtc tctgggttga 600

gagcccaggg cctgtcctga gtctgagctg cccttggatg ggcttgagct ggtgtcttca 660

gacgagcccc acagtctggt gcatttcaca tcttaccctt ttgcctgcct gcttgggcct 720

ttggagctgt gcccagtgcc ccgggctacc ttccctgtcc cacaccacct gacacaaagc 780

ctctcacatt g 791

<210> 285
<211> 453
<212> DNA
<213> Homo sapiens

<400> 285
cccacccgga ctcccgaggt gtgggagccc accctgccgc tgcccactcc ggccaatgcc 60

agcgcctaca cggccaacac ctcggcgtcc ccgacagctg cgtggccagc gggctcagcc 120

cttcggcccc gctaccctac ggagagcgaa gacgtgaaga tcggggtgct gtttgcttcc 180

aaggctatcc tgcagctgct agtgaacccc ttgagcgggc ccttcatcga ccgcatgagc 240

tacgacgtgc cgctgctgat cggcctgggc gtcatgttcg cctctacagt cctgttcgcc 300

ttcgccgagg actacgccac gctgttcgcg gcgcgcagcc tgcagggcct gggctcagcc 360

ttcgccgaca cgtctggcat agccatgatc gccgataagt acccggagga gccggagcgc 420

agtcgtgcac tgggcgtggc gctggccttc att 453

<210> 286
<211> 1337
<212> DNA
<213> Homo sapiens

<400> 286
aagcatacga tggcggcttc cgagtgggag atgggcatgg cctggctgcc ggccttcgtg 60

cctcatgtgc tgggcgtcta cctcaccgtg cgcctggcgg cgcgctaccc acacctgcag 120

tggctgtacg gcgcgcttgg gctggctgtg atcggcgcca gctcgtgcat cgtgcccgcc 180

tgccgctcct tcgcgccgct agtggtctca ctatgcggcc tctgttttgg catagcccta 240

gtcgacacag cactgctgcc cacgctcgcc ttcctggtgg acgtgcgcca tgtctcagtc 300

tatggcagcg tctacgccat cgccgacatc tcctattcgg tggcctacgc gctcgggccc 360

atagtggcag gccacattgt gcactcgctg ggctttgagc agctcagcct tggcatggga 420

ctggccaacc tgctctatgc tcccgtcttg ctgctgctcc gcaacgtggg cctcctgacg 480

cgctcccgtt ccgagcgcga tgtgctgctt gatgagccac cgcaaggtct gtacgatgcg 540

gtgcgcctgc gtgagcgtcc tgtgtctggc caggacggcg agcctcgcag cccgcctggc 600

ccttttgatg cgtgcgagga cgactacaac tactactaca cccgcagcta gcatccccac 660

tcctcctcca gcccacccaa ccgccttggg tcaaggggc tgctctgcaa gcccactggc 720

cagctctggc tcagggccca cctcctccag cgagtacccc agccactcct caaccttgac 780

ttctgcccaa atcccctccc tgtgacccgt tccatatccc tttctctctt gtccaatggg 840

gcttggagca ccgaggccag cgaagccatc gcgctccttg cggaggtgaa gaggaccctg 900

agtccccacc tgcggctccc ctgtgtagag cctgcatctg tctgtccttc cttccattgc 960

tcccagtgcc aaacttgggc cgctgcaccg cggcgcctcc gcccaaatca ataaactgtg 1020

tctgtcccag gaggccgagt ctctttactg gtggggggtg cgtggaggcg cgcagggcca 1080

gagcagaggg gagggtgaac tgggtctcca agtcccaatc cagacctaag ccaaactaac 1140

acgtaggcac ctgtagctgt ttttctacct ggaaaagggg ataggaagga agcaaaccca 1200

acaaaggctg tcacccacgg tcaccaagga gcaccatgct cccctcagcc caggatagac 1260

cctcttttcc aggcctagcg cagagcccgg ggatgccgcc cgggggagcc tgaggacccg 1320

ctccagctag gcacgcc 1337

<210> 287
<211> 504
<212> DNA
<213> Homo sapiens

<400> 287
tgaagcgctg cttctccccc tccctccaaa acagccggta tccagcggcc ggccatctct 60

ctgacttaag tcacccagag cacatttggg aaagtttctc gctcgcgctg tgcgcttttt 120

tccaattggg gatgaaaagg acatggaaag aaatcgcagc aaggttagca ccctagtaga 180

taccccaccc cagcaaaaat gtgcaaagtc ctatttctca agaaagaaag aaagaaaaga 240

aaagcaagaa aagatagctg cgttcttacc acgtgcgcca gcccactccg cctgcgctct 300

ggcgggtccc gcggcccccg cgctgaccat gggcaagacg acgcctcgga agggagtccg 360

actcgcatct cgggccctga gtccgaaaga acgggggaaa gccgaattat gcaaatgcgg 420

atccctgcga tggggctcgg gttacggccc ccgccggccc cttaggtgag gcacccaccg 480

gcagcaagcg cgggcgaggc gact 504

<210> 288
<211> 829
<212> DNA
<213> Homo sapiens

<400> 288
tgcctcagcc tcctgagtag ctgagactac aggggcccgc caccacgccc ggcgtgtgtg 60

tgtgtgtgtg tgtgtgtgtg tgtgtgtgtg tgtgtgtgtg tatttttagt agagacaggg 120

tttcaccgtg ttagccagga tgaacttgca ctgatctctt taccaggctt ccccttaacc 180

ctctgcgcag aaattcgggg tttttctttg tgtttgagac aagagtctcc ctcggtctcc 240

cagactggag tgcactggcg caatcacggc tcactgcagc ctcaacttcc agggcacaag 300

ccatcctttt gcccagcctc ctgtgtagct gggactactc gggagtccca tctactcccg 360

tgcgctgcca tcacgcccag ctaatctttg tatttttgt agagacagca cctcactatg 420

ttgcccaggc tggtctccaa ctcttacgct caagcgatcc tccccctcgg cctctcaaag 480

gcgtaagcca ccgcggccag cctttttgg aacttaaaaa aaaagccata atgtgtcatt 540

aggaaccttg gtgacatgac agcaaaacca aaatgtactt atttattagg tgtcacttgg 600

aagcgcaatt ttcaacaacg caaaactata aaaaaaaccc aacgtcggcc tccacaggct 660

aatcagtctc cgaggcactg acggcgggag agatgaaaac gcgaaggcca gaggagaaaa 720

agaggaagga tttgcagaga gaacggtaac gtggtggaca tgaatacacc caagaaaccc 780

ggatcccgcg cggcacattg ggacgaagcg cgctggcggg cggccagca 829

<210> 289
<211> 725
<212> DNA
<213> Homo sapiens

<400> 289
acatccaata gagcccactt caacttgcat ggtgcatggg gtccaggacc ggtggccccc 60

acacagggtt tcccaaagcc tctccatttg tctgcactct ccactgccga ccttgtagga 120

aatttatttc catgcccaga aatattgttt ggacacagtg gctcggttat cttcgcgcct 180

gcttttctta aatacattta tttcacctct gccacgctgc agatatttaa agatctgtag 240

ataccgtaac cctcagcgat gcgggactca ctctggttat agataatatt ctctgggtgg 300

ttatttgaac ataagttcta tctccagccc agatgacacc caagcagggg ctgtaaagga 360

cacttgctct agaatgtttt caactgaaat atatgtccac acacggagaa tttaagagta 420

tttttatatt tctctctaga tctaaatatt cagatgtgtt aattacatgc cctagaagct 480

ggaagcgatc agtggtgttc acactggacg tggagctgtt tgtataattt tcatctccct 540

gcacttaaac atgactctca gtctaataaa ttcaaccttg tcatttttag aatcgacggg 600

atttctctgg ctgtcgtttg cgctgcattt atccgaatac atccagctcg caggcatcct 660

gcaagaaacg gctcccggct cgcgtgtacg ccgacacctc ggcccaacgc aggactcgag 720

gtggt 725

<210> 290
<211> 729
<212> DNA

<213> Homo sapiens

<400> 290
tcaatgccca tgggatttgt agccagtccc tgcccatcgc ccacgacccc gggaagcgcg 60

cacaactctc gcctttacct agatactccg ttccctcgaa gggacctcaa gtcactggaa 120

ttccccccag agcaactccc agacacaacg caggggggtaa acataagggg ttttaggaag 180

gggtctgagg agcacgtcct gcaagggtag aaaaggagcc tggagcttgg cccagctgtg 240

agcccttggg ccctcacttc ttcactctga gtctgtttcc tcatctcacc aataggaaca 300

ggagcatgta cattcccgta gagtagatac caggcccact ggggatcttg ttttgtaaac 360

gcgccagcca gggacaggaa gttgtgatca aaaggcagct ggaaggtctg ggtcagatcc 420

cagcccaggc ccagaagttc cagctctcca ccctccgcct cgcccgcagg gctcgcgctt 480

ccacagttct cgccgagagc gctatgggac agtgttcaag acgcacctgc tgggcaggcc 540

agtgatccgc gtgagcggcg cggagaacgt gcgcaccatc ctgctgggcg agcaccgcct 600

ggtgcgcagc cagtggccgc agagtgcgca catcctgctg ggctcgcaca cactgctagg 660

tgcggtcggc gagccgcacc ggcggcggcg caaggtgagt ggaaacggga atggaccgta 720

gatacgtcg 729

<210> 291
<211> 652
<212> DNA
<213> Homo sapiens

<400> 291
gatgagccca gatactgcgg cggcggcgct cgcttcgcgc gagccaaccc ggcacgggct 60

gggcgcacca cctgccgctg ctgtcgctcc actccgcctt cctttcttct cctcctacca 120

ctcgcccgcg cacctgaggc tctcaggtga gtccagcaat gggattgcga gcgtcagtgt 180

tctctgctgc gtcgccgacc tgggcgcgct cgaaggcaag tcttggcact tcggggaaga 240

agggctcgcc ctctccagga gactcctcca gctttcctgc tgcctcgatt taaggacttc 300

caaccttaaa tgatggccga ccatcgcttt tttggacagg cttccttggc ccccgccctc 360

cctcaattca gttctcttat tctctctccg tgcgccccgt catacttttc agcataccta 420

ccgtatttgt gcttttgcct ttatttgtgc ggcaattatt gggcgattat atcattgatg 480

tctggctccc tcactggact gtgggttcca gggggaagac gccgcagttt tgctcaccag 540

gggctccgat gtgcattcag taattgtgtg ttgaatgaat gagtgggcaa caggcttgtc 600

cccggtttgt ttattggaat atctgcttcc tgtgcaagcc tcctggctgc tt 652

<210> 292
<211> 377
<212> DNA
<213> Homo sapiens

<400> 292
ctgcccctgg aggagaggcg gcagggaggt ggttgggctc gcgggagccc ggggccaggg 60

aggcctccgt ggtagcggcg acggcggcgt cggactggcg ggcaggagga ggccgtttcc 120

cgccgtgggc ttgtcgctga gggctaacgg gaggatctcg gccctgctcg tgggggttca 180

gctgctgcgc tcgctgactc gcggggtgtt gggcctggga cgcttcctga cgctctgctg 240

cttgcctctg ccgtctgtct gtctcccgct ccagtggccc tctcaccctt cactgtaaca 300

cgccgtatag gtcaccccta tcagaaccgg acgcccccca agaagaagaa gccgcgcacg 360

tccttcacac gcctgca 377

<210> 293
<211> 398
<212> DNA
<213> Homo sapiens

<400> 293
cacaacgggg aagggagaga caaagccgag ccgcctctcc tggccagccc ggaaccgaaa 60

gtgggtccgt agagagccca gagtcctggg ggaacagatg ggcaaagggc tctaggaggt 120

gatggaactc aggcccgaag ggcaggcagc ccacaggata ccaagcctta acgaactacg 180

ccagaagaat gggcgtaggg cccacagccc ctcacacaac ccaggccaag ctccctcgta 240

cactcagacc aggagaagaa acagacacgc aatacacaag ccccgctccc ttggagccta 300

cacttgggcg aacacaccac tgctgtttgc agtatcccga gacaggcccg gcaggcgtga 360

gctaagaggc ctcgggcccg aacttgggat ttgaggct 398

<210> 294
<211> 435
<212> DNA
<213> Homo sapiens

<400> 294
atgtgtcaat cttctctgca caagaaagag ttcagccccg ggaatcatcc gggaagcgtt 60

gtacctcttc ctacatcaat gagtcccctg agatgtagag gatgtttaca ggcaccttgc 120

gagttcatgc tttgggcagt aatacgctta cattttgcct ggttatttca tctggtccaa 180

acatcgagag gttttattat ccctgattcc gtttgtggcc aatcatattt tccgcttttc 240

aagcatctcc ccgtaaatca ccacctgtca gacttgatgt gcattaagga agtcaatgag 300

ttgtatttca cttgtcatgt ttgaagaggt gcatgacact ctatggttac tgctaactaa 360

agaaagagat gacttttgtg cgcaccggct aattggtgtc aactcacatc accttgcatt 420

gaggtgacag gcaca 435

<210> 295
<211> 312

<212> DNA
<213> Homo sapiens

<400> 295
cgcagcggag gcggcgccgg ccagggactc tgcagggacc ggggtgcccc ggagggagtg 60

gtgctggtgg aggaggaggc ggtggctgag gggagtagg tggggaaaga ggaggaggaa 120

gaagaggagg aggaggaaga agaggaggag gaggagaaga agaaactgga ggaagaggag 180

gaggcgaagg aggaggagaa gccgcagcgg gcgccgcagg agcgagtgag ctgggagcga 240

ggggcgaagg cgcggagaag cccggccgcc cggtgggcgg cagaaggctc agccgaggcg 300

gcggcgccga ct 312

<210> 296
<211> 352
<212> DNA
<213> Homo sapiens

<400> 296
caccagccgc gcccctcccc catcgcctga gtcctccctt gggaataacc ggccacagaa 60

caacagcaaa agtagcaaaa agtgttgtcc tctcacctct gtaaaataaa cctaagcacg 120

gttagaacat acggtttatt taaacgcttc cgcctctatt tacggtcctc gcagtccacg 180

caggcagact caggagcgcg atcctacctg ggagtggggt ttggagagcg ggaaagcact 240

cgcgggaaga aggggcagaa tttctgctgg ccctcggtag tcctctgccc acgtcggtga 300

tccggtctcg cttctccgcg ggagctgaac ggatccaaat gtttcccagt gg 352

<210> 297
<211> 318
<212> DNA
<213> Homo sapiens

<400> 297
ggcagaattt ctgctggccc tcggtagtcc tctgcccacg tcggtgatcc ggtctcgctt 60

ctccgcggga gctgaacgga tccaaatgtt tcccagtggg ctagcagctt cttagaggga 120

ctgggagcgc ctcccctcag ctccgccccc cactccagct cccatgtatg gaaaataggg 180

gtggagcgcg ctctgccatg ctcggtgctt agagaactag tgaagaggac agtgtgggga 240

ggatggaatc cgaaacggga ggaggggccg gcaaacctgg ccagagcagc gaagaggacg 300

aatctctact gcaggagc 318

<210> 298
<211> 596
<212> DNA
<213> Homo sapiens

<400> 298
gctcagagtc tcagtaacaa acccgttttt cagaagtgtc tgccaacccc ggggtgggga 60

atgaaaacac cagcccgtat tgggaaagaa acctgggtgg cagatcacga aagaaaaccc 120

ttcaaaagta attttccatg atgtgaccgt agagataaac atttcccatc atctggcttt 180

tgagtttggg gagatgagca gacagaaaaa acctcagatc ttttttcatt tcacagacgt 240

ttataccctt ttgtttcttc cagtcacctc tgacaaaatt ctggggacgc tgggaacact 300

gaatcaacat gggcaatgag aacagcacct cggacaacca ggtgggtgtc aggaagcttc 360

tctctcgagc tacgtggtgc tcttggcaga ccttgactag gttcttttta cagcgtctca 420

tcttcaccca ggaaggagca aacaagacag cttctgcgta gatgtgtgcg ggcatttatg 480

gatctatgca gccatttttc atttcattgg aattctctta cgattaaggc tagctctagt 540

taaagccgga aacgctaatt ctacttatta tttattatta tttttaaaga catggt 596

<210> 299
<211> 795
<212> DNA
<213> Homo sapiens

<400> 299
caggtgtggc ggactggcgc tccccgcaac agaaggtcgc gcaggacacc ggaagtcgtc 60

ctggagtcag tctgagcgga tcgcaggggt tccaggcgcc ccaggccccc cacaccgcga 120

gtcacaggaa caaggaggaa aaacagcctc tttatgactt gggggaggca attcaatatt 180

tcttcctggc ataaaatagg aattgctcta tttacccagc ccgcaggagc gtcgtgaaga 240

ttaatgactg tgaaatgttt ttaagatcaa aaacattccc agattttact tgtaggcact 300

tatatgtgca tattggtatc tgtacagcaa aatatactct ttattgattg ccctcctagt 360

gtgacttcta ttccgtaatc tgaaagcccc gagataaagg aatcaggtct gtctctgcct 420

gctcctgaaa cactaggaaa tatgggtagg aaatgtcagg tcagctgggc atctacgctg 480

agcctctatc cagtccgcag ggcagataga gcaatccaga gtgcggacca cagctgaacg 540

tctggagaaa gggaaaaaac tttaagatta ttggtcaccc cccactggga agaaaacaca 600

gtatcctccc cagctcatta ggaggtgctc ccaaagcctg agtctccatc agggtctcgg 660

gtgtttgcag agccttggct ggcactggga gccaatggtc agccaagttc tgcctgtgcc 720

aggagcccac gcagcccatc tggcccggca gctccaacag caaggcctcc ctgttccgag 780

cagagtaagg ccaca 795

<210> 300
<211> 796
<212> DNA
<213> Homo sapiens

<400> 300
ctgcctcagg tcctcgctca tctgacggtg ccgggattcc taatctgccc ggaagtaacg 60

caaatgctct gtgaatgttg actccgctgc ggttagccag agattgcttg gcgtgatgtg 120

cacgagactg agcaatggca cacggtaggg gcaaggaggc cctgttttct cttgacttat 180

tctcatcaat catctgggag atgactatgg atgaaatcat ttttgtgttt tgtgatgcca 240

gggtctcctg caaatagaaa aagatcccga ttattcaagc agtctattga aatatagaca 300

gtaaactctc tttcaagggc caccaggatg ccagagcact gggttcataa cactcagatg 360

tcttgtccaa ggctttttac tgcacacctg atgctcagag atggcaggaa gggcaggaaa 420

actagatgtt tatgacaagt tacacgttct ggaaacattg gcctgagtca tgaattggtt 480

ttctctgctg ggcatctatt cttaatttat ctaatgatgt gtcaacgggg atcatctaat 540

tcgataacca cctccgccaa gcccaggaac ccctgaatac aggctcatat cctcaatgga 600

gaggaaagac agaattctgg aggtggctgg aacttggggc cacaggttta tgtgggagat 660

tagtcagtag tcctgaaagg aaacagcaac ggtggttacc aggagccccc tccctacctt 720

ggtcctttgg agtcctccac atttgccggt ttagactcta attcagttgc tccctgctgc 780

ttggcatcat ggaagt 796

<210> 301
<211> 543
<212> DNA
<213> Homo sapiens

<400> 301
ggtggtctgg catgtggtca agttacacca atgtgggaac caaaatggcc ggggttgagt 60

gctgtgtcct gtaactataa tttcaggccc tctatcaaga caaagaaatg gtatctaaat 120

tgggatatgc acaattgcaa aatgtcaaca gcgagtacta cagagggaca aggatgcaag 180

aagcgagatg ggaggagagc tgagtgggga ctctgcaaag ggcgctccct tctccctcca 240

cgtttaaata gaacacgggc agtttctccc tgtgcttatc aaacccagat attttcagat 300

gctgttctag ctgcaaactc atgaagtgat ccttgaaatt atattaccaa cagaaatttt 360

ccttcattgc ttattttgat ataatttcaa taaatgtacg gtatataaaa ttatttcttc 420

atttcctcct gattctgaaa atgaaattca atttatgaac cagcctgtgg aactccagca 480

gagtgcaata caccggcagt gtattgttgg ctgtgggacc aggtccccac gggagaacaa 540

atc 543

<210> 302
<211> 531
<212> DNA
<213> Homo sapiens

<400> 302
atattagtgc tgtaacaata tgtctgctca gaaatggcat ttaggcaacc ggcatcatag 60

ccaggcggag tatgcccctc agagtatgcg gcctgctact gtagcctgga agcctgcaac 120

gctttccttg ctattccttg caactatatt tcacatgcca cacatacaaa gtcaatgatg 180

catttttatt tgccatgtag ggtgaaact cttgtggtag ttagaaaaaa caggtgggaa 240

attgctcttc tgacagagat ctcaagtaac gggcacgcta tctaacaacg acacgaacgc 300

tgcatcgctg ctctagggaa gaggttaact gacagaatca caatccaatt ctcacataca 360

cgcgcgctat agtaagtaca aaagagtttc ctttttatca ataacagaca attgtatatc 420

tcaggatgcg agcgcctgag aaggaagtag taaaatgatt ccgagtgatc cctgactcct 480

ccggcaccgc ggacggatgc atcacccaga cctgttttca tttttgacta c 531

<210> 303
<211> 421
<212> DNA
<213> Homo sapiens

<400> 303
gggagctttg ctgccgctgc ggccttgtgt gaatttggag ggacaaggcc ggggcgcgaa 60

ctgaatctac tgtcatatgc ggagtcaatg caccatctcc aggacctcga aagcctcagt 120

ttccacgtat gcaaaacggt ccatggtgcc ctcgtaccct ggtcgctgct gcaccatcga 180

ggtcagcgcg cggcaagtcc caggtaggcc acgtcggctg cagacctcgg cggcgggcgg 240

tggcggcgcg tgtccacgcg agggcgctgc gcaccgcgct tcagcccgac ctcgccttct 300

gcaggggcgg tctcctggcg ccaggggcgg tgcgaggacc gaggagagcg caagagtaag 360

cccgcgggtg ccggagcgcg cgccccgccg tgtgctgggt ccagggcgcg cggccggcct 420

g 421

<210> 304
<211> 578
<212> DNA
<213> Homo sapiens

<400> 304
cctcatcaat gcatctcaca cctctcagga caccagaatg gccaccctcc gggcccctgc 60

cccgaggaca tctcagaaag ggagcacgtc tactgcatta atgagctcaa atgcctttgt 120

ggcggcccaa gcctctggta ctccaagctg tcacagtggg tgggacccca gcccctgtgt 180

ccagcccagg gccagaaggc atgccaagtg gactgcctgg ggggagcagc acacggcttg 240

gctctgcgct cactcaccag gctccactcc agcacgccag cctgcacgtc ccgtgcaaga 300

aaatcacaac ttcctggggt gcttgcactc ccagacgcat gcatgactcc ccctaaactc 360

cagttagtaa agagaagtct catttcctca aacggaagct ttcaccttgg ctttacagca 420

ggaagtgcca gggtggacct gacaaggtgg ctcagtggag tcgggtcgat ttgcagggtc 480

cgaagtcaag caaagcccaa acggccccag ccgaatgttt ccctcaaccg gagccgggct 540

tcaggtccca cagcgcctgg gcccaggcca ggggcatc 578

<210> 305
<211> 360
<212> DNA
<213> Homo sapiens

<400> 305
gcacaaatgg gccaaacagg ttcgtaaggc ccccgcgggt cccctggccc ggaagttgag 60

ccgcgggtgc ctggtctgaa gttccgcgcc ccacagtggc ccgcgggcct cggcggctcc 120

tgccctacgc cctccaaggc accaggcagg tggccccagg gcctttgccc acgacacccc 180

ctgggtgggg caggaccacc cctctgtgaa ttcgtcctgg atgcccccag gcacagtctg 240

tactcctccc tcccccagca cacgcggcct ctgacgctgc acgccgagct tcctgggacc 300

caggacgaac cgggctccaa ggaccctcgc tccgcccgga ccacacgcag gcagcgccgg 360

<210> 306
<211> 434
<212> DNA
<213> Homo sapiens

<400> 306
agctccaggg ctgggttgcc aatgcgacaa agcagacggg ccccccccacc ggcacctacc 60

tggggcctgc tgctccttcc tccaaggtgc tctggggtgt gagtcggagc tgcaccccag 120

aaacggctcc cgaggaacag tgtctgtgag gctgggtgct ggggccgcct gcaccccccct 180

tcccctccca gggccctgcg ggagactgca ggtgggcacg gactcacagc agcctctgga 240

aagggctgca gaggggcttg ctggctgact ggccagggac caggttgggc agagaccccc 300

aggcgggagg gttggggctc accaagatgc tcaactcaga tgagaggcca cctgcccaca 360

ctctggcagg ccagctatca agaccggggg cttctgtcct ctttcacccc tcctctccct 420

ctccctgccc acaa 434

<210> 307
<211> 425
<212> DNA
<213> Homo sapiens

<400> 307
agctgacgag gcttggaccc gccgctgcca gctcggccct ggcctgcgcc ggatggggtc 60

ttcggctgcc cccgcgcggc ctgctgctgc tgggctggtg tcccttcgag ggctccgcgc 120

gcctggagcc ctcacacacc tgctggccct aaggccctcg agtcgggagg ggggacaggg 180

gtcagctcca ctctttcccc gagggaccat gtgtgggaca ccttgagaga ctcccaccag 240

ccccctggag ctggagtctg aagccgctat ctgcggaggc ccaccctgcc cagtagatgg 300

aacaggggct gagcccatca tgtcactgtc gggggtccca gcccttctca gccttgggcc 360

agaccactcc agccccggga tctgcctgtc caggctggct ggaagctgtt gtactgggta 420

aaagt 425

<210> 308
<211> 666
<212> DNA
<213> Homo sapiens

<400> 308
gagaaggaga atggagaccc tctgtgccca gtgaggctgg gccaccttcc gggtactcca 60

ctgatgttgg gctgctcctg ctagtctttg gttggctgtg ggcacagggg gcctagaact 120

gtggacaggg gcctggctgt gtggccttga gagaataacc caacctctct gtgccttggt 180

ttccccctct cactggactg accgtttatg gacagctcga agcctgggtt cccaggcttg 240

ggactggctc tcctaatgtg tcagccgacc actgcgtctg tgggtggtct gcaggggcgg 300

cccccacccc ttcctaagga gaccccctca ctgtccatct gtttgcctca gagcctgggc 360

tcccagccgt tgacggattg ttttccgcat tttgctctgc agaacactgg acagtaagat 420

gttcttgata agagagtttt tccttccacg gactgcgttc cgcagctcct gggtggggtg 480

cagagtgtgt cagctcaagc cagaaattcg gtcgtaaaga tgctgtttct cttcacgttt 540

ctcaaatgga cttggcttcg gaaatcactt ctgcaacaca aaccagcagc ctgtgcagct 600

ctgggcgggg gcatgccggc tacccagctc ccggcattgt tgcacatggc tttgaaacgc 660

acggca 666

<210> 309
<211> 697
<212> DNA
<213> Homo sapiens

<400> 309
tcgggggcgc actttcccgg cctccagcga cactgcgcac gcccctatcc ggagtgggtg 60

tgtgtccctc tgggcgtacg tgcctgaggc tgtgccacct cctgtgcttg ttcttgtgtt 120

tctgactgtc ttgtttgcgt gtctctgtgg gttcccctct gcgtgattgt gtctctgggt 180

gtatccccgc gtctgggtcc ctctgtgttt gctgttggca tgggtgtctg tgaacgccct 240

ctccaggttc ttgtgtctga ataccgctat ttgtggatcc ttctgtaggc gtctctgtgt 300

tttcggtctg tgtgtcctcc tctctgtgtg ctagtcttta ggtgtatcta tgagcgcgtg 360

tgttttctgt ctgcgcctct gttttctagc tacgtctgtc tccgaatgta ggtccagtac 420

ctgtctgcgc gggtccctct gtggaggcgt gcttcctcgt ctgtgatctg agcttctgtg 480

atctgtgtat atggatgtgc gtctgtaagt ctgtatgtgt gtctgtctgc atgtctgcga 540

gtatctgctg tgcttgtgtc ggtctgtgcc tctttgctgt gcctgtctgt ccatacgtgt 600

gtatgtgttg gtgggggtgg tgtaggtgga tcggatccga gccaagccgg gggcaactag 660

gccaggcctg cggttcccct ggctctcgct ctcccac 697

<210> 310
<211> 349
<212> DNA
<213> Homo sapiens

<400> 310
ggccggggcc ggctgcggcg gccgcgccag tctcgcttca agacgcagcc ggtgaccttc 60

gacgagatcc aggaggtgga ggaggagggg gtgtccccca tggaggagga gaaggccaag 120

aagtcgttcc tgcagagcct ggagtgcctg cgccgcagca cgcagagcct gtcgctgcag 180

cgggagcagc tcagcagctg caaactgagg aacagcctgg actccagcga ctccgactcg 240

gccctgtaag gggcgccgcc cgcggggggg acgcgcgcgt ccgcggtccg cgcggggacc 300

ggcgtgtgaa ccccgagagt gcccgcgccc tgctcccggg ggacccgca 349

<210> 311
<211> 420
<212> DNA
<213> Homo sapiens

<400> 311
cctgtggctg cgttcaaggc agcttggagg tcatttaggc cagctccccc gggttccccg 60

tgggctcaaa tcaggaccca gacccttctc tcttgccaca gctctgggcc acctgggact 120

cgggacttcc tgtctccctt cctactctgt cctaaaccat tgttcagagg cctgccgcct 180

ccaagaatcg ccgagggaga agctccaggg gaactggcct gcccgcatcc ccactgccag 240

tagcgctcag ggtcccagga ctcgggtctt gccagaccac agtgtcccct gggaccctag 300

ggtctctggc tccgcactgt gttgcgccag tgcgcctgga tttcccgtga gctttcggcc 360

aaccacgctc cgggcttccc gcctggctcc gcacctgcgg cgctctcacc tgcggcggcg 420

<210> 312
<211> 706
<212> DNA
<213> Homo sapiens

<400> 312
tcacacctgt tatctctcac cctattctct cttcacccct actcccgccc ggcagctccc 60

cgctgttagc accagaaata gttatctgct aagaaaacga aaagtgtaat gctggcttta 120

ccccgcgacg ctttaaaaaa ctccaaaaac ccagcgtgat gggggtagg tttgcctaga 180

cacagctgag gcagccttaa taaactgaga ggaaggttaa ttgtcaaaga gggttgagcc 240

actaggagca gcagcaacct tggactaaca ggtgtatctt cagcgagcac atgtcccagc 300

acatgtcccc acgcccatcc ttccatttag cgccacaaac gcagcctctt aagagaatgg 360

gaagtttggg agtcacagaa cggaagctac cattggcctt aatggatgtc aaccattaag 420

gtaagcttgg gagtcggaga acggaagcta ccattggttt tagtgaatgt caatcttcac 480

ccgccacttt tcttcctggc ctccttgccc cttcctattc tgctggctag ttctctctcg 540

tcatctgctg gccgcactcg ggaacatacc aaacgcggaa acgcgcctta gcttgccctg 600

aatacctgca aaccgagggc tcggcgtggt ttttaaaggc ttggctagcg tgcccccggc 660

ggtactaaaa gagggctgct tgcgccaatc tcattcccac aggatg 706

<210> 313
<211> 328
<212> DNA
<213> Homo sapiens

<400> 313
ccctgtcctg tgctccttgc aggtattcag gctccaggcc aggtggggcc ggacgccccc 60

agccatccac catggtggtg gcacacccca ccgccactgc caccaccacg cccactgcca 120

ctgtcacggc caccgttgtg atgaccacgg ccaccatgga cctgcgggac tggctgttcc 180

tctgctacgg gctcatcgcc ttcctgacgg aggtcatcga cagcaccacc tgcccctcgg 240

tgtgccgctg cgacaacggc ttcatctact gcaacgaccg gggactcaca tccatccccg 300

cagatatccc tgatgatgcc accaccct 328

<210> 314
<211> 578
<212> DNA
<213> Homo sapiens

<400> 314
tatgtctcac acactgacta ggggtccttc ggcctcagaa aagtggcccc ggacggaata 60

cccactaaga agtgctctct gaaaaaggcg ggcgccaacc tcgaacgaag tcgttttgag 120

ggactggctc gccagctcct aagcaacttc ggggctcgtg ccgcgcaccc accaggcctt 180

ctgcgcacgc gcaagtgcgc ttcccaccgc cccttcgggc gcagctccaa ggcctggccg 240

cggtctgcgc gtgcgcattt cattgtcgac accgagagga cgccttggcg ccttcaggtc 300

cctcctggat ttaccagccc attgtttatt tcgcgcctgc tttagcgtgg tgtcgtcggg 360

aactagcgac tgcagagtcc ttactgtgtg ctaggcgtta tgcagggtac cttcctttat 420

tcctcggcga tatgctctgc ggcaggcatt aatgccccaa atttaggatc gcttgagctc 480

aggaggccga ggctgcagtg agccgagatc gcgccactgc actccagccg gggcgaccaa 540

gcgagacctg ttacacacac atacacacac acacacgc 578

<210> 315
<211> 509
<212> DNA
<213> Homo sapiens

<400> 315

ggatctcctg tcgccttcgg ggcgctgccc atggtgctga cggctgcgcc ggccgtgtat 60

ggctctgtcc atggttctga acccacagtc ggcttcggag ctctgtccgc ggttctgaaa 120

ttcagagccg ctttggagct ctgtccgcgg ttctgaaatt aagagccgag aggagccgac 180

cccgctttag aagtcgaggg cttgtgggct atggagatac agcaacaggt tccctggcca 240

agagctgcgg gcagcgggtc aacaggtgtt tgcagaggca ggtccatgag aaattcctct 300

ggattctctg aaactcagac catgccttcc tcacttcttc tctgcctccc agtcttactc 360

ctgacgcact acgtcttctc gccctacagg cactcctctg gcgggcccca cggggcttct 420

ctcagctcaa gagcttgagg acatcgtagc ggcactaccc ggctttctgc ttgtgttcac 480

agccgagggg aaattgctct acctgtctg 509

<210> 316
<211> 458
<212> DNA
<213> Homo sapiens

<400> 316
gttccctcgc cccacaaaca ccccggcaga tggacacctc ctggcccgcc ggcctctgta 60

cccctccgaa atgggagtac cgcagcatcc cacaggagca gccgcgccca cctgatgagc 120

acaggtgtgt agagcagggc ggcgatggga gtcacgttga tgcccatcag cattttgctg 180

tcgatgtcgg gcagccccat gttgccatac ttcacctcgc ggtaggtctc gtcgtagtgc 240

aggatcagct gcatctgcag gaggcctggg gacaggacag agagcggcgt gcagggagca 300

gcctagcttt gggcgccacc gagcagaaga cggcatgcag gcctcgcagg gagctccaat 360

caccgaaggc attcccgctg acagcgcccc tcaggacagc ggggttcccg ggctgcgcca 420

cgccttctgc cagcccgcgg ccacttggcc agctaagc 458

<210> 317
<211> 584
<212> DNA
<213> Homo sapiens

<400> 317
ttgcagcctg gccgtcactc ctcggtacgt gttttggact taaacgctcc ggatgtttac 60

tgagtgcttg attaataaca tggaaggcct ggtctcattg ctgtgggagt gaaggatgca 120

cagccaggcc tgacatgatg agaacaagaa cctggagtct cgctgcctgg gtggtaatcc 180

tggccctgcc acttagcaac tgtgtgactg tagccaggtc acttaatttt gctagatcct 240

gcctgcgctt cagtggatct tgctggtttt ccaaggtggc caaacacttt aaggcattca 300

tgtggtcgct aggctgcagg gttgaaccct ggctcacccc gcagggcgcc gtgtgctctg 360

tggcctggct gtgcctttgc tgacaccgtg cccgtgtgtg ttcatgcagg tcaggagcgg 420

gtcgtgattg ccgacgatct cccgcacccg ttcggtctga cgcagtacag cgattatatc 480

tactggacag actggaatct gcacagcatt gagcgggccg acaagactag cggccggaac 540

cgcaccctca tccagggcca cctggacttc gtgatggaca tcct 584

<210> 318
<211> 636
<212> DNA
<213> Homo sapiens

<400> 318
ccaggcatgg cccggcctcg gagctctgcg gccccgaagc cgcctcaccc ggggctgcag 60

ctcggagccc aaccaagagc tcacggctgg cgccctggat gggaccttcc cttcctggct 120

cgcggccagg ggtacctgtt ggggcagcca gggtgagggt gatggtgggg gtcttgggtg 180

aaggccaagt tggtcgccac tccacgatga tggtaactgc caggccccac tgcttcacta 240

tcctctgggg ccagagtccc gagtcctcac ttgccagaaa cgcatattgt gcccagggca 300

ctgggcctgg taccctggca ttggcaccga aactctcaaa gcccagggtg tcaatcattg 360

gagattgctt tctaccctcg cccctgtctc ccctgggcgc ccaggcctgg ttcaggtggg 420

ggcactggtt ggaagccagt catgtaagtt ggggacccca tcctgaggtc cccaatatcc 480

ccaccaccct ccccagggcc agacccagag gtctaggtaa ccctatgttt tagagagggg 540

ggcagaattc tcccaggaag ggtttggctt gaaggacaaa gtttgccgga agaggatagg 600

acagggctgg gactggctat ctcctcttgg ggtgag 636

<210> 319
<211> 765
<212> DNA
<213> Homo sapiens

<400> 319
cacagactcc tacagcacca gtgcagtgct gcggcgccca cagaaaggcc ggctggcagc 60

cctgcgagat gagccttcca aggcaaggtc tttgtgtgaa atacctctgc ttacgtgaaa 120

gttacctact tatcccgtgg ctttcagact actttgggat aaaaatgttg ataggtcatt 180

gctttcttgc cctcctctca ttttctattt tctgactaag agtttagctt ttagagcatt 240

ttttaaaata gggttttgtt tttgtttttg tttttgtttt aaagaggtag ggtctgtgtt 300

gcccaagctg gtcttgaact cctggcctca cacagtcctc ttgcctcggc ctcacaaagt 360

gctgggacta caggtgtgag ccaccgtgcc tggcctctgt agtttttttt tttttttcct 420

tttaatagga attcttataa aaatacagtc acttttaaat acaatgataa tcatggattt 480

aaagtagtga ttaaaaataa atgagtaggc caggtgcagg tagctcacgc ctataatccc 540

agaactttgg gaggccaagg cgggtggatc acctgaggtc aggagtttga gatcagcctg 600

gtcaacatgg tgaaaccccg tctctactaa aaatacaaaa aattagctcg gcacagtggc 660

aggtgcctat aatcccagct actcagaagg ctgaggcaga agaatcgctt gaacccggga 720

ggcgttgcag cgagctgaga aagcaccatt gcactccagc ctggg 765

<210> 320
<211> 775
<212> DNA
<213> Homo sapiens

<400> 320
aactaggaac ttcagctctc acttcctaga acggggttgc taacaaggcc ggcatcggcc 60

ctgatccctg cacagatggc ccctcttgct cccccactgc ggggccttaa catccttgtc 120

acccgacatc tcagggctgg cctcagtggg gaggagggaa ggtggctctc agcactagtc 180

ctcagagaga cagccaagtt gatggggtaa gtgtgaagga ttttaaagtg gcaattacac 240

caacctacag tgttgtaaaa acaaaacaaa aaactaagtt tttaatgaac aaaaaaatct 300

caacaaggta ataggagatc ttggcaaata aaaggcaaca ataaaaacag ataaacagaa 360

aagctagcaa acatgattta tgaaattata aaactcaaaa gacggactta acagctacag 420

ggacatttca aaggaaaggg taagtgaact taaacaataa ccaatagaaa tgatctaatg 480

taaagaaaaa tgactgaaaa aaatgaatag agctccaggg acttgtttga tgctatcaaa 540

tgatgaaata tgtgtgcagc agaaacactg gccatcaggc aagagggcgg ggctctgcca 600

tcattagaat gttgcagtaa cccaggtcaa gctgacctga tcagtgtgtc gcgcagagaa 660

aggaactgag tcaattacct gctgggggcc aggaggcagc cagctgggga aggcgggatt 720

tgaaccgggc ctactgacct ctaagggccg caccttccac ccatgccact ccaat 775

<210> 321
<211> 284
<212> DNA
<213> Homo sapiens

<400> 321
cggagggggg cgcggtggct ctaggagccg caggtccaca gaggaaatcc ggcctggccc 60

cgatttccca gctgctggcg ccgccgcact taacgcgggg gcaaacaggc gaggtgacgg 120

cggggctgaa tgcggaagga aatgaccctt cgctggccct ggggcggccg cggagggggt 180

tccccgcacc aagccgtcca ctctggtggg tcctggcagg tcacttttcc tctccggatt 240

gtgctctagt gtctggaccc gtactcctcc atcatctgcg ggac 284

<210> 322
<211> 305
<212> DNA
<213> Homo sapiens

<400> 322
cgacggtgac gcgctctggc tctgacaagc ctggctcccc ccaccctacc gggtggcgcc 60

gttgctgacc ccacgcccag ccgttcccac tcccgcctga aactcctccg acagggcttc 120

gcttcggggg ctcagtcccc agcgcgtaca cactcgcgtg gtgtcggctt agcctcttgt 180

aaaggaagca gggcaggcag gtgggaggaa gcccacaggg gcacttggga tgatgagggg 240

gcatgctggc aaagccgggt cagtgacaag gatatattcc atttgtttcc acctaagcct 300

tagcc 305

<210> 323
<211> 519
<212> DNA
<213> Homo sapiens

<400> 323
gaggtaggtg ttaattgtct cttgtgcttc tctgggtatg tctggtagcc ggttttgctg 60

ttaacttgaa cacggattag atgtgaggaa gaacaaaagg atcaggagga tttctaggca 120

acacaatctc ttataaaatg gcatggctat tgggggaaga agataatgct ttgcagaatt 180

ctaattttga ccctcttctg aaaaaggaat ccaattttaa taatcattgc ccgatgaaat 240

ggtcaccctt gcagtgctgc aggctgtaca cgtgcctgaa tttgattaga cggcatcccg 300

agaaagggtt catctgcttt tgtctctggg tgattacgtc tgtgcaagtg tctgtcaagt 360

ctgaattatt ctgcctcaca ttatatttct gcccagcaac atagtgctgc atgttgtgac 420

aatgacctta ccttccaaac acctttcaaa caaaatgtct ggttcacccc ggctcattat 480

aactagatat gccaactgtt atccttcaat ctgaaaaga 519

<210> 324
<211> 328
<212> DNA
<213> Homo sapiens

<400> 324
aaaattctag tttacgacgg tcgtaattta acaaacaaac cgggacttcc ggacccgagg 60

aaggaggagc acggctccac caacagggca tgcgcacatc tgaggtcgct tagggctcgg 120

attaccgcag cccagatttt aaagacccga agggtgttgg agggtttaat tctttcaggt 180

gttgggtgcg attgtgagca ggattaatac ccgtatatat ttattatgca ctttttacta 240

tattaacagc tttctcacca atttcgtctt aaaatcaccg gagtaagtta caagctcttg 300

gaagttaagt tggtacctct caaaagtt 328

<210> 325
<211> 274
<212> DNA
<213> Homo sapiens

<400> 325
ccgcccccctc agcccgcccg gacgcccctt ccccaggcgt ccgccccgcc ggccctggtc 60

gggagaggct ctggcccttc cctctcgaaa gtgcaaacac tccattctcg cttccccttt 120

gcccctcctc ttccagggac gacttcctcc tccgcaattt ctcacttctt tctttggcgc 180

ccatttcctg cgccccttt ccatctttcc tttcccgcct gaaccggggt cgtcctcgcc 240

gcccctctt ccccaactct ttcagcccct gtgg 274

<210> 326
<211> 715
<212> DNA
<213> Homo sapiens

<400> 326
gccaagttct ttggatgcca tccgcgcggt gtcccataac tttcctggcc ggtgggcaca 60

ctgcctgcgt gagtgtttgt tctctgcatc gaccattccc tgctcaggcg tcgcccttcg 120

tgctgggact gcggcctgct gagccccagg taggagtcca cgcatcgcct ctctctccag 180

gtgcacccgc acagctggcg aacttggcta aggtcagcgg gagaggaggg gtgagatgtg 240

gtgctagttt taggcacact gtgctcgtta tcagattgcc taggggcccg actgaagtat 300

tcctccgccc agcgcctcgg gcaaacgcgg gatctcccag ggaagctcgg agggggacag 360

ggagctggtt cccctctgcg actcgcccct ctctggctgg ccgagtgtct ggcgccaacc 420

tcgctggagg aggctgtcgg gggcgggcca gccagcgggg ctgccctcct cgcagtgacg 480

tcccaggggg cggaggggcg accgactgac tccgcgccct ccccccgcc tcccgtgcgg 540

tccgtcggtg gcctagagat gctgctgccg cggttgcagt tgtcgcgcac gcctctgccc 600

gccagcccgc tccaccgccg tagcgcccga gtgtcggggg gcgcacccga gtcgggccat 660

gaggccggga accgcgctac aggccgtgct gctggccgtg ctgctggtgg ggctg 715

<210> 327
<211> 605
<212> DNA
<213> Homo sapiens

<400> 327
tgttacctgg tggataatac ttgttgcctc gagggtaacc actgttaccc ggtggataat 60

aagcattgcc tcacaggtaa ccactgttac taggtggata ataagtactg cctcatgggt 120

aaccactgtt acccgatgga taataagtac tgcctcgtgg ataaccacgg ttaccctgtg 180

gaaaataagt gttgcctcgt gggtgggtaa gcactgttac ctggtggata ataagtgttg 240

cctcttgtgt aaccactgtt acctgctgca taataagtat tgcctcgtgg gtaaccacta 300

ttacccagtg gataataagt attgcttcgt gggtaatcac tgttacctgc tgcacaataa 360

gtgttgccta ttgggtaacc actgttacct ggtggataat aagtattgcc tcatgggtaa 420

ccactgttac acactggata acaagtgttg cctttggtaa ccacagttac ccagtggatg 480

ataagtgttg cctcgtgggg agccactgtt acccagcagg taataagtgt tgtctcatgg 540

ggaaccactg ttacccggtg gataataagt gttgcctggt gggtaaccac agttagccgg 600

tggat 605

<210> 328
<211> 648
<212> DNA
<213> Homo sapiens

<400> 328
cgatcccatt ccatccgttc ctcgtctcct cccggtctga cccgttgccc ggccgtggtt 60

cgccacacca ggcatccaaa gctgaggtcg ctcctacggc ctgggctcgc cttcgcttta 120

gagatgtttg gcctcttccc tcccaaacag cccatcttca aaacctggac tcttggactg 180

gcacctggcc acctttccct ctaccaagac tccacttccg tcttacccac ttcttcctca 240

gattcttggt accccctggg ttggagactg ctcattttcc ttccaaatta atcccagacc 300

ccctaaaata ttgacaacct tgacaacccc ccaaccgagg agccagactt tgttttggac 360

taacttccat agccctatca tggaggcagt gtacctggta gtgaatgggt tgggcctggt 420

gctggacgtg ctgaccttgg tgttggacct caacttcctg ctggtgtcct ccctcctggc 480

ttccctggcc tggctcctgg ccttcgtcta caacctgccg cacacggtac tgactagtct 540

tctgcacttg ggccgcggag tcttgctttc attgctggcc ttgatcgaag ccgtggtccg 600

gttcacatgt gggggcttgc aggccttgtg tactctgctg tatagctg 648

<210> 329
<211> 375
<212> DNA
<213> Homo sapiens

<400> 329
agcggccggc cgaccagggc tctccccacc gactgcctgc tttggcaccc ggctaccccc 60

cttggcctgg ctttcctggg gcctttcgcg ttcgctcgct ttctactgtc ggtatttgca 120

gcagcgctcc ctcaggtccc gagcagagct aagccagcca gcacccaggg gaaggggacc 180

cacagccccg tggaaagagg tctatcgaga tgctttgcag caaagtgctc tgcacgtggc 240

cctggccaaa atgtgcacca ccccctttca gaaagaacat aaggcagagg gagcagaatc 300

cccctggtgc cagggcctgc gcgcccgggc tccgggaacc cggcccagga ggctcgcgga 360

gggcaggagg ctggc 375

<210> 330
<211> 715
<212> DNA
<213> Homo sapiens

<400> 330
cacgtgtgtg tgcatgtgca cgtgtgtatg tattatgtgt gtgtgtttcc ggtgggcagg 60

tgtggctgga ggcatgaccc cttttctctg gttgatttca tctccagtag ccaagtagaa 120

actacagcat gacctgactt ctatgccccc agcactatgg ccatgacagc ctccagatag 180

gagaagggtc tggcttcatt gacatttcca cggtagtgca tacagccctc gagacctact 240

caagagagga caaaagagag gctcgggtag agcttatcta gtggatgaac ctgctcctag 300

acacagagca ctgcctggca tccctgctgc agaatcgggc taggataggg ctgagtattc 360

taattgcctg tgggatagga tttcactata tccctgcaca gctgttttca tgctgtggtt 420

ggattttgac gctgtcgtaa tttccccgt tgtttgatat tctcgcattt acattaaaga 480

agtacaggag aagaacagtg ttcttgggag aagatttcag catttaggat tcatacaaat 540

aaatgcatgg actgcccacg cttccctcac atccgtcaaa caactcagtt aaaaacacct 600

tttcaaaagg ctcttctata attttacttc ttttaaaaat agtgatgggg ttatgtctga 660

tgccccggaa cgtccgtcat gcattgattt tatccagttc agtgccaggc agcaa 715

<210> 331
<211> 677
<212> DNA
<213> Homo sapiens

<400> 331
aaatactggt ggtgcaatgc agccaaccgc tgcatctccc aaaagatgcc gggaaagagg 60

cagacaccca aacccacccct gggcctgggc agacccctct agcctctgac aagtccaggc 120

actgctcaga ccttcacatg cacacctagg gccccttcat tattcagcaa gctcttccca 180

ggctcccatc tggcatcagg catggtgctg ggagctgctg ggcacacgga tgagcaggtg 240

gtgacccttt tccatcagct acccagagcc agctgcagaa cagcgccgag agcacatgcc 300

tataatgtgg ggcagagggt cacaccgagg actctcccag aggggagtta tttgagctgc 360

tctggagcaa tgcagacaag agggtacaga gcacagaggc ctggggtggg gactcccagg 420

gaccaggaga ggcatgaggt gggaagtggg gagccaggct agaaagggcc ttgtgagtcc 480

tctgggggag ttgcagcttg tcctattagc catgtgcagg ctctttggtt ttcagagaat 540

gaggatcatt cacaagggtg actggagctc ctgcgggatg tgcacacctg gcctgaggtt 600

gagatgacac agaaagagtc acagggccgg ggtctcccgc agagctagtc ttctgtttga 660

gtgtcattta tcacctc 677

<210> 332
<211> 378
<212> DNA
<213> Homo sapiens

<400> 332
ggggtcagcc tcccaaagtg ctgggattac aggcgtgagg caccgtgccc ggccaccaaa 60

ctgactttaa atcacactcc ttcactcgga gtttttacct aggaatgaga tgggagcctc 120

cactactgcg gagagggtca gaagtcattt ttgttctcac cctaacccct tcccagtctt 180

gccccagctc cgcgttaaag gggtttgccg cccgcagggg gcgctgcggt gcagaggcga 240

cgctgccctt tcctctccag ggccgagcgg ggggtttgcc gcccgcaggg ggcgctgcgg 300

tgcagaggcg acgttgccct ttcctctccg gggccgagcg gggggtttgc cgcccgcagg 360

gggcgctgcg gtgcagag 378

<210> 333
<211> 477
<212> DNA
<213> Homo sapiens

<400> 333
gacgctggcg gagaccgggc ccctccagcc gctccgggct tgcggttccc ggggaggagg 60

tccgtggggt ccacgcgcga gctagggatt cgggtgcggg atgccctcgg ccgtccacac 120

ccaggcgcgc gctccagtcc gcgcagccca aactttgccc accgccttct gtttggctca 180

ctccgtttcc tctcctgctg catcttctag gggggttctt ttttaccata cctcggagag 240

tcccgtctct tccccgcttc ccatacgtcg ctcctctccc agcctccgtc ctcggctctt 300

tgaatctccc acctcctcgc tgcgccctct ttctcctgtt cgggccgccc cttctgccac 360

ttttctgaac cctgagcaaa tctaccaaac tccccagttt aaggagatga tggacagcac 420

agtggaccgg gcagcaggta gattcctact tctgttttta acttagccaa agccact 477

<210> 334
<211> 418
<212> DNA
<213> Homo sapiens

<400> 334
cgcgctgggc actgatcagg gcgattcggg acaaaaatat ccaggaggcc ggcgcggaga 60

ggcgtgaggc cgacgtttga tgacagttgt cctgaccgct gcctcagtcc ccaacttggt 120

gcagcgtgtg ttgacacggc cgaggcagag tggagacagg cagccgcgga gtcaaggaca 180

ggtagagaca aaaagcccca tcagccccct ccacttctcg caggccagga aggtcccaga 240

gccccagaac cgagcctgcg caatcttggg gcctgcagcc ttggcctcag acggggccca 300

ggttccgagt cccgcagctg ccctcggcct ctcccacgcg gtgttggagg gcgtgctagg 360

attgcctccg gtggagcccc ggccccgttt cctccacacg accgggttca cacctgaa 418

<210> 335
<211> 464
<212> DNA
<213> Homo sapiens

<400> 335

tagaagcagc atgcatctga caattgtcaa tttcaaaaca aacacgctcc gggacttgaa 60

cgcagcgggg cattcagtag cgaatgctgt ctccttgagt tagggcaaag cctgcgtgcc 120

cgccgtcccc tcaccacttc ctcttcccca gcccccacct gagagcagac attcggaatg 180

atgtgtagtg cgaggcggct agcctcccag cagaaagcca tccttaccat tcccctcacc 240

ctccgccctc tgatcgccca cccgccgaaa gggtttctaa aaatagccca gggcttcaag 300

gccgcgcttc tgtgaagtgt ggagcgagcg ggcacgtagc ggtctctgcc aggtggctgg 360

agccctggaa gcgagaaggc gcttcctccc tgcatttcca cctcacccca cccccggctc 420

atttttctaa gaaaaagttt ttgcggttcc ctttgcctcc tacc 464

<210> 336
<211> 512
<212> DNA
<213> Homo sapiens

<400> 336
acatgccagc tctgctgaag gcatcaatga aaacagcagt aggggcggcc gggctcctgc 60

gaacaacaac aaaacaaaca aacaaaaaac cacgtcgcgt gcggggcacc aaggcggtgc 120

ggggagggac agacccagct ggggctcctt ggaggggtgt gagccaggtt ggcggatcgg 180

ttcttagcaa cttccctgga gaaggggtag aacccagact tggtcaaacg tgcctagcgg 240

aggcatccag gcagcgtgac aatcacttgg tttccctcca ggaactggct gtttttggaa 300

taagccagga ctagagcagc acttccaaaa gtggggtgtt gtggacttgc ctgcatttat 360

ggtagatgag agcggcagga gcacgcctcc acgtgtgtca gtgggtggtg gggagcgtgg 420

tagtggtcct agttctgaat tccacacctc tttgtcaagc accggctctg taccaggaaa 480

gctaggtacc caagttccaa acatgaggac ca 512

<210> 337
<211> 414
<212> DNA
<213> Homo sapiens

<400> 337
cactaagccg tggttttcat attggtccag ctggacgagt gtgggattcc ggcagccgtg 60

ggttgcacgg agacggcagg tagtctctgc cttccaggtt ggagtcagca gtgcgaagaa 120

gcgttcgtat tcggtaggag agagagggct gcctggagtg gaggcctgag tcgaatcctg 180

ggctgcggca ggtgccagag gcaggagcag cactgcggag cgggcgaacg gatgatggaa 240

ggaccgaacc ccaaaacccg ctccgagacc aggacagaca gacccagact ggcaaattag 300

gaacggggtg agggttatcc ctgctgacca gggcgggggg agtctagaga cagaggcaaa 360

ggtccggccg cgggcggggg gagatgggag tatcccagga cctagggagc cgac 414

<210> 338
<211> 594
<212> DNA
<213> Homo sapiens

<400> 338
tcgaagtccc ttcccagggc agcacccgga ctgagatgtc taaactctcc ggcaggaagg 60

atacctatcc agacacccgc actggatgtc ctgtctgttt ggctttgagc tggtcattac 120

ggtgtctttg gatcctgcct aacttttctg taatttttat cagaaataag gcttcaggag 180

agcaaacgtt gagttatgcg ctccttctct ggagtctcct gccctggtgt ttgcgctccc 240

actgcagcaa gggaggcctg agtctcttag ggcaggggtc cagggtcttg ggagaggcct 300

tagggcaagg gggatgatct tttcagcct cccctcccct cctcccaccc agcgtcctca 360

tccccaccgc ctgcgataga cttgggtcgc ccattaggaa gctgatccgc agggacgggg 420

caggcttggc tcctggggat cttaggctcc atgggaactt cgggggtctc ggggcccgtg 480

agcggatcta gctactactt cccctaggtt ctcctcgggc ttcagtccct aagagtctgg 540

actccggact gccgcacaga atccttcttg cccggagcgg tattattgag gtct 594

<210> 339
<211> 486
<212> DNA
<213> Homo sapiens

<400> 339
cacggccgcg cgtggccgtg cgctctgcct atgggggccc ggtgggcgcc ggcatccgcg 60

aggtcaccat taaccagagc ctgctggccc cgctgcggct ggacgccgac ccctccctcc 120

agcgggtgcg ccaggaggag agcgagcaga tcaagaccct caacaacaag tttgcctcct 180

tcatcgacaa ggtgagcggg actggacctc gcctctcctc gagccgcgct ccagaccaca 240

ccagccgccc taactagccc tgcctgcgcg cgggccaggc gctggggagc actgccgccc 300

ttctgtcttt ccgccagtgt tggggacacg atctgggggt ccttcctcct tcgcatgaac 360

cccgtggcta ttttatcccc tcggtttcca agccctccct tccagcaagc tgcccagctg 420

cccctccctt tcctcccgga gtgcgggcct gagtcctgta gggctgccct cgctggctgc 480

ctgttg 486

<210> 340
<211> 874
<212> DNA
<213> Homo sapiens

<400> 340
cctgcgagat ggcggaggga ctgcagaccc gggtgcggga gaccagcgcc ggcgaagaca 60

ggcgggcgag ggaggcccct ccagcgtcct gctggggttg agttggggcg gctcgtcccg 120

tggccgctgg gtcgtctggt ttccgctttc cgaaagaaat gagaggagag gcaagtggag 180

tcgctgaact ttaattaaac cgtagagaag acagtggggg gaggggaaaa aaaacgatcg 240

gagaaggagg agaaggcggc cgagagatcg aggaaaggaa gtcctggcgg ctcgggggaa 300

cttggagatc tctccaaggg gctgaaagct ggaagttgta tgaaggtgtt ccttttccac 360

accccagcta ctctgcccca gtcgaagagg ctaattctgg ccctcttata aaaaaaagaa 420

agaaaaggta aagaaaaaga aaatctaatt atgtggcatg tttcagccag gtgttcctgg 480

ttccaatgac tcagacccta ttggagcccc gaggatctgg ataattgggc ctgcatagac 540

agagatgaag gatgccattt ctaaaaggag gaaggggaaa ggaaagtcca tctcttgggg 600

tcttgtagaa aatgccaaag accaaacgag ctaaattatt gtgtccgtgt agatctattt 660

gcctatttac atacagcagg ctgtgtgggt ggggggtgag ggaaggcaag gaataaacga 720

ccaggatgga gcgggctggc agtagagaaa atgagccccc cgccctttgc gggaacagcc 780

aaggggcctc ctcagctcgc agctcaggcg gccgtgcggc gggccggtcc ggggccgggg 840

gccggggccg gggccggggc cggggccggg gccg 874

<210> 341
<211> 861
<212> DNA
<213> Homo sapiens

<400> 341
cctcagtctt ggagccctgc cctgggtgtt gctggggcag ggcagagtcc ggaggaaaac 60

gacatagacc cttctccctg gggaccctga acttacaggg atgacagacc ctaatcttgg 120

acccaagtga atagagaact cttcttgcag cttaatcatg aaaaactccc caagagacag 180

aagtgtagag ccaggattgg gcagagtctg agaagggaga gggttccaag tagcaacagc 240

tgagggaagg tgcagaggtg aggaaggatg agggtagttt agccccagag acttggcgta 300

gggaaagaag agtcattcac tcttgtatcc atcattcact tagcaaacaa tcgagagagc 360

agctctacat cagcatggta ccaggaaaca cacagatgaa ttacacaggc agggacagca 420

actggagagg cttccagaaa ctcagtcctc atccagaata ctagcaccag cttcagagtt 480

ctctgggtcc atgctagctg agcttctacc tccccgcctc tccaatccaa aagaaatcca 540

ttccctagca ttttacttct tcctcagagt cccacccctg cctcagtcct tgccccagac 600

ccactactca aatatgccat gctcctcccc tctgggcaga ccctggagca ccttctggac 660

cacagctaca gcaggaggga ttggggctag acattttgag caccttccca gtggcagaga 720

gcagtggtgc tgaggagctg agcagagggc caaggaaggg atgtgagctt gagtccaggc 780

ccgagatcct cagcctgact ttgcatcttg ccgggagagg catcattcat ccacccaggg 840

gtggagatgg ggcaggggga g 861

<210> 342
<211> 511
<212> DNA
<213> Homo sapiens

<400> 342
ggggttcagt atgaagcccc cggaccgccc cgcccctggc cgcactgacc ggatactggg 60
ggtcatgggg ggcatgctgc gcgcatgcgc cctccctggg caggaggggg taagaatggg 120
ggctcgcggt ggggtcgggg gtctttcggg gccttcagga gggctgcacc cacaactcaa 180
cttatccgaa aacgagttct gcggggcagt cgcaggaata catgaattct attacgaggg 240
ccatagcccc gttctgggcg agccttttat aacaatgtgg gttttgacag gtaggagaag 300
gggaaggggg aggtcagggc tgcctaggag ggcaggttcc ttacagtggt tctgtccccg 360
cagcccccaa ggagaagccc tctagggttg gtgggtaccg agccagagtc tgaacgtacg 420
gagggagatc acagaaggga tcgcgaacat gaggtcctcg ccggggctct gcagcccggt 480
aagaagctgg gggtggggtg gggcacagtt t 511

<210> 343
<211> 398
<212> DNA
<213> Homo sapiens

<400> 343
gctccttgca aagttccttt tgtggaaaat cgcccagccc aagggagccc ggggtatttg 60
caacagcgtg ttcatttcca ggtgcctgtc acgggtctcc tccctgctgc ttctccagga 120
cccatgatga gattattttt aaaaattgtt tttggtcgtc tcccccgccc cctccccttc 180
tttatttttt tcctcttcgc tgcactcttc tcggcttttc ccctgacact actgatgggg 240
gtgcgggggg acgtcgggga tgggggtggc cagcgcggtc ctgggagtgg cgggttcgga 300
tgggctggct gcggtgggcc actttgggca tctcggcgtg gcctgcgccg gggtcacggg 360
gagggctgtc agcgccaggg cggcggaacc cgaggtct 398

<210> 344
<211> 469
<212> DNA
<213> Homo sapiens

<400> 344
ccctgcccct tacctcgtag gtgctggtaa ttcccagttt gtagaacacc gggaggaaga 60
cctccgcgct gatgaccacc acaaagaagt aggtgaaggc aaagatgcta aaaatggccc 120
caaaacggta gacctcggag ggggtgccca ggacagtgac ggctgacatg aagctagcgg 180
tgagggacag cgccacgggc actgcggtca ttctgcggcc gcccatcagg aagtccttgg 240
aggtctgctg gccgccccca gcgaaggcgt agtagatgcc gatggcggcc gagatgacca 300
gcatgcccgc gaacaccacg tagtcccaca ccacgaaggt gccgatgccc cgtggcgtgt 360

ccatggccgc acggtcgcct gagccctgcg cgcaaactgg tggccccgcg gcgcgcagcc 420

ggagcccggc gcgcacttct tatcccggat ccctggcgcg caggcgtgg 469

<210> 345
<211> 524
<212> DNA
<213> Homo sapiens

<400> 345
gggaaaccaa ggacagaagc cgccccctag gacgacgcgc ccgagccacc gggagggtct 60

gaacggcggg ggcctcctgg agcgccttca gcgcctcgtg ttttctttcc ttgggtccct 120

gttagcgtcg ccgcatgcac agcgctccct gtcatttggt ccctgagctt gggtgttttt 180

cacatttcat ctaaagatgc agacccctgc taagtctagc cagccttcac cctgaatctt 240

gaacttaaga tgctgcattt tccacccacc cccacccctc tagtagctgc agattgacaa 300

gcggcttttc ccaaccagat gacattttgg ctgcaagaat ccgtggcccg ttcttgcctc 360

tgtgcagcca agctccttcg ctgtctttct caaactgcag tcctgagttc agcttcgctt 420

agcagtgacc atcccagagg tgggtaatcc aacactggat ccactcactg caaccggagc 480

tggggatggc atgccctctg cttccactaa ccgagtggcc tgca 524

<210> 346
<211> 530
<212> DNA
<213> Homo sapiens

<400> 346
ctataatccc agctactcag gaggctaaag caggagggtc tattgagccc gggaggcaga 60

ggtcacagtg agctgagatc acaccactgc actccagcct ggatgacaga gccagaccca 120

gtattaagga aagaaaaaaa aatccatgcc tctgcctcaa ctgaaacagt ccagatgggg 180

taaagagacc cttcccttct cttgtaattg gaagtcaggg gaagagagcc atcctttctc 240

catgctaagc tctgtgccaa attcatcaca cacatgatca gtttggaact gtaattatcc 300

tcatcttcca gatggggaaa ctgaggctca gagggttgat gggacctgcc caaggtcacg 360

tggctggttg ttttcaaggg ttaggggggt ggcgctgctc ttcttttccc atctgccctc 420

tccccgcctg cactgtgagc ccctgagggc agaagcagtg cccgtttatc ctggaatctc 480

cggcctggca cctggcacac tggagttgcc gaatgaatga aggatgcagc 530

<210> 347
<211> 443
<212> DNA
<213> Homo sapiens

<400> 347
ggagggattt gcctaagctc acagagttag ggtgtaaagc tcagacatcc ggatgtgaag 60

tcggggacag acactgtcgc tctgctaatg aggcagctgg tgtgtcctta catcatgacc 120

cccccaggcc aggtagggtg accccaacat ggccaccgct tgtctccctg ccttgtaatt 180

acccctctga tgcctcccat ccactcaggg tctgtcactg ccccagcatc tagcacaggg 240

cctggcctgg cacatagttg gtgtttaatc tgtcatgccg tccccagtgg ttggagtgct 300

gacggaggca catcgcagtt gtcagttccc atggcgactg acatcactat gctctcaggc 360

tgtaccccgc ccctttctcc agggctcttg cgccgggagc tggccgtggt gctgacaggc 420

ccaggaatgg ccacctaccc cca 443

<210> 348
<211> 759
<212> DNA
<213> Homo sapiens

<400> 348
gttctctctg gccacgttag actcgggaag aggctctgtc gggattggcc ggaagcagaa 60

gggaggtcac tggtgttgtg gctgctctgg aacaactggt gctgatggct cccttggcga 120

aggagtctgt ttttcaaccc aggaaggtta gtgtgttcct gttactaagt ggctgccgtt 180

cctcaaatgc tgtcattgag ctggtaggca ttactgtgag atctaaacct tctcaagaga 240

agaggggaca atgagtattt tctgtatgct tgttattagt tctttcccca ggggtgttaa 300

atcagtgaac acgacagcta agataaacat ctagggagct tggctcttag gtctgcattt 360

gccacattgc tttcggaaag caggagatat cctttctgtt gacttagagt ttgctgcaac 420

agggtgtgct ggagtatctg tctgccttcg atgaagaaac cacggaagtt tgttctctgg 480

acactccttc tagacctctt gctctccctc tggtagaaga ggaggaagca gtgtctgaac 540

cagagcctga ggggttgcca gaggcctgtg atgacttgga gttagcagat gacaatctta 600

aagaggggac catttgcact gagtccagcc agcaggaacc catcaccaag tcaggcttca 660

cacgcctcag cagctctcct tgttactact tttaccaagg tgagggtgcc ggaagagagg 720

gctgggttgc cgcagaggtg tttcagaggt gacccggtg 759

<210> 349
<211> 710
<212> DNA
<213> Homo sapiens

<400> 349
agtcacctcc aggccagtaa aacagaaaca caggcagttt tgtagatgcc ggggctctac 60

cagcttaagg tggtcagttc ccagcaaggc tctttcagtc catctgcatt ttagaatttt 120

ccagtccaca aggtacctgg tgactctctg ggcaaaacca attgacaagg ggacagttac 180

ttggcataca gagctgctgt tcccaccatg taaccctgat gcatttcaac agtcgtcttt 240

actcgacgca gcacttggtc ctgatatgca cgcagattga gaaaataaag aataacggct 300

ccccaggaaa cagttccaca atatacacaa atgaaaaagg atttacacag tagaccctaa 360

atagtttcta aggagccatt atggaaacag gtaaattaaa tttaatttaa ctgtattttc 420

ttcgtttaaa caaatatatc tggtgcttgg aaaacagcag gttataaaaa tgcttaatgt 480

ttctgtgaat attaagcata tgcaaaaaag acttctttgt gacacttttt ctgaagccca 540

atttctcacc atggcaagag ggatgatgcc cacaaaggtt gtctggctga tgaagatctc 600

agagacgacc agctcactcg tggagtcctc agtcagggac tccccagcca ggtaatttgc 660

cgggccctgg taaggccacg tctggtgcaa gatttaacag aaggagccat 710

<210> 350
<211> 681
<212> DNA
<213> Homo sapiens

<400> 350
catgcaccgg ctgactgatt caagcaatac ctgtgtggat aaagccaccc ggagcctcat 60

ctcacttagg gatttttgtt ttgccatttg tttgttctgg gtaaggatta tgagggcact 120

gttagtcaca tccccatttg ttcttgttta aattaaggtg gtttctctgc ccctgataga 180

aaacctgtat gatttctccc cagatgcatg agctactggg tctggatttg aggtcctgct 240

ccctgtttca ttgggcgtcc tcctaggagg gggtttctaa gggaaaaggc atcccccagg 300

tggtcttcta actgcctgtg tccctgcagg aggctgtatt cagcacgtgg ctgcagttca 360

gtgatggctc tgtgacgccc ctggacatct acgacaccaa ggacttctcc ctggcagcca 420

cctcccagga cgaggctgtc gtgtcagtcc cccagccccg ctctcccagg tggcccgttg 480

tggtggccga aggggaaggc cagggcccac tgatccgagt ggacatgacg atcgccgagg 540

cctgccagaa atctaaacgc aagagcatcc tggctgtggg cgtcggcaac gtcagggtca 600

agttcggaca gaacgatgct gactccagcc ccggcgggga ctatgaggaa gatgagatca 660

agaaccacgc cagcgaccgc c 681

<210> 351
<211> 439
<212> DNA
<213> Homo sapiens

<400> 351
cttcgatggt gtctgctggt ggacagcagt tttcaacgtc agtgcagtcc ggtttatcaa 60

gctcttctgt attcagtgct tttatgtctt ggtggaggaa tcctgcctaa cccgaggcca 120

tgaaagtagt ttcctgtgtt acctggaaca tttgttgttt tcactttcgt gtttgggcct 180

gtggtccttc tctgcttgtt tctgtgtctg cagtgcagta ggggttgaga ctgaccactg 240

tccccagacg gatatgtaat ttacccagct ccacgtgtgg gaagaccaac ctctccccta 300

```
gtgctgtggt gccgcctttg ccgtcagtca cgggaggggc gacgtggctg tttctggact      360

ctgctctgtc cgtcatccca tttgcatgcc ggtacgattg caactgccac gtcctatgcc      420

aaggcctgag accggctcg                                                   439


<210>  352
<211>  535
<212>  DNA
<213>  Homo sapiens

<400>  352
agacggggtc tccagccctg gggtaagag atggcccaca gcaatgagcc gggctttcct        60

gggttggttt catagctgag ccgccttgag ctgggggacg ccgccctcga catccccagc      120

acagacgccg ccgtcgacgt tcccagtgca gacaccgccc tcgacgtttt cccagccagg      180

acgccgccct caacattccc agaggggacg ccgccctcga cattcccagc aggcgcagct      240

caggagcacc ttgccatctg cagctccctg ggggacgaga agaaacagca gccacccctt      300

tctcgggcac cttcagctcc gccgagaacg cccgacactc agagaccctg ctctttgtta      360

aatgggaatg agacatgaca cagacgcggg gcagcggcca ctgcagcacg ggttgtccac      420

tgcccagctc tggggccaaa ccctcggcgt cgcgcaaacc atacctcccg cgtgacggcc      480

gtgtccggcc tgctcacctg tgcgaggcag ccctgcagag caggtcacca ggcca           535


<210>  353
<211>  514
<212>  DNA
<213>  Homo sapiens

<400>  353
ggaccagtcc cgttgtaaaa ccgacaaata acagcaggat taggaattcc ggctcaggat       60

tcacgcagcc agacgccaca ggactcctcc ccagccgctc ctgtatatga cgtcaccgcc      120

gtaagaccac aggacaccgc cccagccgcg cctgtagatg acgtcaccat cgtaagacca      180

caggaccctt cccagccgct cctgtatatg acgtcaccgc cgtaagacca caagtcaccg      240

ccccagccgc tcctgtagat gacgtcacca cagtaagacc acaggatacc gccccagccg      300

cgcctgtaga tgacgtcacc atcgtaggcc cacaggaccc ttcccagccg ctcctgtgga      360

tgacgtcacc gccgtaggac ctaagattga tgctggagag gttcttcaga ccctgcgttc      420

tgacggctcc gctggcacca cccagacggg taaactagct cttccggtct gtggccctca      480

caggaaccga ctcggtgcag gaggacagct tcag                                  514


<210>  354
<211>  618
<212>  DNA
<213>  Homo sapiens
```

<400> 354
caagcggatg agggactaag atgtgcagag caggctgggt ggggactccc ggggaggtct 60

cccccaaccc ccgccccacc tcgggcaccc acttcgcgat ttttgcagag gggagccagg 120

tcagaggtgc agcctggtcc cctcgcgctc acgtttttac ccaggtcagt tcgaagttaa 180

gtggaaatga tgattaatcc tgacaagtca gatctggcct cagaatggat ttcccgtgat 240

tgccaccatt attagcattg acttttcctt gaaaaattgg cgccccgtgg ccatgggccg 300

acctaggcag tttctgcagg gacgagcgtg agttttgtac cgcggttacc acctactttc 360

cagctccagg tcttagtcta agagggagtg tctgctcatg aagaggcaaa gccccaggag 420

ctgcgaaaag ccttgcatgg cccatctgag agatgtgctg agtcggcttg ttaaaaatga 480

caggcaaagc ctgtggggtg gggcagcttt cttggcctga gcgcatcttg gttgagccag 540

aggtgacttg gggtggggag tggggcgccg gttggtgggt tctcccttta atttctcaaa 600

ggctgtggtg tttatgag 618

<210> 355
<211> 591
<212> DNA
<213> Homo sapiens

<400> 355
tctggggaag gggagcgcca gagcctgcca gcccagcagc cagccctgcc ggaaggcccc 60

caagccagga tcctgtcagt ctccatctag aaaccagaca gaaagcactt ttgtaaatgt 120

taaccaaaaa ggtccccctg tgatggctgt gctgtaaatc aaagtagaca atgtggggtg 180

ggggcagggc acacgtcttg tctctggagc catcgcaaag cagggagggg atgctccttc 240

aactctcagg tggggactgg agctccccgc tagtccacgc tggagaaagg tttttagggc 300

ctggagtcac ccaggaccca tctctgtcca gggggtgctg tgtaatattc accattaatc 360

ctacacatct gggaagcatt ctcctgctaa agataagata aagcccaaat gatttgtgtt 420

gggggctggc tgggggggctg gggggaggcc agacctggaa ggctgggcac acaggagcac 480

cctaaccagc aagcggggga cgcagcctcc gcggcatact cccacagtct gtctgtgttg 540

ccgggcagat tccctaagca tggtgcactg aggtacacag ggaatctgga a 591

<210> 356
<211> 651
<212> DNA
<213> Homo sapiens

<400> 356
gccggcccac tctgagtgca cagccacact gcttctcgtg cccgcgggcc gggcgccacc 60

tggcgcagaa gtggtgctga tgccgccgcc gcacgagaga ggagagccac gcatcaggca 120

gcgaatggga gggcagcttg aaccctccct gttctggtgg gtgttttggc cagcggctgc 180

atccttctgc tagtggccat catcactgtg acttgttctt gccaaggcaa ggctaggacg 240

ctggtgaaga agatccgtga gcactcccca agcagatttc aggcaacgag tgaccatgct 300

ggtccacgtg tcagagccat aggggctgac tctgggggct tctgctccag gttctttata 360

gacacacgga cagtttccat ttccacaggg gcccaggcgt tcgagtctgt ggaggattcc 420

tagtctgagg tcagtgaggt gtctcggagc tcctcttgtc gggagaggag caggactgca 480

tgggcccagg tagggcaccc ctgccgttct tagtgtcctc attctcttgg agtagttttt 540

catgctagga gccttatgaa aattcagtgc ttgatttggg acttcaaatg gctgatattt 600

ccgggcttgt tttttttttt tttttggaaa ggaaaaatta tccacaggca a 651

<210> 357
<211> 286
<212> DNA
<213> Homo sapiens

<400> 357
gccggactgg ttccccatct aactgatcta atcaatgact ccgaagagcc ggggctcctg 60

gccccgcccc cgccatcctc ttctccgcct cccattggac gcgggccgag aaggcagggc 120

cttgctcctc gctcggattg gctgacaggg aatcagtatc aatgtttaag cggcggcgtg 180

aggtgaatag agtcagtcag caagagacgc tggggagaga gcagggatcg cgagcccggc 240

ggatgcggca gcggcggaag gcggctgggg agacgctact ccaact 286

<210> 358
<211> 574
<212> DNA
<213> Homo sapiens

<400> 358
gcgcgacggc ggcccggagt tgggagtggg cgggcgggcc ggccagcgcc gggaagcctg 60

tcatattctg aagcggctgg acctgagccg ttgccaaatc cgctaatctc agcgctggcg 120

ggttcctctt gctcaaaggg ggctccatca gaactacaca atcaggccca tagaccctca 180

ctggggggac tttattccct ctgcccgcct tcaaaaacat gtatgtattg ggcgctcttt 240

aacttctttt gtcctggcct cttaactctg cttattcctg ttcccactct atcctccagc 300

gattggcaga gtgaacacca catttgagct gcacagtggg accgagattt tggaaatggc 360

acgggtggga ttggagggag ccgcgtgatt ggcagtagcc tcggctgctc gattggctcc 420

cgttcaggcc ctcgacccag cggggattcg cccccgtgct tgcggccgcc tgcgctttcg 480

cttcgcgccc cctcctccgc tggagccaca cgtccccgcc tccccgggga tgcccaagtt 540

gcacttgcag aaagtttgag cctggcctgc gcgc 574

<210> 359
<211> 571

<212> DNA
<213> Homo sapiens

<400> 359
gcgcgcaggc cacgcccccc gggtgcgcgg gccgggccgc agagcacgcc gggagcgtgg 60

gtctcccagc ccgccacatc ccagggctgt acgcgcggac tagcggagct aggggaaggt 120

gccagggtgg ccgccgcctc agtagcacgc ggacggctgg aaagtcagcc cccgtttccg 180

cctcacccag gattgctcca ctcggggcct ttccgcatcc cagccaccac tccattccga 240

ggcgcgatca gctctgacca gtcttcctca cacccatagt cgattcgtta ctcccgttaa 300

ggtcaaacgc tgacagaaac cagccgcttc gcacacttct acatggggtc aagccccctg 360

ttatctttgc ggggatgatt gtaacaatca cttaaatggt cgcttcgtac catcttgccc 420

ccgcctgggc cttgtcggcg acgccgcagc cacgtggatg ctgtggaagc ctgtcccctg 480

gatcttccaa aggctccgcg ctcattgcca cagccagcgg ccggcagtgg cctaggctgt 540

tgcacgcgat cggtccccca catctcccct c 571

<210> 360
<211> 657
<212> DNA
<213> Homo sapiens

<400> 360
aaaaaagact ttttcttccc ttctatatta gcactattct agacgtttcc ggtagcatga 60

tatgagaaaa atacacatac acacttttag gctggtgcgt ttttgtttaa aatgtgagcc 120

tatctccaaa tacatgtgta tgcaggacca tgacattgag gaccacatct gaccctccaa 180

tccagcccag tgctcagtgg aatgccagca tgtggtagat gctaatgaaa tatttttgga 240

tgattcagta cgcaatttat ttgaccaagt gttagttatg agtaaatttc agagccaata 300

ttcaaacaca gtttagtgtt tatccaatgt caaagtttaa acaactaccc gatcttactt 360

ctctgtgata gaaaacagaa tcactcatga tgcctggcta acctgattta tggggttgtt 420

aggaagacta aagagtattt tattccatat gaatgtgttt tgaaaactat gaaacactgg 480

acagatgtaa ataattgaat tattgagata caataaatct atagcactcc tttcaaactt 540

aaaaaaaaaa tcagtgtcat ctagcaaaag agaattggaa aagagacttg tcttaaaata 600

ggttttccgg ttgggcgcag tggctcacgc ctgtaatcct aacactttgg gaggccg 657

<210> 361
<211> 759
<212> DNA
<213> Homo sapiens

<400> 361
aatgactgca aaaacaagca cctgcactcc gggtgagcac gcggagctcc gggcgccctg 60

cttttcatgc tctctcttca gtcactgcat ggatgggcaa agaacctaag cctggaattc 120

tggtgctggc tccagcgcca acccctggga cgctctgaaa atcagctttg ccatcatctg 180

taagtcaggg atgactccac ctgcctgttt tacctggcag cattatttta aaaatcaaac 240

gtgctcaagt attcagttaa atgctgtaca aatggttctg aaagaaactg gtctgacttc 300

actgcttttt aaacacgtaa atattttgtg tttattctca cgatttctat gtccaacaag 360

ccagctgcat ttcctctgaa gccacagaat gttttaaaag agaatgaaaa ggccacactg 420

ctagtgttcc cacaagtcat gacccaggaa gtatctcatt cctgtttttg tggctaagac 480

agacataaga aaattggaat ttcttgagta tttaaaaaag atgacagttt caagaaactc 540

ccccctttat cttatactca aggttttgtg ttttttcctt aataaatatc tctgtaattc 600

aggcagttca agcatagaac attccattta ttctgtcagg tcagcctggg tcttagtaaa 660

gccaccacac tcctccatgg ttagcctgga agtagctaac gacatacacc ggtctgaaat 720

ccccagatta cttccttacc cccaagcaag ttacttgtc 759

<210> 362
<211> 321
<212> DNA
<213> Homo sapiens

<400> 362
caaacctatc tctatatcct cagcactggg ctctgcatct ccacatgacc ggacctcagc 60

aatgtttact ggatgaatgt gagagggagg gagtggatgg cctttgaggc tttgcagcca 120

cagttttctc cattaccagc tctgatacat tttctgttgt ttccaactcc attccccttt 180

cacctgctca cctgctctcc tgctctccag cacccactcc cctttctgga gcagcactaa 240

tgattcctaa gttgtgtcct gatgagctgc ccggccaact ggccgggcct ctaacctcct 300

gaagcgctcg ctgatggccc c 321

<210> 363
<211> 388
<212> DNA
<213> Homo sapiens

<400> 363
gaaacagcaa tgttcatcac aaaggaatca tgatccaggg gatgggcgcc ggttcctgag 60

gacacagaaa gggtgtgtct ggggagcaag agccgtagtt tcggcgccag acaccgaggc 120

aggagctgcc gcagcagccc ttggggaccc cgaacctgcc agtggagagc caggtggggc 180

gttctctgag gagggtgaat agagttcccg cccccagccc tcccgttcct gcttccttta 240

gggtcaccgt gcgttcctgc ttcctttagg gtcaccgcgc gttccctgca gagaacacgc 300

tgggaaggct ttgttgggac aacagcttct gcagccgccg gcgccctcct ggccgggctg 360

cccccacgtc ttttccctct gtgtgctg 388

<210> 364
<211> 592
<212> DNA
<213> Homo sapiens

<400> 364
tggatgcgca gagctgtgag cggtcggacc tctgtcccat gaggaagccc ggatgacctc 60

tgcgttctgg aaactgcttc tgctttagtc atttcatcgg ctgacctgtt acagggcttc 120

tgtctggatg caggggaggc atggcagggg cctgtggagg agcccaggag ggaaatgggg 180

cccgactgga gggtggtgga gcgtcatggt gcctctttca tcaggccttt aggaaacaca 240

gggaagtctg ctgctgtgta tggacaccat tatgaccctg acttactctc aaggactcac 300

tcctaggctg gagctgacct ggcgttccac ggccaccagc ccaggttcat gaggcttcag 360

ctgccatcac tagtgtttct ctcccgcttc ttcagtttca gaaacgatca tcatccccct 420

ttatcctgaa agccccagtc cctgttttca cagaaataac aatgttcccc taaagaaaag 480

gaacaccaga gaattggatt caagatctaa atgacctcag taaccacact gtttcggatt 540

tccgggaatg ccacgtcagc aaggagggtg gaggctgaac actcaccgcc cc 592

<210> 365
<211> 405
<212> DNA
<213> Homo sapiens

<400> 365
gtgacggggc gcggcgcgtg ggacgccaag ctgacgtcac tgtctgtgcc ggtgtctcgg 60

gcgggggcca gtgtggctgc caatggggca aggtgcgtgg gacgtcaagc tgacgtcact 120

gtctgtgcca gtgtctcggg caggggctgg cagggctgcc caggaggctg tcggtggggc 180

agttcctcat gaatctctga gctgagtttg taaaagtgtg tgagagtggg gaaaatatgg 240

gtgaaaatat tttcagacat ttggattccc ctttggtgag ctgtctcgtt tcttcccgct 300

ggtcagggat gtggctgctt gaacagctgg gcgcggtgac caatgtgtgg agagccggga 360

agcctgcggt gaggccgcgc gatgtccttc atcccgggca gtaaa 405

<210> 366
<211> 396
<212> DNA
<213> Homo sapiens

<400> 366
actacaggat agaaacccca tgggtgtttt cttgtttgct tttgtatccc ggggactaga 60

atacctcctg catgaggtag ctggtcagta atcctgcagc ctaagtgagt aaccaggctc 120

tgtacctgag aaactgtaag gtaaagaagg ttgaaattgt cgtcatggga ggtgcagaga 180

tgattgaata gtgaactttg gtaggttgac tggagccatg aggccccagg gggcataagc 240

```
tagtttagca gctctgtgac gtcggaaacc tacaggtact gggtccaagg atggagtcat      300
tggaacaccc cagtgtggcc cccgagtagc agcaaggggc atgggccggg aacctatgat      360
tctggcacga aagtgttgaa aagagggact gggtgt                                396

<210>  367
<211>  493
<212>  DNA
<213>  Homo sapiens

<400>  367
gctttgcgga tggtggaact gaggtccaga ggagggcagg ggcaggtccc gggtgctccc       60
taggcaaagg gagccgatct cggggagggg gtcacaggga gcgtccctgc gacttctagg      120
gcccgaaagc tggggaggat gagagaccag gggtctttcg tcgccccctg gggctgggca      180
gaggctcagc ctgtgttgcc acccaaagct gcttctggca agtccgagcc gcgtcccttt      240
aagagggggt ggagcttcaa cctggcacga gggatgctgc cagcgtgcgt gtccctacgg      300
aagctgagac tgcacttcct gcgaggcccc tgcagcagca gcggcgtggt cagagcgagc      360
ttcggagaag cagtggtggg ttccatgtga tggtggagta ggaggcaggt ctccgcggta      420
agtggcgggg gcgtggaccc caccgggaac cctcccggct ccttccctgc ctctccctgt      480
tttcgtgctt tca                                                         493

<210>  368
<211>  790
<212>  DNA
<213>  Homo sapiens

<400>  368
caaggctgac gtgggacgac gtgggacgcc gtgggccgcg agcacgcccc ggcactactg       60
cggctaagtt tggcctttcc tggttgccgt cctcccctac aggctgtgcg ccgtagcggc      120
tgttatcctt cttttccgtc tcaggaaaac agcgtgcttg gtgggtttga gagaacttct      180
gtttttctgt cctccatgtt tgaggcggag aaagaacatt ttgttcttga ggtgatgatg      240
gattgaatgt aaagttacgc acaaagtggg atgtttagtt ggaactgttc cctcaggaaa      300
tacgattttt tgggcgtggg ggacagggtt tcgctctgtc gcctaggcct gagtgcagtg      360
gcgcaataac ggctcactgc agtctggaac tccaagggat cttcccacct cagcctccga      420
gtagctggaa ctacaggcgc gcgcgaccat gcttggctta tttttatttt gtagagagga      480
ggtctcacca tgttgcccag cctagtctgg aactcctggg ttcaagcgat cctccatctc      540
ggcctcccaa agtgccagga ttacaggcaa ccgcaccgag ccaggaagtg attaattttt      600
ttttaagtca cttcacattc cagaaacatt aatctcgaca actctaggtt gctgtaagca      660
gcttgtgacg gtggccccca gactgcgtcc cgctttagac tgcacccagt gaatctccac      720
tctaaccacc gcggagtgac cgggtgtatt cttcgccttg tatagggcac attggttcag      780
```

tcttgaaagt 790

<210> 369
<211> 538
<212> DNA
<213> Homo sapiens

<400> 369
gcccgcttcg aggagctcaa tgccgacctc tttcgcggga ccctggagcc ggtggagaag 60

gcgctgcgcg acgccaagct ggacaagggc cagatccagg agatcgtgct ggtgggcggc 120

tccactcgta tccccaagat ccagaagctg ctgcaggatt tcttcaacgg caaggagctg 180

aacaagagca tcaaccccga cgaggcggtg gcctatggcg ccgcggtgca ggcggccatc 240

ctcatcggcg acaaatcaga gaatgtgcag gacctgctgc tactcgacgt gaccccgttg 300

tcgctgggca tcgagacagc tggcggtgtc atgaccccac tcatcaagag gaacaccacg 360

atccccacca agcagacgca gaccttcacc acctactcgg acaaccagag cagcgtactg 420

gtgcaggtat acgagggcga acgggccatg accaaggaca ataacctgct gggcaagttc 480

gacctgaccg ggattccccc tgcgcctcgc ggggtccccc aaatcgaggt taccttcg 538

<210> 370
<211> 376
<212> DNA
<213> Homo sapiens

<400> 370
gaggcggcgg cagcccggcc ccctcccgcc gtcgccccgc taagagcccc ggccgctgtc 60

cccgcctgac aacccgcacg ggaaggaaga agccccagga ctcacgtcgc tctccacctc 120

gatgtcatcg ttatcgctca tttcctacgg cccagggagc ggccactgca gcggcggcgg 180

ggaggggaag gggtgaaggg gagggggaag tcaccgacaa caacaagccg agtccccccc 240

acacacacac tcactcactc actcactcgc tctctcactc acacacacac acaacacggg 300

caagaaccac ctcctcactg cagcaccgga tcaacggcgg cacgcacgcc cggtcggccc 360

ccgccacgtg accagg 376

<210> 371
<211> 668
<212> DNA
<213> Homo sapiens

<400> 371
ccagagttcc agaggaggac cagggagagg ctctggacag gaggcgatcc ggcctgagag 60

cgggcagtga ggttagcatt tcaaagatgg agatattctg ggacagggg agagctgtgc 120

catggacagg tagctggaga cggtctaggc agctaccagg atgacaccag ggattggagg 180

gagaggatgt taggttggag ccaaagcctt caggatacga aaggagtgac tgggggtggc 240

agctgcaaag gtgggagcgg gacactgggc cgtgggggct ggagaggtgc tggggtgtta 300

gagacgtcac ccgccgccct tcctgacaca gagtccgtgg aaagagcgag aaggagccca 360

cgtcctggga gagggctaag tgggcgagga ggagctagga gggcagccag gacaggaggc 420

aggaggggag gggtaggagc acaaggacgc tggcactggg gggcagggag gacaccctga 480

gcaggctgga gaaacaaccg tgggtgcgat caggggactt ctgtaggaag aaggctggcg 540

aatgactcgg gagagcggga ttggcaggca cagggtcacc tgactgcaac tctgcaaaga 600

gcccagtccc atcagccccg ggaatggtgg gatgctgggg gcactgagtt ctctcgggat 660

cacttaca 668

<210> 372
<211> 660
<212> DNA
<213> Homo sapiens

<400> 372
agctaacagc tcctgtgggt gacgatgagt aagctgcagt ggagaagccc ggggacaggg 60

actgctcaga aggcagaggt ttcaaggtct catggagatg agatccactt cccaggttat 120

gtactgatgg gggtggtggt gacagaccca ggttatggag gctactggat gaagagtgca 180

ttctagtctc tgctgacctt agacgctggt tctaagtagt tcattcatag tagaaactct 240

gctcttttgg acctgtctga tcaacctaag ggaaaaggat ggaatatagt cctagcatag 300

aaccagcgtg ttgtgcaggg aaactgaggc ccagtctcct aacttcagat aaggtggcat 360

gaaggaggga agggatttgg tgagcaggcc agtcctaggg gtccattcag gaaagccaac 420

ttccagggcc agtatgtaca gctgtgtgag tgcataattc cagggataag aatgttcatg 480

ttgtgcttgg cgtggtgcag tgcataacct gcacaactgt atatgatgat cctccttgga 540

attcacgcta ccacaattct ggtccctact tattggtagg aatgatgact tgctaatccc 600

agtgacaggc cggtcccaat tctacattct ccaaggctga tctcaggaac tggagaaggc 660

<210> 373
<211> 322
<212> DNA
<213> Homo sapiens

<400> 373
acttcagcga taagtgtttc ggtgttcctg cgtgcggatt tgtgctctcc ggggaggtct 60

gcggcccagg ttcgattcct gcgacttgtc ctaggcaggc ctgtatgtgc gcggcggccg 120

cgtgctgtac agtgtgaggg aacgtgtacc aaacgctcgc gggatacctg tgcccgtcta 180

gccaagagtg cacccgtgtg cgcgagcggg cttctgggac gccgccgtgg tcgggggcgg 240

ccctgcgagg ggagggggtc acagggactg gccggcgccg gccccgtgcg cacggaggcg 300

ggggcggggg gcgggggccg cg 322

<210> 374
<211> 476
<212> DNA
<213> Homo sapiens

<400> 374
ttgtatcccc aggagtgccg tctgcctggc ctccccattc ctgcagtccc ggttgtcatc 60

tatgtgggcc aggccatgag ccctccaggc aggctgtgct tcaacccatc ccctgcacca 120

acagcagcct gctcatctcc aagctcagct cagcccaaga gcagaaaggg agtgtgctgg 180

aagcaggttc aggaagatga gcgttcatgg gccgtctgca tgccctgggc tggggagctc 240

tcagtgggag ccacgtgggg tcgaggattg ggggaggcta tgcaggccca ggggcctcgg 300

gctttcgagc tcaggctctg gaatagcctt cttcatgcac ccccacacag ggactgacac 360

tgatttgcaa gaatgccatt tcgaaatttc acatcacact tccaccatgg ccccagacag 420

caagcccggc ccagccccag acaggcaggc aatgggcatc tgctcatcat ccagca 476

<210> 375
<211> 612
<212> DNA
<213> Homo sapiens

<400> 375
gagcaggacc taggttcggg gcggctgttc ccctctctgg catggcctcc ggcagtggcc 60

aggagacggt tttggacaaa gcttttctca cagtggttgt tccagttata cccactgtga 120

ctcggggctg ttcagaatct gcccaggtgc cctgagctct ggggcctcct gggtgggggc 180

tgggcttgtg ggcaggatct cctttggggg ctctggaggc tgtggctcac tttggttgtg 240

gggtgagcac tggaagcccc agctagcaga acacccacag agactggggc ctgcacacat 300

tccgccccag tgtgtggggt gggcccaggc ccctctgcgc aggtcagctt caatggggag 360

ggtgctcagg tcctgcttgt tttcctctgg gttaatggga ttcatctcct ggccccagat 420

cctcacaggc tgcccctgtc cctccagcaa tgcaggacat ggcaggtcac cctggaggga 480

ggcatgttct ggtctgggtg tcaggtgtgg cacctcagat tttccatgca tgctgtgggc 540

tgagcaggac agcagatgac cccgggcccc caccctgtct atggacattt tttgctgcgg 600

caactgtggg ag 612

<210> 376
<211> 518
<212> DNA
<213> Homo sapiens

<400> 376
cagggcggat gtctgagggg tctgcgctgg gctttccggt ggcttcgccc ggaacgcgcg 60

cttgttcctg aggcggtgcc tgcctgtgtc gtcgtccctg tttgttcttc aaacgtcagg 120

atttctccgt gtttcccaga ccgtaacgta catttaaaaa gcttgctttc tgtcacatat 180

gcccttttat attatggaca taacaattgt tacttaccta cgtttcctat ttgtgaatta 240

ttcatagata aagaatttac caaaactttt atttttagtt gacacattgc gtatatttat 300

ggataacagt gtgatatttt gattcttgta ttcattgggg aagattcaat caagctaatt 360

aacatgggca tcaactcacc agctaatact ttatggtgag aacactaaaa atcttttatt 420

tagctattct aaaatgtaca gcgtgacttc ggaggaaatg atactgcccg gcctcagtag 480

cccgagactg atcatttcta aatcttgagg ttcccatt 518

<210> 377
<211> 783
<212> DNA
<213> Homo sapiens

<400> 377
aataaacgga atttgggccc taaagtcctt tgttctggag aaccagatcc ggaatgttca 60

gaggcttgct gttgtgccgt tctgccccga tggtatcctg tccgctcgca ttggggcgcg 120

tccccccatcc gcccccaact gtggtgtcgc gacaggtcct attgcgggtg tctgcggtgg 180

gaagggcggt ggtgactggg agcatgcggg gtaaccgcag tgggcaaggg acatgggtgg 240

aggtgggtac atcagggtga ttgcagttcc gtgtggcgag ggtacgtggg gggaccagtg 300

catagggatt ttaggcggag gtaggtatat tggagtgatt gtggcggggc gaaggtacgt 360

gaagtgatcg gtatttaggg ggtgttgagc gcaggtaggt gtataatagt gaccactgcg 420

tggtggagca gggtacgtgg ggcgactggg gggggaattc gtcgcagtga ccgaggcgag 480

gaggctatgg cagtggacca gggggatgag tcggtgtgac agtggtgggg gttgtgttgg 540

tggtcgcggc gcggggagag tccgtaggaa gtggctggga ggtaggggga aggcggcgac 600

agtgggtatt ggcggcggtg ggcattggca gcgggtaggc atggcggtgg tggactttgg 660

cggcggtagg catggcggcg gtgggcatgg catggaggcg gtgggcatgg cggccgcggg 720

gcctgtcgct gtccggagtg actaagggac gctgaatgat tgctgtgtag aggaggggggc 780

atc 783

<210> 378
<211> 433
<212> DNA
<213> Homo sapiens

<400> 378
cagggtgctt tggagtttaa gaacgtgcat tttgccgatc ccgcttgccc ggaggcgccc 60

atatttcagg atttcagcct ttccattccg tcaggatctg tcacggcact ggttggccca 120

ggtggttctg gcaaatcaac agtgctttcg ctcctgctga ggttgttcga ccctgcttct 180

ggaaccatca gtcttgatgg ccatgacatc cgtcagctaa acccagtgtg gctgagatcc 240

aagattggga cagtgagaca ggaacccatt ttgttttctt gctctatcac tgagaacatt 300

gcttatggtg ctgatggcct tcctctgtga ccgctgagca agtccagaga gtggctgaag 360

tggccaatgc agtggtcttg atccggaatt tcccccaagg gttcaacact gtggttggag 420

aaaagggtgt tct 433

<210> 379
<211> 152
<212> DNA
<213> Homo sapiens

<400> 379
ggcgctcaca tccgcagccc cgggatctta gagacagagc gctccgctcc ggcagcggcg 60

ccgcgcccat tggctgggct cctcctcgcc ccgcccagcg accggcccgc tcagagccta 120

cgcatgcgca cgagcacccc gccgccgctg cc 152

<210> 380
<211> 524
<212> DNA
<213> Homo sapiens

<400> 380
cttcctgccc accccaaagg gtgcttccct catggacatc tacctggacc ggctggccac 60

cccctggggg cttgctgaca actacacctc tgtcactggc atggacctgg gcctcaagca 120

ggagctgctg aggcacttcc tggtccagga cacggtgtac cccttgctgg ctctggttgc 180

catcttcttc ggcatggccc tgtacctgcg ctcactcttc ctcacgctca tggtgctgct 240

gggggtgctg ggctcactgc tggtggcctt cttcctttac caggtggcct tccgcatggc 300

ctacttcccc ttcgtcaatc tggcagccct cctcctgctg agcagcgtct gcgccaacca 360

cacgctcatc ttcttcgacc tgtggcgcct tagcaagagc cagctgccgt cggggggct 420

ggcgcagcgc gtgggccgca ccatgcacca cttcggctac ctgctgctgg tctccggcct 480

caccacgagc gcggccttct atgccagcta cctgagccgc ctgc 524

<210> 381
<211> 642
<212> DNA
<213> Homo sapiens

<400> 381
cccacatgct ctacctggct ggaaaggtag taggaggtgg gggagggccc ggggcagcct 60

gtgggagagc cctaccctca atgcacccct gcacctgccc cacccctgg agagtagcca 120

gcctaacctt ccaagttcct gggcctcacc tttcccatcc acacctgccc tcaagtctga 180

cacccattct tactcaagtg tctgctacca agaattcctt caaaggtgag ctggagcggg 240

cttttacatt ggaataaaga tcagagcctg cagtgtagtc agtgatgaat gatggaattt 300

gagatacccg ccaggagatc cttggtgaat acagccttgt ttgtcagaaa ggctgtctac 360

acacacacac tcacatcagc ccatccacct ccacctctcc acccgcagtg agtcccctgt 420

ggtgcaccca ctaggagcct agaagccagg tttccttggt ttttttaact gcccatgagg 480

cacaggggag agctgtcttc agctggctcc tgggaccagg ccctgctctg ccctccctac 540

ctgccacctt cctcaactcc atctctcctg accccagggt gatggaaaca tccggtacta 600

cgagatcagc actgagaagc cctacctgag ttacctcatg ga 642

<210> 382
<211> 397
<212> DNA
<213> Homo sapiens

<400> 382
agaaggaccg gaggcccgcg cagccaccca gccgacccat tccccggccc ggcgcggggg 60

ctcgcagtca cgcgaggggg gagggagtcg gggcggggct cttaaagggc cagcctcgcg 120

cgcggcgtgg ggttggggtg gagggagagg aactgtcgaa gggtggcggg tgcgcgattg 180

aataagggga tgcggtgcaa agggtctagg tctgcgtgtg gcttctgcct gcgaggagag 240

gcagcctgca tgtgtccgca ttttcggtcc acgcctgtgg cacgacacga agccccagtg 300

ccatttggca tggcctagct gcctgcctcc gacgctgtcc cgccctccgg aaccttcctg 360

ttacagggtt tccaggaaaa aaaaagttgt atttgcg 397

<210> 383
<211> 591
<212> DNA
<213> Homo sapiens

<400> 383
ccgagggtag aggagcgtca ggcgtgcttc ctgtctgtct gcagcagccc ggctggccca 60

aagagactct agggacccaa gctctgagtc atggcttccc aggccttagt cagaacggcc 120

cctggtggca ctcccttcca aggggtgggg agcagggcct gtcgacttgc tcctgaccca 180

cacacggagc cagtccctgt cccagctctg ccccagctct gccgccgccg ctctgctgcg 240

tgactccagg aacccccagc ctcgctgaat ccatgtccgc agaagagaaa agcctgccag 300

gccttggcct gccctgtctg ggaatccttg ctggagccac cccgaaacac ccctatggga 360

acccctttgg tctttatcat tcttgggtgt tttccactga tgactgcggg tgtgcccagt 420

ggcagctggg cctgagctgt cagcccaggc aggagacaaa caagcctgtg actcaggcag 480

ggcatggcac agctggcgga aggaggcatg cctgttcttt gtggctgggc ggggggttat 540

ccggacacag ggagtctcca gcttccggct ttcccacctc ggtgccccca g 591

<210> 384
<211> 323
<212> DNA
<213> Homo sapiens

<400> 384
cgcccctcca ggcagtctca cgggaatgca cgttcaggcc gcctttcccc ggaacccgca 60

gcggcctttc tggaggctgc ttcttggtga ctgggggctc cttggagccg tccccagcct 120

caagcggctg cattgcagct cagggacccc gaagggagga ccacgttctg ccgctgaaga 180

ctcggagtcg cctggcaaga cggagcgaga aggggagggt ggctggcatg gggactctgt 240

ctgctgcctc gcaccgcacc ttggaggacg ctccggccaa ggctgggagg ggcgagtgcg 300

gctgcaggcg cgcggtggcg agg 323

<210> 385
<211> 386
<212> DNA
<213> Homo sapiens

<400> 385
gaagaacttg taaaaccaag tggtgcattt cattagaact caagtggccc ggagaaaaga 60

aaaagaactc atttcccatg agctcttaga gccactgaaa gctcagaaca aaaccaactg 120

agcagggttt caagtattct ttttggtttc attttctcct caaattttct gaagaacagc 180

tgagagttta gcaaatacgg gtgcaaacga ttctgcctgc cacgtcaacc ctgcctcggg 240

tgtcatctga cgaggatgcc aagctacaag ggctgcggtg agactcagag acacgctacc 300

aatggcaccg cggccccacc tcgccgaagc agctgccggg gagacgcgat aaaactgccc 360

cacgcggaaa agaaggaagg gcggtg 386

<210> 386
<211> 493
<212> DNA
<213> Homo sapiens

<400> 386
gagtggagat gacggggtgc gccgtgtgca cgccttggcg tggtggggcc ggtcagtttt 60

ccagaaatta ggactgcaga gtgaagaact tgttgctcag gacaggccgc ccccaccgag 120

tgtcctctgg ttgggtttca aatggttctc ttggtttggg gctctggttc ccgtatcctg 180

ctcctcacct ggaagaaggg agcttggagc ttgcgtattt gcacctcctg ccttctgtct 240

ggatctaggg cgcagccacg gggcgctgtg gacggcaggc ccctgctccc ccaagtcctg 300

ccgcacaggc ggttggaggt gagcctcggg aagagtccca aggagtgtgt cctgtggctc 360

agtgaggacg ggcgcctggg ccctgccagg gcttctgggt gtctgggaat ggagggacag 420

cagcccagct gcccgccgaa ggccggcacc gttcccggga agcctcgctt ttgtccaccg 480

ccggctgggt cct 493

<210> 387
<211> 475
<212> DNA
<213> Homo sapiens

<400> 387
gttgtcccag caagagatgc ctttggggag gtgccctgtg gctcagcccc gggagctcag 60

ggctgaatgt gcctggactc aagggggcag gaggagggga ggggcccaga ggagacacag 120

ctgcttgcaa gccaggtgcc ccttggggca aaaccagctt ctgtggttaa ggggccagat 180

ttcgggactc aagggtcttg tgtgcctgta gcagccacgt agcttgtcca catgaacggc 240

caggctgctc ctctccgtgg ctgcacgtcc tggccacccc gaggccgcag cactttcagc 300

agctccgtct ccacgtagtc gcccacgagg taagggtgcc agcattgctc gtggtactgc 360

aggttgttgg tggcgatgga gaagggcacg gtcatggtgt ccgaggcatg acacagctgc 420

ggctccgggg ccagcagcag gcgggtctca gcggcggtgc taccacctcc cactc 475

<210> 388
<211> 444
<212> DNA
<213> Homo sapiens

<400> 388
cttgccagcc atggcgaagg gagtgctggg gactggggtc gctcagtgcc ggtcggcgag 60

gggcagagtc ctgcccgcgt gggggtcaaa gacaaatgga caaactgtat gacgtgggcg 120

agagccagcg ctctgcagaa aggagacggg agactcgtgg gagtcacctc gggggcggc 180

caggacttag gacagggctg gggaaagccg ccaaggtggc gcccatctga gcgacaggag 240

ggagcggccc tggcaggacg gacgtgggaa ctgcaggggc acaggcccta cgtgagctgc 300

gtgggtggaa accgaggctg ggacaggccg tctgggacag gccgactggg cctgtggagg 360

gtgctgctca gagcagagct gggctgacca tgcccgggga agggtgttgc gggccaaggc 420

agggaggatg tttctcctca tcag 444

<210> 389
<211> 614
<212> DNA
<213> Homo sapiens

<400> 389
ctggccaaca tgccaaaagc ccgtctctac tgaaaattta aaaattatcc gggtgtggtg 60

gcacaagcct gtagttctag ctactcggga ggctgaggca gaagaactgc ttgaacccag 120

gagacaagag gttgcagtga gccaaaattg tgccactgca ctccagtctg ggagacagag 180

tgagaccctg tctcaaaaat aaaaacaaaa caaaacaaaa caaaaaaaca aaaaacaatc 240

acttaggtaa cccaaaatag tgcccatcat ttgtgaaaag aaatactcac tagttgtatt 300

ctactcagaa aatggttgcc ttgcttaaaa agttttgcag aaaactgcaa aagttcctca 360

tttctggtac tattcggtaa tatatatgta ggtttctttc ctcagttccc actctggaaa 420

aaaaaaaaaa aaggaaaacg aaactttttt tttttttttg agacggagtc tcgctttgtc 480

gtgcaggctg gagtgcagtg gcgcgatctc gactcactgc aagcttcgcc tccagggttc 540

acgccattct cctgcctcag cctccggagt agctgggact ccaggggccc gccaccacgc 600

ccggctaatt tttt 614

<210> 390
<211> 314
<212> DNA
<213> Homo sapiens

<400> 390
gtctctcggc tgccctgagc tggggctgga tgggccggac cctccattcc ggctgcagct 60

tcccgcgcag tgagaaggca gcggtccctg agagcccagc agtgccctgg gagcctgcag 120

aacgcagggg cggattccgc gtcgcactgg ccgtcaaggg gcggccgcgg agggaagggg 180

tgggtcggtg ggtctgacag cgggtctgcg taggcggcag cgtctgtccc tcccagcctc 240

tcgctccgcg ccatgggcgg gccccgggct ctgctggccg cactctgggc gctggaagcc 300

gccgggaccg ccgc 314

<210> 391
<211> 328
<212> DNA
<213> Homo sapiens

<400> 391
cagccggaga cggtcaaggt ccaggacttc agtgactcga cgaagatccc ggtcaagagc 60

atccccagtg agcagaaggc gatccagatc cagaacgcca ccagtaaccc gccgtcgttc 120

aaggtctaga acgccaacaa cacgccgccg ctcccgttct agaactccac cagtgactcg 180

cagaaggtcc agatccagga ctccaccagt aaccaggagg cgatctcgaa gcagaacttc 240

gcctatcact cgcagaagat caagatccag aacatctccg gtcacccgaa ggagatctcg 300

atctcgcaca tctccagtaa ctcgaaga 328

<210> 392
<211> 777
<212> DNA
<213> Homo sapiens

<400> 392
caaacacctc caccgccgcg tctgcctctg cggacgtggc cctagatccc gggtggccat 60

ggaacttggt ctgggcgtgt agttttcctt tgagtgggtc ggttctaagt ttaaatttcc 120

gacgagccca gggtttcgaa cttccttcct tttttccttc gtttctcttt cttttctttt 180

ctttctttct ttctttttct ctctttcttt ctttttctct ctttctttct ttctttcttt 240

ctttctttct ttctttcttt ctttctttct ctctctctct ctctctttct ttctttcttt 300

ctttttctct tttattttcc tttttcttct ttttttgttt tttgtttttt ttgacagtgt 360

ctccttctgt tgcccaggct ggagtgctgg agtgcagtgg tgcgatctcg gctcactgca 420

acctcaacct cccaaactca agggatcctc ccacctccac ctcccaagta gctgggacta 480

caggtgctca ccaccacgcc aggctaagtg tgtgtgtgtg tgtgtgtgtg tgtgtgtgtg 540

tgtgtgtgtg tttgtgtgtg taaaaccgag gtctcgctgt gttctggagg ctggtctcca 600

acttgcactc aagcaatctt cctacctcgg cctcccctca caaagtgctg caattacagg 660

ccagaaccac catgctcagc tggttccttt tccttccagg tcctgaggtc gcagcggcgc 720

gtccctccgg tacatggcgg ggaagaggtg cggttccggg agcctgcgac gcctctg 777

<210> 393
<211> 288
<212> DNA
<213> Homo sapiens

<400> 393
gctcaggagc ctgcatctgc gctatggcgg gtaagccccg gtggctgacc ggaagaagcg 60

cccacggagg cgaaccccag gcgcccctca gcctacttcc atttcccctc gcgacccttc 120

gccccgccca ctctagctgg ctgtgcctga ctggctgggc tgccgcatat tcgcgctgcc 180

attggtccat aatcagctgg gggtgggcgt agggagacct acttcagcag gtcctggccg 240

gaccagcatt gttaacggct gcggttcgaa gtggctgtgg gtattgct 288

<210> 394
<211> 867
<212> DNA
<213> Homo sapiens

<400> 394
aattgtccca gacaaactag gatggcccgt caccctagtt acaggtgccc gggccaggtg 60

ggttccatgt ggaggtggag gcagaagccc aacgggagga ggaacaaggg gtagtgggag 120

gaggagccca cggggacagt gacactcaga gattcactga cggggtgacg gacagatcat 180

gcggggggcc ctgcaatgga atattcctta gcagtagaaa ggaaaggaac tatcagttcc 240

cataacacag gtgaatctca gagcagttat ggtgactgaa agagccgagc aaaaaagact 300

acatgcaaca tgattccttt atgtaaacat tccagaaagg acgatcagta tatagggagg 360

gaccataagt gggtggttgc ctggagccag ggaaggagga aggggagaca ggggttacca 420

aggggcacga ggaaacctta gggtgatagg tacgtttgtt ctcttgagtg tgatggcttc 480

attggcgtag acacatgtta aaactgatca agctgtaccc gttaaatatg cacagtttat 540

tgtatggggt gtgtattcta ttccccgata aagctggaaa aagagttttg ctgtaaagag 600

gatgacacaa gtgaaacagt agcaggagat gtgattttat cttaagcatg gaaggaagag 660

agttgaacct gcgtgacata ggggcattta ggctggcagt gagagaggag agcgaggtgg 720

ggaccatttg tgaaggttac tgtatgtcat gtggagagct ttggaattta tcccatcatc 780

ggcatggttc tgcaccccgt cgtgtgaaga aatcatccgg agaacttgtt ctgagtccag 840

aagttaccta caggctgcct tgaggca 867

<210> 395
<211> 233
<212> DNA
<213> Homo sapiens

<400> 395
agggcgcttt ttcggcgtct acctgctcta ctgcctgaac ccccggtacc ggggccgcgt 60

ctacgtgggg ttcactgtca acactgctcg tcgggtccag cagcacaatg ggggccgcaa 120

aaaaggcggg gcctggcgga ccagcgggcg agggccctgg tgagaggggg aggccttctg 180

tgccgggagg aaggcgtccc agaggaggcg gaccccgcgg ggcacaggcc tgt 233

<210> 396
<211> 602
<212> DNA
<213> Homo sapiens

<400> 396
tgcggccaca gtttctgccg cgcctgccta ggccgcgtgg ccggggagcc ggcggcggat 60

ggcaccgttc tctgcccctg ctgccaggcc cccacgcggc cgcaggcact cagcaccaac 120

ctgcagctgg cgcgcctggt ggaggggctg gcccaggtgc cgcagggcca ctgcgaggag 180

cacctggacc cgctgagcat ctactgcgag caggaccgcg cgctggtgtg cggagtgtgc 240

gcctcactcg gctcgcaccg cggtcatcgc ctcctgcctg ccgccgaggc ccacgcacgc 300

ctcaaggtgc gggatccgcg cgcatcgtgg tcggaggggc tgttcggtgg cacggggccg 360

atggggaggt cgctgcagtg atttggattc tgagtctcta gagaggctca cgagctcctg 420

gagttgcggg gggcgggggc ggggcgaagc agccaggaaa gagggtctga ggagcttaag 480

gcggggctgg cagaggaaag ccgcgcaggg atggagcctg gcgataaggg cgctgagcaa 540

acgtaggttt cccggcctta gtccctagag gaaactggag atgggcgggc ggagccccag 600

gg 602

<210> 397
<211> 662
<212> DNA
<213> Homo sapiens

<400> 397
caaaaagccg taaaaagcca cagcatcggg gtcagaaagc cgcgacggcc gggataaaaa 60

ccgcggtggc ggggtaagaa gccgcggcgg caaaaagatg cggcggcggt ggaaaaaggc 120

gcggcggggg caaaacggtg cagcggcagc aaaaagccgc ggcgtcgggg gcaaaaagcc 180

tcaaaaagcc acggcggagg gggtaaaaag ccgcaaaaag ccgcgacaga gggggcaaaa 240

agcaggggcg gcaaaaagcc acgacggcgg ggacatgaag gcgcaaaaac cctccgcggc 300

aggagcaaaa acccgtggcg gcgggggcaa aaagcggctg gggtgataaa aagccgcggc 360

ggtgggggca ggaagccgcg tagggggcaa ggagccgcgg cagcggaggc aaaaagctgc 420

ggtggcaggg gcaaatagca gcaaaaagcc gcggcggcgg ggggcaaaac gacacaaaaa 480

gccctgacag tgggggcaag aagccgcggc tggaaaaacc tgtggcggag ggggaaaaaa 540

gccgcggcgg cgggggcgga aaggcgtaaa aagccgcggt ggcctcggcc aaaagccatg 600

acggcaaaat gccggggcgg cagggcaaga agccacggcg ggaaaaacct gcggcggcgg 660

gg 662

<210> 398
<211> 647
<212> DNA
<213> Homo sapiens

<400> 398
cctctgccct gcgaccaaca gccgggagcg gaccagacac cagaactccc ggaacggttg 60

aagacggttc cgctccctgt cccgcctttc gcagcccagc agtttcgccc tgcggagagg 120

agccttgctg tttccaaatc tctcctgctg aagagacatt ggagctaggg cggctagttt 180

cacctggtaa ttgtgacacc ctgtctcctc gagctgcagg cttttatgct tgtcatgttc 240

gaagtttgat accttgcaga tcaacaaagg gtcggtggcc tctcactgcc tccgcggcag 300

ggttgtcaag gtaacgctcc tcagacaagg gtggggcgcg gcccgcccct tttctcaccc 360

cgcctcctcc tcagccccac cctccttcgc tcctcctctt gtcactccct ttcagacatg 420

cgcagtgcgg cccgtcccta gggctgggtt aagggccgcg gatgtggcag ttctcaggcc 480

tcttgggatc gcctcaagaa gccccctcac gagtgtctcg atttcctgtc agccaacaaa 540

gggccgttcg cctttcatgg cctccacagc agcgttgccg tggtaacgat cctccgccgg 600

acgttggccg caccgcgccc ctattcttgc ccatctcccg ctccgcc 647

<210> 399
<211> 534
<212> DNA
<213> Homo sapiens

<400> 399
cgcctttcat ggcctccaca gcagcgttgc cgtggtaacg atcctccgcc ggacgttggc 60

cgcaccgcgc ccctattctt gcccatctcc cgctccgccc cgtcccttct cgctcctccc 120

tcttgtcaca cccgttcaga catgggtagt gtagcccgtc cctagcggcg ggataaaagt 180

cctgcccttt cacacatgcg cagtgcaccc attcctaggg gtggggctaa ggggcctgcc 240

ctttcggaaa tgcgcagtgc agcccgtccc taggtgtagg gtaatggcgg ccgacctggc 300

aggtctcagg ctccttggga tcccctcgag aagcccgttc atgagtgtct gaaactgtca 360

cttgactgcc agaagtgaaa acatcgtgtc cctagtcacc tgccatttgc cttttcaaaa 420

ccatttcctc tgttctctag gctgtcacaa attctctgca ccccaggagt gccgcttgga 480

cccccgggct cgctgcgtgg tccatatcta ggtcgggcct ctcacggaga cttt 534

<210> 400
<211> 526
<212> DNA
<213> Homo sapiens

<400> 400
gaaagtctcc gtgagaggcc cgacctagat atggaccacg cagcgagccc gggggtccaa 60

gcggcgctcc tggggtgcag aggatttgtg acagcctaga gaacagagga aatggttttg 120

aaaaggcaaa tggcaggtga ctagggacac gatgttttca cttctggcag tcaagtgaca 180

gtttcagaca ctcatgaacg ggcttctcga ggggatccca aggagcctcc aggtcggccg 240

ccattaccct acacctaggg acgggctgca ctgcgcattt ccgaaagggc aggcccctta 300

gccccacccc taggaatggg tgcactgcgc atgtgtgaaa gggcaggact tttatcccgc 360

cgctagggac gggctacact acccatgtct gaacgggtgt gacaagaggg aggagcgaga 420

agggacgggg cggagcggga gatgggcaag aatagggcg cggtgcggcc aacgtccggc 480

ggaggatcgt taccacggca acgctgctgt ggaggccatg aaaggc 526

<210> 401
<211> 424
<212> DNA
<213> Homo sapiens

<400> 401
gggcggagcg ggagatgggc aagaataggg gcgcggtgcg gccaacgtcc ggcggaggat 60

cgttaccacg gcaacgctgc tgtggaggcc atgaaaggcg aacggccctt tgttggctga 120

caggaaatcg agacactcgt gagggggctt cttgaggcga tcccaagagg cctgagaact 180

gccacatccg cggcccttaa cccagcccta gggacgggcc gcactgcgca tgtctgaaag 240

ggagtgacaa gaggaggagc gaaggagggt ggggctgagg aggaggcggg gtgagaaaag 300

gggcgggtcg cgccccaccc ttgtctgagg agcgttacct tgacaaccct gccgcggagg 360

cagtgagagg ccaccggccc tttgttgatc tgcaaggtat caaacttcga acatgacaag 420

cata 424

<210> 402
<211> 322
<212> DNA
<213> Homo sapiens

<400> 402
tgaggagcgt taccttgaca accctgccgc ggaggcagtg agaggccacc ggccctttgt 60

tgatctgcaa ggtatcaaac ttcgaacatg acaagcataa aagcctgcag ctcgaggaga 120

cagggtgtca caattaccag gtgaaactag ccgccctagc tccaatgtct cttcagcagg 180

agagatttgg aaacagcaag gctcctctcc gcagggcgaa actgctgggc tgcgaaaggc 240

gggacaggga gcggaaccgt cttcaaccgt tccgggagtt ctggtgtctg gtccgctccc 300

ggctgttggt cgcagggcag ag 322

<210> 403
<211> 322
<212> DNA
<213> Homo sapiens

<400> 403
cctctgccct gcgaccaaca gccgggagcg gaccagacac cagaactccc ggaacggttg 60

aagacggttc cgctccctgt cccgcctttc gcagcccagc agtttcgccc tgcggagagg 120

agccttgctg tttccaaatc tctcctgctg aagagacatt ggagctaggg cggctagttt 180

cacctggtaa ttgtgacacc ctgtctcctc gagctgcagg cttttatgct tgtcatgttc 240

gaagtttgat accttgcaga tcaacaaagg gccggtggcc tctcactgcc tccgcggcag 300

ggttgtcaag gtaacgctcc tc 322

<210> 404
<211> 424
<212> DNA
<213> Homo sapiens

<400> 404
ttatgcttgt catgttcgaa gtttgatacc ttgcagatca acaaagggcc ggtggcctct 60

cactgcctcc gcggcagggt tgtcaaggta acgctcctca gacaagggtg gggcgcgacc 120

cgccccttt ctcaccccgc ctcctcctca gccccaccct ccttcgctcc tcctcttgtc 180

actccctttc agacatgcgc agtgcggccc gtccctaggg ctgggttaag ggccgcggat 240

gtggcagttc tcaggcctct tgggatcgcc tcaagaagcc ccctcacgag tgtctcgatt 300

tcctgtcagc caacaaaggg ccgttcgcct ttcatggcct ccacagcagc gttgccgtgg 360

taacgatcct ccgccggacg ttggccgcac cgcgccccta ttcttgccca tctcccgctc 420

cgcc 424

<210> 405
<211> 526
<212> DNA
<213> Homo sapiens

<400> 405
cgcctttcat ggcctccaca gcagcgttgc cgtggtaacg atcctccgcc ggacgttggc 60

cgcaccgcgc ccctattctt gcccatctcc cgctccgccc cgtcccttct cgctcctccc 120

tcttgtcaca cccgttcaga catgggtagt gtagcccgtc cctagcggcg ggataaaagt 180

cctgcccttt cacacatgcg cagtgcaccc attcctaggg gtggggctaa ggggcctgcc 240

ctttcggaaa tgcgcagtgc agcccgtccc taggtgtagg gtaatggcgg ccgacctgga 300

ggctccttgg gatcccctcg agaagcccgt tcatgagtgt ctgaaactgt cacttgactg 360

ccagaagtga aaacatcgtg tccctagtca cctgccattt gccttttcaa aaccatttcc 420

tctgttctct aggctgtcac aaatcctctg caccccagga gcgccgcttg gacccccggg 480

ctcgctgcgt ggtccatatc taggtcgggc ctctcacgga gacttt 526

<210> 406
<211> 322
<212> DNA
<213> Homo sapiens

<400> 406
cctctgccct gcgaccaaca gccgggagcg gaccagacac cagaactccc ggaacggttg 60

aagacggttc cgctccctgt cccgcctttc gcagcccagc agtttcgccc tgcggagagg 120

agccttgctg tttccaaatc tctcctgctg aagagacatt ggagctaggg cggctagttt 180

cacctggtaa ttgtgacacc ctgtctcctc gagctgcagg cttttatgct tgtcatgttc 240

gaagtttgat accttgcaga tcaacaaagg gccggtggcc tctcactgcc tccgcggcag 300

ggttgtcaag gtaacgctcc tc 322

<210> 407
<211> 424
<212> DNA
<213> Homo sapiens

<400> 407
ttatgcttgt catgttcgaa gtttgatacc ttgcagatca acaaagggcc ggtggcctct 60

cactgcctcc gcggcagggt tgtcaaggta acgctcctca gacaagggtg gggcgcgacc 120

cgccctttt ctcaccccgc ctcctcctca gccccaccct ccttcgctcc tcctcttgtc 180

actccctttc agacatgcgc agtgcggccc gtccctaggg ctgggttaag ggccgcggat 240

gtggcagttc tcaggcctct tgggatcgcc tcaagaagcc ccctcacgag tgtctcgatt 300

tcctgtcagc caacaaaggg ccgttcgcct ttcatggcct ccacagcagc gttgccgtgg 360

taacgatcct ccgccggacg ttggccgcac cgcgccccta ttcttgccca tctcccgctc 420

cgcc 424

<210> 408
<211> 526
<212> DNA
<213> Homo sapiens

<400> 408
cgcctttcat ggcctccaca gcagcgttgc cgtggtaacg atcctccgcc ggacgttggc 60

cgcaccgcgc ccctattctt gcccatctcc cgctccgccc cgtcccttct cgctcctccc 120

tcttgtcaca cccgttcaga catgggtagt gtagcccgtc cctagcggcg ggataaaagt 180

cctgcccttt cacacatgcg cagtgcaccc attcctaggg gtggggctaa ggggcctgcc 240

ctttcggaaa tgcgcagtgc agcccgtccc taggtgtagg gtaatggcgg ccgacctgga 300

ggctccttgg gatcccctcg agaagcccgt tcatgagtgt ctgaaactgt cacttgactg 360

ccagaagtga aaacatcgtg tccctagtca cctgccattt gccttttcaa aaccatttcc 420

tctgttctct aggctgtcac aaatcctctg caccccagga gcgccgcttg gacccccggg 480

ctcgctgcgt ggtccatatc taggtcgggc ctctcacgga gacttt 526

<210> 409
<211> 578
<212> DNA
<213> Homo sapiens

<400> 409
tgctgggcac cctggagcgc gtcgggatgc ccaaagtggg gcgtgaggcc ggtgggctct 60

ttgctcctcc gcttcgcccc tgcgcctccc gcgacccgtc tcacttccct gcgtctctga 120

gcggcccgag aggcgcagcg ctcagagtgg ccttgatgga agaacctggc cctcggcggg 180

aacgattttc ttttgcttta cctcttctgg atcagccgtt ttccacctga cttgagcagc 240

cccctccccc atgccttttt tttctcgttc cctccgtctg tcctcacaat ttcctctatc 300

ttcgatttct agatcattcg cttttcttct ctttcctccg cttcctcatt gtgctgtttt 360

tatgcctttg ccctggacca cttttcagtt cacttttctc ccttctgctc cctccctcac 420

ccccttctta atgtctcttt tttcctcgcc ctgagtctgt gactgtgtcc ctttccccca 480

tctgcagttg ccgtttttga ttttctttct ttcctcccca cttccatccg gatcctctcc 540

tatttcctcc ttggatttct gtctctcagg ttctgtct 578

<210> 410
<211> 551
<212> DNA
<213> Homo sapiens

<400> 410
ggtcctggaa gcctggaacc ccggcctgag ccaggcatcc cctcggggcc ggcttccacc 60

tgccagggct gagccctatg ccctgacgtt tgggcagcgg ggaacggggg cacagggagg 120

gtgacggggt gaaggaaaca ggcctgctgg tggctccgaa ggaaggtttg ggccaggcct 180

agctggaatc ccaaaactca cctgccagca accgcgagcg ggaagccctg ccaggctcgc 240

ctgcccctgc cagcggccca gccacgctgg gtcccccctct ccatcccgca ctttgctctt 300

tgcccgcgac aggggtcccc tgctcaaatg cccagcctag cctcggtcca acctgtctgg 360

ctgtccttcc ctgacacctc gtttgtctac ccttctctgc acgtgtcagt ggctccccgc 420

tcgcgtccgt gtgtcattcc ctacacacgc ctgtgtgtcc tcttcacacg tctgactgga 480

gcgtcggcct cccgcatttc ccggcctcct cgccgccctg ccccgcctgc tgctgtgtgg 540

gtgtcgggct g 551

<210> 411
<211> 488
<212> DNA
<213> Homo sapiens

<400> 411
gcagcgcggg gtcgcggagg tttagcagct ccctcagggc gagcagcacc ggcgcgcagt 60

gcgcgttctc ctccaggccc tgagcacgtc ggggtagggt ctcgcgccag ccctctctcc 120

cacccagcca ctgagactct cgcccgtctg cccgcccaag aagctctcac caaactgagg 180

aaaagctgct gaagctgggc agcatcgtgc cgcaggcgct cggccgcctg ggtcctctcg 240

tcggctccgc ggcacaccag gcggccttgc atcagcgcgc tcaggtactg gagcaccacg 300

gcacgctcgg cctcagccag cagcagctgc ggggaggcaa tcaggcgggt gcaggtcgcc 360

tctcacccgc gccgcccctt ccccaatctt ttcccccaac ccacctgaac cgtggggttc 420

cgcacgcgcc agaagtcccg gcagaagcgc cccgtccgtt cacacacact ttgcaggagc 480

tcagggct 488

<210> 412
<211> 193
<212> DNA
<213> Homo sapiens

<400> 412
cgcaatgcct cctggggctt gaagtccaca ccgcccaggc cggtgaaccc ggcgggtttt 60

gcagtcaact tcgctccgaa tttacagaaa acagctacta tgctagggac gcggaacagt 120

gtattagaga ctgaaaaaca ggccgggatc ggtggctcta cgcctgtaat cccagcactt 180

tgggaggctg agg 193

<210> 413
<211> 884
<212> DNA

<213> Homo sapiens

<400> 413
gaatggaatg gctagttttc aacgtgcctt ttagaaatac ttcactagcc ggggcgcggt 60

ggctcatgcc tgtaatccca gcactttggg tggctgaggt gggaggatca cttgagccca 120

cgagttcgag accagcctgg gcaacatagc aagatgccat ttctaaaaaa aaaaaaaaaa 180

aaaaaaattt aaaaaattag cgggcgtggc agcctgcacc tgcagtccca gctactccag 240

aggctgagac gggaggatcg cttgagccca ggagtctgag gttgcagtga gccaagatcg 300

catcactgca ctccagccta gacaacagca agaccctgtc tttaaataaa acagaacaga 360

acaaagtaaa acaaagagaa tagggacgac tcctgggttg tgttttgtta gtctgtttgt 420

ttcttgtttg tctagtcttt caactgaaga tgtgaatggg tgaagatact ttcaacgaga 480

cggtttgtct ggggaagacc aagaaggggg aacagaaaga tttcttcagt aagattcatt 540

ctcaccccgt tcgggttgag atccctaagg aaagaaagtt ctgagaggag aggatgggtg 600

gacgtcacaa tgaggcaaaa agcatcaaag gggaactttt ctatcagaac tttctaacaa 660

ccaggcggag agcggacagc tctatcttgg acggggtggt ggccagcagc tctctcgggc 720

cagggctgaa aaaaacctcc aaggctcgga ctgccgcccc gacttctgaa gcagcggtgt 780

cgttcggggc tgaccgacga gcgtccctag cgacaagggg ccgtgtccat gtgccggttc 840

ccgcgctgca agagggggcg gcggccggag ctgcccaacg ggag 884

<210> 414
<211> 659
<212> DNA
<213> Homo sapiens

<400> 414
ctctggcagt cggcagtagc gacccccttcc tgggaacaga actttcctcc ggaggccagg 60

gtctcctcat ctgcaactgc catttgccac acacacacac acaaaagttg ggagagaatg 120

tgagaatgct tgtgttcgtg gacgcgtgcg cacgtatgga gcggcggtac ctatgattat 180

ttgcgcgtgg cgcctggcta tgcgtgggcg aacgcgcagt ggagaacatg tgggcgtgca 240

tgaagtgtgc acttaaacca aagtgtgccc tccccgttgg gcaggcctgt gtgttggtcc 300

aagggttggg agcgcctgag ctgtgtgagg gtcgctggga agggtgtttg aggccgtggg 360

cggtgcggag cgtgtctgtg tgcgcggagg ctgagctggt tgtgtgaagt ggtagcgggt 420

ctgccagaga gcgtttacct gcgtgaatgc acaaccacac actacgggtc acgtcccaca 480

ctcattcttt ccctaggcgg gccaggctgt ctgcacgcgg agacccagag aggccagcgg 540

aagggcagcg cgggaaggcg gctctcttct cccgcgagcg cagatctggc ggaggaggcg 600

gcgactgccc ggcaggctgc gccggcgagc cccccagctc ctctcaccga cgtgccagg 659

<210> 415
<211> 483
<212> DNA
<213> Homo sapiens

<400> 415
cgcgtccccc cgtgctccgg gggggtgagt cccgcgtccc ccggtgctcc gggggggtga 60

gtcccgcgtt ccccccgtgc tctgggggtg tgagtcctgc gtcctcccgt tccctggggg 120

tgtgagtcag gcatcctccc gttccctggt ggtgtgagtc ccgcatcctc ccgttccctg 180

ggggtgtgag tcccgcatcc ccccatgctc caggggtgtg agtcccatgt ccccctgtac 240

tctgggggtg tgagtcctgc atcctcccat tctctggggg tgtgagtccc gcgtctccca 300

tggaccagcg ctgtaagtcc catgccctcc tgtgctatgg tggtgtgagt cccgtgtcct 360

cccattctct gggggtgtga gtcccctgtc ttcccgttct ctgggagtgt gagtcccatg 420

ccctcccgtg ctccggtgct gtgagtcccg cgtcccctca agctccggag ctgtgagtcc 480

cgt 483

<210> 416
<211> 309
<212> DNA
<213> Homo sapiens

<400> 416
acctcggacc cacccacgtc cccaccccgg cctggacctc cacgtctgcc ggccacagcc 60

tttcgtccac ctccacaaaa gcagctgtgt gtatgtacgg ggtgccctgg cggcggtcct 120

caaattccct tcaggtggca gacagaacga gggtgacagt gaaaacgtgt ccgcggcatt 180

ggccctcggg gaggaggcga ggggtccacc cagcccgcta aaccctctcc agggcataag 240

ccaggtgggg cttctccacc ggcctccaac gggtgggcag gccctggaca ggcgacaggg 300

ggctggaat 309

<210> 417
<211> 320
<212> DNA
<213> Homo sapiens

<400> 417
gagtttgtca tcctcctgcc tctgctgcag ctgccgctcc agctggcgcc ggtagcagat 60

caggtagagc gggaggcaga agcccagcag gctgagaagg agcagcccca cgttcacctg 120

gggaggcagg gaggccgcgc atcacagggc gtggtggtgc gccgaggctg cagacccgcc 180

ctccactcgg gtttgggaaa ggggagaagg tcacggggtg gaagggggaa cgaaacagga 240

aggcccacga ggtcccatat gccagcgtcc cggttcccaa ctcttcacag gccatgtggc 300

caccctcagg gcctgggccc 320

<210> 418
<211> 837
<212> DNA
<213> Homo sapiens

<400> 418
ccctaaagca cactaagcca ggaaagtcta gcccaaatat taatgtgtcc ggaaagtctt 60

ccatgacaac tctagcttat actgatgctc tctttctctg aaattgtacg aacgttagaa 120

caattgataa ttcgtttcac aaatattgaa gtattatgat ggttactgct attgaaaaga 180

ctgaatcaaa catgttttgt cctcgaatta cttacagaga tgtaagaaag atatgttcta 240

tagagcaatg actgaactgg aaggtaaagc atagtaagtt gcatgaaata atatgtgtgg 300

gagtttagga gagaagcagc aggagggagg caaagacagg gaaagagaga aacagagaga 360

caggcaggag tgagagggag agtgatcctt ttgagatggg aaagtttcac ggaaagatga 420

gttcagcagc cagcaactgg cgtcaagtct tgaaatattt gggaagtgga cttggaggaa 480

aactatcgca aaaatatctt cttcaaaaga acatatgtac agagctgtta cctacccact 540

gatttgccaa tttgcaaagc atttacaatt accaatttca tcaagataat cataccagaa 600

gatgatttct tcccactccc aagtcatgtg atcttagaat tttaaggacc tggatggatg 660

cggtggctca cgcctgtaat cccagcactt tgggaggccg aggtgggcgg atcatgaggt 720

cagttgttcg agaccaacct ggtcaacatg gtgaaaccct gtctctacta aaaatacaaa 780

aattagccgg gcgtggtggc aggcacctgt agtcccagct gctcgggagg ctgaggc 837

<210> 419
<211> 448
<212> DNA
<213> Homo sapiens

<400> 419
ggatgacacg ggtccgtacg cccagtcggt gaacttcctg gacaggctcc ggagccgctt 60

ctccttgatc tcacgactga ggatgtactt caggatctgg gacaggagga ggaacaacca 120

tcagctgcag aacaggggct taaggccaac agggctggac cagccagagg ctggctgggc 180

ccaggggata cacccgccca gaatgggtgg agacctgcct ctgcgcctgg cgccatggcc 240

cctgggcccc gtctttatct gtggaatgcg cacaaagctt gctgttccgc ccatctcact 300

gggggttgtg aataccaggg acaaagggtg tgaaggttct tcgcaggctg cagcaagctg 360

cacaatcagc ttgaaatgcc tcacgccagc tggggacccg gttcggtgaa aaatccaggc 420

actgatgatt ttttagaaac agtttcca 448

<210> 420
<211> 355
<212> DNA
<213> Homo sapiens

<400> 420
tgtgcccctc ccacactcct gtgcccgccc tgcggacacc accaaggccc ggtcctgtgg 60

cctgtgacac agccttctct gcacagccct gcaacgccaa gacttgagca cacccoctcc 120

cctgacaggc acacgcccgt gtccgcgtga cccgccctca cacaccactc catcctgtcc 180

tgcacccccc acgcagacac aggcacactc ccgcgtccac gtgacacacg cacacccgcc 240

tccaaacagc acacgcaggt ccctgcgcac gtggacaccc ccccagccac cagccctgcg 300

cctcccgggc cccgaagccg cagcgcccgc ctctgccgcc ccctctcgcc ccagc 355

<210> 421
<211> 162
<212> DNA
<213> Homo sapiens

<400> 421
ctatctcccc gatctccctc tcctttccat ctccttactt ccgcccctcc ggtgtctctc 60

cgagaggtgt ccccccacgc cccgcgccct ccgcaccgcg ggcctcgctt cccggtcccc 120

cctggcttcc tcgccacgtc cgccccactc taggtgcagg ac 162

<210> 422
<211> 573
<212> DNA
<213> Homo sapiens

<400> 422
tccgccctct ccgtaccccg cccggccaga gcccccottt ttgtaggccc gggtagagta 60

gcctgtcatt cacaccacac cctccccgtc tgagacccta cgggagccca ctgtcgtctg 120

cccaaacctg ctgtctcgtt gggctccccc ttttctccct taacacagta caagagaggg 180

agagggggtt gccgtatgca ctttgtcctg cagcaaaggt tggctcctgg caaaccctcc 240

caaaagccaa cacaccaatt ttcactggcc caggccttcc gacgactcag actcccgtat 300

tgccaacctc gatatcccta acctcctccc caattccaat atccccgtcc tcgtgtccac 360

ttgcagtgac ccaagacccc ctacgttacg accacccctt gtttctgagg tcccgccctc 420

gagacggaga ccccgccctg gactcgccat ctggagagcg agggaggaag gaaggataca 480

gacccaggcg ttcaccccac ctccaccccc gcccccgaca tcccggccag ataaaggagc 540

cgaggccaag aggggagaga gaccccgccc ccc 573

<210> 423
<211> 385
<212> DNA
<213> Homo sapiens

<400> 423
acgccagtcc cacggggatt gaattatgta cacacacaac ccccaggccc gggggagggg 60

ggagcggcga gaaatgaatg cagaggggcc ctgccctact cacacgcatg cacctgccgc 120

ctgctgcgcg caggggagac acgtggattg caaaccccag cgagcccagg gtccaccgcc 180

aacgcgtgga aatagggatg gtccccaaaa cctgagggga tgagtagttt tgttctcagg 240

aacgttgccg aggaccaggt aaatctcgga tatcaatcag atcacagacc tccaccaagt 300

gccagctctg tgcttagaat aacagacccc caggccgggc gcggtgcaag cctgtaatcc 360

cagcactttg ggaggccgat gtggg 385

<210> 424
<211> 849
<212> DNA
<213> Homo sapiens

<400> 424
gctgggtggc tgcgcgcgcc tcaggcggca ggcgaacgac gtgcggcacc gggcggagct 60

agggggcggg ggcggagctg ataccgaagc tcctattggc cgtcggggac gccccgcctc 120

tctctggcgt tgcagcccag gagcctccac cgacatggca gcgaggttcc cgagccaacg 180

ccatcctccg ttccttccgt cactcactca ctaatcactg ttcctgagtt ccttcacttc 240

tcacccgtca tttattgcgc acctattctg tgccaggcac tcggccaagc actgcgaggc 300

cttcaaagat catttctttg tctctcccaa acccttccgc ccagtcccac ccgcagagct 360

gcaacgctct gccattatcc ctgattctta accgccgcct ttctcctttc cgttcttgcc 420

tcactgggcc tccctgagac acttgatttg ccccaacctt cttcctctgt cttggttctc 480

ccactctcat cagctctgca tcccctgggt cctctcccac ttctccccat tccgttgggg 540

ctagtccaat gtggtgccat cctctttcca cctgcgtctc tctctcttct ctctctctct 600

ctcaacaaat gtcatccctg actcttccct tctccttccc tttcccacac gccaacaatg 660

gctgaatcct ctgatttgac ctcattgtct ctcagacacc tcaccctctc tcccttctca 720

ctatccctcc ctcggctcag ttccacacca tttgtaatgg tcaagacctt cttacccttt 780

ccccagcgtc cagtgttgcc ggtttccagg ccatcctgtg ctgggccacg acatcaactt 840

gtgatacta 849

<210> 425
<211> 468
<212> DNA
<213> Homo sapiens

<400> 425
ctgctcctgc tcctggatca ggcgctgcag cgcctccagc tgctggatcc ggaactgcag 60

cggacgggtc ctgcccgagc tgaaggcggc gcgggcgcgc ttcacggcct cgctgatctt 120

gctcatggcg cctggggaca gagagcacct gcagctggct gaggggcacg agcgcgccct 180

gcctcccacc ctgcctgaat tctataggaa gggacttccc tgggctcggc cttggggaaa 240

atggcctttc cgctggaagg tccactttct gcctcgcctc ctctcccgcc cctcactcag 300

acgcctgggc cctggctgcc ttgctagccc ctgagaaggt gacacccgcc gcaacccgag 360

tcctaagccg aactgctgct gggggctctt ggcagcgtgc tcgcggcagg gcctttcccg 420

gccttttccg ctcccttact tgcttgcaaa gctgatggca caatccca 468

<210> 426
<211> 400
<212> DNA
<213> Homo sapiens

<400> 426
ggcagtgccc accagggtag agaggggctg ataattggct ggattatccc ggcggggagg 60

cctccttggg gaagctattt ttaactctga gagatgctgc agccacttcc ctccctctct 120

ttattgccct ttattcctgg ggcctgcagg ctttgcccgc cttcctggaa gctctgtccc 180

tttagaggca gggagggatg aaacgctccc ctcacatacc atagagtgtg gtccccacat 240

ggttctggca gggttctcat gcgccccttt ttagagaagg agagagagct tcagaaagct 300

tgaggggcat tttggaggac ttgctggtct ttttacgtct attgccttcc cggcagcccc 360

ttgaggctgt tagaaatttc ctaaattaac aaaggggtgg 400

<210> 427
<211> 480
<212> DNA
<213> Homo sapiens

<400> 427
gcttgccagc atcccagcca ccccgatgct ggcttccagc agggccatcc ggaaggtgcg 60

gctgcgactg gagctgacat ctgccacgga cgcaaagcta gcagccagaa ggccaccgaa 120

gtcgccgagg agggcacaaa ggatgcgacc cagcacgaag tagccgacgt ggagctgcag 180

ctgcaccaca aaaacggaca ctagggcctg gagcagcagg cccagcgagg ccagcactag 240

cagcgggcgg cggcccacac tgtcgctcca agctcccagc agggtggacg agaagagccc 300

caccaggaag ccgcccacgt tcatgtagag ggtccagtgg gaggtaaggg tctccacttc 360

ctgtaggggc acaatgacca gggtgcggtt cctcactctg ggttccacaa tccccaaatc 420

ctcgccatcc cgggagctac agggatgagg acttcctttg gagccctaaa cctcagagaa 480

<210> 428
<211> 324
<212> DNA
<213> Homo sapiens

<400> 428
gcacagcgat actagctgag tccatcgatg acctttaaac ccgcttcccc ggttctgtct 60

agattacagg gtttggaagg ctttggaaga tgtgttcaga gaactttgcc caaactggcc 120

cacgcctgtt ccaatgctat gcatagctgt ttctgattct tagccagctc aggccctcta 180

ttctttccat gctggtctgc ggcctccgtt acccacctgc tgagggcgat cttctggtgg 240

agcagaaagc cccgctcgta gggccagggc aggccggcct cgaaccactc tcggcagcgc 300

agcacgggcg agatgcaggc ggcg 324

<210> 429
<211> 787
<212> DNA
<213> Homo sapiens

<400> 429
cagcagcagg cccagagagc ccacgaaggc cactgcccac agcacacgcc ggatggtcag 60

cggcccatac acgaagatgt ggcggatgcc atggagggtg gaggtgttgg caaagatctg 120

gatgctagaa ggttgcaggc tgccctcact ggggctttcc ttgaggtcca tcgggacccc 180

gtgcagttcc gcaactggct tcaggttgat cgaatttcag agaagagctc ccagtcctcg 240

ggctctgctt aaaccttgac gttcagggga gagaacgcaa ggcaagcatc gcgccagatg 300

ctgtgagttt atcctgagag cccagccgca cgctgacagg ggtgagtgtt tatataaaac 360

ccattcaaac tctagctctt gattttttcc aggcgataag gagggctggt tttatttagg 420

gagacaccaa ggtgatgtgg aagagatgtg ggtgggaagg gagagcttag ccaatggaaa 480

taaagtcctc ccccctacac acgccactag agacaagatt aaaagcgaga gcgagagaga 540

aaatgcttaa aacaagtcgc tgagcagctc ggagcctgtt aaccagcgca taatgcccca 600

tgttaaccat cctgggggat gtcagggcgg ccacgctgat gcactgcggt cgggcgcagc 660

ggcacagcct tcagagccgc cgccgctcgg ttccttttcc catgatgttt tggactccga 720

atgccgtttc tcgccgccgg cctccgtccc tcctctcctc ctcctccagg ccccctttg 780

tgggcag 787

<210> 430
<211> 790
<212> DNA
<213> Homo sapiens

<400> 430
ctggacaaca tggggaaacc ccgtctctac taaaaataca aaaaatagcc ggatgtggtg 60

ggggcgcctg taatcccagc tactcaagag gctgaggcag gataattgtt tgaacctggg 120

aggtggaggt tgcagtgagc tgagatcatg tgccattgca cttcagcctg ggcgacagag 180

caagactctg tctctcccac ccccgccccc cccaaaaaaa ttccagatgg aggcttagaa 240

atattattat aacttagtat gtctgcaaca tgatctctct ctctttctat gtattataaa 300

atacatggga attgaagaca tcgatgaggg atagaggaga gccaggcccc attaggagca 360

tgattgcttt ttttgagaca gattcttgct ttgttgccca ggctggagta cagtggcatg 420

atctcagctc ctcactccaa cctctttctc ctgggtttaa gcaattcttg tgcctcagcc 480

tcccaagtac ctaggattac agacgtgcac caccacacct ggctaatttt tgtatttttc 540

cgtacgccac atttcccgcg ccccaccgcg gggcggggat tcggcgctgg gctcttccct 600

gttcactcgc cattactgag ggaatcctgg ttagtttctt ttcctccgct gacaaatatg 660

cttaaattca gcgggtcgcc acgtctgatc tgaggtcgcg ggcctcgatc agaaggactt 720

gggccccaca cgagcggcgc cggggagtgg gtcttccgta cgccacattt cccgcgcccc 780

accgcggggc 790

<210> 431
<211> 624
<212> DNA
<213> Homo sapiens

<400> 431
agtctgcaca gccacccagc ctatgtggcg cactgcgttc tggatcaccc ggatctgggt 60

aaggcggggg ccgctgggaa ctcctgagcg cccccgctcc cagcctttgt gccctccatc 120

atttcctggc cccagacccc ctaccgacct tctctcttgg agcgcggagc cctcttcgac 180

gcattccgca ggaccgcccc ttctcagctg ctgcccagag cgccccccatc cgtcaagaag 240

gcccactgtt ggtgcttggt tcccatctgt cacctagcgc ccccagtgca gagcccagca 300

taacctagag cccgctgtca acagcgccca ccgagggctc cccgcccccca cgcagcacca 360

gcgccaccgc acacctgact ctgtccgctc acacagcctc accttcacct cttgggtatt 420

tcagatccgc tctggcccag tgggcagagg catcgcctag agaaaataac ctcagggttc 480

cctcatccct ctgacaaacc tgcctctttc cctgaaacca ttcaataaaa cctctgatca 540

aagtgtcagt gctttgaggt ggcgcaggtg gctccgggtg gggatgatcc tcctggggtt 600

caagccagag gtaaaagtgt gggc 624

<210> 432
<211> 585
<212> DNA
<213> Homo sapiens

<400> 432
gggattgacc tgggattggg gattggcggg gtggggagct ggtggggtcc ggcggaaagg 60

ggaggacgtg aacttgggcg ggttgccctg gagaggcctg tagatgctgg gcaggtggga 120

aggcaggttt ccaggtggca gcgggtggag aggaggtggg ggacttgtgg tcagagagcg 180

cccctggcgg ggatttgggg atcagcatgc aggaagctct ggtgatgaca ccccaggggc 240

gtgtgtgaaa cggattcagg ctgccaagcg ttattcactg tggagagatt gtcatcacca 300

gagccgtgtc taaaggattt agccagggct ggatacggaa aacagaatgg aagggggctt 360

tgggagacca gcccacctca caagaaagag ctgagagcct agatttgggc cagcgggggt 420

agtctctgga cggagggcgg cacggggctg gaggaggagc gttttatgat gcggccgtgg 480

gtgctggcct tggctggggc ttgtggcgac tgggtgccgt gacgtggggg tggaccgggt 540

agagcggggt cggcaggggg ccgagtccgg gcgccccccg catcc 585

<210> 433
<211> 329
<212> DNA
<213> Homo sapiens

<400> 433
cgcccgcccc ccatcccctc ccctcccccg ccggccccag cccggcaccc ggcggcccct 60

ccgcccagtc ctcgccgccc aactttgatt taggaggtcg cctggtcccg ccccctgccc 120

tacgccgctc ccccgcgcag ccctgcaaat caggctctca gctattggct gcctcggtgg 180

ccaggagggc gaactgggcg gagtcttggg gggctcgaca cgatggggcg gggcttggcg 240

gggaaagaag cggaggaggg agtttcacac tgaaaaaccc gggatggagg ggcgtgacca 300

gattgaggga agcttgggga gatcatagt 329

<210> 434
<211> 565
<212> DNA
<213> Homo sapiens

<400> 434
tctgagatta ctaaactaac agtttaaaag attctgggaa ccaggtgacc ggggttctgg 60

tcccaccagt atcctgtatt gtggctttgg gaaagtctgt ttcccatctc tagacctcag 120

tttccacact ttgaagagga agtttgtgtg gtctctaaga tccatttctg cgctctgtaa 180

tctccataag ccaaggggtt cgaacctccc aggcttcaca ctgtgcagct ggccagcacc 240

attcatcgcc caccaacagc cctgggcatt ggcctctgat tccccgtcac ccagagggaa 300

gacccttttg gggccgaggc gctcaccttc agccagtccc cgtagtggac gtagcccccg 360

atgtaggcgg cgtaggtgct aatggccaat tggagtgcgg cccccagcgc gaaccagcgc 420

cgcttcacgc caaaggacat gaaactagcg cacagcacgg ctgcccccat gtcgaaatac 480

aggtaaggca ctgggatgtc gggcttcctg cgagccggga gtggggaagc aacgaggaca 540

gaatgaagcg gtgggggaag aagct 565

<210> 435
<211> 559
<212> DNA
<213> Homo sapiens

<400> 435
tgttcctcct cagccaggct gtgggtggga acatctggaa gggatccccc ggaactgggg 60

gaatttccag gcacatgagg ctctgtcaac ccagccagga acatccgccc ctgccatctg 120

ctccagacgt cattgcagag tctgtgtgag aggaacccga cagtttccag ctcctccaag 180

gagcgtctgt ctgtgtctct gggtcctgcc ctgggcatgt gaggggcctg cctctgctac 240

tgctggggga gtctgttggg ggcctaagtt cccaagtgca agtcctgcgt tttatctgct 300

gggagatgag gtgtctccaa attccaaaga tgccctgggg gtcttcccta ggacgatgaa 360

ggagggagaa gctgccctgg ttgttgagaa gcctcagccc agaattttct caccctcatg 420

aggcaaattt gtccaacaca cgctgtgctc tgccctgtgc aacatctcag taataaatgc 480

ccagttttgt gcactgatgg cctgtgcacc ggaggcttta cagcttgacc tcacttgatc 540

tgcatggaaa ctgtgaaac 559

<210> 436
<211> 306
<212> DNA
<213> Homo sapiens

<400> 436
ggtgtccaca cccgccggcc tgcaggcacc tcggggccca cgtgtcctcc ggccacatcc 60

caccctgggg ccacctgtgt ccatcccaga gcgtgcgcgc aaggtgaggc ctgggggcag 120

gtgcctattt ttgccccccct caacctgcat ctttgctatc ctgggactta ctgctcttca 180

gaactttcgg gcccctggta gaaggagatg tctctcctgt gtctgagatg ggtggggcag 240

gaactcccca cagccccggg cttcttaagt ggagaagggg tgggaaagtc aggagttgga 300

ctttct 306

<210> 437
<211> 536
<212> DNA
<213> Homo sapiens

<400> 437
tctccagctc ccaccatttc tagccagccc tgaggaattg tctgagaccc gggagcccag 60

agatcatcag cgggggccaa cagccatgac ctttgagttt tcttggccgc tgggccacag 120

aaagggtgca agaggcagct ggacaggtag acagaagtag gcacaaagaa aggggccctg 180

gacggttgcc agagaaaaga cggagaatga caaagagaga agtgaaatag ctggactgga 240

ggcgggaact ggagcgcagc aggcaggtgg agtggccaaa acccgcgtgc taagaagagc 300

ccacaggggc ggcaggggcg gcaggggcgg cagaggcggc caggcgcgtc tccgttcccc 360

atctttacgg ggaggttaat gacctcctca gcgccccctc gtcccaaacc acccaattac 420

aactcctcag gctccttaat gatctgatta attggttggt tagggcggat ggaggtgctt 480

ctctgccggg gacctggcgg ggtttcgcta gatcatttgt taattgtggg aaggaa 536

<210> 438
<211> 471
<212> DNA
<213> Homo sapiens

<400> 438
aggggtagcg ctgttcttca ccttggcgag aaccttcttc tctttgaccc ggcggttctg 60

aaaccagatg gtaatctggc gctccgagag gctggtggct gccgagatct tgcgcctctt 120

gtccttggtg atgaacttgt tagccgcata ctcccgctcc agctcccgca actgcccctt 180

gctgtacgga atgcgtttct tgcggccgcg acgaaaggcg caggcgtcag gagggtgctg 240

cccgctggag tctgcgcggc gtgaaaggga gggaggaaaa ggcatggtca gatacccacc 300

catgcagacc caggccttgc aagccccaag ctaagtcatc tcacaggtgc acacaggtca 360

ccctacaggc gcacgtgcaa tcctgttcct ccaaagcata ccaggacagc accctggctt 420

ccggcttgtt ctctcaccac cgctcagcct tggctcctga aggccaccct c 471

<210> 439
<211> 647
<212> DNA
<213> Homo sapiens

<400> 439
ttatgcacaa tgaccccggc aggatcacac tccttacatg agcatcctcc ggtgcatgca 60

gacccacaca gcatgtgtgc aaacacactc gcctatacat gcacacagac agtctgcaca 120

catgcctagc ctggagggaa aacacgatgt ggactcctac taagtgacta gcactgctgg 180

gggacttggg agctgggaat tctggagtag gggaacatac acagttgcac gggttcacaa 240

ctatgctcac ttgtacacgt gctaacgcag aaccttccca ccgaagttga acctcgcagg 300

cccccatccc aaaggcaaga gttccaggag cccgcagggc ccttcagtgg ccagccaggc 360

tcggcagcgc gcggggaact acggggtccc gcgcgccccc gcggccctgg acagccaaac 420

accaacctgt gtctgcgtga ggcccagctg cgcggccagc tcggcgcgct cgggcagcgc 480

caggtactgg gccttctgga agcggcgctg cagggcggcc agctggtagc tggagtagat 540

cgtacgcggc tttcggacct tcttgggctt cccattcacc atgcgcacct ctgcttccgg 600

ctcctccttc accgacactg cggggaacgc accgggcgga agagggg 647

<210> 440
<211> 498
<212> DNA
<213> Homo sapiens

<400> 440
ttcttgttca aataactggc gtggtttctg tctcctgact ggaccgcacc ggatacacta 60

gcaatctctt taggggaact tcgggcttga ttctggctgt agctgggatc ctcttgggct 120

agggcaggct ggccttcaag tcttccactc cagggtcact ttgctgcttg ccatagaaat 180

gcctggtcaa gcatgcccac caagactgtc gccttccgtg tatctccctc tccactgagc 240

acggatacct tctgggtctt ctctgtgtca ggtgcaagct gctagggggg cttctgccct 300

ctcttgtctg gaaatgcttg ctgccatctc tttattgtcc tgcccatgtc ttgtactatg 360

catggcattg gtaggactct caatttctaa tgttaatcaa gaaagagcac agtagggagc 420

ttggtgcagt ggctcatgca tgtagtcccg gttacttggg aagctgagtg gggaggattg 480

cttgagtcca ggagttca 498

<210> 441
<211> 509
<212> DNA
<213> Homo sapiens

<400> 441
cagtagaact gggtcagcta gagggaaagg tctttatccc ttgggtagcc ggagtctgag 60

gcattcccaa gggcgagtgg gtacagtgtg gttcccatcc acagagtggt ggcatgcatg 120

ccatgccagc tactgtacca gggaggggcg atttcaagaa gagactcagt cttggcctca 180

agcatttctc agtccagaga gagagagaga aacccagctc ttgcaggcag agctttataa 240

attacaatgc atcttcctgc ccacttcctt tctgaaccca aacaatagcc cttctgtgaa 300

ttaggccagg atgctttgaa actgtctttc agacaggagg agctgaggac ccaaagtcac 360

tcggttgttt atcaacagat ctagacttaa attcaggtct ctgattctca aacacagtct 420

aatttcgatc tgtagacttg gtcaagctgc tgttttgtcc ggtgtactgt cctgatattg 480

agaggtgaca atgtgctagc aacccttgc 509

<210> 442
<211> 516
<212> DNA
<213> Homo sapiens

<400> 442
tcccagagcc gcgccgaagc ctcgtgcctg cccaggcttc ccggctctcc ggaaggtctg 60

cgcgctccca tgtccagggc ggggccacgg gctgggcagc ctccctcccc gtggcagtgg 120

tcagtgccgt gtaagcgttc ctagaatcag agttctcctc ctcagatcac ccgcccttct 180

caggggcacc cagcaagccc ttatgcccta ggcgctcacc caggctggcg gcctttccgc 240

cttgctcttt agccctgcac ctgctcaaag gggtcactcc aggacccacc gctgcccacc 300

ctcggacagc agggcttcca gtccagctgg ggagtcagag cccctgtgag cccctactgt 360

ggtgtgactc cagaactggc ttttgtctgc tgccaaatta aagaccctgc ctccccagag 420

tcctccagct ccagggtggc caccctcccc atccctgctg ggtgaccggg agctgctggg 480

agtgagccca tccctaacag cagcagcctg atgagg 516

<210> 443
<211> 635
<212> DNA
<213> Homo sapiens

<400> 443
agcccaatag aaataaggtg ttatacagac aattgcacta ttgaatctcc ggagacagag 60

ttgagttaag gaaaaatgca ctttcggaaa gctttggcga ctgaacgacc tgagttctcc 120

aacgccaagg ctggaagttt ggagaccttg aaacaagtaa attaccatta atatcataat 180

tttcagtttc cttttaagaa atgagagtaa caccactttc ttcacagaat caagtgagag 240

agaacctatg caaaggaact tatcacttgg taggtatttg aagctccctc tgcctgaaaa 300

ctacttattc aaagactgca acaaaaggtg ttcacagtgt ggtatctttc aaaacatacg 360

ctaaggcaag gaacagataa ggaaatggaa gtacatccaa ttgggatcca agcaacccaa 420

gtgggaacca acagctagac ttggagatgt cttagctcac tcttaaccac gtgagggtag 480

ggtatccaac caataaagac attcagagtt ggagagcggg cgagtggagt gtcctttaga 540

ctccgcggac tcgacgggag ggaccctgca atgcttaatt agaaccggtt gccatggcga 600

cagtctctag gcagcgtggg ctgagctctc cgtag 635

<210> 444
<211> 530
<212> DNA
<213> Homo sapiens

<400> 444
aaaatgttca ggcttggaga acctacccct ggaggagtgt tagggtttcc gggctcggat 60

gtccgcgagc ttgacgcctg acctagttgt gtctgtctgg gcggtgacgt ttggtctgag 120

ctgacctttc ctcacaatcg tgaatttggg ctgtgaggat caggccttgt cagaacccga 180

gatgagagga gagaggtgcg gagagtctgc gtggggtgga agtagaagtt agagttgaaa 240

aacctgtctt gcgtggggta tagaggaccc tactgccctc aaaataatta tgttaatcta 300

aaactgttct aaaattaaaa ggttattaag aaaacgaagt ttaggtcggg cgcagtggct 360

cacacctgta atcccagtat tttgggaggc cgaggcgggc gggtcacttg agctcaggag 420

gtagagacca gcctggccaa catggtgaaa cccgtctcta ttaaaactac aaaaattagc 480

cgggggcgtt ggctcacact tgtaatcccc gctacagata ctcgggaagc 530

<210> 445
<211> 390
<212> DNA
<213> Homo sapiens

<400> 445
gctggaggaa ggatgggcag aggggccacc tggcaggcgc cctggcagcc ggggtgatct 60

gtcttctcag gagatgtcag gacctgggca gtctccttcc tctttggtcc tggggcctgg 120

ggtggtggcc cttgggcaga cctacagagg cctggctcat aggtaagggg ctgccgcacc 180

tccgccctat tcaacataca ctctctgtgc actctgtctg agccagaccc tgggccagat 240

gcgaggttca aagagcaagg ccattccccc acctccaggg gctccgctca gacgggcagg 300

caccgtccca ccgtcactag gacagagaat gtggagaggc cgggaagtgc gccaccggga 360

gcctgcgcag gaaacggagg ggcgctgcgg 390

<210> 446
<211> 484
<212> DNA
<213> Homo sapiens

<400> 446
tcctgcaggc aacaaggaag agggccgcgg aagtcaagac cactgagccc ggaatagccc 60

agcactcgag gagagacttg ggttcgagtc ccgatgctgc acttgccgtt cgccttcggg 120

cctcagtttt cccatttcta aaatgaggac ttgaataaaa tcacatgcaa ggcttccatt 180

cttacactca gtgctggaat taggacaacc tcttcattcc gtggggttga ttctaagatt 240

tcagcctcgc ttcagtcctg cggtggggag attgtgggta gtcttggtcc aaatcgactg 300

cgagggaaac tttggcttta aaacgagggg tgattacctg ctctaaggtc gacaccacgc 360

ttgcttgata gttgaccgtg ccttagtttt cccgtcagtc aagtgggaag agcagcccat 420

ttcctgccgc tccccgggcc ctgtcccgcc tggacgcctc cctccaggag cctgcgcccc 480

ggcc 484

<210> 447
<211> 551
<212> DNA
<213> Homo sapiens

<400> 447
gactctgcgg attctctgcg ggcgggaggc gtccaggcgg gacagggccc ggggagcggc 60

tggcaatggg ctgctcttcc cacttgactg atggaaaact aaggctcagt cacctgagta 120

agcgtggtgt cgacctcaga gcgggcaatc acccctcgtt ttaaaaccaa agtttccctc 180

gcagtcgatt tggaccaaga ctacccacaa tctccccacc gcaggactga agcgaggctg 240

aaatcttaga atcaacccca cggaatgaag aggttgtcgt aattccagca ctgagtgtaa 300

gaatggaagc cttacatgtg attttattca agtccccatt ttagaaatgg aaaaactcag 360

gcccgaaggc gaacggcgaa tgcagcatcg ggactcgaac ccaagtctgt ccccgagtgc 420

ggggctattc caggctcggt ggtcttgacc tccacggccc tcctccttgt tgcctgcaga 480

aaaacgccgc ggccacgatg ccggcgtatg tggaggggga tgtctacctg gtggtggagc 540

accccttcga g 551

<210> 448
<211> 830
<212> DNA
<213> Homo sapiens

<400> 448
tgcccagcac atggacagca ggggtgggac acctagggga gggggcaccc ggggtgtctc 60
ggcaaccagc ttgttttcct ttccattaca ggcagtgggc tgcagccgcc agttgctgct 120
ggcccatttg ttgctattgg catcccaagg ccccaaatta tgatgactaa ttcccaccgc 180
tttaggcagc tccagggctg tctgtggcaa catgctccag ggggagcggg gtagaaaccc 240
cgcagggcgc acactaacac ccgacatcaa ttccaactca gcctcctgcc tcccagaggg 300
gctgggagtc cgtagggaca gggagagggt tccagccaat cagaggaccc cttcaaccac 360
agccagatcc taggaaaacc agctataagg ggacaatttc ttcatcagca taaaacatag 420
ctaacagcca gctggtgttg agcagtagtg agtcctccat catcaagggt atgcaagcag 480
agaccagatg gccttgtggg cagacgacgg tcctagatga cttttcgtgt gtctttcaag 540
tcaaatgcaa tgattcccat tagtccttag gacagaagat aagcccattc tcccctcctt 600
gttagcttac agcccgactg cgtcctgtgg tacaaagtca aatgtggacc aatatgcagg 660
ttaacacttc attttaaagt cagccattca ggcgctgttt aaacagagga tcgctgtaaa 720
gaatttggcc tccttcccag cctcctttcc cttgtagaac aaggttcctg gcccctatgc 780
cggccttcca gtagaggaca cgaggaagag gcagtcgata aatatttgcc 830

<210> 449
<211> 756
<212> DNA
<213> Homo sapiens

<400> 449
aggcccccca ttgtcgcaga acttcaggaa taaagctgga ttgggcaccc ggtctgtctc 60
tttctggttc tgtaattgaa caggctgttt aatccaggga tcggcaaaca tactctgcca 120
agagctggac agtaagtcct ttaggcttta tggaccacat agtctcagtc atagctactc 180
aactctgccc ttgtagctca aacacagcca tggactatct ataaaggagt gggtgttgct 240
gtatgccaat aaaactttat tgacacgaac aggaggtggg ccacatttag cccatggacc 300
ataggttgct gaccctgatt tacccttctg acccagtttc ctcacctata atatagggac 360
aataatgcct taacatgacc actgagagtt gatacaaaca aaagtactta caactgcaca 420
atcatgttgg gtttttgtgt tttatggcaa tccctcacct tgcatccttg cccagaggag 480
cggggcaggc cccagcagtg cctcgctgag tgctgtggcc aagcacatca ctttgacttc 540
gtggctggga gactgcgaac ctcagactac ctgcgttatg atttatttca gcgtgactag 600

tatacatctt ggttctgcag cgagtggagg gaatgaggag ggcctttcaa gggaggcggg 660

attgcattcg cacctgatta cgctgcgcgg acacaacaga ggcgcccgga cccgcagccc 720

tgccttcgcc gcctttgctg ggctgtttgc gcttcg 756

<210> 450
<211> 783
<212> DNA
<213> Homo sapiens

<400> 450
ctgcaccctt gaccttctgg gctccagcga tcctcctgcc tccgcctccc gggtagctag 60

gaccacaggc atgcaccacc acacctggct aattcttgta ttttttgtag agacagggtt 120

tagccatgtt cccaggctgg tcttgaactc ctgggctcaa atgatcctcc cacctcagcc 180

tccctagtag ctgagactac aggtgcgtgc caccaggccc agcttttttt tttttttttt 240

tttttaattt tttgtagaga tagggttttc ccatgttgcc caggctggac tcaaactcct 300

gttctcaagt tatccgcctg tctcagcttc ccaaagggct gagattagag gtgtgagcca 360

ctgtgcccag ccttcacatc atccagcagc tccagtgcct ggggtaaggt ccagtcccca 420

tgctaaggct ctgctgccag gtgtttggct gaggtcccct tgtcaccttt ggaaatgcaa 480

cagttgtggg tggccccagt ctctgtccca agaacatcag cccagaccct tggtcagaaa 540

agactcagaa cagattcaga gaagccacca gttctctctg cctcacagtg tcccactggg 600

tctgccaagt ctaattccga attgtatgaa ctgaccactg gtttgggcat cgactcgtcc 660

aagcttcaga gacaaacagc acaggtttct ggggccctca ccgcgctgta gctcccttac 720

ctcatccacg tgccgggact aatcgacagc ttagcctccc cactctcacc ggttgcatta 780

aga 783

<210> 451
<211> 544
<212> DNA
<213> Homo sapiens

<400> 451
tgccctgact tcccaaagtg ctgggattac agtactgagc caccacaccc ggccagctcc 60

tggccaagtt tctgagcgcc cctgcactca agcctgactc cttccttgca tgacatgatc 120

ttgtgtccac gaagctgcat ggcctgggac cagcaacagc cttgttctaa cttgttgtct 180

catgatttaa agtggatggc acatggtctt actcttctcc cagccccctt ggtacaagtt 240

gggactcatg cccacagtac agcaagttaa tcagagctgc agggccaacg cagtacccct 300

gtactggctt caactccacc tagagaacga ggggtcctct gcagaaaggg atccatggct 360

gcttcgttgt gagaactttc cttttttttt cttttctttt ctttttttct tttttttttt 420

tgagacagag tcccactctg tcgccaggct ggagagcagt ggtgcaatct tggccattgc 480

aacctccgcc tccccggttc aagcgatcct cctgcctcag cctcccaagt agctgggatt 540

acag 544

<210> 452
<211> 549
<212> DNA
<213> Homo sapiens

<400> 452
ggaggaacag tgacctggct aaggcgtggc ctcagggtgg ggcctctacc gggggcgtgg 60

ccttgggctg cttggggacg gactcttagc gggcgtggtc tagatctgca tggggtggga 120

ccctagtggg gcgtggtctc agggtgcgtt gggggcgggg cccttggagg tcgtggtctc 180

gggcggggac gtggaaggag tggcatctgg ctgcgttgtg gacgggactc ctgagggcgt 240

ggtctagggc tgcatcgggg cgggaccctg gtggggcgtg gccctgggag ggtgtggcct 300

cgagcggggc gtggcctcgg gcggggcgtg gcctctggct actttgggtg cgggctggcc 360

catgggccgc agagttgctg ccgctgctgt cctcacaccc gctcgtggga agcagcgcct 420

gtcgcaactc gccacttgtt ctcctcacag caggttcagg agaagtggca cctggtggag 480

gacctgtcgc gactgctgcc ggagccaggc ccgagcgact ctccgggccc caagtaccct 540

gcctcccaa 549

<210> 453
<211> 482
<212> DNA
<213> Homo sapiens

<400> 453
ccatgctcta cgggacaggt gagggtggcc tcacaccggg ccccctgtcc ggggctctgc 60

tggcggatcc tgctgggcgc gtgtagctgg gctcagcaac agggccttcc gcctgctccc 120

cagggaaccc tggagggaag cccttggggc caggatcctg ctgggcagca acatgctctc 180

cacggggcca acccaggcag ggcgcgaagt ccatcctggc ccctgtccaa acgggtcggc 240

tgtatttata gaccgtctca gaggacaggc tcctgagagc cgttcctgaa aaggtggctg 300

ttcctgagct gcagggaccc aaggggccca gcaggcggcg ctcacgtgtc atcatggcct 360

gcgctcgggg agcctgccca cggggtgccc ctggtgcccc gcggggggag gggtggtgtg 420

ggaaggggag gccggctcca ggctgtgagc tcagtcggct ggcgggggta gagctggctg 480

ca 482

<210> 454
<211> 462
<212> DNA
<213> Homo sapiens

<400> 454
gagcccaagg gggcagcacg gcgaggagca gtcgccaccc ctccctgccc ggcagcaggc 60

ccaggggggca gctctcctgc tctgccagct cccagacacc cacaggctga gaaagaacaa 120

acagccatct caaagaagta ttttcacaca gttcacctca acacagtgaa aactctcaag 180

ctgatgaaat aaagaccaca tggaaagggg agggaagaaa ggtagaagtc attatgaatt 240

tattatttac acgattgtta aagtacacaa atacagtggc gatacaaacg cacagctcgg 300

agactggccg tcagtgcaca gctgacacga cgtcctacct acgtctcctg cacggggcga 360

cggggactag atactgggca aggactgtca caagcactcc gaagacgcga cccggcgagg 420

ctcgggctgg aacccgggcg cagagctgcc tcgcacaaac gt 462

<210> 455
<211> 359
<212> DNA
<213> Homo sapiens

<400> 455
ccttgtccca gctttcctgg gctgggggct gggctccacc tgcctttgcc ggtgacttcc 60

gtccctgcag actcacaatt ttcaaagccc tctccccgcc tcctctggta gtcacaaccc 120

agttattcag acttgtacag gtcgttcgca agtcctcctg gaaggtatct tggctggaaa 180

aggggacaag gccaagcttc cccaccgctg gatgctgcgc tcggctctgg aactgacacc 240

aggcgtctcg ggcagggcca ctgacccgca gcacacagaa gccagctttg ccatcgcagc 300

ggggaccgcc gggcgggcca gctccgccct ctcctgcctg gggggccctc ggcaccctc 359

<210> 456
<211> 314
<212> DNA
<213> Homo sapiens

<400> 456
ctgccgtgca atctaagtcc ccgcagcagc gtccccaacc ccaccctgcc ggtccctccc 60

accctgttca tgctagctcc tgagagacct gcctgagatg tccacacccc agccctcccc 120

ctccgaggag gatggaccag gattccaacc ctttgccgac cctccccacc gccctggaga 180

cgaggtgcaa tgtgtccgtg agcagcgcgt tccgcagcct ttatgagcct ggctcctgtt 240

aataattgtc tcccgattgt ctcccggcgt ccaccggcgt ccaccctgca ttctccgtga 300

aggtgacttc tgct 314

<210> 457
<211> 808
<212> DNA
<213> Homo sapiens

<400> 457
tcagacactt ctcgcatcta cgtaagcagg agcctcagag gtcctgagcc ggtgcgtact 60

aacgactctg gtgaattggt gacatctgca tgctcaatta gctgagacac tggtttgttt 120

tggcacaagg tatttggtct aacaccttga aaaaaagtgt accatagcca ccctgtcact 180

gggatgaaga tgacctaagt caacagcaat tctgatcact gcacattttt agcaccgaga 240

aaggatcctc tcttttggca gaaacagcaa gtcctgactc tccccttcct gtgcacaaag 300

taacctcttc aagaaaatag attcctggga tgacggggtg gtaaggggac tcagggacat 360

cggttcagcc accatcggaa gcctgagtcc tttccctaga atcctgtgat ctttggtgac 420

ttttggtgag ggttcccatg ctgtgtgcat cagccgcaga cctgtcctcg ggccccagag 480

actgggaact cccaggggaa agtcattcaa tccaatcacc cacttaggtt ctctgtccag 540

aaatgacaca ggaaaaagag ttttattaat attataatgt actggctggg cgcggtggct 600

gaagcctgta atcccagcac tatgggaggc gaaggcaggt ggatcacctg aggtcaggat 660

ttcgagacca gcttgcccaa catggtgaaa gcccctctct actaaaaaca caaaaatgag 720

tcgggcatgg tggcacatgt ctgtaatccc agctgctccg gtggctgagg caggagaatt 780

gcttgaaccc aggagacaga ggttgcag 808

<210> 458
<211> 477
<212> DNA
<213> Homo sapiens

<400> 458
gatagaaaaa taaaagccct atatttaaaa gtgagtagca gcaagtgacc ggtcagtaac 60

ctccttcaaa ggaaagacca caaaattcaa cgcaatggtt aatccaggca cagcccattc 120

atcaatctgg atgtttcaat caatttccct caaaagtctc agtcttcatg accactatag 180

aaaacagacc aagagcccag agtttagagt gggaaaatct gggaagattg gtaactgtct 240

cttaactgga tatttaactt tgaacaagct gtcaaattct ctgaacctca atttaaagaa 300

gccataccat tatctgaggg gatcactgtg aacattaaag tgagatagta tgtaaaggtt 360

ccctgctaca acattcctga ttaagttctt gtctgtgacg gagcaacgac aagagtatgg 420

ttagtcccgg gttgttacca ttcttaaaac gttgctttgt agttttactt aggtaga 477

<210> 459
<211> 670
<212> DNA
<213> Homo sapiens

<400> 459
aaacagacct agcgcaaaac gagcactgac acagcggggg cccacatccc ggaggttctt 60

tagggagagg tcggggagtg cggttaccta tggctccgaa gtccccgctg gcatcctgaa 120

aggcatcgta tttctcgctt aaggcgttac ccttgtggta gtgggcgcat ggacgtttac 180

agttgggcgg ggcaggattt aaattccgcc agtggtggac cagtattggt gataggagag 240

atctgggaag aattattaag ctgtcgcttt taaataaccc taatagtgtt tgtgacaatt 300

ttacccgttg acttttctca gcttgcttag gacagttctt tgtggggaac tgaactgtag 360

gacgtttagc gtccctggcc ccacacgttc ccaaagtcgg ggtgagaact agtgggacgg 420

gggatcatgg agtgcatggc cgctgtcccc acctttggta gcaccaactg ctaactgcct 480

cctgaaaggt tgttggcgcc atcatggttt ctgggagcca ctttcttttt tattcttttt 540

tttttttttt gagacggact ctagctgtgt cacccaggct ggagtgcagt ggcgcgatct 600

cggctgtcag ctccgcctcc cggattcacg ccattctcct gcctcagcct cccgagtagc 660

tgggactaca 670

<210> 460
<211> 613
<212> DNA
<213> Homo sapiens

<400> 460
ccttggaact tcctttccgc tgttacttcc gtcttgatgc ttgaaaaccc ggcctggact 60

ccaagctgta gctggcttct ctcggtgcaa tctaaaaggc ggagctcgga gtagcctgtg 120

ccgtcgggac gggaagatgt cagcgggcgt tgtcctgcca ctcgggcgct ctgggcgtgg 180

gccttgggtg gggcacgtgc ctggaaatcc ccattgatag aggaatagaa aatctcccaa 240

tctttttaag taactacgtt ttaaaaaatt aatgagcgtg ttgtcttttc tcatatagaa 300

gagtgattac ttttgatgtc tgtaagccta aagctggggt cgttctcagt agaccgccaa 360

gctactggct tctccctcta tccctccctg ccttcccacg cggaaaaggt ttgtatataa 420

atagcgccca aatggacatg aagggtagag tggttcttaa tctctccctt tttttttttc 480

ttttatttga gacggagttt cgctcttgtt gtccaggctg gagtgcaatg gcgcgatctc 540

ggctcactgc aaactccgcc tcccgggttc aagcgattct cctgcctcag cctcccgagt 600

agctgggatc aca 613

<210> 461
<211> 826
<212> DNA
<213> Homo sapiens

<400> 461
cactgattaa ttctaactgg gccacacccc acctcctcgc cagggaatcc ggatttgaca 60

gaagaatata ctcaaggggt gacttttcag tgctgtcctt taggtgtggt gacaaggcca 120

agagagtggg gatgggggag tctttctggg agttcccata agctgtccag catgcccagt 180

gtcaggcctt ctttctctgc ctgcagatgt ggctgaaggt ggcttgggag gcctgcaagg 240

ctcaaaacca tggggaaagc ccaaaagctc actatggcgg aagcaggctt gaatgttctg 300

gtgaacagtc acctcagacc ttttcaagcc acccatggcc ccagagaagg caccctgtgg 360

aatggctgcc tctcttgttc ctctctctct cacgtgatct tatccaaggc cacatcccca 420

acaacctgtg ttcccttctg cccccgcatt cctctgctct caccacctcc tcacctggta 480

tacccctctt ccccatgttc acttctccga aacccattga tctttcaaga atctgatcaa 540

gttctaccta ctccaggcag ccctccttgg ctggtctagt ccatccccag ccagtctcca 600

cagccagcca tttggtcatt gccatggcca tttatttcct gagtgctcgc tctgtgccag 660

gctctgtgcc agacctcgtg gcccaagccc tcctctcatg aggcccacag gctgctggtg 720

ccagtatggt acctatgtca gcccttgtca ccaggcttgc tttagactct ggttgccggc 780

cacagaatgc tcccggtgtc ctctgcagct cctgactctt ccacca 826

<210> 462
<211> 576
<212> DNA
<213> Homo sapiens

<400> 462
acgggttccc cgggctgtgg cttaatccgt tcatgtgccc ggcactgccc gggctgctgc 60

cgagcccgtt ggtactgagg cttgcgttgt cagcctgtcc gtttgctaac gtggccatct 120

ttatatacat agagaaaatc ttctttcctt ttattctttc tcgctcacac tctctcgctc 180

ctctctctcg ctcgctcgcg ctcacacacg cacacgcata cacacactcg ggttctctcc 240

ccctcggttt ctctacacct cgctctccgc tcgctctctg ctctctctct ttctcctctt 300

ctctcgctcg ctcgcgctcg cgctctctct ctgctctccc ccctctctct ctccctccct 360

ccctctcctc tctctctccc tctctctctc cctctctctc tccctctctc ctctctctct 420

cggtctgatc tgattttttt tttctttttt tctttttttt tttttttttt ttttttttac 480

attgaacaga caggatctct gtcctttccg tttaaacagg ctggaccggt tcaagttccc 540

agccagacca ggggtggggg tggcgagtgt gcgcgc 576

<210> 463
<211> 441
<212> DNA
<213> Homo sapiens

<400> 463
tggaaacacc aggtgcacgt tgtgaccttg ttggcaaaaa gcaaggtgcc ggaaatccat 60

gcacccacag cagataactg tccccctcct gtgcccatcc tgaagctggc ccaatctgag 120

ctccccacag gagtttattt ttgatagatc gagaagacta gaactatcca aggaacgaga 180

actgccccgc ttgggcctcc tcctcacctg gtgcgttcag tcaggctgca gcatgccctg 240

aggatgtgct gcaagtccct ggggccaggc tgagagagct gaggcccttg tcttgatgaa 300

ctgcaggagt tgctgtcagg gcctgctggg ccctgaaggc ctccagtctg ccaagaggga 360

ggtggccgtg agctgagtgg tgccaagtct ccggtgccag gcattggaaa ggtcatcttg 420

acatccgtct tcccttccct g 441

<210> 464
<211> 479
<212> DNA
<213> Homo sapiens

<400> 464
atattcccct tccacctctt gatccgtttt aagctttaca aacacactcc ggggatccgc 60

ggcgggatgc ctgatgggct cgggaacctg gtcgcggcgc acccctagtc ctgcctcagt 120

ggggccgacg cccttgggct catctctccc cttgcgtttg tctccctcta cttcgggctt 180

accctctcac ttcagactac cccctggggg tcacctccct ccttggacgc acccctcccc 240

agcttcagac tcgcccctct agccccctct ggctcacctc cgcggggccg ccaccctggc 300

ctgtgccccc tggaagcgcc gagacccagc cgaaggcttc ccagccccgc actcgtcgca 360

gtttgaattt cccctcgctg gctccctttt cgggacccac tcctttcttg gctgggttgt 420

acgaagtccc ggacctcgcg tttagtttgt ccgtctatat ctgttgtaac tcctcccag 479

<210> 465
<211> 437
<212> DNA
<213> Homo sapiens

<400> 465
ctctttatca gcggaatgag taagacacac acacagcccc cagagctccc ggtctgctgc 60

gggaagctta ttaacgctga taatagatga ggctgactta cagacccaca tttttcgggt 120

aaaaggctgg ttccctctgg tccctaattg cgggggtcca ctggctttcc tcaccacaca 180

cataaactgc tgcttttaaa taccccactc ggctgccatt taacaaactt attcatacaa 240

agtttcgtta aaacttgagg cggtttaatc taaatactga tcgtaactat tcagtctgtt 300

agttctccct ggaaatggca gcggaggcgc ctccccccac ctcccgccca accacacaca 360

ccccaccgca ttcttccttc atacacccgg gttcatctcg ccaccgaggc ctcccccacc 420

cgcacccggc ggggcgc 437

<210> 466
<211> 586
<212> DNA
<213> Homo sapiens

<400> 466
ggtgtgctgg gcaacagcct agtgatcacc gtgctggcgc gcagcaagcc gggcaagccg 60

cggagcacca ccaacctgtt catcctcaac ctgagcatcg ccgacctggc ctacctgctc 120

ttctgcatcc ccttccaggc caccgtgtac gcgctgccca cctgggtgct gggcgccttc 180

atctgcaagt tcatccacta cttcttcacc gtgtccatgc tggtgagcat cttcaccctg 240

gccgcgatgt ccgtggaccg ctacgtggcc atcgtgcact cgcggcgctc ctcctccctc 300

agggtgtccc gcaacgcgct gctgggcgtg ggctgcatct gggcgctgtc cattgccatg 360

gcctcgcccg tggcctacca ccagggcctc ttccacccgc gcgccagcaa ccagaccttc 420

tgctgggagc agtggcccga ccctcgccac aagaaggcct acgtggtgtg caccttcgtc 480

ttcggctacc tgctgccgct cctgctcatc tgcttctgct atgccaaggt gcacgccggt 540

cgcggggccg agacgcgcga gggagggcgg agggccggtg ggggcc 586

<210> 467
<211> 431
<212> DNA
<213> Homo sapiens

<400> 467
cggcctcctc gcgcctcagc cctctagcgc cagcgttttg tcacatctcc ggtttccttt 60

ctttgcttca gcttccatgc gaccccctag ctgcggacac tccacacgct gagagtcagc 120

tgagcttgaa caaagagacc tttctcctct gcagaaatcg ctcgagccgc cagcgccaga 180

attcacagca gctttctctt gaaatgcttg gcatttcccc acaatttctt aggtatactt 240

ggtgggcgaa gtgcgtcatc tctgtttgaa tttatcgcac agaataagcc attttctcca 300

tttaatgtta tcccctcggt aaccaggagg tcggaggcca caacgcaaaa acagcaaatt 360

tgtcaacgga atgtgcaaag ccgggcataa atttatcttg tcctaatccc cggccccctt 420

tcatgcggtg a 431

<210> 468
<211> 462
<212> DNA
<213> Homo sapiens

<400> 468
tccctgggta ccacagatgt gccctggttc ctccgtgccc accagcagcc gggagaggcg 60

aaggcactgg cctggcctgg cccctgcttt gagtcttatc atttctgcat cattctgagc 120

tggaaacctc cccaaaatca ttcaaggacc cacataacca cacaatggaa gccttgcagc 180

cacctaacag caggaaaggc catagccagg tccccccagc tgcctcctgg gctccgaccc 240

acccaccgta gccacagcct atttacacac gtggcgtcaa agccacggcc gtcaatcact 300

gcgtgtcgct cccagctgca gacgctggac aaaagggcga cggggtggca gcccccagcg 360

aggacggatg ggccataggg ctgctctctg ggcatctctg agcaagaccc tccggcgcca 420

cagcagaaat ggccagacgg gcctgtaccc aaaaagccag cg 462

<210> 469
<211> 587
<212> DNA
<213> Homo sapiens

<400> 469
tgggcgagga ggcgggcagg acgggcctga cactagggac ctcgggcccc gggaatgcct 60

ctgggggggc gtgtacaccc gttgctccca ggaggcacac actgcggttc gcttcgccaa 120

gaatgtttaa ttgcatttga tgactacggt ttccattcat tcatttgtag agatataaca 180

ctcagaccac aaaatgcata aaatgcggtg gcttttagta ttaacagagt gctgcacccg 240

ataccacagc ctcactccag aacattctca tgggcccaaa aggagacctg gggtgttagt 300

caccagctca ctccccgtcc ccagcccctg gcaacccacg ctacttagtc attatttagg 360

tgtttaggag ttgcaaagtc aaatctttaa acccacatat ggccaggcgt ggtggctcac 420

gcctgtaatc ccagcacttt cagaggccga gacgggcaga tcacctgagg tcaggagttc 480

gagaccagcc tggccaacat ggtgaagccc cgtctccact aaaaatacaa aattagccgg 540

gcgtggtggt gggcgcctgt aatcccagct actctggagg ctgagac 587

<210> 470
<211> 788
<212> DNA
<213> Homo sapiens

<400> 470
ccgtggggct gctggacacg tccgtttcca cctgcggggt gggcaatgcc ggcacagcga 60

gtggccagac acaggcatgc ccagcagaca cccaaccgct ctccaccacc tcaaccttct 120

gtattttcct gaaagcattt accactcctt gaagttatag attaatttgt ctctgcattt 180

tgttgtccaa accaggagct cggtgagggt gtgatggggt ctgccttggt caccgtggca 240

ccccagcacc ccgcagggtc cctctggcca aaagagatgt tcactgatgc cctagaagca 300

ggaacagagg ggctcccctg cgctcgccaa gggcgcctga caaatctgga ggccccccatg 360

acagaatcag ctccagagta ggaaatgtgt ctggctgcac gaaagccccc tcgggcgcag 420

cgcctccagg cccacgcaga tgtcgctgtc atacattgtt ggaaacgcct ctgctggaaa 480

cagtgtttgc tgaatcttgg agggagagga aaaaacctag taaacattct tgttgaggtg 540

atctggggga atgtctgcaa aggcttggag ggctgatggg gcagagctgc ccaggggccc 600

aggacaaagg ccaggcgtgc gcgcgcacac acacacacac acacacacac acacgcagtg 660

gcctccctgg gggccgtggc tgggcgaggc ctgctgttgt ccgagatgct ggaaggagct 720

gtgtctctgg gtctgtcccg gccgcacccc gttctgtccc acacccaacc actccccacc 780

cacaggcc 788

<210> 471

<211> 450
<212> DNA
<213> Homo sapiens

<400> 471
tgcgggctca gggcctcgag tctctcctgg agcacgggct gcggtgcgcc ggcagcttac 60

ggggcggcca gtccttgccc acaacgatgt ggagccctgt gaaagtcgga ttcgaataaa 120

gggccacgtg tgcacccaga aagccgagtc tgtggttcag gggggtctgt cggcggagcg 180

gggccactgg aagaaaagcc tgcggacctc ggttcagcgc acgagtagga cccgacaggg 240

aagactgcaa gggtcattgt ccgagcagtg accgcggggg gctcgccact gagggggttc 300

gcagcgcgga gactccagtc tcgcgggatc tgaggcgcac tcggcttcga gggagcggcg 360

gccgcgcagc cgctgtcagg ccccgtcttg ggccgagtcc cgggttccct gtagcaggct 420

tgggagcggg gcgccacctt cctgggccct 450

<210> 472
<211> 580
<212> DNA
<213> Homo sapiens

<400> 472
tccctgcagc acccgcggcc cttggctggg gagcccctgg gaccccggcc ggcagcgcgt 60

gctgcagaca tcaatcacag aaggcaccac gtcactgaag cgccaggcct gggccgtccc 120

aggctccgct gggtccctgg tatgccgtcg gtgctcaata agcacttgct gcagaaatgt 180

gtgaaggaaa aaggggggcta gggaagggct ccaggaaagt gtaactccca tgtcagagct 240

gggaggggct tcgagggcat cactcactga gcgggaaact gaggctggag aggggctgca 300

cctggcctgg ggtcatctgc aagatcaggg gccgtgtcca ccaatgtcct gcaccccttg 360

ccgactccct ctgcctcggc cgcctccttg cccttcaccc tctctggttg ctccctgcct 420

ggtgggggta gcttgtccgt gcccagtgga cagcggggac ccatggaccc caggcacccc 480

aggccccagg gagaaatgca accactggtc ccacacagag tgggttgtcc cgggtggcag 540

catgagccat gcatcaggcg tcagaggcag agggttggca 580

<210> 473
<211> 490
<212> DNA
<213> Homo sapiens

<400> 473
gtgcgccctt atggtcggtg cgggagaccc cgtatcctcc agctgcttcc ggtggccctt 60

cctcaggtca tgtcccgcag tcccctgcct ctgaaccgcg ctctcgcgag tttccgtagc 120

cttcgcgtcc ttcctcccca gctctgattc gcctttatgt ggagattccc ctaggccttg 180

gtacatcccc agaattgacc atcgttcctc atcgttacct agagtatgag tttttcaggt 240

ttttgtatca caggccccca gttatctccc acacactacc ccgaggaccg aggttccccg 300

ctgtcttgca gagaaggcct ccttgtaggg tggccagaaa cagaggatcg actccgtgga 360

aacaaggacg gctgggtgca gttcctcacc ctataacccc agcactttgg gaggccaagg 420

cgagtggatc acctgaggtc cggagttcga gaccagcctg tctaacatgg tgaaactcca 480

tctctgccaa 490

<210> 474
<211> 517
<212> DNA
<213> Homo sapiens

<400> 474
tagatacaca aatccttatt gtggtggagc cccttccttc cgagctttcc ggaaaaccaa 60

cgacacaact ccccaaacag gaagcacaac ccgcttgcac ctgtgcacca tgctcggttg 120

gagctcccag ctgcctccag ggcacagcgg ctttggagga ggggccgcgt tgcatgctgg 180

gagcaatctg aggggcggga ccctagcgtt taagggctgt cctttttga accacatttc 240

caaagcgctc gggttccccg agtggtgccc caagcagtgg acgagcaagg tgcccgagaa 300

ggaggggacc acagacaccg cctggtgtcc acggactacc aggaagccag ggggcggtgg 360

cctccgtggg gaatgcgcat gtgcggtccc gagcctcagg ctagcgttcg ggactagagt 420

ggggcgtcgc cgccgcatgt tgcacctgtc ggcagcgcgg cctctcccgg atgtgacgta 480

gcgtgacgcc tgcaggattg atgctgaaat gacgagg 517

<210> 475
<211> 504
<212> DNA
<213> Homo sapiens

<400> 475
tgggcggccc tgcggggcaa agccccctgt gcgcgcagag ggggaggccc gggaatgaat 60

ggagggatgc ctggcgcctg aggtcaccta cacgccgccc gaggtcagag tgcagggagg 120

attttctgcc ctgggggtct cggtgtcccc agtgggaagg ggggctggac actgagccca 180

gtgtttcatt ttttaaatgg aaatgattcc cgccaccttc caattatact cagcataaaa 240

aaaaaataga ccagacacgg tggctcaggc ctggaatcct ggcactttgg gaggccgagg 300

cgggtggatc acctgaggtc aggaatttga gaccagcctg gccaacacgg tgaaaccccg 360

tctctactaa aaatacaaaa attagctggg cacggtggtg catacctgta atcccagcta 420

ctggggaggc tgaggtggga gaatcgcttg aatccgggag atggaggttg cagtgagtgc 480

gatcatgcca cttcactcca gcct 504

<210> 476
<211> 621

<212> DNA
<213> Homo sapiens

<400> 476
ttttaagggt ccgttttgtt ttattttgct ccctggtcta gctcagctcc ggaatcggtg 60

cagacacgca atatcctctc agtagatatt tatggaataa ataaatctgt ctcatctttg 120

gcattgctat gatggggtct attttgtctt tttctctatt tatccccatg tttaaaatat 180

ttttttatta gaaaaaaaaa cggaagtcac cttaggatga aagcgcctcc cctcccaacc 240

ccccagtggg gtttggcatg tctctcgctg tccccatcac ctccctggcc cgtgcaggtg 300

gcctggccgt ggttctgcac gggccgccgc gtttaggcgg cactgcagct aatgacaccc 360

cgcagcgtgt gcctggctgg ccgcccgaga tgtgcctgcg catggcatgg ggacgtttgc 420

aagcctggat cctggcgcgg gggcaggtag cgggagaaca ggcacgcaga tggcagaggg 480

gctggggcgg ggacgctcgc ctggcgtggg ccacagtcac gacccaatcg tagcccccac 540

tgtgtctctt catagttgca tccttggaaa ccggcggggc tgcaggaaaa aaattaaaaa 600

tcagatgaaa gaaaataaag c 621

<210> 477
<211> 608
<212> DNA
<213> Homo sapiens

<400> 477
ctgaatctgg ccccccagga gccccgtgtg aggcactttc aggggccgcc ggaggcctgg 60

aagacccgag tgtggcagta ggaatgtccc gagcggcttt cagtgcctcc ttttcaaccc 120

tccccgcctg gagccctcct tctggatttg cgtctcggca gattgtggga tgtcacttct 180

gtcctcttcc gccctgcggg attttcccag agccttcggg tttgagccca gctggaagtg 240

gccctcgttc acgctgcctg tcagcacctg cctgggctgt gtcacctaga gtgtggctcc 300

gtgtgtgcgg cgcagacacc gtgggggtgt ttggagcttc ctgcgtggct cccagatgtc 360

tttactacac agtgcccgcc gtggaaggga cagatgagca atgtagcccg aggcgttctt 420

tcaagagcag aggaatcggg agcgaagtgt ttttgttcat ttagcagact aggatcatgg 480

gcgagtttcc tctaatagca ctgaagaggc gaattcacgg cttcccctcg gccagcggct 540

ttggcgtgga tttggagccg ggccaggttc atggaaccct cctgcagaag cagactgggg 600

cttcctgg 608

<210> 478
<211> 430
<212> DNA
<213> Homo sapiens

<400> 478
tgccctgcac tgtcctccaa gggccgctag gtggcgctcc cgcccttccc ggggccgcgc 60

tcatgcctgg aatcttccca ggccgtcctc gcgctggagg aggacggagc ctttgcctgg 120

agggcgtggg agctccgcct tgcctggccc ttccctgggt tggagttgta agcggggaga 180

gggggggcg ggggagaatg ctgtacctct tcgttaaaaa aaagcacaat gagcaggaag 240

gggcgagctg tgcgcatcct cacctgcgtc cgaagcgcag atggagccca agggaaaggc 300

cctgtagagg accccgtgtg acttggggca aagcgtggcc tcccaggggt gcgtgggcag 360

cgggacgccc atggtgtgac cgggtttgtg cctccatagg gaccgtctgc cctgtgcaga 420

gagcccctgt 430

<210> 479
<211> 471
<212> DNA
<213> Homo sapiens

<400> 479
caccaccccc agtcaccacc tcctgtccca gccccccatg tcccaggccc gggaggctgt 60

catcgcagct gcatttgcca agctggaccg cagtggggac ggcgtcgtga cggtggacga 120

cctccgcggg gtgtacagtg gccgtgccca ccccaaggtg cgcagtgggg agtggaccga 180

ggacgaggtg ctgcgccgct tcctggacaa cttcgactcc tctgagaagg acgggcaggt 240

gggtggctgc gcggggggcc ccctccccag gcagttcctc ggccgtgccc cctcacggcc 300

ctctgttccc aggtcacact ggcggaattc caggactact acagcggcgt gagtgcctcc 360

atgaacacgg atgaggagtt cgtggccatg atgaccagtg cctggcagct gtgagcagct 420

ccggctcagc cctgctgccc tggcctgtca ctccccaccc ctgccggaga c 471

<210> 480
<211> 802
<212> DNA
<213> Homo sapiens

<400> 480
tgtagtccca agctactcgg gaggctgagg catgagaatt gcttaaaccc gggaggcgga 60

gggtgcagtg agccaacatt gcactgcact ccagccaggg cgacagaggg agactcaaaa 120

aaaaaaaaac aaaaactttt gtacttggtt aacgtgaacg ctgcctcctt aaaaaaaaac 180

aaacaaaaaa aaacccagga acatttaacg tgaatgtcag ggactttggg atgagtccag 240

ggacctagaa gtgcggaatg cacaaaggag ctgggaaagt gtgtgagcct gagcccaggg 300

ctgcaagttg ggagtgcctg agtgcacagg gaggccactc caaccctcca gctgcctcta 360

ggggtatcag agagggagcc cgtccccact ggccaccaat acctcttagg ccagggcgaa 420

cacgtccacg gggtatatt caacttgtga ccccagtctc tgcgagagcc tgcagtagca 480

tggggggggag agacctcgcg ttctctaagg atggaggaca aaggaggaga gactgggagg 540

gcagggacgg ttgcggaacg gtcacaaggc ctgggcgcga gggtttggaa ggcaaaggtg 600

cgagaggcac caggtgaccc gtcctgcgtg attagcggtc ggacaatctg agcctgctaa 660

gagctttcct gaccacgctg gtatgaaggg cggcttggag gtgacaatgg gggacgacag 720

ggcacagcag gtgtggagac ggtgtgcagg gccggagcaa aaccgttaaa ggcaggtgga 780

gacaccctcc tcccctcccc ca 802

<210> 481
<211> 311
<212> DNA
<213> Homo sapiens

<400> 481
gtgctgcgct cccggggcac tcacgccgtc gctggctact ccgcggggcc ggctcagctg 60

ccgctgcagc gctacgcacg cctccctcag acgcgggctc agctcgcacc tgcagaggag 120

gccaggagag actcaggatg gatcggggac agcgccgacc aggaggaggg cagccgcctc 180

ctctccaaac aactagcgag cgccgctcac ctcccctgtg ccacctgctg aggctctgcc 240

tccgcctcgg gctcagggtc ccggatgtcc ccgccgctgg gagggccagg ggtgcccgag 300

tcggatcccc c 311

<210> 482
<211> 470
<212> DNA
<213> Homo sapiens

<400> 482
ggcttggccc ggtgccactg tcgggtacac aaagtcctgt ctggggggcc ggttgttcag 60

gcagagcccc aggcccgagc tgcctcgaga gaaaagcaac tgtcatgcta ggtggctgca 120

aatcaagagc tcaggagcct cagctggatg gggtcccag ggagcatccc tgatttctat 180

cgtctcccgt gtaccctgcc ccaccatgag tgtgacccgc tctgagggac acccaggtgc 240

atcctcactc tagaaagctg gtgatgtagt cacggctgca ggatgaccac acgaatgggt 300

tggtcttcat ggtaatgtgg gcagccatga gcttggctgg gtcctgacca cgggccccac 360

agctgtttcc cacgccgtca tggttcatgc cgaatctggg gaaaggggtg tcggctctgc 420

cgggcgctga gggacccgcc cagctcctcc cctcctcccc ctcttgtgtg 470

<210> 483
<211> 452
<212> DNA
<213> Homo sapiens

<400> 483
ccctccgttt ttttttaaat acctggactt aattgtttaa actggagtcc ggaccaaaac 60

atctctgacc tcgggggtct tcctttggtc aggaagaggg gagggagcaa ttggacccct 120

ttccctctgc ggctgcagag tccaattttc ctctggggac atttgctttt tcccttgggg 180

aagacagtac ggattctagt tctgtggact cttttactta gctttgagac ccaagcaagt 240

taattggctt gtgcctctta ttttactcta atgcaatgaa taaagacagt cccagccttc 300

gccctaaggg agcaggagca cctgcgatgc cccgttccca agtcctcagg gcgaatccgc 360

caaatgtgcg agctgggcag cccacccctt tcagctgctg gccggaagcg gaagtgggcg 420

tccgtcgcct cgccatctcc catagctgtc gc 452

<210> 484
<211> 510
<212> DNA
<213> Homo sapiens

<400> 484
tcttacgtct aagcctctgt aaccccacgc cctgcccgca gacgctcccc ggctagacga 60

ttcggactgc cccaggagtt ggacgtggcc cgagggccca gagcagacgc tgcgctgcga 120

ggcccgcggg aacccagaac cctcagtgca ctgtgcgcgc tccgacggcg gggccgtgct 180

ggctctgggc ctgctgggtc cagtcactcg ggcgctctca ggcacttacc gctgcaaggc 240

ggccaatgat caaggcgagg cggtcaagga cgtaacgcta acggtggagt gtgagtgggg 300

gtgcgcaggg tgcatttcta tctggttcaa ggtctggagg gtggccagcc tccagggaag 360

agtaggagta gggtatgagg tgtccctttg ggtgaggttt tgggaaaggg aagaggctgg 420

ttagtggggt tggagaaaga tcttggagga tggaagggac cgggtgggcg tgcccctagc 480

ctagggcgtg gtatttgggc ggagtcgtgg 510

<210> 485
<211> 501
<212> DNA
<213> Homo sapiens

<400> 485
tgcctcagcc tcccgagtag ctgggatcac aggcatgcgc caccacgccc ggctaatttt 60

gtatttttag tagagataga gtttctccat gttggccagg ctggtctcaa actcccgacc 120

tcaggtgatc cgcccgcctc agcctcccaa agtgctggga ttacaggcgt gagccaccgc 180

gcccagtcta attttttttt aagagacggg gatctcgctg tgttgcccag gctggtcttg 240

aactcctggg ctcaagcggt tcttccgcct cgaccttcca aagagttggg attacaggca 300

tgagccactg ctggcctcct tttggatact acctcgagaa ggcgtcccag ccccgacgta 360

gtaacgcctt accctcgatt tggaggcgtt gttatcccag cctgatgttt ggggtcgcgt 420

tccagcggtg atcgcgggag tgttcgcgcc ccggcccata gagggcgtgc cagccctgga 480

cccgcaggtt tccctccagt g 501

<210> 486
<211> 395
<212> DNA
<213> Homo sapiens

<400> 486
gctccgccac agcagctctg cgagggactg gcttctgctc acctgagacc ggagggggcg 60

tggcctcacc tcacctctgc ccaacaaacc ctgcgcgtcc aggccagggt ggtatcgcga 120

gtcacctggg ccccacaggc accacgtgac caggccaatt cacctgtgtc ctcgccccac 180

gtgaaccaac cacagagggg cggggcctca cctgtccttt ccacgcagac ttgccacgtg 240

acgggggcgg gtgctcgggc cagagtccaa cggtccagca agtcgacgac cccgcacctc 300

ccagccccag caccaagaga ctgaccgaac cagggccagg gatcccgggc aagaggagcg 360

ggcgctcacc ttcccctagc cccgccccac gacct 395

<210> 487
<211> 334
<212> DNA
<213> Homo sapiens

<400> 487
gtgagtgagt gagggagaga gagagagaat aggtgtgtgt agaggctccc ggtgcctctg 60

tctggctgct gaggctgaga tgggagcaag tggctggcga agctggtggt ggcttcaaac 120

cacactttcg tagaacaatc gcaagagaaa attgttgggg ggagggagga ggaggagaag 180

gcggttttcc ttgtgccccc ccttctaacg ctgcttttct ccttctctct ttccccctca 240

tcccgtcttc ccctcctccc gtcctccctc gccccgcatg ctcccggctt gccgcctgca 300

ggatgagttc cacccgttca tcgaggcact gctg 334

<210> 488
<211> 326
<212> DNA
<213> Homo sapiens

<400> 488
cggggctgag gctcaggtag ttggggagta ggtgcgcgtt taggaacccc ggggagatgc 60

tgcgtctcag gaagttgggg agggcgctca ggcttgggac tcctctgggg acaaggctca 120

ggaccttggg gagggagtgt tcgctgggaa accttgagag attccgtgtc ttagaatgct 180

ggagagaggg tgcatgctta ggacccgtcg gggagcgtgg ctgacaacag tggggagtgg 240

accttgcgct cctccgaccc cctgggggtg aggatccgga ttgtgggggg agttggggat 300

gtagggcaag gatccctcag ggcgc 326

<210> 489
<211> 398
<212> DNA
<213> Homo sapiens

<400> 489
gaggctccag gcggcgaaca ccctgccctg tggtcaccca gcacaggtcc ggagactccg 60

attgtcactt ctgtttattg agttacaagt tgagatgtgc aggttcggtg gcgccagccc 120

ccccatcccc ccccttggg caaaaatagc tcccagcgcc gaggaatggg gggtaggaag 180

ggtctcggat aacgggatgg ggcctcgagg gtccctgtgg ggctcgtggg tcccaggatc 240

aagcacggct gacacggaag acagcggggt ggggggcctg ccttggccgt ggcgttgggg 300

ggaaggtgag ggagagcttc tgtacaaggt catcttccgt gagggtcccg gctgcggccc 360

caaaacgccg atgggccccg cgggacggaa gcggagag 398

<210> 490
<211> 481
<212> DNA
<213> Homo sapiens

<400> 490
cgcttctccc ggccctccag cacgccacgc cgctcccgct ccatcgcccc ggcccgctcc 60

gacctcaccg ttcttggagc cctcgctcct tctcaaggac ccgaggcctg gcacgccttg 120

gcacagtctt gggaccatcc cgtggccctc ccgcattcag gaggcgtagg ctcaatgaaa 180

cattctcatt cttcaaaagg acacggccca cgccagggcg ttgggaggcg cacctagtgg 240

agacgcagct ctctgcccta caccaggagc ttggcaatgg ggttccagct tggacgcggg 300

taccctgggc acttcccagg catcttcttc tgcaggtctg cacagcgggc tactgggaca 360

aaggtgtgat gggggctgct gagattaggg ggttctctga gggtcacttg ctgctgtgac 420

cctggggaac ccgggggggg ggctccccaa catcaggtta atacatgagt tgcagagagg 480

c 481

<210> 491
<211> 346
<212> DNA
<213> Homo sapiens

<400> 491
ctaaagagca tccgctgcag cgcaaccacg aagacacttt ggcaaacgcc ggggttctgt 60

ttgataagga gaagcgggag tacgtgttag tgggagcaga tggctccaag cagaaaatgc 120

ctgctgattt ggttcacagc actaaagtgg gcagccagag agacctgcca agtaagctcg 180

accctttaga aagcagtcgg gattttttgt cacacgggct gaaccagact ctcgagtata 240

acctgcaggg tcctgggaac atgaaggaga agcccaccga gtgccccgac tgcggccggg 300

tgttccgcac ttaccaccag gtggtcgtgc actcccgtgt ccacaa 346

<210> 492
<211> 432

<212> DNA
<213> Homo sapiens

<400> 492
cagactcgcc atcccagacc ctactcccag ttcattcctt cctgaggtcc ggggtttgag 60

gcatggggag ggatgggttg gaaacagcgc ccaggaactc gtcctagcaa ggattggcgg 120

gtgggagtgg cggcagatcc tccaccaccc acagcttacg cgaattcacc tccctcctct 180

gcgctttaat agcgggtaat ggtcgctgaa atgagaatat ccattaccag cactaaaaaa 240

gccgcttttt actgtgccac acagtcaact cgtttgatcc tcacgcaaga gttagaatga 300

acaatagccc cattttccca gtgaggaaac tgaggcccag aggggatgag tgatttcccc 360

agggccaccc agcacgggcc tccggaatcc tcaccaccga ccctcccctg caggccgtgg 420

ttggggacgc cc 432

<210> 493
<211> 207
<212> DNA
<213> Homo sapiens

<400> 493
accgacctct cccagaccct ggccttctcc ttcccctccc cttcccatcc ggcgtctggc 60

tcaccttgcg tccgaagacc tgcacagact gcagcgggcc cttggacggc atggctccga 120

gcgtggacta gacaacctca ccgcgcggcg ccgcaaccgg aaaaggaaag ctagggggcca 180

ccctggccgc ttttcagggt ctgcgca 207

<210> 494
<211> 378
<212> DNA
<213> Homo sapiens

<400> 494
caccatgttg gtcaggctgg tctcaaactc ctgatctcaa gtgatctgcc ggtttcagcc 60

ttccaaggtg ctgagattac aggcgtgagc caccaccccc agccttaaga cagactattc 120

tagactgagt ccaagttcca actgcttgat ttggggcaaa tgtcttcctc tctctgagcc 180

tcggtctcct cttctgttta agggacacat tgacagcccc tccctcatac atgttgggaa 240

tcccagactc agcacggtgc ttggcacaca gtagtcactg aatcagttac tccccaaagg 300

actccatcca ccctcccaaa ccccaccccg gaagtacctc gggaagcacg cactcttcca 360

ttttgcacgc cacaaaca 378

<210> 495
<211> 402
<212> DNA
<213> Homo sapiens

<400> 495

ctggtgcctt cggaagatgc acgtcgggaa ccttcggcac ttgcatttcc ggcagccgaa 60

tctctgacac tttcggcagc tgcacctcgg ggaggtgcac atcgggcaca gccatctcag 120

gcaccttggg gagttttatc tctgggagct tcatgtcagg gactttcatt tcacagactt 180

tgggcagctg cacctctggg aggtgcacat cgggcacggc catttcaggc accttgggga 240

gtttcatctc tgagactttt ggcagctgca cctcggggag tcgaacctct ggcacagcca 300

tctcaggcat tttagggagt ttcatctctg ggactttcgg gagctgcact tccgggaggt 360

gcacatcggg cacagccatc tcgggcacct tcgggagttt ca 402

<210> 496
<211> 824
<212> DNA
<213> Homo sapiens

<400> 496
caggtcccac cccaggtgga gtcagggcag ggccagtcac caggtgagcc gggagcagag 60

cagggagcag actctgagct gctcctccct cgtccaaggg gtttcctcct ctcatctggg 120

gaaaaagtgt gagatgtttc aaagcttcct tcttccttgt agctcatgaa ttaggcaaaa 180

gaacacaaag aggtgacaaa aagggacgta ttgatgaccg agagaaccct taaggaccct 240

tcctgctttt ccctctgtga agctctttcc actacctggg gctttggggc tgagggagcc 300

ccctccccac attccaggct ggatccataa tcagccatga gaaattactg aagtacaggg 360

aggagggtct gcagagacag agtgagaggg ttcagcgggg agaatttggg atctgcttgt 420

gcccatggga cacaggctgg aaaagaaaag ttttttcagc tcttccttaa caccagagag 480

accccatctc aaaatatgat gccaacggtc cagggatcca tgtaccacgg actgtgatca 540

tcttgactgt ggtactattg cggtcagtgg ccaatttgtg ggcatagcag gtataggata 600

cactattctt tgcagtgctt tggggataaa gagctcttgc atgtgttgca gaaggtttcc 660

attactcagc caaaaatacg gagagggtgg gttagaggcc acatggatga agagtctaag 720

gtttgctcct aactcgtaag atatatctgg ggtagaaggg gtgtccaagc cacccggagc 780

aaagagaata aagccgtaag tgatgtcctc agagggaagc ggaa 824

<210> 497
<211> 781
<212> DNA
<213> Homo sapiens

<400> 497
gccccaaccc ctatgccccg cccaccacga gtcaagtcag gctcccgccc ggttcggctt 60

acctcgctga acgccccgcg tccgatcacc ttcagaatct cgaagtcgtc cctctgcagt 120

cggacctcct taagcctcac cacgatgggc tccgctgggg gggtggtggg ggaaaagaac 180

cgagggtcac cagaaagggc actggagaca agggggaaag ccccaccctc tgtctgtctc 240

ccccttctctc tgcctctcag cttcacccta ggactgtctg cttcccaggg gcttccccac 300

ataaacaccg tgtaaggttc tgggggccaa aaatgccctc ccatagaggt gagatgcctg 360

agaagccctt tgcgggacag ggcattggcc cagagggctc caagggtgtg caggatggtt 420

agggtggggt aacggagtct gcagaaggac agaccctaaa gaacaagggg agaaggaaat 480

aagacccagt tcttccacct tccaccctga ctccaggtga cagttcaggt gcagccaggg 540

ccccaccccc acgttctcat gtagaatgtc ctgggtaacg gcccagacgt ggggttgtat 600

ccagtacctc tagattcaga tgcaggtggt tcttgaacca cactttggaa aaccctggat 660

ttggggctgg gggaaggaac aggacaggga gacaaaggta gcatggtcac atatcccaga 720

ctcaagtgct ccggtccagc ccatctctca gtcctccagg aggagtgctt tagtcctacc 780

c 781

<210> 498
<211> 618
<212> DNA
<213> Homo sapiens

<400> 498
aaggtcttca tggggggggg ggggtgtgga ctgtaccatg ttgcgaagcc gggggtgtgt 60

gagattcctg ggctaaacca cagggaggca gaggtggagg gaaggggttg taccttgtag 120

attctcagta agggggaagg gagcatcaga ttgtggaaca gcgtaaggta gtacgacttg 180

gaaagggaga gaggatccaa attgtaccat tttggaaatt cacagtaata ggaagcacag 240

aacagtaggt aattggacca taggaaggca ggggtcaagg gttatagacc aagttacaaa 300

gcagtgaaga ggaaagacaa atcgtgggct agcaatggcc tagggctggc aggtggactg 360

taccacatag acatgtaggg aaagagagaa aaagagacag ctcacgaggc tagaccatat 420

agacgcagga aggaggatgg gactgaatca cactgagaca ccaaggggag acacatataa 480

gagggttagc ccaagactta gagggggtgc agactggacc ataccatgca ggcggtagtt 540

ggggcctcgg cagcgcctct cggggtgccg ggggtccctg ttgcccctca gtgccctgtg 600

ggccaagggc tgcagggg 618

<210> 499
<211> 319
<212> DNA
<213> Homo sapiens

<400> 499
caaggccgcc ctgctggtca ccacgcggcc cagggccctg agggacctcc ggatcctggc 60

ggaggagccg atctacataa gggtggaggg cttcctggag gaggacagga gggcctattt 120

cctgagacac tttggagacg aggaccaagc catgcgtgcc tttgagctaa tgaggagcaa 180

cgcggccctg ttccagctgg gctcggcccc cgcggtgtgc tggatcgtgt gcacgactct 240

gaagctgcag atggagaagg gggaggaccc ggtccccacc tgcctcaccc gcacggggct 300

gttcctgcgt ttcctctgc 319

<210> 500
<211> 296
<212> DNA
<213> Homo sapiens

<400> 500
cacgcccact tccggaaagg acccaaagcc gggcgagtgc acgtcccgcc ggttgctgag 60

gagaagggag gtctgggcgg ggcagcggcc gaggctggac tggggcgcgg cgggggcggt 120

gtcctggttc ctgtgtgggc ggcgggcgtg agcgtgcgcg tgtggcctgg tggctgtgag 180

tgcccatacg tggtgagcgt gcggtgctgt gctgaggagg ggcgagcgcg cgcggggatg 240

gcggcccggt gtgtgactgt ccggtgcgtg gccgcgaatc tgcgcctgcg tgcatg 296

<210> 501
<211> 800
<212> DNA
<213> Homo sapiens

<400> 501
gaaggaatag attttacccc tgcatctgct ggttccattg cccctcatcc ggctcccagg 60

aaagctttga attgtgggca aaaccaggtg gcactggcag agacattctt ggggttggtg 120

tctcccctaa aacacactgt gccttctaga gaggttccct agaagtcgcg atctttgctg 180

agaaatatcc acacatgctt tgccacattg tcaatcatag gaacatgctg cccaacaaat 240

atttgtggca tagtaaaatg tttttaaaag cagttgctat ggattgaacg tatccccccc 300

caactccccg ccatcaattc atgtgtcctg atgtgatggt atttagaaac aggccttctg 360

gaggtgatta ggtcatgagg atagagccct catgcatggg attagtgccc ttacaagacg 420

agacagaaaa gagcttcctc tctctctcag gaataatggt gctcaggaga acccagccat 480

actggcaccc tgatcctaga cttacaactc ccagaactgt cggaaataca tgtttgttgt 540

ttaggccaca caatctgtgg cagacaagct aactaaaact gcagtcttgc aggctagtgt 600

ctccctagtt aggatttgat ctcagagcct caagggctgg gcatggctct tgcctcaagg 660

attgaagttg cagggaggga catggaggcc tgtccaggac ctgggagttg tgccttggta 720

cagtgccctg gccgctctgt cctgtgcccc cggggctgcc catcccatgt gtgattccta 780

ctggctgcac tgtgcaggca 800

<210> 502
<211> 812
<212> DNA
<213> Homo sapiens

<400> 502
ggcctcactc tccatcctga agatggagca cagactggct ggctgaggcc gggagctcat 60

cacgggatgg agacagattg gcagacaaag aggaggcggg cggttggggc tgggtgcccc 120

tggggaggaa ggcggggaga gagcgtagga gcaggaggaa agaaggctgg gcttatgtgc 180

agcctcacag actgggcctg cagagtctcc agctggaggt tgggactgga ccttcctcct 240

gaatgggcag ggagggaggc ttgttggcct agccctcggg gttcttgaaa taaatgaggg 300

agggccttgg ggggtggtaa attgctgagg ctgtgataaa cattctcccc ttcccacaaa 360

acacacacag gcacgtgtgc gtgcatacac atacacacac acaccctggc cacactgctg 420

tgcatatctt actgattgtt gtcctctgtg acagcagttc tccaagttga gtgcattgga 480

aggatcagtt aaaatgcaga tttccaggca tttccatctc taaatgtagg gtgtggcctg 540

agaatgtgta tgtcttacag gatcctggtt gatgctgatg ctgggggttc tgggacacac 600

tttgagaacc actgctccat tgagcagcct ttcagtttgg gtgcccctcg gtgctggtgc 660

tgggagagag ttaggagggc aagggggtta gtgagggctg ctacctgtga aacacaaagc 720

aggagggaat ggaattggcc aggagagcct cagaccacag cccgggcctg cagagtcacc 780

ctctaggggt ctctggagta aagattatcc ac 812

<210> 503
<211> 386
<212> DNA
<213> Homo sapiens

<400> 503
gaccagggtc cctggctacc tgctacgggg gccggcagat ggtggagccc ggaaaccgag 60

cgctgtggag cgcctggagg ccgacaaggc caagtacgtc aagagcctgc acgtggccaa 120

cacccgccag gagcctgtgc agcccctgct gtccaaacag ccgctcttta gccctgagac 180

tcgccgcaca gtgctcacgc ccagccgccg agccctgcct ggcccctgcc gacggcccca 240

gctggacctg gacatcctca gcagcctcat cgacttgtgt gacagccccg tgtcccctgc 300

cgaggccagc cgcactcctg gacgggccga gggagccggc cgtcctcccc cagccacccc 360

tccgcgaccg ccgcccagta cctctg 386

<210> 504
<211> 519
<212> DNA
<213> Homo sapiens

<400> 504
gaaagctcag aaggcgagct taccccggac ccaaacgcgc aatactaacc gggagtgtaa 60

ggaggccacg ccctcctcgc gtatttgtgc ccaaatgagc tgcctcccca gcgtgcacca 120

aattcctgag ccacctgctc cgagctcgat ttccctttca agctgtgtgt tctgcggggg 180

cagggagagc tgcaagggct catctttcag gacgtgctgc aaaggccctt tgaagtcatc 240

cgccctgctc ttggcacctt gcgcctggag ctgagctctt ccaaaaatga cccctgcgca 300

ttccgtggtg cggccgccca gcccaggcag tcgcggtcca gagcagggga agctgcagcg 360

cggccgacag ctgggagagc cgaggccctg agggaggacc ctgcctcaag ccaagcaacc 420

aggccctgcg gccctctccc cacccctgtg cggccctggc gcagggaacc gggacggggc 480

tttcaggcct tgaagggcag acttactcca ccaaacatt 519

<210> 505
<211> 551
<212> DNA
<213> Homo sapiens

<400> 505
ggaggcccgc cagtggtggc cgtagacacg ccagagaggg cggcggcgcc ggcgacgacg 60

gcactggcag cgcaggagac ggaggaaggc gcgcttgaac tcgcggctgg aacaggggta 120

gatgagcggg ttcacgcagc tgttgaagta gccgagccag aagatgacct tgaagacgcc 180

ctccgatggc ttcagctgcg ggaacaagga gcctgtaggg agcagagacc gatactattt 240

agctgcttgg ggagggggag gccaggcggc tctgggcgca aaggggagac ccttcagtag 300

ccttggctga gtcattgact aggagttctc aagggatccg tgctgtaaat caggaggtcc 360

atgaacttgg atagagaaaa ataataattg ttttcactca cctctacctg atattgagga 420

ttaccttcaa tgaatgtagg caacaaacca gagtagagtt agacaaaacc acgtgcaatt 480

gctgtttttg tagctcaaaa ccggtgcaat attggcagtt tcgtattatg agacaaagta 540

gcaaacatag c 551

<210> 506
<211> 421
<212> DNA
<213> Homo sapiens

<400> 506
tcggccgagg gctccgggga cttggagctt gagtcctgag gggcgccccc ggcagccaga 60

ccgtgcactg gggggagggg gagagagaag cgcgtcaggc gtctggaggc cgcgcgcagc 120

ctgcaccaga ctcggagcac cctggatcct ccgtgcgacc ctggacctcc gcgcctggca 180

gcccagcccc gctgcctttg ccatgacccc tgccttcctc tacgcctgac gaagacgtcg 240

ggacctccca gggtaaagag ggaaatccgt aggagtgggg gctaggggct cgcctttcaa 300

ggggctcaca tagtgtgggt gagaatttgg ggggggaagt ggcactgaag atcatcgcgg 360

gttaagggct ccggcgccct taagcggttt cctctccggc gagacgtggg cagattttta 420

a 421

<210> 507
<211> 580
<212> DNA
<213> Homo sapiens

<400> 507
aaggtctgcc cagaaggctg ccgaccaata acgctcaccc tcctgcgccc ggtagtgagg 60

acctgggaca aatcgcagtc tgtgtcttcg ttacaaaatt gccatcattg cccagtggtc 120

cagtgacgtc acggaagagg ccgaggctca ttctcctccc tctaatcacc ccctcgccag 180

ttagccatgg aatagaagaa aacagcttgg ggggtgcggg gagggtcttc tccagctgta 240

atgccagcta aggtgctttg gtagcaaagc tgggggtgag gaggcacagg ctcctggacg 300

gagccaggct cctggacgga ggccgctcag agtctctgcg gcgtccgctc ggtccgcacg 360

tgctgcagca ctacctcctt ggaaggggcc gcgcacttgt actccatctt ggccctggcg 420

gcgccctgct tcttgcgcag gtggcagagg agcgccaaaa gcgccaccac caggcacagg 480

ctcgcgatgg agatgcctat gagcaagccc gaatgcacga gccccacggc cggaggagtc 540

aaggtggagc cgggcgtcgg gctgggcggg ggctcgccag 580

<210> 508
<211> 491
<212> DNA
<213> Homo sapiens

<400> 508
tcgggcccgc agatgcactc gaaggtaccg gggaggttgt ggcacacccc ggagcagaag 60

ccgccgtttt cgcactcgtc gatgtccgtg cagatgaaac cgtcgtccag gatgtagcct 120

tcagggcact cacagctagc ctgggtgttg gggtcgcagt cggctggaca ggcagtctgg 180

ttgcaaaaca tctggcacct gtgcggctcg tggggaatgg gcgcgaagcc ctcggcgcag 240

acgcagaggt agctagtttg gttcaggggc tggcactggt actcgcagtt ggctctgaag 300

cacgggtcca cgggctccac acactcgccg tccaccaggt cgtagttagg gtagcagtgg 360

cactcgaagc caccctgtgt gttgacacag cgctgcggac acggactggg ctccagtatg 420

cagtcatcca cgtcctcgca ccggtgttgg tcggccgcca gccggtagcc ggtctcgcac 480

atgcacgagt a 491

<210> 509
<211> 810
<212> DNA
<213> Homo sapiens

<400> 509
actccaaagt gtccattcat tttggttgct aagaattgct cttcgtcccc ggtgggtgtt 60

taaaaagaaa gatctgccct tcctgtccta gctcttacaa tcttaaacct cctcatttgt 120

tcccaggaac ttttgcttag agaatgatat tgatgacagt agtttctatt catgagcact 180

tactacaatc caagagttag aagctgttac tcactagcta tgtgatcttg gataaagttc 240

accttcctga accttaattc ctcattttta aagtagcatt aatggtttca cacgcagaca 300

tgtttaggag aacttactat gaaatacatt ttggaaaaag atgatgatgg atggatggat 360

tgatggatgg atggatggat ggatggagag ttgggtagat acataaatat agcaaaatga 420

taatggtaca ccaaagtggt gagtatatga gtgttgactg tagtttcttt taacttttcc 480

ctatgttaac ataaaacatt gctgaggaaa acagtattga taatggtacc tactacatgg 540

tgttgtgcaa tggaagaaat gagatgaggc aagtaaagtg ttgtccgtat ggtgtttgac 600

ataaagctgt aagtcagtgt gttattgttg ttagtatttg tcgcaaccag caaaataggt 660

attataactc tattttaggg agaaaaataa aactagatac tcagataaaa tagtaacagg 720

acgtaaacta gttaacatac gtgctagact tgagctgggc cggcctatac caaagctgtg 780

ccctgcccac ctcgccacca ggcagccaag 810

<210> 510
<211> 516
<212> DNA
<213> Homo sapiens

<400> 510
ccagccttcc agaggagcct gtcccctgct ctcctgggga ccccagcacc ggctgaagcc 60

tccagagtga ctcatggctc tatgtccatc actgcagctc tggaggagta accacggcac 120

acataccctt ctatgggaca cctgcaaata cttttcaata agctcgagtc catttcatca 180

tcaacacaaa cagaaagacc ctccactagg tctctcaccc gcttgacata aatttgtttt 240

tcatattcct catggaaaat agagctacgc ttgatcagga aggtgaaaat ggaatgggtg 300

agggtattta aattctctct tctctctctt ccctccttgc tatgtgtact tggaataagt 360

gcacacgctc agctttaaga gctgtttggg acttgggaac cagcttgcct ggacatcaga 420

ggccacaaag tccgtccctg tctctttgca gtctgtgcag ttgccccggt tggtggagcc 480

atggtggctg tgccctgtgg gttgtgaatg attgca 516

<210> 511
<211> 497
<212> DNA
<213> Homo sapiens

<400> 511
tggaaagcct aaggggcgag ggcggacgcg ggcgccgtgc ctgcagcccc gggtgccgac 60

caagtggcct cacctgggac aggtcttggg gtctccctcc cctaggtctc cctggaaggt 120

aggcgatcac gggagtgaca gaccccgagt agatagccag tgatccaaag acaccaaaag 180

gccaggcggt aggagcccag cctcagacac tctctatagt gaccgcggcg cctcgggaag 240

gatcgtctta aactcatttt gcaggtgaga gcaactggga cttattaacc cagtcacatc 300

ggacacagag acgtggcgag ggcgacacag agcaggagac tcaatcaagg agcccgtatt 360

ggagctctaa ggggactcag agatgcagaa ggaacgaact tctaaggggt cttgcaattc 420

ctggactggg gcgttcctaa tgcatcccgg gaccccaatg ccagggaggg gcctgcagga 480

ccccagcggt gggcgag 497

<210> 512
<211> 738
<212> DNA
<213> Homo sapiens

<400> 512
ttctggggga cacaaaccac tttctctggt ttgttctctg ctttgtcccc ggttctggca 60

ctgtaggccc tcaataaatg gtggctgtta tgcactaggt ttaaaggaga ctgagctccc 120

ctctaggcac cagccagggc aatgacccca agacctctct tgcaggtaag atgccaggat 180

agttgcagct tcttttacat tcattgtcac gtcccaggag ccccctgagc cgaacgggac 240

ttagcgctgg agtcctacag gtgagaagtg agaagcagga tgcaagccca tgggcaagct 300

tcatggggac agaatagaca ggaggcatcc gcctgccagc tagaggccag agtccagttc 360

cccattgtgc agatggggcc gttgaggccc aaaggcacac agagaggtga ggtgaactga 420

acccaaatga ggggatacag aagctggcat gggaggagtc aaagcaagag ggccaggctg 480

caaaggttgg aagacccaga aaatgtgcag tgggagcccc agttcagagc tgggaaggcg 540

cgcaagccaa aatccagctc ccattgcaca gatggtgacc tccgagactt agagacacac 600

gaggactgct ccagggcaaa cagcaccgcg gcatcggact tcgaactcgc gcctctgacc 660

ccaggcagga catccctcag ccccagcccg gcccgacggc cccactcaca cagccccgtt 720

ccggaagccc ttaagcac 738

<210> 513
<211> 892
<212> DNA
<213> Homo sapiens

<400> 513
gtgggaaaag agatgtggcc tttcctgcac tgccggtttc tctggccccc gggcccagca 60

tttcggcgaa atctcccaag gtccacattc tcccatttct ctcgccgatt tgggcaggat 120

gcaggcagct ggccaagaaa tgatcctggg cttcaggtca ggagtcaggc cctcgagtcc 180

gcttcctgcc tgcttctctc tacacgtgac tcaccgcgga catcgaccaa gtcatcgcgc 240

tctgccactg cgcctgtgtc ctcgtcaggt cactccatct atagattctg ggagcctgat 300

caatcgtagt tcctggggct ttctctgcgc ccgacaccaa accatgtcta ggggaaaggg 360

agggtgtctg ggatacagtg gtgagcagaa cggcctggga tcctttatca cgaaatgcag 420

gttacagggg aagacagcag atccatacac aaagaaacgc agtctcaaac tggataagtt 480

gtgtaactgg gtaggttgtt gtgaaattaa ataatggaag ggaaagggat tagtaaattg 540

tgacatcata ttctctgtca gatgaaaacc ctgtcctaat ttctatccac cagatagcat 600

gttgtctcta atactcattt tttcagctaa cttctgtggc aggatggcac tgactccccc 660

cacccaacac acacacactt tttcccttcc tagcaggggg cctgtaataa aaatgaaaga 720

ggagagctaa gtgtgatgta acagggaatg gtgaagattg tgactcataa ctaagatgac 780

tgtaacttac tgctcaaacc agaacacttt aaagaatgaa aagacacaat aaataattac 840

accgggacgg taggcataaa agtgactctc ccaggtgaac tggcactatg gt 892

<210> 514
<211> 685
<212> DNA
<213> Homo sapiens

<400> 514
gcggggaggc ttagtgcccc cttgagaaac ggcttggcaa cgctgtgccc ggggccttgc 60

acgcgggctg agggggctag agacctcgcg acttttactc cccgaccccc gatctccgac 120

tcctgacttt ggaagccccc aaagctgctt ttcggttgcc tacaaagtgt cggtcattgt 180

gccacgtcct tggaaccgtg aactcatttt ctcctcaata acctttaaaa agtagctgta 240

aaacctccat tttacagacc agttggctga ggtttaaatg gaggacgctc ccagggcccc 300

agagggggcga attccagact cctttgccag gaccttgtac taagcccctt ccctacgctt 360

ttccctgcct aggcctcgtg ggctgtgcgg ctatcctgga gacagatgac agctctccct 420

tggatggctt tgctggttcc gcaccagcca gcgcccccat ttttcctgca gcaccctgat 480

ctgcactccc tgagggggctc ccactgtccg cggtgtgagg atgtccctgg gtaagtggtg 540

ctggtcatga gcactttggg ggacccttca aattcccttc tgggggaccc cagcggaggg 600

catggtcatg aattccgaga actgtatggc aaagccgggt gcagaggaga gcctggctaa 660

gttctttcat tctcctgagc ttgac 685

<210> 515
<211> 339
<212> DNA
<213> Homo sapiens

<400> 515
aacaaatctt acttgtatct gcttgagtgg atagaggtcc tttggtatcc ggcatcttcc 60

caaatggccc aggtgccagg atggcagatc tttaaatgat attctttatg ggcccagaaa 120

acatatgtat tcctctgatt agatttggag gccagcccct gtcccttctg agctttgctt 180

tctctgctag aagtggcttt cattttagat gcttaccaga ggctttcgca ttgtcagata 240

actgggaatc actatttgga tgtcatggtg tacatgtgca gttaggaacc ggtacaacca 300

tttgcccctg tgaaagcagc agccccagga taaagccaa 339

<210> 516
<211> 484
<212> DNA
<213> Homo sapiens

<400> 516
atgcccggca gggccaggac cagagtcccc gcacgagggt tcctctctcc ggtcgcacgt 60

gcccttcacc cttcctgagg ccgttttctg acgctcctga gctcccctcc agggtgaggg 120

caggcgcggt gggaaggcgt tggacagagc agacactcgg ctggggtgca tgcctgggtg 180

cagcctcgtc ttttgtcact aaccactcag ctcggtagcc ctccaggctc tctcttcgag 240

ctccgcggtc cctgcgcccc cgctgcgctc tgctagctgt gatgcagggg ttcgggggca 300

ggaaaaatct cgacccgcca cgcccgtgcg gcaaacctgt cctaggttga gggggcatga 360

ggcgcgcaca accagggcct cgaaggcagg caagagggcg cgggcgccac agaagcctgg 420

gcctgcgcct gggccgggca gatgtgcccg gcgctcgggc cccgcgctct gcgtcctgca 480

ggga 484

<210> 517
<211> 695
<212> DNA
<213> Homo sapiens

<400> 517
aggctggagt gcaatggcgt gatctcggct cactgcaacc tctgcctccc gggttcaagc 60

aattctcctg gctcagcctc ccgagtagct aggactacag gcgtgcgcca ccacgccccg 120

ctaattttgt atttttagca gagacggggt ttctccatgt tggtcaggct ggtctcgaaa 180

caccgacctc aggtaatccg cccacctcag cctcccaaag tgctgggatt acaggcgtga 240

gccaccgcgc ccgaccaaca cttgaatttt aatacttact ttgctactaa ctgtgtcgtt 300

gaaacagtat gttgctttcc ttctctggcc cttagcttaa ctatctgtac agcgggaaca 360

ttggcaaact cctacgtgga ggagcttgca ggtccacttc aagttatcag actctaacta 420

ggagaaaaca ttgagaattt aggctgaaaa taacgacaca gtttatcacc tcgttaattc 480

ccaaacatca ttaccgcgtc ctaagccacc ccacttgcct ctcccctagc cgccttcctt 540

taaaaaaaga attcccctct agagggcgcg caagctctgc ggccttcgtt cctctcttcc 600

gtttgccttc actcttcctt ctgcgcatgt gctcagttcg ctatccggct gcttgtacct 660

ggttcaggag gttcaggcag aggttgcacc cacta 695

<210> 518

<211> 837
<212> DNA
<213> Homo sapiens

<400> 518
ggcgacggcc ccggggaggc ggcggccagt ccagaggccg agcagtttcc ggagagctca 60

gagctggagg acgacgacgc cgagggcctg tcctcccgac tcagcggcac cctcagcttc 120

accagcgccg aggacgacga ggacgacgag gacgaggacg acgaggaggc tggccctgac 180

cagctgcccc tcggggatgg gacgtcagga gaagacgcag gcgagtgcag gaggacggag 240

gcgggaaccc tggggctcga ttaggcctcc atcccagccg cgttcccaga gcatccaggg 300

cgctaagttg atccagctga aactcccaca cttcttaaag cgcctgggcc acctcttggc 360

tgtgtgacct tgagagagtc ttaacccccc tctgagccaa gttttccctt gtgtcaagtg 420

ggagaactca ctctcatctc agaattgctg caggcattaa atgtacaggg cttgggatcc 480

gaatggaaat gatgtattaa cgatattttg agggtggagc tattaattta gcccctactg 540

tctgccaggc acggtgtttg gctcctcggt gcctctctgt ccctcagttt ccatttctgt 600

aaaaaggggg taagtcctgc ctcacactgg gaggatgagt gaggccacgt cagtggttga 660

tgtcgaccct ggccctaggc ccattgatat gactggtcat gtgtctgcag aacggagccc 720

cccacctgat gggcagtggg gcagtcagct cctggcgcgg cagctgcagg atttctggaa 780

gaagtcccgg aacaccttgg caccccagcg gctgctcttc gaagtgacca gcgctaa 837

<210> 519
<211> 283
<212> DNA
<213> Homo sapiens

<400> 519
aagcagaggt gcaatttgaa gctagattct cccactctta aactagtgcc ggacagacga 60

aggcctagca catgggctac ggagccctag tagattccag tccccacact gagccaggag 120

gggtcgctgc ccagggatac ctacagccat actcgagctg gacaaggcgc acgctctttg 180

tggaagcaaa cacgtccacc accgcgtaga ggggctgcgc agctggcagt ccccgggcgc 240

tcgggcccat gtcctcgccg ttgatgatga tgtgcatgtc ggc 283

<210> 520
<211> 380
<212> DNA
<213> Homo sapiens

<400> 520
gggacatcgt gcttgagagc tttgcagttt ggccttttct tttctctacc ggagatggga 60

ggcctctgtc cgtgtttctg ctgtgttcac caggaggcca gtctggggca attactgctc 120

cccatctccc aaaggagcca catcagcctc atagcagtgt ctctagcttc gagctgttgc 180

ctgtggcagg cagaacagtg tgtactcgca acaagcaaat ccccagatgt gagcccattc 240

ggggctgcca cgttttttcc tgatgaaatt cctgacttgg tgtgttgtgt ttttttaaaa 300

aacacgccca ctgcctccaa aaacctcagc cggggaagag aaacatttct cgttgaattc 360

ccagtttttg aaacggtgtc 380

<210> 521
<211> 413
<212> DNA
<213> Homo sapiens

<400> 521
agtgtttcat ggcaggttga tcagggtttg catcggatgc cccatctgcc gggctagttc 60

tggctgggag ggtgttagga ggagatgctg tgctcggacc tgcgaagtca cgggggagat 120

ggggacccac ttgggcctta attataacac atcccataat gatatggtgt tataatacgc 180

acacaaagtg tcctaatctg atattatatc tgggttttgt acaatagata caatcggctg 240

gctgcagttt gctaattcat tctcgaccta tctccacgct ccacctagat gactatcggc 300

ttccttaatt aagtggaagg caagctctgc aggcgatgct tgcagtccca gcaccgacga 360

gtccggcctg gccctcccat aagctgggat aatagctact cccacatggg gcg 413

<210> 522
<211> 534
<212> DNA
<213> Homo sapiens

<400> 522
tgggctgcgg tgggctcggc tgtccactag cgcagtactt ggcagcggcc ggcgtgggcc 60

gccttggcct tgtggactat gacgtggtag agatgagcaa cctggcccgc caagtgctgc 120

atggcgaggc actggctggc caggccaagg ccttttcggc cgccgcctcg ctgcgccgcc 180

tcaattcggc agtggaatgc gtgccgtaca ctcaggccct tacgccagcc actgccctag 240

acctggtccg ccgatatgat gtggtggctg actgctcgga caacgtgccc actcgctacc 300

tggttaatga cgcatgtgtg ctggcgggtc ggcccctcgt gtctgccagt gccttgcgct 360

tcgagggcca aatcacagtc taccattatg acggtggccc ttgctatcgc tgcatattcc 420

cccaaccacc cccagcggag acagtgacca actgcgcgga cggcggggtg ctcggtgtcg 480

ttaccggggt cctgggctgc ctgcaggcct tggaagtgct gaaaatcgct gcgg 534

<210> 523
<211> 444
<212> DNA
<213> Homo sapiens

<400> 523
gggacagaca gtgatgaggc caagtctcag ccccctggga aacacattcc ggcgggaaag 60

aggctgaaac agggaaactg tggcaaatga taccagcgag ggctaggtcc ctgacacaag 120

gaaacccaag gaagcatttg gcttaagcgg tgacaatatc cctctcctca gatgccaagg 180

tcaaggcgag ccgcggcaac accatctccc cagacacaca cgcaaaatcg gcccttctcg 240

gggaaagggc aggggctgaa gggggaggcg gggtgggcgg cggctggaaa aaagtgaaat 300

ttcctctcct cctaaaataa gtcattggag cgaggagagc gagcgctatc tcagtaggct 360

gtgggcgagg tgcgggggtg gcgggggcac gctccgggtc cgccaggagg ggagggatcg 420

gaaggggaag cggggaggga aagg 444

<210> 524
<211> 479
<212> DNA
<213> Homo sapiens

<400> 524
taaacttgcc agcacagaca agacagcttg ttcaagctgc acctcaggcc gggtcagaga 60

ataaaaccga gggctagaag gcccagaatg tcggacagcc cagcggcacc cgtcagggag 120

tcccaggcgc ccgaaagagg cgccgcacct ctggcgagtc taggacccat cttcctggac 180

ctgtgctctg gagtgcctgc gggcctgggt ctaatttctg cttctggcag gtgtcccccct 240

cccccgccgc taataccagg agcgctgctt ctgcggtaac ttattttacc cccagaagcc 300

tgttttggga ccgaagtgtc agggtcctgt gtgtcttttt atgcactgtt ctccttagtg 360

caaagccctg cagatatgct tggccgaaaa gtctcagtgg tttcaacttc cagattttgt 420

tcccgcgtcc ggtcggaaaa tcacctggga tttggctgct gtctaaggcc gggggaaag 479

<210> 525
<211> 436
<212> DNA
<213> Homo sapiens

<400> 525
agaccgcccg ggacccgctt ctatcgcggc accgtggtgt agctccctcc ggggtcgtat 60

gctgtatgtt tactagccga attctcagtc ctggacgctc cgtggggcca gggaatatga 120

tctgtgctcc gctgcagcct cgcgcccaga gtaaatatcg gtcggatgaa ctaatcttac 180

atataaaaca gaaatcattt cccttttttta atatggcacg tggctcttcc ctaagtcgcc 240

ctgggttaaa taccgactca attaacaaga tgctaaaaac cgtttacgtt tttttacgtt 300

ttttacgttt aattcaccag acggtgcgct ccacgaggga aggggcctgg cctccctagt 360

tcagggttgt gcttagtgcc tgcaaccggc cgggcccgcg ggggcagcca ggagagaaag 420

gcgcgcccag cgcccc 436

<210> 526
<211> 507

<212> DNA
<213> Homo sapiens

<400> 526
tgggctctgc ctcgtgcacc gcgtgctgca gcccacagcc ggccgccgcc gggctggcgc 60

gagagtggag ggtggcagtg cgcaggcgcg gagcggaaaa gagggggcgt gaagggggcg 120

gagccaggca gggttggggg agggggtgat gtactgtggg ggcagggggg aaggggagta 180

gggaggggga gggggagcac ggatgaggag gaccccagag ggggcactgg tggacctggg 240

ggggtggatg aggaggggag gagggtccca tacagggcac tgggagacct ggggtggggg 300

tggataagga gaggaggaga gcctgcaggg cactggaaga ctgggtagtg ggggagcgcc 360

taaggagggg aggagggtcc tgcaggggca cggaaatacc ttggggttga agtggatgag 420

gaagggagga gggccccgca gggggcgctg ggagacccgg gggggaagta aggagggcac 480

gagggcctgc agggggcctg ggagatc 507

<210> 527
<211> 449
<212> DNA
<213> Homo sapiens

<400> 527
cctcaaagag cgtgcgcacc tgcccgcgcg cgccggagct gacctctccc ggcggcgggc 60

ggttagggga aagtacctgg gggaaaggta gaggaggtaa ggggaccctt ggggatgccc 120

ctacgggcta ccagggttga acgcacaggc atggtcacgt cggggtattg ccaagctttt 180

ggcgcagctg gtcgggtggc caggctgcat gcggcttagc aggagacgtc ctgggctgtt 240

tgcgcaggac ctctggaggc cctcgagatc gtcgcaagtg gaaaggtaaa gcggaacaag 300

ggacaggctg gagacggggg tcgcgtctaa catcaggata acttacaatt cgtttccaaa 360

gagcgcggta cgcgcagagc tggggaaagg tgttggatcc ggcgccagtc cttggacgat 420

cagtcgtcga aaaggaggca ggggccggc 449

<210> 528
<211> 527
<212> DNA
<213> Homo sapiens

<400> 528
tgaaccccgg ggaggtttga cgatctgccc aaagtccctg ggaagtttcc ggccgttcta 60

gggctaaagc tcagctctct cgactgcatc tccttcccac ccccatgtgg ctacccccat 120

agaatggcgc atccacgtag atggcccaga tgttgggttt aagggagaga gagaaaggaa 180

gtttggggca tctgtttatg agagaaaatc gtcagccgtg cgtggaaatc ttatgcatag 240

caaataaatg atgggcaaat acccacttgc atattcagag gcacatttca gatgtgaaca 300

tcctgtctca ccaaaacaac cccagctaag aagttccgtc gggggtggag gactttttcc 360

aagcctttgt ttctgtggag tctctgtgca tggcctgctg tgatgaggta ggggttgacc 420

cagaagtcta aggaggccac caccccgtag gaaagaggag ccagcagaca gcagcgccgg 480

gggttattgg ggacccccaa atggggaaag gtaggggtgt agacaaa 527

<210> 529
<211> 793
<212> DNA
<213> Homo sapiens

<400> 529
aggaagtcaa gactgcagtg agccaagatg gcgtcaccgc attccagccc gggaaacagg 60

gagactctgt ttcaaataaa aaatgttgct agtattctaa acacttaggt tttaaagggc 120

ttgctagctt tgctatcggg gtattatctg aaggcacgaa aactcttagg ggagtgtcct 180

gcttaatgat agccagcatt gatccatatt tcatagtctt attaattgga gatgtttggg 240

atgatttcat caatttaatt agatcttcac ttgtctttac ccctaatgtg gctgacagat 300

agcttggatt aggactggat gtggggccgc caaatttgtt tgagtaaata atacataagt 360

tttattggcg ctcaggccca tccgtcgtgc cgtctttgac tcttatgcta tgacagcagc 420

agcgtcgagt agttgctgca gagaccgtat agccctgaaa gcctaaaata ttatagttac 480

caggtggcct tttgcagaaa aagtggttat tcccaaactc aggtcggggt ggagggtgag 540

ggccacatcc tggtaccatc ctggctacat cggaatttcc agggcccttg aagaccgagg 600

tcgaggcccc ttccttggag acctagcagg cagggagcct gggactgtgt atggaactga 660

gcctctgggg ccatcatgga gctcggcaag aggctcagga ggatctggaa agcacgtgga 720

cttcccagga tgctggcagc gtccggtgtt tctcaaattg cgttctgcgc atagaccacc 780

tggggtgctg ccc 793

<210> 530
<211> 539
<212> DNA
<213> Homo sapiens

<400> 530
gcttggaaag ggccctgcgc ccagcacaca gcccccagtg aaagagaacc ggttccagga 60

actccgttcc tgtaaacgga tttcttttga taagtgccct gtcaattaaa acccaaactg 120

attttctgtt ctttctcaac tggtgtctcc cttccagaaa gttcagcagt acacagtcat 180

cgttcaggcc acagatatgg aaggaaatct caactatggc ctctcaaaca cagccacagc 240

catcatcacg gtgacagatg tgaatgacaa cccgccagaa tttaccgcca gcacggtgag 300

tccctcgaag ctgcccagtg acgcatggcc cgtgcagggc aggaatgcac tggcgtgcgg 360

cacagcctcc tctctctctg cggctctgcc tgacgggcac ttggcagata gcgtgtaaga 420

aaggccctgg gcagcggagg ctgctgtgtg gagtggctcg attcctgatg tgcgcagtag 480

gagagaggcc ggggctcagt cagggagggg accggcggcg gaggtcggtt gccacctgg 539

<210> 531
<211> 433
<212> DNA
<213> Homo sapiens

<400> 531
ctccctcatt cacccctccc cagactttga tgaaaccatc tgtggctccc ggcctctgca 60

ggacccagcc cctcagctcc tcagcccctg caggtgtccc tcggtactgc ctcacgccat 120

cctgccccag gtgataacta tgccctgagc ccccaccatt tccactgggc acatcccaga 180

aatacgcatt cacagaccag gcacgcccat gactgcggcc agattttaaa aggaatcacc 240

agaaatgtaa tctccagtgg tcctagagcc ccctgggcac ccagcttctg gcaccctgtg 300

gacacccagg agtgggggtc ggcccaacca gctgcgcagc cgcccagccc tgccagcggg 360

gagcttcctc cctccctgcc ctccggatac accctcaccc caactgagcg agcccagggt 420

tgggggaggt gca 433

<210> 532
<211> 403
<212> DNA
<213> Homo sapiens

<400> 532
agacagtctc tgagctttgc cgcccctgga agcagaggcg ggaaacgacc ggataaaaca 60

gaacgatggg gagtttcatc ctgtcattta ttttgagtct gactacagat cattatttct 120

cattttggat ttcagatggt ttgaaatgca agtctagctg ctcaaccatg ggctctaga 180

aagaggtgcc tggctatcac tcaaggggtt gtgggagccc aggacggtcg gaggcgctag 240

gagctctggg attgcctgtg aggccgcgca gagacaggag tttaagatga atgaagcacc 300

ccaaccttgc gctctgggaa tgcgccgtgt ttaaggctcc cctggtcctc tcccgggtgc 360

cccggaccct tcccaagggc tgctctccct ctcaccgaga gcc 403

<210> 533
<211> 403
<212> DNA
<213> Homo sapiens

<400> 533
cctgcagccc ctcctcacgt ctctgctctt ccggctgcca ccaggctccc ggcacgcggc 60

ccttcttcct tctgagttgt ttttcagggg ctgcgctggg gtcccacgga cacctcgcca 120

gaatcagctg ggttcccatg ggaaggggaa gtgccgctcc tgcgtccctg caccccttgcc 180

ctctgtgtgg tgtggcgtgt ctatcccgac tggcgaggcc gcgagcccat agtttgctgc 240

tgctccttca ccgtccgtgc tcattcctca gcctggtaca gccccaaccc cctcccacct 300

ttgtgtggtt ccaggcccat gcatgacacg cgtgttccat ttccacgtgc taccggcacc 360

agagcccatt ctgtacatgc tcgtttttac aaccgctttt taa 403

<210> 534
<211> 383
<212> DNA
<213> Homo sapiens

<400> 534
cgcaaggagg ggcaccccta gcacatctgg gttactccat cgcgccagcc ggtgtctgca 60

gcctgaaaat cgcgtctctg gtcttgttcc ccagcgagcc gttcaggact ctctgtccgc 120

ctgcagcccc cgccttccct ggtacctccg cagagaccca agaggcgtcc agaggggagc 180

ccaggcgcac ggtccccagg gaggggcccc gccctcaggt gggtgccgag gtggccgccc 240

acagagcagc aggtgcgttg gaggaggggc gccttgtgcg gctgaatctc gtgtgcagga 300

ggagagggcg aggctctgca gaaacatcag acccggccat ccctgtagcc gcgcccctgg 360

cccagcctca ggctctgcag ctg 383

<210> 535
<211> 437
<212> DNA
<213> Homo sapiens

<400> 535
agccacatgt ggctggcagc tcctgtatgg ggcaacgtgg tctgagagcc ggggagagct 60

ggcagggagg cgctgcaggc acagcccccc aagaccccca aagctttgtg ggggacccgt 120

gcagctgcac ctccccctgg ggtccgcagc tggcactggg cttccttcct gccccatcag 180

ccacagggag ctccacactg cgtgagaagg ccctgtgacc cgaggtcgga caggcggaca 240

ggcccacagt gagctcgaga cccacagaca ctgaccgaga cccgacagga cagacatgca 300

ccaaaaacac gcggacacgt gtgcttgaga agaggagggg gagcccgaac aggcacgggg 360

gagcccgcac aggcatgagg gggcagccgg tgttgggggc tcctcctcca gcacgggggc 420

aggaggcagt gagatgg 437

<210> 536
<211> 425
<212> DNA
<213> Homo sapiens

<400> 536
caccctggag gaggagatgg aaccctcctg tttgctgggg ctcggccccc ggtgaggctg 60

gctgcaggtg accacagaga cctagcctgg tgtcgtgcgg ccgctgggtc cgtgaagggg 120

agtgaggcac tcctcaccct gtggcagctc ctagtcagga ggcctctccc ctccacctgc 180

acgtcatgtg gctgtggctc ccaggaccag cgcctggagc gggtgagtga acgtccctct 240

cctccgtggg cactgcctcc gctgtgagcc acccttcagg ctacgtgcat ttccgcctgt 300

tctcagggtt taaccgaaac cgaacattaa ccgtagccct aacgcggctg agggatccag 360

gccttgcctc tgggccgggt gagagacaga cactcgccaa ccagtccgca gtggctcccc 420

gtgtg 425

<210> 537
<211> 465
<212> DNA
<213> Homo sapiens

<400> 537
tgccaagaca gctgccgctc ctgccggcct agggccgcag ccggaatccc gggccacatc 60

ccctgccccg ttgtgccttc cacacctcgg gcagtcacta ggaaaagggt cgccaactga 120

aaggcctgca ggaaccagga tgatacctgc gtcagtcccg cggctgctgc gagtgcgcgc 180

tctcctgcca gggggacctc agaccctcct ttacagcaca ccgagggccc tgcagacacg 240

cgagcgggcc ttcagtttgc aaaccctgaa agcgggcgcg gtccaccagg acgatctggc 300

agggctctgg gtgaggaggc cgcgtcttta tttggggtcc tcgggcagcc acgttgcagc 360

tctgggggaa gactgcttaa ggaacccgct ctgaactgcg cgctggtgtc ctctccggcc 420

ctcgcttccc cgaccccgca caggctaacg ggagacgcgc aggcc 465

<210> 538
<211> 507
<212> DNA
<213> Homo sapiens

<400> 538
agtgtccttt tccgagggcc cgaggctccc cctccagcag ggagagctcc gggacggccc 60

ccaccaaggg ttgggtttct tccttcacaa ttccacagag gcatccctgt ccttcctacc 120

tgggaaacct cgaggtgcgg tgcccgtgta cttctggtac tttgcgtggt gccatcaggg 180

accccagagc cacagctgcg tgtgtgtgtg gatgtgtgtg tgtgtgtgcg cgcgcgcgcg 240

tgtacggcga aaggatgtgc ttgggggagc cgagtacaca acgtctgctt gggcagctgc 300

tgggcaggcg ttgggcctgg aggtatctca cacccacgta tcttccagtc ttcaaacacg 360

gcattgctct gcctcccgta gcgcgcttcg aacctgcctc gcggacacgt gaacagaggc 420

tgtccctggg aagataagtg cgctttcccg taaaatccgg gaaatttgcc ttgaggaaag 480

tttccgttct tgttacttgt cgggttt 507

<210> 539
<211> 323
<212> DNA
<213> Homo sapiens

<400> 539
gtgcgcggag gactcctggg ccgcacaccc atttccaggc tgcgggagcc ggacagggga 60

gggcagaggg gggacaaaag gactctttag gtccaaaatg accctgaagg agagtccaga 120

atgcccagtg gccgcgtctg caacggagtc ttctttctcc aattgccttc tgccccatca 180

ccatgggccc cacctgcgcc acctgcgccc accctgtgac cctggctcag cgaccttggc 240

ccttaatcgc ccaacgccga ttcctcaaaa ttccggctgc gctgaatcgg gctgcttttg 300

ccgccgcccc ggcagttggg ccc 323

<210> 540
<211> 582
<212> DNA
<213> Homo sapiens

<400> 540
gcaggcagac cctgcctggg acagtcaggg tgcaccggtt ctgtggctcc ggctcttaag 60

cactgctctg gaccactcct agcttgaggg ggttggggga ggtcaccaca ggctggcgga 120

ttgcttgggc agagtcagag gtaggattgg gcttgggaag aaaggtgcag ggtgcctgaa 180

gcaggggcag ccaggcagtg tggaaccagg gggctctgag gacagcgtcc actctcttgg 240

cagaagtgtg caggtcctgc tgtgtctgtc ccgtccctcc tcccatccac ctcctacttc 300

attgggcaaa ctgtttcctt ctttggctca ctcgggatcc cccagcaatg tcctttgttc 360

tcgaactccc agtgactcga ggggtgtcct ctgattgctc acctcaggtg gagcctcgaa 420

aggatggcgc tgctgttcct acctcgggtc tccccagtgc acggcatcct tacctgtggg 480

ctccacaggg ttccatgcag gcattttgct cccatacccc gatgtccaag cccggctgga 540

caaggagaag caatgtgatg accgaggggc tgagatggct gg 582

<210> 541
<211> 842
<212> DNA
<213> Homo sapiens

<400> 541
cccgcccttc tgccccttcc ctggcctgca gacctgtgac ctgccacacc ggcctgctgc 60

tgatggatta tcctgataat caactctctg ctattgttta gcaaaagcct ttccctttgt 120

cctattatca gaatccccct gcacttctca gtccctggtg acagatccac ctcccactgt 180

ggctggaatt tcccctgaac gttcttgtgc aggaaggctt agttctctat caaaaataag 240

tctctctgct ccttaatagt gggtcttttt aaaaatttcc ctgaagctgt ttctcgttgt 300

cctttgcctt cctgaccaag acaatgtcag catggggttc tgtccttggt cactgctcag 360

aggtggagct tgaccctggg atgtcgggaa gacgctctgg gaaaagctgt ggctgaagct 420

gctgagtcca aaggggtgt ctccttccca gtagtttcag acgaggatct ctcagccagg 480

actcttctgg agcctggaaa ccaagccaca ggacgaaggg ctgcgggaag aaggaggctg 540

agcctgggga acctggtggc tgccttcggg tgtcagaagg gctttccccc agcacgcgtt 600

tccccagagg ctggattggg gccactgagg cggtgtgaca gggaggtaga tttcagctca 660

gcaggaggaa gggtcccctg aaagccaggg ctcccagtga cacactgggc gcctggtgat 720

tactcagctt ctgggtgctg agggctctga aaggcaggca cacttgtccc actgtatcat 780

aacagaaaaa accggaacgg cccatgtgcc cattagctgg tggatggata aacacagcgt 840

ga 842

<210> 542
<211> 414
<212> DNA
<213> Homo sapiens

<400> 542
gttgggactg tctaggcgaa agctcagtgt gaattgtctg ctgtgttccc ggtgtccaag 60

gcccccaggg gctgggtgag tcccaagagg gatgtaggcc gagcagagtt gggcactgaa 120

cccccaggct ccccttgttg accacccagc cagaaattcc cagggcagaa ggtggggtct 180

gctcacccat ggggcaggcg gtctggggat gtctgacgcc tgggcctctg gccagcagga 240

tgtggacaga agtggccgct gggtggggct gagtgcagag ggcagtgccc atctgcagcg 300

tggctatgcg ccccacagag tgtgggcctg aagcggggaa aggacccccc tcaacacctc 360

aggccggcgg gtcccattcc agctgccacc cggactcagc ctgaggcctc ctca 414

<210> 543
<211> 601
<212> DNA
<213> Homo sapiens

<400> 543
gcttcagcct cccggagtag ctgggattac aggcacttgc caccacgccc ggctactttt 60

ttgtattttt agtagagagg gttttgccat gttggccagg ctggtctcca atgctgtctt 120

cttttaagat ttgattttcc gttgctggct ggtgtgggga tgtggagcgc cctcaccctg 180

tgaaggcatc ccttcccgtc actgtgtcgg tccctgtggt tttcgctgcc cgtgtcccct 240

gtgctgctgc tccttgctgc tggcctctgg ctgtgtcacc agctctggag tggccctaga 300

ctcacgagag agcatgcagg tcccaaaccg agagtggctg gaggcaggag tgctgcagga 360

gtgcctgggc cagccccctg tccaagtagg gagctctgcc cagctcccaa aagagcctcc 420

tcacaaagcc agtcactgag cccgagcagc gcggtcgccc ctgacgcggc accttccttc 480

caggctccta cctggctcag ggcgtccagc ggcagccaga caggctgggg gcccctccat 540

gttgcgcgct ccggctgact caccggggct gggggccctt ccacgcggcg cgctccagct 600

g 601

<210> 544
<211> 588
<212> DNA
<213> Homo sapiens

<400> 544
agctgccatg tgtgagggct tgctctgagc taagcatgat tctgagcacc ggcagggagt 60

acagcaagtg gatgctgagg cctggagggc aggccccaga gagctgagtg cccctccagg 120

tttcatggct ggcactgtgg accagtgtgg cccactgcct gcttgccacc ccacaacaaa 180

gcttcagagg tgcagaacca gtggcctgag ctccagggac aggaagcatc ttggcgtggg 240

gttctcatgg tggcacttgc atgccctagt gcacaggggc ggaaggggaa gctctgttgc 300

ctgggccctt tctctgtggc aacagatggg gaagtctgcc gtgaggatag agagtgcatg 360

gccatcccat cacagcacag gttgagtgct gagccacccc gagccctccc cagcccagcg 420

gctctcccca aggaggcttc aaaaccccgt gctccctttg tacccccaag cccatgcttg 480

gctctcagat ccacctcggg ccctttgcac cccagactcc tccccagctg cacattcccg 540

ggattccagg catctggtgc tgtgtgaggt cactaggaca gggcagtc 588

<210> 545
<211> 446
<212> DNA
<213> Homo sapiens

<400> 545
caagtcgagt ctgcgctata caaacaagag actcttaagg cgattagccc gggctgctgt 60

gagtttctgt ctgctcccct ccccgtgggc acctgggagc ccaggctcct aattaggctg 120

catgggccct tgcaaaacgc tctcactgtt cccaggtcac cgcctggcag ctgccccagc 180

ctctcccgac gttaccgtgc aggctgagca ggaggctggt tcactggccc ctgacagagc 240

ccaccgattc tctctccttc ctggtgtagg ctttacatac gaaccagtgt gagtgatctc 300

aggcatagcc atggttctaa gactcgccca ggagcaccta gggatttgcc acccacttga 360

gaaatgaatg tgatgtgcgt cctctgaggg cagctccggg aaacctggct cccccacctg 420

gacgccccgg tgtcccctgt gcatcg 446

<210> 546
<211> 399
<212> DNA
<213> Homo sapiens

<400> 546
gccgcccacg gcgtcgctaa cgaggatgcc gcccacgcga tcgctaagcc ggacgccgcc 60

cgcggcatcg ctaacgaggt tgccgcccag ggcatcgcta ccgaggacgt cgccgacggc 120

atcgctaacg aggacgccgc ccacggcgtc gctaacgagg tcgccgccca gggcgtcgct 180

aacgaggacg ccgtcgaggg cgtcgctaac gaggacgccg cccagggcgt cgctaaagag 240

gtcaccgccc acggcgtcgc taacgaggac gccgtccagg gtgtcgttaa cgaggacgcc 300

ctccacggcg tcgtcgctaa cgaggatgcc gcccaagcga tcgctaagcc ggacgccgcc 360

catggcatcg ctaacgaggt caccgcgcag ggcatcgct 399

<210> 547
<211> 318
<212> DNA
<213> Homo sapiens

<400> 547
gccgcccacg gcgtcgctaa cgaggatgcc gcccacgcga tcgctaagcc ggatgccgcc 60

cacggcatcg ctaacgaggt cgccacccag ggcatcgcta ccgaggatgt cgccgacggc 120

atcgctaatg aggacgccgc ccacggcgtc gctaaagagg tcaccgccca cggcgtcgct 180

aacgaggacg ccgtccaggg cgtcgttaac gaggacgccc tccacggcgt cgtcgctaac 240

gaggatgccg cccaagcgat cgctaagccg gacgccgccc atggcatcgc taacgaggtc 300

gccgcccacg gcatcgcg 318

<210> 548
<211> 396
<212> DNA
<213> Homo sapiens

<400> 548
ctccacggcg tcgtcgctaa cgaggatgcc gcccaagcga tcgctaagcc ggacgccgcc 60

catggcatcg ctaacgaggt cgccgcccac ggcatcgcga acgaggacgc cgcccagggc 120

atcgctaacg aggtcgccgc ccacggcatc tctaaggagg acgctgtcca gggcatcgcg 180

aacgaggacg ccgcccaggg catcgcgaac gaggacgccg cccagggcat cgcacacgag 240

gacgccgtcc agggcatcgc taacgaggac gccgtccacg gcgtcgctaa agaggtcgcc 300

gcccacggcg tcgctaacaa ggatgccgcc cacgcgatcg ctaagccgga cgccgcccgc 360

ggcatcgcta acgaggttgc cgcccagggc atcgct 396

<210> 549
<211> 426
<212> DNA
<213> Homo sapiens

<400> 549
gccgcccacg gcgtcgctaa cgaggatgcc gcccacgcga tcgctaagcc ggatgccgcc 60

cacggcatcg ctaacgaggt cgccacccag ggcatcgcta ccgaggatgt cgccgacggc 120

atcgctaacg aggacgccgc ccacggcgtc gctaacgagg tcgccgccca cggcgtcgct 180

atcgaggacg ccgtccaggg cgtcgctaac gaggacgccg tccacggcgt cgctaacgag 240

gacgccgccc agggcgtcgc taaagaggtc accgcccacg gcgtcgctaa cgaggacgcc 300

gtccagggcg tcgttaatga ggacgccctc cacagcgtct tcgctaacga ggatgccgcc 360

caagcgatcg ctaagccgga cgccgcccat ggcatcgcta acgaggtcgc cgcccacggc 420

atcggg 426

<210> 550
<211> 777
<212> DNA
<213> Homo sapiens

<400> 550
ctccacagcg tcttcgctaa cgaggatgcc gcccaagcga tcgctaagcc ggacgccgcc 60

catggcatcg ctaacgaggt cgccgcccac ggcatcggga acgaggacgc tgcccagggc 120

atcgcgaacg aggacgccgc ccagggcatc gctaacgagg tcgccgccca cggcatctct 180

aaggaggacg ccgtccaggg catcgcgaac gaggacgccg cccagggcat cgcgaacgag 240

gacgccgccc agggcatcgc acacgaggac gccgtccagg gcattgctaa cgaggacgcc 300

gtccacggcg tcgctaaaga ggtcgccgcc cacggcgtcg ctaacgagga ccctgtccag 360

ggcgtcgcta acgaggacgt cgtccagggc gtcgctaacg aggttgaagt ccagggcatc 420

gcacacgagg acgccgtcca gggcatcgct aacgaggacg ccgcccacgg catcgctaac 480

gaggtcgccg cccacggcgt cgcgaacgag gacgccgccc agggcgtcgc taacgaggac 540

gccgcccagg gcgtcgctaa cgaggacgcc gtccacggcg tcgctaacga ggacgccgcc 600

cagggcgtcg ctaaagaggt caccgcccac ggcgtcacta acgaggacgc cgtccagggc 660

gtcgttaacc aggaggccct ccatggcgtc gtcgctaacg aggatgccgc ccaagcgatc 720

gctaagccgg acagcgccca tggcatcgct aacgaggtcg ccgcccaggg catcgct 777

<210> 551
<211> 399
<212> DNA
<213> Homo sapiens

<400> 551
gccgcccacg gcgtcgctaa cgaggatgcc gcccacgcga tcgctaagcc ggacgccgcc 60

cgcggcatcg ctaacgaggt cgccacccag ggcatcgcta cccaggatgt cgccgacggc 120

atcgctaacg aggacgccgc ccacggcgtc gctaacgagg tcgccgccca cggcatcgct 180

aacgaggtcg ccgtccactg cgtcgctatc gaggacgccg cccagggcgt cgctaaagag 240

gtcaccgccc acggcgtcgc taacgaggac gccgtccagg gcgtcgttaa cgagtacgcc 300

ctccacagcg tcgtcgctaa cgaggatgcc gcccaagcaa tcgctaagcc ggacgccgcc 360

catggcatcg ctaacgaggt cgccgcccac agcatcgcg 399

<210> 552
<211> 720
<212> DNA
<213> Homo sapiens

<400> 552
ctccacagcg tcgtcgctaa cgaggatgcc gcccaagcaa tcgctaagcc ggacgccgcc 60

catggcatcg ctaacgaggt cgccgcccac agcatcgcga acgaggacgc tgcccagggc 120

atcgcgaacg aggacgccgc ccagggcatc gctaacgagg tcgccgccca cggcatctct 180

aaggaggacg ccgtccaggg catcgcgaac gaggacgccg cccagggcat cgcgaacgag 240

gacgccgccc agggcatcgc acacgaggac gccgtccagg gcattgctaa cgaggacgcc 300

gtccacggcg tcgctaaaga ggtcgccgcc cacggcgtcg ctaacgagga ccccgtccag 360

ggcgtcgcta acgaggacgc cgtccagggc gtcgctaacg aggttgaagt ccagggcgtc 420

gcacacgagg acgccgtcca gggcatcgct aacgaggacg ccgcccacgg catcactaac 480

gaggtcgccg cccacggcat cgcgaacgag gacgccgccc agggcatcgc gaacgaggac 540

gccgcccagg gcatcgcaca cgaggacgcc gtccagggca tcgctaacga ggacgccgtc 600

cacagcgtcg ctaaagaggt cgccgcccat ggcgtcgcta acgaggatgc cgcccacgcg 660

atcgctaagc cggacgccgc ccgcggcatc gctaacgagg ttgccgccca gggcatcgct 720

<210> 553
<211> 828
<212> DNA
<213> Homo sapiens

<400> 553
gccgcccatg gcgtcgctaa cgaggatgcc gcccacgcga tcgctaagcc ggacgccgcc 60

cgcggcatcg ctaacgaggt tgccgcccag ggcatcgcta ccgaggatgt cgccgacggc 120

atcgctaacg aggacgccgc ccacggcgtc gctaacgagg tcgccgccca cggcatctct 180

aaggaggacg ccgtccaggg catcgcgaac gaggacgccg cccagggcat cgctaaggag 240

gaccccatcc agggcgtcgc taacgaggtt gaagtccagg gcgtcgcaca cgaggacgcc 300

gtccagggca tcgctaacga ggacgccgcc cacggcatcg ccaacgagga cgctgcccag 360

ggcatcgcga acgaggacgc cgcccaggac atcgcaaacg aggacgccgc ccagggcatc 420

gctaaggagg accccgtcca gggcgtcgct aacgaggacc ccgtccaggg cgtcgctaac 480

gaggacgccg tccagggcgt cgctaacgag gttgaagtcc agggcgtcgc acacgaggac 540

gccgtccagg gcatcgctaa cgaggacgcc gcccacggca tcgctaacga ggtcgccgcc 600

cacggcatcg cgaacgagga cgccgcccag ggcatcgcga acgaggacgc cgcccagggc 660

attgcacacg aggacgccgt ccagggcatc gctaacgagg acgccgtcca cggcgtcgct 720

aaagaggtcg ccgcccacgg cgtcgctaac gaggatgccg cccacgcgat cgctaagccg 780

gacgccgccc gcggcatcgc taacgaggtc gccgcccagg gcatcgct 828

<210> 554
<211> 814
<212> DNA
<213> Homo sapiens

<400> 554
gccgcccacg gcgtcgctaa cgaggatgcc gcccaagcga tcgctaagcc ggacgccgcc 60

catggcatcg ctaatgaggt cgccgcccag ggcatcgcta acgaggtcgc cgtccacagc 120

atcgctatcg aggacaccac accgtccagg gcgtggctaa cgaagtcgct gcccagggca 180

ttgctaccga ggatgtcgcc gacggcatcg ctgaggacgc cgtccagggc atcactaacg 240

aggaggccgc ccaggccatc gctaagcagg acgccgccca cggcatcact aacgaggacg 300

ccgcccaggg cgtcactaac gaggtcgccg cccagggcgt cgctaacgag gacgccgccc 360

acggcgtcgc taacgaggtc gccgcccacg gcgtcgctaa cgaggacgcc gcccagggcg 420

tcgctaacga ggtcgccgcc cagggcgtcg ctaacgagga cgccgtccac ggcgtcgcta 480

acgaggacgc cgtccagggc gtcgctaacg aggtcgaagt ccagggcatc gcacacaagg 540

acgccatcca gggcatcgct aacgaggacg ccgcccaggg cgtcgctaac gaggacgccg 600

cccagggcgt cgctaacgag gtcgccgccc acggcgtcgc taacgaggat gccgcccacg 660

gcgtcgctaa cgaggtcgcc gcccagggcg tcgctaacga ggacgccgcc cagggcgtcg 720

ctaacgaggt cgccgtccac ggcgtcgcta acgaggacgc cgcccggcac atggctaagg 780

aggacgccgc ccaggacatc gctaacgagg acgc 814

<210> 555
<211> 372
<212> DNA
<213> Homo sapiens

<400> 555
ctggaccccc ctctgtggca ggtacaagcc cccaaccccc tcccggtccc ggggcactca 60

ctggggcccg tggacgccag ggctgcacag gcagcgcccc gactggaagt cgcagtgtcc 120

gctgccgcag tcggcgcaca cgaaggcgca gtcctcgccg taagtgccat tgctacactt 180

ggtctcgcac cttggaaaga ggggaggagg cgtcagcaga aatggaggca aacggaccac 240

agcgccctcg acattggggc ctttgttagg gaggcagggc gggggactgc gtgtctgggc 300

cgacgcaggc agggctgctc accggtcgcc gatccagccc gcgttgcagc gcgtacactt 360

gccggtgaca tg 372

<210> 556
<211> 504

<212> DNA
<213> Homo sapiens

<400> 556
catttcacat tcacacaagc atttcacaaa agccgtcatg tgtgtctacc ggatttgctc 60

attataatcg tctatttttt ttgaaatagg cttgctcata tttaattagc ttgaattgtg 120

ttacaataaa tattatagtg ctttaagaaa aatgtctgaa acctttgagg caggtacaga 180

gggaaggttt tcccatatag gctgcacgtg tgcatgttcg ggcccacgct accttcaggc 240

tgatgtctgt ccagtagatg tggttgctgg acacatcaaa gtccagggcc gaggcctcct 300

tgacgccgag agcaggatgg ccacatcgtt gttgttggtc tcgagggaga tcctgtggat 360

ggcggctcta ctggtgaaga ccaagaaggc cttgggcatg atgcaggtct tcatgtcact 420

cagcagttcc aggccctaga tggggcagcc aaaccgggtt gtgtggggcg tgaagaagca 480

caggtggctg cacccccatt cctg 504

<210> 557
<211> 422
<212> DNA
<213> Homo sapiens

<400> 557
tggggccgcc ccagcgggca gcagccaagg tggtgggctg cggggcgccc ggggcacagc 60

cgtgacctgc ccacacctgc aggtgctgag gagccacgtg atggtgcgag tgggtggtgg 120

ctgggacacg ctggagcatt acctggacaa gcacgacccg tgccgctgct cctccactgg 180

tcagtgccag ggtggggctg ggctggacgg ggcaggggac ttgcttctgt ggctctgtcc 240

ctcacatgct gcctgtcctc tctccccgca gctcatcgcc caccccagcc gagggtctgc 300

accttttctc cacagagggt gtcgcccacc accagtcccc gccctgctag cccagtccct 360

gggagtgagc gccggggctc ccggcctgag atgactcccg ttagcttacg aagcacaaag 420

ga 422

<210> 558
<211> 714
<212> DNA
<213> Homo sapiens

<400> 558
ggccagcggc tgcagtccgc ggctttcagc acccctccct tctctgtgcc gggcttttcg 60

gccgaacgtt ctcgctcctt ctgcgttgac tgttttatgt gtggttaacg cgcggctcac 120

caggggggat tttgaagcac ctgattcggt cgggcgcaaa ttgccttggt tagtgggccc 180

gaccgtgccg cactgtccag ggagcgtcaa gggtaggtgt ctcgctggga gtcacatttc 240

aagtggaaag gagaatcctg ttgcagttcc ttggtgtcgc atttcggttc actacacaca 300

agccactgcc ttcttgcctg gggctcccc gctcttctca cggtggcttt cctctctgca 360

gagacctctg cgcgaggtat acctaagcgc attgtgtgcg tcttcgggaa aaggcgaaaa 420

ctactcgcag tcccacgtgg atttcatttt ttcccctttta cctactctta gggtgcgtgg 480

taggcatcat tatccacctt agctgccctt cttttgggtt ttctgtaaaa ctggaaaaac 540

gagaagaaac tgttgcactt caaatttagt tgttttgttt tgttttttgg agacggagtt 600

tcgctcttgt tgcccagact ggagtgcaat ggcatgatct ccgctcaccg caacgtccgc 660

ctcccgggtt caagcaattc tcctgcctca gcctcccgag tagctgggat tgca 714

<210> 559
<211> 687
<212> DNA
<213> Homo sapiens

<400> 559
gaaatggtaa ggggaggctg ggaaaacatc cagctgtcag cggcctcccc ggggcacgct 60

gggatcagcc aagagaatgc aacagccagg ggtgggagcc ccctccgcgg ttgcacacgc 120

caggtccctc cagtcttgca ggttcaaatg aaggcttatg tgaaggggga atcgctctgg 180

acagctcagt gcccctcaga gtcacatcac caccattttc caccacgcgg aggtggttga 240

aaaaaaggag tcacagatgg gtaaggtttg aggggcgtcg ggctgacgag gcaaaaaaaa 300

aagtaggtgc tgtggtgctg atgggaggtt ctccctccct cacaggacgc caggaaggaa 360

atggggccgc tacagaggtg gtgaggtgcc agggcccacg gagggcccac ctgttgcgcg 420

ggggcgagca ccaggcgctg ggtgcacggg ggcccgtgcg tgcctgtgta cgtgcgtggg 480

tgtgcgcgca cgtgtgctca ggccgcgcgt gcaggggccg tgcgtgcgtt tgtgtgcggt 540

gcgcgcgcgt gtgtcccgcg tgtgcacggg cgtggcagag gcgggcgcgc gacctggccg 600

cggtagtgcg tgcccgtcgc tctgcgcgcc cgcccgccgg agcgcagcat cgcctccggc 660

caggagtgtg catgcgtggg gtgagtg 687

<210> 560
<211> 546
<212> DNA
<213> Homo sapiens

<400> 560
tcagcgagag gagccgaccc ttgggggaca gcggggtcgg gttccaagcc ggggctgctg 60

gggctggtca ggccgcccgc cctgcttgac ccctctcacc tgatggctcc cttctccaat 120

cttcaagaag gtgaaacgtc actgtggtcc gcagagggca ggccgcgtgt gcggagtcgc 180

tgtcatccct tagtggccac gcccgcctcc ctgtccactt cccgtcttcg tcgttgcggt 240

tcaggtgttc gctccactct gcggcaccaa aggcccgtca cgtgcttccc ctgcccagac 300

accccaaaca ccgaaactgt catccgaagg ccccgcacct gccgcctgtc ggatgctgaa 360

gaacgaatcc caggtggaga ggacagcacc ccaaaaggga tgggggatgg attggggcgt 420

ctgagccccg tggagtatgt cagcctttca gagcgacccg ctccacgctg cgatgtggtc 480

cttaagaagt tagggccggg cacggcgcct cacgctgtaa tcccagcact ttgggagcca 540

aggtgg 546

<210> 561
<211> 817
<212> DNA
<213> Homo sapiens

<400> 561
acaaaaaatt agccaggcgt ggtggcgggc gcctgtagtc ccagctaccc gggaggctga 60

ggaaggagaa tggcgtgagc tcaggaggcg gagcttgcag tgagccgaga tcacgccact 120

actctagcct gggcaacagt gagactcttg tctgaaaaaa aaaaaaacaa aaacaaaact 180

ctcatttcat catgagttat gcggggaaat gggatttgaa atatggacat tttgccttct 240

gccagttaga agcatgtctg ttccctaaat gtgggcgccc cctgcgtctg tacgtagatc 300

tccacctcgc tgctttattt tccagaggtt actgtagctc cttactttca gtcctcaata 360

gcgtcatgaa atttcttcca ttatttttc ttttctggat tccagttagc atttcccccc 420

aactcatgac ttctgctgaa taagagaaaa gagcaatatt taggttcatt tcctgtttat 480

tattaaagtg attttcacaa atgtttcatt acatgcatat gggggacatg atggataaaa 540

caaaactgaa tatttgtggc aactaaaagg aagatgtggt tatgttggta tttgccactt 600

gcaggcagag tcgttgccaa gcgtctggac aatacagtgc cctcacagga gccagaagtg 660

aagtctgcgg gccgctagca cagcgccctg gtgtcctgtt ccccgagcaa tgtggcctgc 720

gtctgctcgg catgttggag gttctgatct cagcggagta agacacccgg catcggtcag 780

ctccctcttc ctcctcctgc tttctgccac ccatctg 817

<210> 562
<211> 280
<212> DNA
<213> Homo sapiens

<400> 562
gccaggctgg agccaaggta cggcgcgcac acacccggcc ccgtcctgcc ggcagcaaac 60

gcccagtgct gtttcatctc agctttcgac ctgcccacgt ggggacgtgt atcacgcccc 120

tttcatagag gatgccactg agtccgcctg gaggaacttc ctggggtcgc cccatacctg 180

cctccgacta accctactgc cccacagagg ggcccccaca ggacaggccc cggcagagag 240

ccccaacagc cgcgaggctg cccttcctgg cctctcgtca 280

<210> 563
<211> 469

<212> DNA
<213> Homo sapiens

<400> 563
tgccacgatg actcatacta gggcaagttg agggcccgca ctgtccagcc ggagtcggtt 60

agagaccctc aggaggctca caggctccag ggcctggatg ttaggatgtg aggcctgggg 120

ttaccgagat cccacaccca ggagatgctc gcgtggagcc ttcgctcaca gcggcctgcg 180

cgaatggggc ttgctccgag aggttcagct ggcaccgaga gctggcaccg agagcgtttc 240

cagctgcaat ctgttcccag caattaataa ctgtaaataa caagtccgct cctccgcctg 300

gctccgcgct actctccgct tccctcccag cccctcagct gctccccttc ccgtgcccac 360

cctccctctc ctcccctctt tccttccatg tcgtcgagag gtccctgccg tcccttcccc 420

gggtccctct gagaacaccc agctcccttc ccgtaggcca tgggggaag 469

<210> 564
<211> 363
<212> DNA
<213> Homo sapiens

<400> 564
gagcagcgac cctgccatat gggccccgca cacggagccc ccgccagccc ggggcctcgg 60

cctcgggaag cacctgggag gcggcgggga aggttggccc tggctggtct gacacacgcc 120

tcggggctcg tccttcatgg gccaggcctg gtgctccacc taccactgct gggaagccat 180

ggaggcttct caagggtgac ctgcgaggtg gggtgcctg ccttccctct tgaggtaaga 240

ggggtctggt cccaccgctt gccgtaaaaa aggaggagca gctcaggtct gcacgtggcc 300

ctgagcagct cgccggaagg ttccagcctg gtctttttat tccgtttccc ttcccagggc 360

cgg 363

<210> 565
<211> 563
<212> DNA
<213> Homo sapiens

<400> 565
taccaggagc ccgggaagca ccgagctcag ggctccttgg ctcagcaccc ggctttgcca 60

gaccactcgg gcctctgcgc ctcccgacag cgccgtctgg ggcattcccc tgccagctga 120

gcccagagat tgacacgggc tgggccaaag gggagccatt ccagcctcgc tcttccctgc 180

cagaaccaga aagccacctc tctgccctcg tcctggaaaa atgggccaaa ttccccaagg 240

caaagattaa acgacaaaac aaatttcaaa gaacaagtgg gcacctggtt ctgagccaaa 300

tgatgccttc cagcatgaca atgcggccct gagcacctgc caagggacaa ccatatgttc 360

cctggcccga cgtgtccacg agccactgcc agcccaagag agctgtggac aataaagatg 420

agaatttttt tttttttttg agatggagtc tcacactgtc gcccagtcta aagtgcagtg 480

gtgcgatctc ggctcactgc aacctctgcc tcccgggttc aagcgattct cctgcctcag 540

cctcccaagt agctgggatt aca 563

<210> 566
<211> 426
<212> DNA
<213> Homo sapiens

<400> 566
gctcaccagc ggagccgacg tggcctgcac caccagcaca tactcccgcc ggacctcaaa 60

gtccagctcc accatggcac gcaggtcccc gttgagcagg tccagctgga agaaatgccg 120

catgtcccct tccacaatct gatacatgat ctgggcatta gggccttcat cagggtcgtt 180

agcacgaatc tttgccacca ccgaccccac tgggttgttc tcctcaacaa acagctccag 240

ttcgtccttc tcaaacatgg gggcattgtc attaatgtcc aagatggtca cctggatttc 300

taccgaggcg ctaaggggag tgggactgcc ccgatccaca gccagagccc aaaggttgta 360

cacggccaca ttctcccggt ccagccggcg ctgggtgcga atcacaccgg acgtgggctc 420

gatgta 426

<210> 567
<211> 666
<212> DNA
<213> Homo sapiens

<400> 567
gtccccaccc tggaaggtgt acagcagacg cccattggga cctgagtccc ggtccgtggc 60

agagacctgg aggatgctgg tcgagggtgg agcatcctca aagatggaac cctggtagaa 120

atcccacagg aactggggtg cattgtcatt ggcatcgagg atgaggatct ctagggtggt 180

ggtgtctgat ttctgcggga tgccgttgtc ctgggccatg atggtcagcg tgtaggcgac 240

ctggttctca tagtccagct ccatcatggt gtacatggtg ccactgtcgg ggtcaatgcg 300

gaactgcggc acggggtcct gaatcacgta ggtgatgcgg gcattctctc ctgtgtcctc 360

atcgttggca ctgagggtag caatggaggt gcccacaggc ctgtcctcac tgacactcac 420

tgtgtaatgg gagctctgaa agacaggcct gtgggtgttg gcatcagtga cgttgattag 480

gacatgcgca gtgtgcgacc gtgtgccgtc ggatgctgtc accgccagca cgtactgctg 540

ctcctgcttg tagtccagag gtagcgccag ggtgatgagg ccgccccctc tctggctgct 600

gagtgcaaag cggttccggg tgttgccgcc tgtgagctgg taggtaatca cactgttggc 660

gtcacg 666

<210> 568
<211> 473
<212> DNA

<213> Homo sapiens

<400> 568
atctagctgc agatcccggc tgtctacaca gccatctagc tgcggatccc ggctgtctat 60

actgtgatgc ggggacactt gtcatttcgg aaggttgcta atgaaaagtc agaaatttcc 120

cagagacccc acacaagcta gaaaaatggg ccgtttgcat gagagatcct ccgtcagcaa 180

taataactta gcaggagaga ttccatacga tgggaaatga ttgtttggat ggtaatttgc 240

ttagggaaat caaaaggagg gagagaccct catgctggag tcagtttcca atgaaaacca 300

ggaggattgc atctggttct ttaaggtagg catgaaagtt ctcgggagaa agaggaggga 360

ggaaggaggg ctggagagag ggaaggatgg aggacaagag agagtggcag gtggcctcat 420

ccccggggct cttgggctgg agcagagatc ctgaaagcca tcgggtgccg acg 473

<210> 569
<211> 428
<212> DNA
<213> Homo sapiens

<400> 569
ccgaccccag ccttgttagg ggactggcca cactcacaga catgccagcc ggcaagagct 60

ggtcatgggt gtgtggaccg tctcagggaa tcagtcagag gtggctgggg tagccaggag 120

ccaccacact gcctgccgac gtggctgggc ctgtttcaga tccaggaagt gtgggtctgc 180

atggggaacc ttctgctttt gtgatgagct gagcggcccc tggccaaccc tttcggcctt 240

gcatggagct cagatgtgtg gccgcaggag ctgagcccca gtcccaggct tgtcctgccc 300

tggctctgcc tcttgggagc ctgtcctggg cagggtgggg ctgtgatgaa gactgggcag 360

gtgaaagctc tggggcaccg gcctgcctgt ccccttcctg gagcggacag tgagaggggc 420

tttcttgc 428

<210> 570
<211> 505
<212> DNA
<213> Homo sapiens

<400> 570
tgcgaagcgg tcctgacctc ccggccgacg ggtgttatct caggacagcc ggtgtgcttg 60

gctacatcac ccctgtctgc tggtgggccc ctgtctgggg cccccaggcg ccctctcctg 120

aagggcctgc gcagagctgg gggcccgaac cattcctcac cccaactctt cattgtgaaa 180

attgtcatag atacaaaacc gtgaagagaa tcacactgca gacacccatc tgccccccac 240

aactgtttat gccgtgtgtg ggttccacag ctgcttaaaa aattgctaaa tgttcttcat 300

tcctggatat gttgatacac agacgtttac ccattcacag atgcactgat gcattcttag 360

acacccttat acttgggaga cccacatgtc ctggtttgca cgacacttgc tggtttcaaa 420

gctgaaaatc ccgccttgta ccaacccta agtcccgggc aaggcaggag gaccccgggc 480

aggagacaag tgccctcccc caggg 505

<210> 571
<211> 454
<212> DNA
<213> Homo sapiens

<400> 571
cccgcagccc tggcagcaag gcggagggcc tggctgacac acaccttccc ggatttgatt 60

atcctcacgg ctgatccaat cacttgggca gtcaggaacc acgatcatag ccaaaaacct 120

caattccacc aaaaatcgag tttcccagat ggcattttca ctctttagtc ctgaaccact 180

caccttcaca ggtgctaact gtgtttgcct ctggagtgat gggaccaacc agaaggcatc 240

tgggatagaa agggcactca gaggagcatg aggccctcga tgaaggggtc tgggatagaa 300

agggcactca gaggagcgtg aggccctcga tgaaggcgtc tgggatagaa agggcactca 360

gaggagcgtg aggccctcga tgaaggcgtc tggatctcca aggccgggag ataagcccgc 420

taaccggcac cttagtgctc caggacgctg ggct 454

<210> 572
<211> 608
<212> DNA
<213> Homo sapiens

<400> 572
aggaaaattg ctacaaatct ggattaatgg aagtcaaatg gtagtgagcc ggatggtaat 60

taagctgagg ttaaaagctt tgaagccagc ctttcgggag cggacttttt caaacacctc 120

agggacctaa acaaactcct gatatttatt caggctgaaa ttctgactcg catttagact 180

gctaaattaa taagcttgga gacctctccc cttccctgag tagacactgc ctttcattta 240

tgccatagag gtgcattaac gctccgtaat gccatctatt atttgggatt attaattcat 300

cagttactca attgaaaaat ctgaggagtc aatacagcaa gtcgttggga aagtgtggga 360

ccctagtgaa aaatcagcag attgttgagg aaataatgtt aaagaaacac tgagtaacaa 420

aagacattct ttattaaaat atcaaagagg ataaatatct ccttaagaag ctctggggtc 480

ctaagtaaac tcccaagggg tggggggtcct gacagcagga catggtggtc tcacagttta 540

acctttgcac gaagccaccg gtcttcggct ttctgggcac aggactcggt gcagcaccct 600

gggaggaa 608

<210> 573
<211> 508
<212> DNA
<213> Homo sapiens

<400> 573

aacactgaca cacaggatgg tcccgctgct tgcctgtggt cacagtatcc ggagcatggc 60

tgcagctggg aaccacctcc aggcgacaaa gcaacccagc acgggtgggt acggaggggc 120

cgttcgtctc gctcacagtc tgggggtggc ctcatggtgc tgctcatcac agcgggcatg 180

ttggctgggg ctgactgtga gaacccagct ctgccccaga ggcctggcct tggctggttc 240

tcacagggag gaagagagcc cagagcagta agcacagccc gtcagagagc gggcagagac 300

gcactgcgct tcctgagacc gcggctcacc gctcccctgc attctgctct tcaaagacac 360

atgcagaacc ttcagggtgt tgaaaggggc cgcaaagatc acagctctca gcgggggcgc 420

ggtcagtcgg ccacagcggt ggagatggcg ttgaaagccg gggatgatga ctgctgcgct 480

gggatgactg acggctgagc caggccct 508

<210> 574
<211> 474
<212> DNA
<213> Homo sapiens

<400> 574
tacagtttgc agaacacacc ctcctgccct gtggaagctt ccaggctccc gggcaggtcg 60

ctgaaggatc ctcttcctgg cccctggagt ccacaggtgc gacatcccgt cccgacatgg 120

gtgcctgccc cagccactca gtcgtcacct gtgagcacct gaaaaccagg ccacagccca 180

gactcacaag gagccacggg cctgggagat ttattaaaca ttcgcacgta attttccatt 240

aacatttcct attttggcat cttaaagagt gaaagttgat ttcggttcaa catcctgtgg 300

ggaaattagt tcattttgct aacgcatctt aataattaca gtgacatcaa ggttaattgc 360

ttaatctgga ttgccacagt gcaggccgcc gaattcccca cgcaatctgt gctataatta 420

gtaccggggc atccagattt agaaggctct ttcaccggaa cagcggaatc aggc 474

<210> 575
<211> 632
<212> DNA
<213> Homo sapiens

<400> 575
gtgtgtccgg ggacacgcac cgggtggaag tagatggcgt cgaactgtcc ggacagcgcc 60

tgcagctcct ccttgcctag gacctgggtc tccctgacca gggatacata ctctgggctg 120

catgcagatg ggcaggcagg agaggcaggg tcaccagcag gagcaggggg gcgggaggcg 180

gggacctggg attcatggtg ggagtggcca ccctgtcact tcctcaagcc tgtgagccca 240

cccgaggtgc atagggagag gctctgtatt cgaggctggc aggctcgggg taggcccagg 300

ttcttaggtt tctagctggg ctgcccgccc cgccccccat cgtggggcct gccccgtcc 360

tgacctgtgc accaggccca gctcctcttc cgaggcctcg cgggctgaca accgcagaca 420

cctctgttcc aggccgcgct gccgcaggcg atccagggct gcggtcaggc gctcaggacg 480

ctcgatctcg cactcggggc tggggcagat gaggagctca gttcagaggt ccctccccgc 540

accccacctc ggccaggtcc cacttactcg tcccagagca gccgggtggc cgtcatgtcc 600

tcatggtaca caagcgcggt ccccatggct gc 632

<210> 576
<211> 536
<212> DNA
<213> Homo sapiens

<400> 576
tcagcccccg ggcgcagacc ccgcccttcc tgggcacgat cccatgggcc ggtgccagag 60

ctgagcccac gccaggactg accgtcatgg gctgcaggga ttcagcctcc cgcggcggct 120

gccaggactg caggggctcc gcgatgacca ccatggcaaa cctctggatc aggctgaagc 180

cctgacccgt gacgttgatg ctgcggccac cactgcggag ggcagtgcct tcgtgggctc 240

ggctggcagg ggtggtccct gcagcctccg ccctcccgtc cccgcccacc ctggccctga 300

ccacacgggg cttccaggtg catgtgtggg tctcggtggg caaaggggca acgcggtggg 360

acgtgctgag cacgagactg tgccgcaagt gggcgggggg cacacggggc ctggtgagcg 420

tggagcctct ggtgtgccca tctgggtttc taggaggaag tggatctgga gtgcagtggg 480

gttttccgga ggccgcctcc cgctgcacag accagcccca ccacattcct ttcagc 536

<210> 577
<211> 333
<212> DNA
<213> Homo sapiens

<400> 577
ccgtgacagg aagagggggt ctcgggccgt gacaggaaga gggggtctcc ggccgtgaca 60

ggaagagggg gtctcgggcc gtgacaggaa gaggggatct cgggccgtga caggaagagg 120

gagtctcggg ccgtgacagg aagagggggt ctcgggccgt gacaggaaga gggggtctcg 180

ggccgtgaca ggaagagggg gtctcgggcc gtgacaggaa gagggggtct cgggccgtga 240

caggaagagg gggtctcggg ccgtgacagg aagagggggt ctccggccgt gacaggaaga 300

gggagtctcg ggccgtgaca ggaagagggg atc 333

<210> 578
<211> 359
<212> DNA
<213> Homo sapiens

<400> 578
ccgtgacagg aagagggggt ctcgggccgt gacaggaaga gggggtctcc ggccgtgaca 60

ggaagaggga gtctcgggcc gtgacaggaa gaggggatct cgggccgtga caggaagagg 120

gagtctcggg ccgtgacagg aagagcgggt ctcgggccgt gacaggaaga gcgggtctca 180

ggttgtgaca ggaagaggga gtctcgggcc gtgacaggaa gagggagtct cgggccgtga 240

caggaagagg gggtctcggg ccgtgacagg aagagggggt ctcgggccgt gacaggaaga 300

gggggtctcc ggccgtgaca ggaagagggg gtctcgggcc gtgacaggaa gaggggatc 359

<210> 579
<211> 333
<212> DNA
<213> Homo sapiens

<400> 579
ccgtgacagg aagagggggt ctcgggccgt gacaggaaga gggggtctcc ggccgtgaca 60

ggaagagggg gtctcgggcc gtgacaggaa gaggggatct cgggccgtga caggaagagg 120

gagtctcggg ccgtgacagg aagagggggt ctcgggccgt gacaggaaga gggggtctcg 180

ggccgtgaca ggaagagggg gtctcgggcc gtgacaggaa gagggggtct cgggccgtga 240

caggaagagg gggtctcggg ccgtgacagg aagagggggt ctccggccgt gacaggaaga 300

gggagtctcg ggccgtgaca ggaagagggg atc 333

<210> 580
<211> 615
<212> DNA
<213> Homo sapiens

<400> 580
ccgtgacagg aagagggggt ctcgggccgt gacaggaaga gggggtctcc ggccgtgaca 60

ggaagaggga gtctcgggcc gtgacaggaa gaggggatct cgggccgtga caggaagagg 120

gagtctcggg ccgtgacagg aagagcgggt ctcgggccgt gacaggaaga gcgggtctca 180

ggttgtgaca ggaagaggga gtctcgggcc gtgacaggaa gagggagtct cgggccgtga 240

caggaagagg gggtctcggg ccgtgacagg aagagggggt ctcgggccgt gacaggaaga 300

gggtgtctcg ggccgtgaca ggaagagcgg gtctcaggtt gtgacaggaa ggagtctcct 360

gtgtgtaggg actcatccat tctgtacaac tcatgtgcat cctcccatgt gcatcctcct 420

ctgtgcatcc ttccatgtgc atccttgaag ggtggcctg ccctccaca cctgtgggca 480

tttctcatca ggaggaacga gagacttgaa aaaagaaaga gacacagaga taaagtagag 540

agaaagaaaa atgggcccag gggaccggcg ctcagcatac agaggacccg cgccggcacc 600

tgcctctgag ttccc 615

<210> 581
<211> 618
<212> DNA
<213> Homo sapiens

<400> 581
acgccatcca tcattcaaat aatcgcaatt tagagcccgc gtttcatgcc gggctggttt 60

aatcaccagc agcagtttgt gaagggcctc attcgcctgg gattcgggga tattgcgtga 120

tttagcagac gtgacccctg acctctgcgg ggggcggaga gcctgccttg gaggcaaaag 180

cggaacagcg agggtgactc actcagggac aattattatg cttaacagcc tcactgtcat 240

cttagaagga aaaagagcca aaaaaaaaaa aaaaatccat aggatgctga gcattccttc 300

cagatccttc agcattcctt ccagatcctt cagcattcct tccagatcct tcagcattcc 360

ttccagatcc ttcagcattc cttccagatc cttcagcatt ccttccagat cccagtttag 420

aagaaggcag tggggtcggt ttaaatgcac ccagcgtgct cccgaagata atgtttttgt 480

ttctgtagac gcggtggcag ctttctggcg atttctaggc aatgtacagg aggaaagaaa 540

gaaggaaggg gagggcacgg agaagctccg gaaggctgga tgcttgggga aggaggtcag 600

gaggcaggac cggccggc 618

<210> 582
<211> 478
<212> DNA
<213> Homo sapiens

<400> 582
gtgcctcatt ttccaatttt taaaaaattg cctaatcatc aaagaggccc ggaagctagg 60

ctaccaaggc gcgacgctgt ttacacccca gacccaggtt ctcagctcgg ctttgcttgg 120

gaaaggctcg gaacctgtct gggcctgggg gtttagcaca gaaggctttg gttagctgca 180

ctgtgctgag cgcagctctg cagcgaggtt tagggagctg cggtgcgtta ggcacaaggc 240

acgtggcgcg cctcgctcca gcactttgca aacgcacagc agcgcagggc cagagcggct 300

ggcccccagg ggtgggggcg accagggaca ggaggtccca tatggtgtcg tcgcgttaaa 360

aacgtaggtc ctaaggggcg agccgcgtcc gacctccgcg cccctgcccg acgcggcgcc 420

tcgctcgccg gccccgctcg ctggattcat ttccatctgg agcgcggcgc gtcccaac 478

<210> 583
<211> 437
<212> DNA
<213> Homo sapiens

<400> 583
ttttttgaa aaagtgaaag caattttatt aagaaagtaa aggaatagcc ggctgtgtgg 60

cagcccgcga ggcggtccgc gggagcacac tctgtgcaga gactgggcgg ccagcctgcc 120

cttgctgtcg ctgacggtga ctgcgggagg ccaggttgtt tttcatcatt cagaacattg 180

cctgaagcag gtccaccgtg ccgttagtaa cgaggaacat cgagccaagg cacctgtgcc 240

gtcagacgtt gcctagcgtt agaagcgagc tggaatgcgt gaccaacatc accctggcaa 300

atgtcatccg acagctgggc agcctgagta aatatgcaga ggacattttt ggagagctct 360

ttactcaggc aaataccttt gcctctccgg taagctccct tgctgagagg gtcgatggac 420

tacaagttaa agtcact 437

<210> 584
<211> 516
<212> DNA
<213> Homo sapiens

<400> 584
ccatcagcca ctgtgcctga ccgtgcgcga gcgcgaagac ccagggaccc ggctctacga 60

gtgcatcccg ctgtactctg taagagccgc actctctagg tgagcttccc agagcggtcc 120

ttgatgcctt cctgccttga tcgtcccaca accgatcgac tgcccctttg caggttccta 180

atttattcag atctgtagaa cggagacggg cgtggccaaa aatgggacta gaggggtctc 240

cgagaagtcg gagaatgggt gtcagcttgg gcctgggtgg aaaccaggag gacggattgc 300

cgcctggggc gctcgccgcc gcgcccgccc tactgtcgaa aggtggggct gatttcgttg 360

gtgggcgggg gtgggcttta ttgtccctcc acacacagca tttgtattct tacaaaatag 420

aagctccctt cccctacggt atctcgcata atggtgtgtt catttccggt acccgcaatg 480

gtatgttaca tcctccacgc ctcatcccca gctgca 516

<210> 585
<211> 480
<212> DNA
<213> Homo sapiens

<400> 585
ctccgcctga gccccgccca tgacggtggg cgcctgcgcc tacgcgcgcc gggaagcaga 60

aggtgatctc tgccttcgag acacggagtg tgctgcctca gtctgtgatc cctcgcgttt 120

caatccaatt caaaccatgc tgcagccaat tcgttcgcgc gtgcgtgtgc catcattgtg 180

agtggaggag actgcccgat atgatcccag tcattgcggc gacccctgga gatctccaac 240

gcctgcgtcc cccaaccata ccctgccccg catccacaga tatcttccct tccttgatcg 300

ccagtacctg gcactgaccg tgctttgaat tcaaacaccc tgggaggcat agtccctgtg 360

atttaaacta acattgaaga agcgaccaga cgcggtggtt cacgcctgta atcccagcac 420

tttgggagac cggggcgggc ggatcacgtg aggtcaggag ttcgagacca acctggctaa 480

<210> 586
<211> 595
<212> DNA
<213> Homo sapiens

<400> 586
aagatcaccc gccgctcatt gggctacgcg tatgtcaact accagcaacc ggtggacgcc 60

aagcgggccc tggagaccct gaactttgat gtcataaagg gcaggccagt gcgcatcatg 120

tggtcccaga gggacccgtc gctccgcaag agcggggtgg gcaacgtctt catcaagaac 180

ctgggcaaga ccatcgacaa caaggcgctg tacaacatct tctcggcgtt cggcaacatc 240

ctctcctgca aagtggcctg tgacgaaaag gggcccaagg gctacgggtt cgtgcacttc 300

caaaagcagg aatctgcgga gcgggccatc gatgtgatga atggcatgtt cctgaactac 360

cgcaaaattt tcgtcgggag attcaagtcg cataaagaac gagaggccga aaggggagcc 420

tgggccaggc agtccactag tgctgacgtc aaggatttcg aggaagacac cgacgaggag 480

gccaccttgc gatgaagaca tcccaggagc tagccagcca gcagagccaa accttggctc 540

acacccggtt tacaaccccc cacccccagc cctcccccgc caacccacca gcagt 595

<210> 587
<211> 595
<212> DNA
<213> Homo sapiens

<400> 587
cactgctggt gggttggcgg gggagggctg ggggtggggg gttgtaaacc gggtgtgagc 60

caaggtttgg ctctgctggc tggctagctc ctgggatgtc ttcatcgcaa ggtggcctcc 120

tcgtcggtgt cttcctcgaa atccttgacg tcagcactag tggactgcct ggcccaggct 180

cccctttcgg cctctcgttc tttatgcgac ttgaatctcc cgacgaaaat tttgcggtag 240

ttcaggaaca tgccattcat cacatcgatg gcccgctccg cagattcctg cttttggaag 300

tgcacgaacc cgtagccctt gggccccttt tcgtcacagg ccactttgca ggagaggatg 360

ttgccgaacg ccgagaagat gttgtacagc gccttgttgt cgatggtctt gcccaggttc 420

ttgatgaaga cgttgcccac cccgctcttg cggagcgacg ggtccctctg ggaccacatg 480

atgcgcactg gcctgccctt tatgacatca aagttcaggg tctccagggc ccgcttggcg 540

tccaccggtt gctggtagtt gacatacgcg tagcccaatg agcggcgggt gatct 595

<210> 588
<211> 699
<212> DNA
<213> Homo sapiens

<400> 588
ccagctgccg cctctgcaca gagcctgcac ccggtgctcc gagagccccc ggatcacggc 60

gaactgggct cgcaccattg ccaggatcac tccgacgagg agacgccaac ctctgatgag 120

ttggaacagt tcgccaaaca attcaaacaa agaagaatca agttgggctt cacgcaggcc 180

gacgtggggt tggcgctggg cacactgtat ggtaacgtgt tctcgcagac caccatctgc 240

aggttcgaag gcttgcagct gagcttcaaa aatatgtgca agctgaagcc cctgctgaac 300

aagtggctgg aggaggcgga ttcgtccaca gggagcccga ccagcattga caagatcgct 360

gcacagggcc gcaagcgcaa gaagcggacc tccatcgagg tgagtgtcaa gggcgtactg 420

gagacgcatt tcctcaagtg tcccaagcct gccgcgcagg agatctcctc gctggcagac 480

agcctccagt tggagaagga agtggtgcgt gtctggttct gtaatcgaag acaaaaagag 540

aaaagaatga ctccgccagg ggatcagcag ccgcatgagg tttattcgca caccgtgaaa 600

acagacacat cttgccatga tctctgactg gaggaagcga ggaggcggcc ggccgcactg 660

ggagcagcgc ggatttctct ttctctctca ctctcttcc 699

<210> 589
<211> 319
<212> DNA
<213> Homo sapiens

<400> 589
ttttttaaat cttggtacag aggaagcggg aagttactta gcacggttcc gggtttctcg 60

cgccccgcct gtccccctc cctatcactg ctactggctc ttggtccctc cgttggactg 120

tcctgcggag agaaacccca gcccatcggt ctgcgctggg accgcccgcc gcgcatctgc 180

ccttcttcgc tgactccgcc ccgcatctgg ccagacccgc ctcgcgtcag agctgaccca 240

ctcactgcgc gtttgccagt cagtctctcc ggacctgcct cgagcctcag gctgctgaaa 300

tcaccgcgcc tcactcggt 319

<210> 590
<211> 342
<212> DNA
<213> Homo sapiens

<400> 590
gactgtgttc ccgcagcaga cgttaggccg ctgcccacgc cttgagtgcc ggacgaggtc 60

aacataggct ttccgtcaca gaatatgttt gggcaggaag atcggaacac ttggggctgg 120

ggcatctacc gctccccacg gcacacacga gtcgtcaggg aaatgccgcc tctgtgtgtg 180

tgttagcagc ctggcgtagt gtcttcccac tgttttgtct gtcttgagaa tagcattcaa 240

cgcgactgtg ttcccgcagc agacgttagg ccgctgccca cgccttgagt gccggacgag 300

gtcaacatag gctttccgtc acagaatatg tttgggcagg aa 342

<210> 591
<211> 579
<212> DNA
<213> Homo sapiens

<400> 591
cctatcgtcg tcagtcatgg ctagcatcat tgcacgtgtc ggtaacagcc ggcggctgaa 60

tgcacccttg ccgccttggg cccattccat gctgaggtcc ctggggagaa gtctcggtcc 120

tataatggcc agcatggcag acagaaacat gaagttgttc tcggggaggg tggtgccagc 180

ccaaggggaa gaaacctttg aaaactggct gacccaagtc aatggcgtcc tgccagattg 240

gaatatgtct gaggaggaaa agctcaagcg cttgatgaaa acccttaggg gccctgcccg 300

cgaggtcatg cgtgtgcttc aggcgaccaa ccctaaccta agtgtggcag atttcttgcg 360

agccatgaaa ttggtgtttg gggagtctga aagcagtgtg actgcccatg gtaaattttt 420

taacacccta caagctcaag gggagaaagc ctccctttat gtgatccgtt tagaggtgca 480

gctccagaac gctattcagg caggcattat agctgagaaa gatgcaaacc ggactcgctt 540

gcagcagctc cttttaggcg gtgagctgag tagggacct 579

<210> 592
<211> 800
<212> DNA
<213> Homo sapiens

<400> 592
cacttccaag ctgtgtctct tgacattgta cagcagagca tgctgcctcc gggctgcaca 60

gaacaccacg gagtagcagg caatcatgac aatcagtgga atgacgatga aggacaccac 120

gctgagaata gtgtagctgg ggctggcccc ccagatcatg gagcagagag cattgcgctc 180

atcaaaggca gcctggcccc agccgtagag tggaggagtg ctctgcagga tggccacaat 240

ccaggtgcca tagaggagca ggtaaccgcg gcgctgggtc atcttggacg ggtaggagag 300

agggtggatg atggacaagt agcgatccac tgacaccacg acaatggtgt tgacgctggc 360

gaaggcgaac aggtgggtga ggctaaccag ggccgtgcag aagtggctgt tgaggggcca 420

gaagagaggc acagaggtgg ccaccaccca gggggccacg agcgaaatct gcagcaggtc 480

ggtgacgagg aggttaaaga taaaacggtt ggtcacctgc agcagctgcg gcttgcgctg 540

caacactagc gccagcacta tgttgccgac gaaagaggcg gcgaggaaga taaccagcac 600

ggttgagcgg atgatgccgt gggccaggct gatgggcatt ttggagaggg gcatgcacgt 660

gtggctgctg ttactctcgc gcgtgctgtt ggtgcaggtg gacgtcatgg caacagccag 720

agggcgtgag acaggttgca ggctcagtgc cggcaccggt gggtggcaaa ggggtgcaca 780

gaccgcactc agtgcctatg 800

<210> 593
<211> 853
<212> DNA
<213> Homo sapiens

<400> 593
ggggcgggaa gccttttcct caccttggcc tcggccccaa gtggtgtccc ggagatgcag 60

cagcgtctcc cagtcctcac cgcggacgcc gccccaggtg tccccaggtg tccccaggtg 120

tctcgaagcc gcggtctctc ccatgcgggt cctggggaga ggaagctcag tcctcatagt 180

ggccgcaaag tgactgccgc tgtgtggatg agacgcggtg cgtgcacggc gccccgagtt 240

gccaggggcc atgcgccccc ttgcggcagc catggggagt ctctgaaaat cagcgtctag 300

atttttccag gctgtggatt ttggaaatta cgactgaaat ggagcccacc atcccatgcc 360

ttgatcggaa ctcatggagc aaggaagcaa agaagatggc cagttcatgc ctgtaatccc 420

agcattttgg gaggctgagg cgggcggatt acttcaggtc cagagtttga gaccagcctg 480

gctaacatgg tgaaaccgca tctatactaa aaatacaaaa attagctggg catggcggca 540

cgtgcctgta atcccagcta ctcaggaggc tgaggcagga ggattgctcg aacccaggag 600

gcgcaggttg cagtgagcca agatcatgtc actgcactcc aacctgggtg acagagccaa 660

actccatctc aaaaaaaaga tggccagtag gcaaaactta attgatttc tttcttttct 720

ctttaagaga cagggtctcg ctctgtcacc catgctgaag tgcagtggtg taatcacagc 780

tcccgagttc agcctccagc tcccgggttc aagaaattct ccagcctcag cctcccaaca 840

cactgggatt aca 853

<210> 594
<211> 333
<212> DNA
<213> Homo sapiens

<400> 594
ccgtgacagg aagaggggt ctcgggccgt gacaggaaga gggggtctcc ggccgtgaca 60

ggaagagggg gtctcgggcc gtgacaggaa gaggggatct cgggccgtga caggaagagg 120

gagtctcggg ccgtgacagg aagaggggt ctcgggccgt gacaggaaga gggggtctcg 180

ggccgtgaca ggaagagggg gtctcgggcc gtgacaggaa gagggggtct cgggccgtga 240

caggaagagg gggtctcggg ccgtgacagg aagaggggt ctccggccgt gacaggaaga 300

gggagtctcg ggccgtgaca ggaagagggg atc 333

<210> 595
<211> 359
<212> DNA
<213> Homo sapiens

<400> 595
ccgtgacagg aagaggggt ctcgggccgt gacaggaaga gggggtctcc ggccgtgaca 60

ggaagaggga gtctcgggcc gtgacaggaa gaggggatct cgggccgtga caggaagagg 120

gagtctcggg ccgtgacagg aagagcgggt ctcgggccgt gacaggaaga gcgggtctca 180

ggttgtgaca ggaagaggga gtctcgggcc gtgacaggaa gagggagtct cgggccgtga 240

caggaagagg gggtctcggg ccgtgacagg aagaggggt ctcgggccgt gacaggaaga 300

gggggtctcc ggccgtgaca ggaagagggg gtctcgggcc gtgacaggaa gaggggatc 359

<210> 596
<211> 333

<212> DNA
<213> Homo sapiens

<400> 596
ccgtgacagg aagaggggt ctcgggccgt gacaggaaga gggggtctcc ggccgtgaca 60

ggaagagggg gtctcgggcc gtgacaggaa gaggggatct cgggccgtga caggaagagg 120

gagtctcggg ccgtgacagg aagaggggt ctcgggccgt gacaggaaga gggggtctcg 180

ggccgtgaca ggaagagggg gtctcgggcc gtgacaggaa gagggggtct cgggccgtga 240

caggaagagg gggtctcggg ccgtgacagg aagaggggt ctccggccgt gacaggaaga 300

gggagtctcg ggccgtgaca ggaagagggg atc 333

<210> 597
<211> 615
<212> DNA
<213> Homo sapiens

<400> 597
ccgtgacagg aagaggggt ctcgggccgt gacaggaaga gggggtctcc ggccgtgaca 60

ggaagaggga gtctcgggcc gtgacaggaa gaggggatct cgggccgtga caggaagagg 120

gagtctcggg ccgtgacagg aagagcgggt ctcgggccgt gacaggaaga gcgggtctca 180

ggttgtgaca ggaagaggga gtctcgggcc gtgacaggaa gagggagtct cgggccgtga 240

caggaagagg gggtctcggg ccgtgacagg aagaggggt ctcgggccgt gacaggaaga 300

gggtgtctcg ggccgtgaca ggaagagcgg gtctcaggtt gtgacaggaa ggagtctcct 360

gtgtgtaggg actcatccat tctgtacaac tcatgtgcat cctcccatgt gcatcctcct 420

ctgtgcatcc ttccatgtgc atccttgaag ggtggcctg cccctccaca cctgtgggca 480

tttctcatca ggaggaacga gagacttgaa aaaagaaaga gacacagaga taaagtagag 540

agaaagaaaa atgggcccag gggaccggcg ctcagcatac agaggacccg cgccggcacc 600

tgcctctgag ttccc 615

<210> 598
<211> 618
<212> DNA
<213> Homo sapiens

<400> 598
acgccatcca tcattcaaat aatcgcaatt tagagcccgc gtttcatgcc gggctggttt 60

aatcaccagc agcagtttgt gaagggcctc attcgcctgg gattcgggga tattgcgtga 120

tttagcagac gtgacccctg acctctgcgg ggggcggaga gcctgccttg gaggcaaaag 180

cggaacagcg agggtgactc actcagggac aattattatg cttaacagcc tcactgtcat 240

cttagaagga aaaagagcca aaaaaaaaaa aaaaatccat aggatgctga gcattccttc 300

cagatccttc agcattcctt ccagatcctt cagcattcct tccagatcct tcagcattcc 360

ttccagatcc ttcagcattc cttccagatc cttcagcatt ccttccagat cccagtttag 420

aagaaggcag tggggtcggt ttaaatgcac ccagcgtgct cccgaagata atgtttttgt 480

ttctgtagac gcggtggcag ctttctggcg atttctaggc aatgtacagg aggaaagaaa 540

gaaggaaggg gagggcacgg agaagctccg gaaggctgga tgcttgggga aggaggtcag 600

gaggcaggac cggccggc 618

<210> 599
<211> 195
<212> DNA
<213> Homo sapiens

<400> 599
cgaaataaga agcaaatctt gctgggaccg agttcaggtc gaggaactcc gggaccctct 60

gcagcctcag ccctgccgcg ctccccgcag cttcgggcct gttgcgggga gttggggagg 120

aattcaggga aggggggaac caggccggga taagaggctc cggcttgctg tgggctgcat 180

gacggggtgc aaagg 195

<210> 600
<211> 448
<212> DNA
<213> Homo sapiens

<400> 600
tcgtccactt gaggacgtcg aagcaggtga tgatgcccag ggcgtgcacc gggtaggtga 60

gatcggtgcg cgccagcggg gacagggcgg tcaggagcag cagccaggtc cctgcacacg 120

cggccaccgc gtaacgacgg cggcgccagc gcttggagct gagcgggtac aggacccccca 180

ggaagcgctc cacgctgata caggtcatgg tgaggatgct ggaatacatg tttgcgtaaa 240

aggccacggt caccacgttg caaagcagca ccccgaatac ccagtggtgg cggttgcaat 300

ggtagtagat ttggaaaggc aacacgctgg ccagcatcag gtccgtgacg ctcaggttga 360

tcatgaagat gaccgacggg gatctgggcc ccatgcgccg gcacagcacc cacagagaga 420

agaggttgcc cgggatgctg accgccgc 448

**Description**

FIELD OF THE INVENTION

This invention pertains in general to the field of biology and bioinformatics. More particularly the invention relates to the field of categorization of cancer tumours and even more particularly to identifying methylated sites, which may aid in categorization of cancer tumours.

BACKGROUND OF THE INVENTION

Worldwide, breast cancer is the fifth most common cause of cancer death, after lung cancer, stomach cancer, liver cancer, and colon cancer. Among women, breast cancer is the most common cancer and the most common cause of cancer death.

Breast cancer is diagnosed by the pathological examination of surgically removed breast tissue. Following diagnosis, it is important to analyze the tumour type in order to aid clinicians when choosing the right therapy. Within the art, such analysis is performed according to two categories.

The first category involves the use of immuno-histopathological variables, such as tumour size, ER/PR status, lymph node negativity, etc. to define a clinical prognostic index such as the Nottingham Prognostic Index (NPI). The problem with such an index is that it has been shown to be very conservative, thus typically causing patients to receive aggressive therapy even when they are a low risk of disease recurrence.

The second category involves the measurement of the expression levels of a large number of genes, typically around 500, and calculating probability of a subtype based on the relative expression levels of the genes. This method is very costly in terms of tissue handling requirements. It is also hard to perform in a clinical setting, due to the demand of laboratory equipment.

DNA methylation, a type of chemical modification of DNA that can be inherited and subsequently removed without changing the original DNA sequence, is the most well studied epigenetic mechanism of gene regulation. There are areas in DNA where a cytosine nucleotide occurs next to a guanine nucleotide in the linear sequence of bases called CpG islands.

CpG islands are generally heavily methylated in normal cells. However, during tumorigenesis, hypomethylation occurs at these islands, which may result in the expression of certain repeats. These hypomethylation events also correlate to the severity of some cancers. Under certain circumstances, which may occur in pathologies such as cancer, imprinting, development, tissue specificity, or X chromosome inactivation, gene associated islands may be heavily methylated. Specifically, in cancer, methylation of islands proximal to tumour suppressors is a frequent event, often occurring when the second allele is lost by deletion (Loss of Heterozygosity, LOH). Some tumour suppressors commonly seen with methylated islands are p16, Rassf1a, and BRCA1.

There are reported epigenetic markers for colorectal and prostate cancer. For example, Epigenomics AG (Berlin, Germany) has the Septin 9 as a marker for colorectal cancer screening in blood plasma. A method for using methylation sites to predict differential therapy responses in cancer and recommending an appropriate therapy has been disclosed in US20050021240A1. However, the results predicted by this method are limited, since they cannot be directly applied in clinical practice. Therefore, it would advantageous to have a method for the analysis of breast cancer disorders, which is time efficient, reliable and cost-effective.

SUMMARY OF THE INVENTION

Accordingly, the present invention preferably seeks to mitigate, alleviate or eliminate one or more of the above-identified deficiencies in the art and disadvantages singly or in any combination and solves at least the above mentioned problems by providing a method for the analysis of breast cancer disorders according to the appended patent claims.

According to an aspect a method for analysis of breast cancer disorders is disclosed. The method comprises determining the genomic methylation status of more than one CpG dinucleotides in a sequence selected from the group of sequences consisting of SEQ ID NO. 1 to SEQ ID NO. 600. The method provides for improved abilities to characterize cancer tumours using methylation patterns.

The regions of interest of the sequences SEQ ID NO. 1 to 600 are designated in table 1 (as "start" and "end" on respective "chromosome").

This aspect presents improvements over the state of the art in that it enables a highly specific classification of breast cell proliferative disorders.

In an aspect a computer program product is disclosed. The computer program product is stored on a computer-readable medium comprising software code adapted to perform the steps of the method according to an aspect when executed on a data-processing apparatus.

In an aspect a device is disclosed. The device comprises means adapted to carry out methods according to som embodiments. An advantage with this is to support a clinician.

Herein, the sequences claimed also encompass the sequences, which are reverse complement to the sequences designated.

## BRIEF DESCRIPTION OF THE DRAWINGS

These and other aspects, features and advantages of which the invention is capable of will be apparent and elucidated from the following description of embodiments of the present invention, reference being made to the accompanying drawings, in which

Fig. 1 is a schematic illustration of a method according to some embodiments;
Fig. 2 is a schematic illustration of a dataset 20 of five measurements 1 to 5;
Fig. 3 is a schematic illustration of a first subset 30 of five measurements 1 to 5;
Fig. 4 is a schematic illustration of a second subset 40 of five measurements 1 to 5; and
Fig. 5 is an illustration of clusters 51, 52, 53, where Fig. 5A is a first cluster 51, Fig. 5B is a second cluster 52 and Fig. 5C is a third cluster 53.
Fig. 6 is a schematic illustration of a computer program product according to an embodiment.Fig. 7 is a schematic illustration of a device according to an embodiment.

## DETAILED DESCRIPTION OF EMBODIMENTS

Several embodiments of the present invention will be described in more detail below with reference to the accompanying drawings in order for those skilled in the art to be able to carry out the invention. The invention may, however, be embodied in many different forms and should not be construed as limited to the embodiments set forth herein. Rather, these embodiments are provided so that this disclosure will be thorough and complete, and will fully convey the scope of the invention to those skilled in the art. The embodiments do not limit the invention, but the invention is only limited by the appended patent claims. Furthermore, the terminology used in the detailed description of the particular embodiments illustrated in the accompanying drawings is not intended to be limiting of the invention.

An idea according to some embodiments is a method using a small selection of DNA sequences to analyze breast cancer disorders. The analysis is done by determining genomic methylation status of more than one CpG dinucleotides, in either sequence disclosed herein, or its reverse complement.

It was surprisingly found that some DNA sequences, SEQ ID NO: 1 to SEQ ID NO: 600 act as epigenetic markers that may be used to analyze breast cancer by subtyping tumours. In prior art, it is possible to subtype breast cancer based on gene expression. Five different subtypes have been reported; luminal A, luminal B, basal, ERBB2 overexpressing, and normal-like. The inventors have identified the same subtypes using DNA methylation.

The DNA SEQ ID NO: 1 to SEQ ID NO: 600 were identified by analysing 150 000 individual genomic loci for methylation, across a set of 83 breast tumours. The availability of clinical information regarding tumour specimens allowed for an investigation of DNA methylation in the context of breast cancer subtypes, histology and tumour aggressiveness. The five major breast cancer molecular subtypes (luminal A and B, basal, ERBB2 overexpressing, and normal-like) were identified. First, an investigation was performed regarding however unsupervised clustering of the tumour set using methylation recapitulates the major Luminal and basal classes that were identified by expression analysis or not. A filtering criterion was used to identify the features to be used in clustering. This criterion was the top 500 loci that varied most across the 83 tumour samples. Then, the top 100 loci that distinguished tumours from normal tissues from were added. These 600 features, displayed in table 1, were used to cluster the 83 tumours for which the expression subtype data was available. Hierarchical clustering with Pearson correlation and complete linkage of the samples based on these six hundred loci gave a dendrogram that is surprisingly similar to the one produced by expression analysis.

Table 1. 600 features for categorization of cancer

| SEQ ID NO: | Frag ID | Chromosome | Start | End |
|---|---|---|---|---|
| 1 | MspFrag4633 | 1 | 32374307 | 32374791 |
| 2 | MspFrag757 | 1 | 1702806 | 1703222 |
| 3 | MspFrag1173 | 1 | 2518915 | 2519285 |
| 4 | MspFrag1211 | 1 | 2622522 | 2623091 |
| 5 | MspFrag1212 | 1 | 2629273 | 2629613 |

(continued)

| SEQ ID NO: | Frag ID | Chromosome | Start | End |
|---|---|---|---|---|
| 6 | MspFrag1241 | 1 | 2871558 | 2871896 |
| 7 | MspFrag1242 | 1 | 2873712 | 2874055 |
| 8 | MspFrag1249 | 1 | 2944491 | 2945100 |
| 9 | MspFrag1311 | 1 | 3036436 | 3036818 |
| 10 | MspFrag1321 | 1 | 3103884 | 3104234 |
| 11 | MspFrag1324 | 1 | 3113132 | 3113448 |
| 12 | MspFrag1326 | 1 | 3118212 | 3118636 |
| 13 | MspFrag1339 | 1 | 3163795 | 3164122 |
| 14 | MspFrag1340 | 1 | 3165605 | 3166112 |
| 15 | MspFrag1359 | 1 | 3218362 | 3218653 |
| 16 | MspFrag1377 | 1 | 3296147 | 3296524 |
| 17 | MspFrag1391 | 1 | 3338689 | 3339191 |
| 18 | MspFrag1534 | 1 | 3642624 | 3643184 |
| 19 | MspFrag1601 | 1 | 4360224 | 4360668 |
| 20 | MspFrag1649 | 1 | 5478055 | 5478432 |
| 21 | MspFrag1650 | 1 | 5490384 | 5490940 |
| 22 | MspFrag1775 | 1 | 6285179 | 6285570 |
| 23 | MspFrag1823 | 1 | 6445812 | 6446063 |
| 24 | MspFrag1961 | 1 | 6949999 | 6950306 |
| 25 | MspFrag2123 | 1 | 9031495 | 9031958 |
| 26 | MspFrag2643 | 1 | 14669841 | 14670071 |
| 27 | MspFrag2886 | 1 | 16695727 | 16696176 |
| 28 | MspFrag3066 | 1 | 18043936 | 18044316 |
| 29 | MspFrag3084 | 1 | 18205071 | 18205589 |
| 30 | MspFrag3535 | 1 | 22625307 | 22625790 |
| 31 | MspFrag4109 | 1 | 27008738 | 27009387 |
| 32 | MspFrag4389 | 1 | 29281582 | 29281828 |
| 33 | MspFrag4819 | 1 | 33768108 | 33768404 |
| 34 | MspFrag4820 | 1 | 33769727 | 33770434 |
| 35 | MspFrag4823 | 1 | 33955400 | 33955873 |
| 36 | MspFrag5071 | 1 | 36908888 | 36909106 |
| 37 | MspFrag5104 | 1 | 37589882 | 37590168 |
| 38 | MspFrag5190 | 1 | 37995046 | 37995631 |
| 39 | MspFrag5455 | 1 | 40267780 | 40268103 |
| 40 | MspFrag5525 | 1 | 40916307 | 40917083 |
| 41 | MspFrag5644 | 1 | 41941498 | 41941965 |
| 42 | MspFrag5980 | 1 | 44977457 | 44977763 |
| 43 | MspFrag6197 | 1 | 47408542 | 47408713 |

(continued)

| SEQ ID NO: | Frag ID | Chromosome | Start | End |
|---|---|---|---|---|
| 44 | MspFrag6914 | 1 | 62496120 | 62496646 |
| 45 | MspFrag7116 | 1 | 65646887 | 65647674 |
| 46 | MspFrag7153 | 1 | 67312523 | 67312727 |
| 47 | MspFrag7228 | 1 | 71223914 | 71224499 |
| 48 | MspFrag7359 | 1 | 79184005 | 79184422 |
| 49 | MspFrag8101 | 1 | 101535648 | 101535994 |
| 50 | MspFrag8168 | 1 | 108527701 | 108527992 |
| 51 | MspFrag8169 | 1 | 108675712 | 108676003 |
| 52 | MspFrag8273 | 1 | 109749595 | 109750084 |
| 53 | MspFrag8710 | 1 | 115926101 | 115926763 |
| 54 | MspFrag8778 | 1 | 116868496 | 116868706 |
| 55 | MspFrag8956 | 1 | 120551325 | 120551421 |
| 56 | MspFrag9029 | 1 | 142697968 | 142698037 |
| 57 | MspFrag9245 | 1 | 145643787 | 145644444 |
| 58 | MspFrag9273 | 1 | 146010092 | 146010549 |
| 59 | MspFrag9278 | 1 | 146064945 | 146066503 |
| 60 | MspFrag9601 | 1 | 148893238 | 148893494 |
| 61 | MspFrag9703 | 1 | 150968906 | 150969531 |
| 62 | MspFrag9928 | 1 | 152077757 | 152078037 |
| 63 | MspFrag9937 | 1 | 152103832 | 152104033 |
| 64 | MspFrag10189 | 1 | 153690285 | 153690897 |
| 65 | MspFrag10393 | 1 | 158225523 | 158225819 |
| 66 | MspFrag10421 | 1 | 158232050 | 158232295 |
| 67 | MspFrag10427 | 1 | 158232923 | 158233174 |
| 68 | MspFrag10490 | 1 | 158246841 | 158247086 |
| 69 | MspFrag10496 | 1 | 158247714 | 158247965 |
| 70 | MspFrag10537 | 1 | 158307786 | 158308067 |
| 71 | MspFrag10623 | 1 | 162330700 | 162331269 |
| 72 | MspFrag10916 | 1 | 172907883 | 172908042 |
| 73 | MspFrag11354 | 1 | 194611559 | 194611928 |
| 74 | MspFrag11474 | 1 | 197984459 | 197984775 |
| 75 | MspFrag11782 | 1 | 202229373 | 202229833 |
| 76 | MspFrag12301 | 1 | 217252591 | 217253153 |
| 77 | MspFrag13394 | 1 | 227605182 | 227605359 |
| 78 | MspFrag13583 | 1 | 232131677 | 232132379 |
| 79 | MspFrag14197 | 2 | 1248326 | 1248943 |
| 80 | MspFrag14202 | 2 | 1293040 | 1293404 |
| 81 | MspFrag14203 | 2 | 1296483 | 1297255 |

(continued)

| SEQ ID NO: | Frag ID | Chromosome | Start | End |
|---|---|---|---|---|
| 82 | MspFrag14231 | 2 | 1703105 | 1703374 |
| 83 | MspFrag14254 | 2 | 1833149 | 1833914 |
| 84 | MspFragl4278 | 2 | 2676636 | 2677246 |
| 85 | MspFrag14289 | 2 | 2812784 | 2813304 |
| 86 | MspFrag14290 | 2 | 2825618 | 2826147 |
| 87 | MspFrag14334 | 2 | 3326870 | 3327299 |
| 88 | MspFrag14451 | 2 | 5957756 | 5957971 |
| 89 | MspFrag14457 | 2 | 6749495 | 6749988 |
| 90 | MspFrag14487 | 2 | 7440522 | 7441007 |
| 91 | MspFrag14609 | 2 | 9553132 | 9553410 |
| 92 | MspFrag14656 | 2 | 10133476 | 10133666 |
| 93 | MspFrag14921 | 2 | 15857512 | 15857896 |
| 94 | MspFrag15066 | 2 | 20312835 | 20313215 |
| 95 | MspFrag15478 | 2 | 26785546 | 26785870 |
| 96 | MspFrag15644 | 2 | 27515565 | 27515896 |
| 97 | MspFrag15771 | 2 | 29699956 | 29700602 |
| 98 | MspFrag17091 | 2 | 65021553 | 65022078 |
| 99 | MspFrag17159 | 2 | 66264144 | 66264933 |
| 100 | MspFrag17697 | 2 | 73589558 | 73590193 |
| 101 | MspFrag17841 | 2 | 74642481 | 74642761 |
| 102 | MspFrag18355 | 2 | 91199543 | 91199793 |
| 103 | MspFrag18856 | 2 | 100492801 | 100493089 |
| 104 | MspFrag19245 | 2 | 108982952 | 108983175 |
| 105 | MspFrag19926 | 2 | 121038231 | 121038980 |
| 106 | MspFrag19965 | 2 | 121259357 | 121259763 |
| 107 | MspFrag20024 | 2 | 122816085 | 122816353 |
| 108 | MspFrag20134 | 2 | 128138182 | 128138536 |
| 109 | MspFrag20225 | 2 | 128792924 | 128793466 |
| 110 | MspFrag20706 | 2 | 139372061 | 139372477 |
| 111 | MspFrag20895 | 2 | 155380949 | 155381434 |
| 112 | MspFrag21537 | 2 | 175420626 | 175420995 |
| 113 | MspFrag21600 | 2 | 176773874 | 176774399 |
| 114 | MspFrag22036 | 2 | 191710645 | 191710851 |
| 115 | MspFrag22213 | 2 | 200159441 | 200159639 |
| 116 | MspFrag22546 | 2 | 209899069 | 209899548 |
| 117 | MspFrag22928 | 2 | 220021958 | 220022344 |
| 118 | MspFrag23536 | 2 | 233077827 | 233078119 |
| 119 | MspFrag23738 | 2 | 236183911 | 236184343 |

(continued)

| SEQ ID NO: | Frag ID | Chromosome | Start | End |
|---|---|---|---|---|
| 120 | MspFrag24273 | 2 | 241696154 | 241696568 |
| 121 | MspFrag25023 | 3 | 13136633 | 13137251 |
| 122 | MspFrag25164 | 3 | 14826516 | 14826916 |
| 123 | MspFrag25187 | 3 | 15081919 | 15082508 |
| 124 | MspFrag25517 | 3 | 28529966 | 28530450 |
| 125 | MspFrag25715 | 3 | 35760405 | 35760961 |
| 126 | MspFrag26073 | 3 | 42996257 | 42996879 |
| 127 | MspFrag26133 | 3 | 44016018 | 44016419 |
| 128 | MspFrag26295 | 3 | 46828327 | 46828820 |
| 129 | MspFrag26333 | 3 | 46909242 | 46909602 |
| 130 | MspFrag26774 | 3 | 50133302 | 50133713 |
| 131 | MspFrag27115 | 3 | 52543768 | 52544136 |
| 132 | MspFrag27268 | 3 | 55492383 | 55492977 |
| 133 | MspFrag27379 | 3 | 58042487 | 58042945 |
| 134 | MspFrag27495 | 3 | 62333914 | 62333971 |
| 135 | MspFrag27677 | 3 | 69184229 | 69184352 |
| 136 | MspFrag27685 | 3 | 69517625 | 69517852 |
| 137 | MspFrag28326 | 3 | 114643147 | 114643394 |
| 138 | MspFrag28887 | 3 | 128424361 | 128424622 |
| 139 | MspFrag29324 | 3 | 135097550 | 135098100 |
| 140 | MspFrag30803 | 3 | 185784594 | 185784860 |
| 141 | MspFrag31913 | 4 | 1192879 | 1193371 |
| 142 | MspFrag32174 | 4 | 1719620 | 1719949 |
| 143 | MspFrag32611 | 4 | 3571688 | 3573129 |
| 144 | MspFrag32624 | 4 | 3776452 | 3776818 |
| 145 | MspFrag32667 | 4 | 3914642 | 3915363 |
| 146 | MspFrag32966 | 4 | 7107197 | 7107478 |
| 147 | MspFrag33006 | 4 | 7629573 | 7630026 |
| 148 | MspFrag33110 | 4 | 9006410 | 9006713 |
| 149 | MspFrag33134 | 4 | 9459349 | 9459626 |
| 150 | MspFrag33136 | 4 | 9459777 | 9459956 |
| 151 | MspFrag33338 | 4 | 15333834 | 15334201 |
| 152 | MspFrag33381 | 4 | 16273567 | 16273855 |
| 153 | MspFrag35700 | 4 | 111901776 | 111901955 |
| 154 | MspFrag36595 | 4 | 152604344 | 152604681 |
| 155 | MspFrag36661 | 4 | 154574444 | 154574685 |
| 156 | MspFrag36683 | 4 | 154962375 | 154962925 |
| 157 | MspFrag37395 | 4 | 187400622 | 187401021 |

(continued)

| SEQ ID NO: | Frag ID | Chromosome | Start | End |
|---|---|---|---|---|
| 158 | MspFrag38281 | 5 | 1011369 | 1011836 |
| 159 | MspFrag38417 | 5 | 1302864 | 1303240 |
| 160 | MspFrag38457 | 5 | 1348431 | 1348617 |
| 161 | MspFrag38485 | 5 | 1440104 | 1440605 |
| 162 | MspFrag38491 | 5 | 1496943 | 1497332 |
| 163 | MspFrag38714 | 5 | 2166920 | 2167677 |
| 164 | MspFrag38815 | 5 | 2919629 | 2920003 |
| 165 | MspFrag38821 | 5 | 3156410 | 3156769 |
| 166 | MspFrag38910 | 5 | 3907742 | 3907967 |
| 167 | MspFrag39470 | 5 | 31716178 | 31716614 |
| 168 | MspFrag39539 | 5 | 33927617 | 33927999 |
| 169 | MspFrag39543 | 5 | 33972064 | 33972687 |
| 170 | MspFrag39760 | 5 | 40871578 | 40871991 |
| 171 | MspFrag40505 | 5 | 71888649 | 71889360 |
| 172 | MspFrag40858 | 5 | 77304521 | 77304932 |
| 173 | MspFrag42441 | 5 | 134394818 | 134395156 |
| 174 | MspFrag42953 | 5 | 140187999 | 140188260 |
| 175 | MspFrag42983 | 5 | 140216007 | 140216482 |
| 176 | MspFrag44192 | 5 | 174111126 | 174111339 |
| 177 | MspFrag44328 | 5 | 175956200 | 175956454 |
| 178 | MspFrag44767 | 5 | 178348383 | 178348602 |
| 179 | MspFrag45007 | 5 | 179673647 | 179673858 |
| 180 | MspFrag45338 | 6 | 1311232 | 1311666 |
| 181 | MspFrag45409 | 6 | 1530339 | 1531041 |
| 182 | MspFrag45501 | 6 | 1625429 | 1625752 |
| 183 | MspFrag45650 | 6 | 3401937 | 3401968 |
| 184 | MspFrag46110 | 6 | 11152853 | 11153148 |
| 185 | MspFrag46277 | 6 | 16237147 | 16237395 |
| 186 | MspFrag46721 | 6 | 27449907 | 27450504 |
| 187 | MspFrag47196 | 6 | 31804402 | 31804867 |
| 188 | MspFrag47435 | 6 | 33353475 | 33353858 |
| 189 | MspFrag47510 | 6 | 33708897 | 33709149 |
| 190 | MspFrag48491 | 6 | 44373563 | 44374341 |
| 191 | MspFrag49687 | 6 | 101001787 | 101002201 |
| 192 | MspFrag50444 | 6 | 123359218 | 123359439 |
| 193 | MspFrag50717 | 6 | 134539380 | 134539767 |
| 194 | MspFrag50853 | 6 | 137860054 | 137860272 |
| 195 | MspFrag52027 | 6 | 168452341 | 168452651 |

(continued)

| SEQ ID NO: | Frag ID | Chromosome | Start | End |
|---|---|---|---|---|
| 196 | MspFrag52146 | 6 | 169670215 | 169670603 |
| 197 | MspFrag52434 | 7 | 580841 | 581190 |
| 198 | MspFrag52666 | 7 | 989299 | 989808 |
| 199 | MspFrag52792 | 7 | 1206082 | 1206625 |
| 200 | MspFrag52897 | 7 | 1460124 | 1460484 |
| 201 | MspFrag53338 | 7 | 4884663 | 4885032 |
| 202 | MspFrag54143 | 7 | 21829594 | 21830366 |
| 203 | MspFrag54400 | 7 | 26916475 | 26916913 |
| 204 | MspFrag54424 | 7 | 26935561 | 26936019 |
| 205 | MspFrag54796 | 7 | 30494831 | 30495180 |
| 206 | MspFrag54824 | 7 | 31149657 | 31149980 |
| 207 | MspFrag54975 | 7 | 35070796 | 35071213 |
| 208 | MspFrag55218 | 7 | 43062129 | 43062415 |
| 209 | MspFrag55275 | 7 | 43877824 | 43878339 |
| 210 | MspFrag55475 | 7 | 47902671 | 47903123 |
| 211 | MspFrag55611 | 7 | 54506521 | 54507157 |
| 212 | MspFrag55649 | 7 | 54862496 | 54862960 |
| 213 | MspFrag55941 | 7 | 63786704 | 63787372 |
| 214 | MspFrag56289 | 7 | 72093180 | 72093418 |
| 215 | MspFrag56402 | 7 | 72563341 | 72563657 |
| 216 | MspFrag56504 | 7 | 73646860 | 73647098 |
| 217 | MspFrag56540 | 7 | 74018306 | 74018544 |
| 218 | MspFrag56922 | 7 | 87208109 | 87208310 |
| 219 | MspFrag57002 | 7 | 90540824 | 90541294 |
| 220 | MspFrag57206 | 7 | 97246402 | 97246843 |
| 221 | MspFrag57442 | 7 | 99419846 | 99420214 |
| 222 | MspFrag57677 | 7 | 100240230 | 100240525 |
| 223 | MspFrag58680 | 7 | 128125215 | 128125598 |
| 224 | MspFrag59067 | 7 | 136989204 | 136989443 |
| 225 | MspFrag60291 | 7 | 155610859 | 155611142 |
| 226 | MspFrag60445 | 7 | 156703792 | 156704149 |
| 227 | MspFrag60779 | 7 | 158289060 | 158289297 |
| 228 | MspFrag60966 | 8 | 1008907 | 1009401 |
| 229 | MspFrag61003 | 8 | 1239397 | 1239831 |
| 230 | MspFrag61051 | 8 | 1470634 | 1471413 |
| 231 | MspFrag61099 | 8 | 1759273 | 1759325 |
| 232 | MspFrag61152 | 8 | 1982797 | 1983256 |
| 233 | MspFrag61161 | 8 | 2062616 | 2063197 |

(continued)

| SEQ ID NO: | Frag ID | Chromosome | Start | End |
|---|---|---|---|---|
| 234 | MspFrag61169 | 8 | 2197099 | 2197693 |
| 235 | MspFrag61173 | 8 | 2324899 | 2325526 |
| 236 | MspFrag61350 | 8 | 7917174 | 7917432 |
| 237 | MspFrag62044 | 8 | 22045386 | 22045723 |
| 238 | MspFrag62294 | 8 | 24826373 | 24826927 |
| 239 | MspFrag62605 | 8 | 29266511 | 29267015 |
| 240 | MspFrag63030 | 8 | 41702523 | 41702937 |
| 241 | MspFrag63043 | 8 | 41774590 | 41774866 |
| 242 | MspFrag63267 | 8 | 49697557 | 49697886 |
| 243 | MspFrag63271 | 8 | 49810071 | 49810539 |
| 244 | MspFrag63597 | 8 | 59220858 | 59221324 |
| 245 | MspFrag64684 | 8 | 97242768 | 97243023 |
| 246 | MspFrag64725 | 8 | 98359395 | 98359772 |
| 247 | MspFrag65670 | 8 | 135559922 | 135560190 |
| 248 | MspFrag65671 | 8 | 135560191 | 135560433 |
| 249 | MspFrag66071 | 8 | 144225273 | 144225476 |
| 250 | MspFrag66146 | 8 | 144444026 | 144444368 |
| 251 | MspFrag67369 | 9 | 988973 | 989201 |
| 252 | MspFrag67459 | 9 | 2613599 | 2614303 |
| 253 | MspFrag68271 | 9 | 34362590 | 34362891 |
| 254 | MspFrag68663 | 9 | 37743792 | 37744031 |
| 255 | MspFrag68970 | 9 | 64167952 | 64168281 |
| 256 | MspFrag69380 | 9 | 76862972 | 76863247 |
| 257 | MspFrag69976 | 9 | 93159730 | 93160221 |
| 258 | MspFrag70538 | 9 | 98551494 | 98551667 |
| 259 | MspFrag71074 | 9 | 112913792 | 112914149 |
| 260 | MspFrag71089 | 9 | 112919236 | 112919593 |
| 261 | MspFrag71090 | 9 | 112920067 | 112920611 |
| 262 | MspFrag71104 | 9 | 112924678 | 112925035 |
| 263 | MspFrag71105 | 9 | 112925509 | 112926053 |
| 264 | MspFrag71120 | 9 | 112930124 | 112930481 |
| 265 | MspFrag71121 | 9 | 112930955 | 112931497 |
| 266 | MspFrag71216 | 9 | 114346043 | 114346380 |
| 267 | MspFrag71581 | 9 | 124112526 | 124112954 |
| 268 | MspFrag71700 | 9 | 125589095 | 125589132 |
| 269 | MspFrag72003 | 9 | 127768596 | 127769001 |
| 270 | MspFrag72461 | 9 | 130337856 | 130338298 |
| 271 | MspFrag72674 | 9 | 131728566 | 131728859 |

(continued)

| SEQ ID NO: | Frag ID | Chromosome | Start | End |
|---|---|---|---|---|
| 272 | MspFrag72675 | 9 | 131728907 | 131729282 |
| 273 | MspFrag72740 | 9 | 132391939 | 132392575 |
| 274 | MspFrag72750 | 9 | 132485893 | 132486113 |
| 275 | MspFrag73062 | 9 | 134431953 | 134432427 |
| 276 | MspFrag73586 | 9 | 136866193 | 136866519 |
| 277 | MspFrag73907 | 9 | 137307963 | 137309295 |
| 278 | MspFrag74424 | 10 | 521032 | 521557 |
| 279 | MspFrag74598 | 10 | 1740057 | 1740811 |
| 280 | MspFrag75026 | 10 | 11420347 | 11420872 |
| 281 | MspFrag76120 | 10 | 35968545 | 35968856 |
| 282 | MspFrag76422 | 10 | 43464543 | 43465148 |
| 283 | MspFrag76467 | 10 | 44201213 | 44201571 |
| 284 | MspFrag76619 | 10 | 47227978 | 47228669 |
| 285 | MspFrag76797 | 10 | 50489052 | 50489405 |
| 286 | MspFrag76801 | 10 | 50489790 | 50491027 |
| 287 | MspFrag77115 | 10 | 64248087 | 64248491 |
| 288 | MspFrag77199 | 10 | 69760469 | 69761198 |
| 289 | MspFrag77777 | 10 | 76836478 | 76837103 |
| 290 | MspFrag78440 | 10 | 94811337 | 94811966 |
| 291 | MspFrag79123 | 10 | 102798099 | 102798651 |
| 292 | MspFrag79169 | 10 | 102883661 | 102883938 |
| 293 | MspFrag79207 | 10 | 102972749 | 102973047 |
| 294 | MspFrag79636 | 10 | 107141635 | 107141970 |
| 295 | MspFrag80112 | 10 | 119291788 | 119292000 |
| 296 | MspFrag80168 | 10 | 120344860 | 120345112 |
| 297 | MspFrag80169 | 10 | 120345113 | 120345331 |
| 298 | MspFrag80343 | 10 | 123771228 | 123771724 |
| 299 | MspFrag80645 | 10 | 126830955 | 126831650 |
| 300 | MspFrag80726 | 10 | 128183447 | 128184143 |
| 301 | MspFrag80728 | 10 | 128234723 | 128235166 |
| 302 | MspFrag80854 | 10 | 131646461 | 131646892 |
| 303 | MspFrag80954 | 10 | 131878295 | 131878616 |
| 304 | MspFrag80975 | 10 | 132947917 | 132948395 |
| 305 | MspFrag80989 | 10 | 133000558 | 133000818 |
| 306 | MspFrag82654 | 11 | 2002464 | 2002798 |
| 307 | MspFrag82859 | 11 | 2864180 | 2864505 |
| 308 | MspFrag82920 | 11 | 3199023 | 3199589 |
| 309 | MspFrag83839 | 11 | 19323892 | 19324489 |

(continued)

| SEQ ID NO: | Frag ID | Chromosome | Start | End |
|---|---|---|---|---|
| 310 | MspFrag84490 | 11 | 43921200 | 43921449 |
| 311 | MspFrag84518 | 11 | 44286856 | 44287176 |
| 312 | MspFrag85089 | 11 | 58487399 | 58488005 |
| 313 | MspFrag85656 | 11 | 63640294 | 63640522 |
| 314 | MspFrag85976 | 11 | 64496008 | 64496486 |
| 315 | MspFrag86495 | 11 | 65945827 | 65946236 |
| 316 | MspFrag86866 | 11 | 67527006 | 67527364 |
| 317 | MspFrag86939 | 11 | 67937373 | 67937857 |
| 318 | MspFrag87160 | 11 | 69602771 | 69603307 |
| 319 | MspFrag87185 | 11 | 69863028 | 69863693 |
| 320 | MspFrag87210 | 11 | 70329201 | 70329876 |
| 321 | MspFrag87698 | 11 | 76059797 | 76059981 |
| 322 | MspFrag88140 | 11 | 93774380 | 93774585 |
| 323 | MspFrag88235 | 11 | 95551592 | 95552011 |
| 324 | MspFrag88395 | 11 | 106833824 | 106834052 |
| 325 | MspFrag88411 | 11 | 107304811 | 107304985 |
| 326 | MspFrag88517 | 11 | 110916170 | 110916785 |
| 327 | MspFrag88655 | 11 | 113989177 | 113989682 |
| 328 | MspFrag88982 | 11 | 118710713 | 118711261 |
| 329 | MspFrag89183 | 11 | 122571813 | 122572088 |
| 330 | MspFrag89408 | 11 | 126267744 | 126268359 |
| 331 | MspFrag89444 | 11 | 128007477 | 128008054 |
| 332 | MspFrag89848 | 12 | 432342 | 432620 |
| 333 | MspFrag89865 | 12 | 440326 | 440703 |
| 334 | MspFrag90004 | 12 | 1887654 | 1887972 |
| 335 | MspFrag90137 | 12 | 3472552 | 3472916 |
| 336 | MspFrag90140 | 12 | 3473198 | 3473610 |
| 337 | MspFrag90376 | 12 | 6626277 | 6626591 |
| 338 | MspFrag91076 | 12 | 28018747 | 28019241 |
| 339 | MspFrag92237 | 12 | 50913530 | 50913916 |
| 340 | MspFrag92520 | 12 | 52761839 | 52762613 |
| 341 | MspFrag92533 | 12 | 52831831 | 52832592 |
| 342 | MspFrag92849 | 12 | 56290306 | 56290717 |
| 343 | MspFrag93471 | 12 | 76221553 | 76221851 |
| 344 | MspFrag93929 | 12 | 100105780 | 100106149 |
| 345 | MspFrag94051 | 12 | 103034912 | 103035336 |
| 346 | MspFrag94345 | 12 | 108603802 | 108604232 |
| 347 | MspFrag94367 | 12 | 108636999 | 108637342 |

(continued)

| SEQ ID NO: | Frag ID | Chromosome | Start | End |
|---|---|---|---|---|
| 348 | MspFrag95107 | 12 | 119463497 | 119464156 |
| 349 | MspFrag95724 | 12 | 126397709 | 126398319 |
| 350 | MspFrag95754 | 12 | 127714235 | 127714816 |
| 351 | MspFrag95908 | 12 | 130037881 | 130038220 |
| 352 | MspFrag96210 | 12 | 131593486 | 131593921 |
| 353 | MspFrag96227 | 12 | 131632939 | 131633353 |
| 354 | MspFrag96587 | 13 | 19666287 | 19666805 |
| 355 | MspFrag97775 | 13 | 43876711 | 43877202 |
| 356 | MspFrag98223 | 13 | 52674273 | 52674824 |
| 357 | MspFrag98264 | 13 | 57102098 | 57102284 |
| 358 | MspFrag98985 | 13 | 99421760 | 99422234 |
| 359 | MspFrag99113 | 13 | 102224202 | 102224673 |
| 360 | MspFrag99150 | 13 | 104803836 | 104804393 |
| 361 | MspFrag99310 | 13 | 109676095 | 109676754 |
| 362 | MspFrag99457 | 13 | 111003520 | 111003741 |
| 363 | MspFrag99472 | 13 | 111623681 | 111623969 |
| 364 | MspFrag99554 | 13 | 111836670 | 111837162 |
| 365 | MspFrag99668 | 13 | 112696646 | 112696951 |
| 366 | MspFrag100018 | 13 | 113964379 | 113964675 |
| 367 | MspFrag100061 | 14 | 18719759 | 18720152 |
| 368 | MspFrag101138 | 14 | 44792484 | 44793174 |
| 369 | MspFrag102005 | 14 | 64078276 | 64078714 |
| 370 | MspFrag102061 | 14 | 64638719 | 64638995 |
| 371 | MspFrag103295 | 14 | 92767021 | 92767589 |
| 372 | MspFrag103518 | 14 | 97286503 | 97287063 |
| 373 | MspFrag103793 | 14 | 100262666 | 100262888 |
| 374 | MspFrag104383 | 14 | 103840309 | 103840685 |
| 375 | MspFrag104955 | 15 | 19487742 | 19488254 |
| 376 | MspFrag105085 | 15 | 22223532 | 22223950 |
| 377 | MspFrag105101 | 15 | 22751446 | 22752129 |
| 378 | MspFrag105266 | 15 | 26323073 | 26323406 |
| 379 | MspFrag105873 | 15 | 38437638 | 38437690 |
| 380 | MspFrag105880 | 15 | 38446968 | 38447392 |
| 381 | MspFrag107570 | 15 | 66794080 | 66794622 |
| 382 | MspFrag108016 | 15 | 72805958 | 72806255 |
| 383 | MspFrag108348 | 15 | 76073603 | 76074094 |
| 384 | MspFrag110494 | 16 | 807095 | 807318 |
| 385 | MspFrag110545 | 16 | 954593 | 954879 |

(continued)

| SEQ ID NO: | Frag ID | Chromosome | Start | End |
|---|---|---|---|---|
| 386 | MspFrag110579 | 16 | 972953 | 973346 |
| 387 | MspFrag110668 | 16 | 1094736 | 1095111 |
| 388 | MspFrag110793 | 16 | 1333585 | 1333929 |
| 389 | MspFrag110848 | 16 | 1408921 | 1409435 |
| 390 | MspFrag111358 | 16 | 2226616 | 2226830 |
| 391 | MspFrag111585 | 16 | 2756264 | 2756492 |
| 392 | MspFrag111802 | 16 | 3149326 | 3150003 |
| 393 | MspFrag112325 | 16 | 10387218 | 10387406 |
| 394 | MspFrag113247 | 16 | 27656752 | 27657519 |
| 395 | MspFrag113614 | 16 | 30112985 | 30113118 |
| 396 | MspFrag113989 | 16 | 31133694 | 31134196 |
| 397 | MspFrag114087 | 16 | 32003855 | 32004417 |
| 398 | MspFrag114107 | 16 | 32172277 | 32172824 |
| 399 | MspFrag114108 | 16 | 32172825 | 32173259 |
| 400 | MspFrag114138 | 16 | 32593842 | 32594268 |
| 401 | MspFrag114139 | 16 | 32594269 | 32594593 |
| 402 | MspFrag114140 | 16 | 32594594 | 32594816 |
| 403 | MspFrag114205 | 16 | 33113217 | 33113439 |
| 404 | MspFrag114206 | 16 | 33113440 | 33113764 |
| 405 | MspFrag114207 | 16 | 33113765 | 33114191 |
| 406 | MspFrag114218 | 16 | 33169752 | 33169974 |
| 407 | MspFrag114219 | 16 | 33169975 | 33170299 |
| 408 | MspFrag114220 | 16 | 33170300 | 33170726 |
| 409 | MspFrag114804 | 16 | 52881971 | 52882449 |
| 410 | MspFrag115251 | 16 | 65017842 | 65018293 |
| 411 | MspFrag115442 | 16 | 65776185 | 65776573 |
| 412 | MspFrag115870 | 16 | 67977524 | 67977617 |
| 413 | MspFrag116223 | 16 | 74023655 | 74024439 |
| 414 | MspFrag116804 | 16 | 85098845 | 85099404 |
| 415 | MspFrag117255 | 16 | 87152490 | 87152873 |
| 416 | MspFrag118129 | 17 | 1424860 | 1425069 |
| 417 | MspFrag118132 | 17 | 1425742 | 1425962 |
| 418 | MspFrag118488 | 17 | 3262975 | 3263712 |
| 419 | MspFrag118491 | 17 | 3380201 | 3380549 |
| 420 | MspFrag118551 | 17 | 3742185 | 3742440 |
| 421 | MspFrag118936 | 17 | 6557888 | 6557950 |
| 422 | MspFrag118976 | 17 | 6866584 | 6867057 |
| 423 | MspFrag118998 | 17 | 6888109 | 6888394 |

(continued)

| SEQ ID NO: | Frag ID | Chromosome | Start | End |
|---|---|---|---|---|
| 424 | MspFrag119665 | 17 | 11841560 | 11842309 |
| 425 | MspFragl20286 | 17 | 19588958 | 19589326 |
| 426 | MspFrag120416 | 17 | 21214632 | 21214932 |
| 427 | MspFrag120581 | 17 | 23756303 | 23756683 |
| 428 | MspFrag120745 | 17 | 24917063 | 24917287 |
| 429 | MspFrag121117 | 17 | 29507543 | 29508230 |
| 430 | MspFrag121187 | 17 | 30501738 | 30502428 |
| 431 | MspFrag121238 | 17 | 31115713 | 31116237 |
| 432 | MspFrag121549 | 17 | 33919151 | 33919636 |
| 433 | MspFrag121727 | 17 | 34635687 | 34635916 |
| 434 | MspFrag122371 | 17 | 39446974 | 39447439 |
| 435 | MspFrag122729 | 17 | 41181205 | 41181664 |
| 436 | MspFrag122955 | 17 | 43222694 | 43222900 |
| 437 | MspFrag123151 | 17 | 44073827 | 44074263 |
| 438 | MspFrag123180 | 17 | 44159203 | 44159574 |
| 439 | MspFrag123393 | 17 | 45425386 | 45425933 |
| 440 | MspFrag123622 | 17 | 46894551 | 46894949 |
| 441 | MspFrag123625 | 17 | 47100530 | 47100939 |
| 442 | MspFrag123786 | 17 | 53294503 | 53294919 |
| 443 | MspFrag123890 | 17 | 54187494 | 54188029 |
| 444 | MspFrag123955 | 17 | 55397186 | 55397616 |
| 445 | MspFrag124390 | 17 | 60203136 | 60203426 |
| 446 | MspFrag124400 | 17 | 60205707 | 60206091 |
| 447 | MspFrag124610 | 17 | 63706209 | 63706660 |
| 448 | MspFrag124812 | 17 | 69147185 | 69147915 |
| 449 | MspFrag124831 | 17 | 69408959 | 69409615 |
| 450 | MspFrag124844 | 17 | 69615375 | 69616058 |
| 451 | MspFrag124893 | 17 | 69990739 | 69991183 |
| 452 | MspFrag125612 | 17 | 73648109 | 73648558 |
| 453 | MspFrag126928 | 17 | 77787428 | 77787810 |
| 454 | MspFrag126936 | 17 | 77793664 | 77794026 |
| 455 | MspFrag127220 | 17 | 78629464 | 78629723 |
| 456 | MspFrag127254 | 17 | 78640698 | 78640912 |
| 457 | MspFrag127669 | 18 | 7278710 | 7279418 |
| 458 | MspFrag127886 | 18 | 11365685 | 11366062 |
| 459 | MspFrag128414 | 18 | 19973409 | 19973979 |
| 460 | MspFrag128737 | 18 | 31331934 | 31332447 |
| 461 | MspFrag128850 | 18 | 33320380 | 33321106 |

(continued)

| SEQ ID NO: | Frag ID | Chromosome | Start | End |
|---|---|---|---|---|
| 462 | MspFrag128857 | 18 | 33399522 | 33399998 |
| 463 | MspFrag129193 | 18 | 44375040 | 44375381 |
| 464 | MspFrag129644 | 18 | 55091846 | 55092225 |
| 465 | MspFrag130161 | 18 | 72334956 | 72335293 |
| 466 | MspFrag130261 | 18 | 73091680 | 73092166 |
| 467 | MspFrag130315 | 18 | 74367316 | 74367647 |
| 468 | MspFrag130916 | 19 | 356947 | 357309 |
| 469 | MspFrag131108 | 19 | 562513 | 563000 |
| 470 | MspFrag131234 | 19 | 626106 | 626794 |
| 471 | MspFrag131881 | 19 | 1225717 | 1226067 |
| 472 | MspFrag132131 | 19 | 1454713 | 1455193 |
| 473 | MspFrag132416 | 19 | 1856758 | 1857148 |
| 474 | MspFrag132985 | 19 | 2839734 | 2840151 |
| 475 | MspFrag133397 | 19 | 3884765 | 3885169 |
| 476 | MspFrag133709 | 19 | 4736010 | 4736531 |
| 477 | MspFrag133765 | 19 | 4987710 | 4988218 |
| 478 | MspFrag133773 | 19 | 4999483 | 4999813 |
| 479 | MspFrag134007 | 19 | 5865969 | 5866340 |
| 480 | MspFrag134481 | 19 | 8278100 | 8278802 |
| 481 | MspFrag134495 | 19 | 8304633 | 8304844 |
| 482 | MspFrag134595 | 19 | 8566758 | 8567128 |
| 483 | MspFrag134630 | 19 | 9334315 | 9334667 |
| 484 | MspFrag134826 | 19 | 10264682 | 10265092 |
| 485 | MspFrag135107 | 19 | 11354200 | 11354601 |
| 486 | MspFrag135257 | 19 | 12746871 | 12747166 |
| 487 | MspFrag135413 | 19 | 12996583 | 12996817 |
| 488 | MspFrag136002 | 19 | 16298270 | 16298496 |
| 489 | MspFrag136153 | 19 | 17263933 | 17264231 |
| 490 | MspFrag136763 | 19 | 18868351 | 18868732 |
| 491 | MspFrag137207 | 19 | 35627974 | 35628220 |
| 492 | MspFrag138344 | 19 | 43973696 | 43974028 |
| 493 | MspFrag138522 | 19 | 44618313 | 44618420 |
| 494 | MspFrag138648 | 19 | 45421947 | 45422225 |
| 495 | MspFrag138677 | 19 | 45593831 | 45594133 |
| 496 | MspFrag138910 | 19 | 46878438 | 46879162 |
| 497 | MspFrag139579 | 19 | 50974863 | 50975544 |
| 498 | MspFrag140214 | 19 | 53833482 | 53834000 |
| 499 | MspFrag141334 | 19 | 60185911 | 60186130 |

(continued)

| SEQ ID NO: | Frag ID | Chromosome | Start | End |
|---|---|---|---|---|
| 500 | MspFrag141818 | 19 | 61770691 | 61770887 |
| 501 | MspFrag142017 | 19 | 63157706 | 63158406 |
| 502 | MspFrag142439 | 20 | 648609 | 649321 |
| 503 | MspFrag142458 | 20 | 773559 | 773845 |
| 504 | MspFrag142557 | 20 | 1875786 | 1876205 |
| 505 | MspFrag142940 | 20 | 4150615 | 4151066 |
| 506 | MspFrag143616 | 20 | 21441106 | 21441427 |
| 507 | MspFrag143733 | 20 | 22976137 | 22976617 |
| 508 | MspFrag143736 | 20 | 22976785 | 22977176 |
| 509 | MspFrag143825 | 20 | 24569612 | 24570322 |
| 510 | MspFrag143827 | 20 | 24742336 | 24742752 |
| 511 | MspFrag143864 | 20 | 25012556 | 25012953 |
| 512 | MspFrag144226 | 20 | 31770902 | 31771540 |
| 513 | MspFrag144360 | 20 | 33144476 | 33145268 |
| 514 | MspFrag144651 | 20 | 36509200 | 36509785 |
| 515 | MspFrag144826 | 20 | 39792506 | 39792745 |
| 516 | MspFrag144856 | 20 | 41569277 | 41569661 |
| 517 | MspFrag145015 | 20 | 43424513 | 43425108 |
| 518 | MspFrag145066 | 20 | 43896344 | 43897081 |
| 519 | MspFrag145069 | 20 | 43952201 | 43952384 |
| 520 | MspFrag145238 | 20 | 44977062 | 44977342 |
| 521 | MspFrag145431 | 20 | 48273066 | 48273379 |
| 522 | MspFrag145469 | 20 | 49009098 | 49009532 |
| 523 | MspFrag145587 | 20 | 52525004 | 52525348 |
| 524 | MspFrag145647 | 20 | 54635914 | 54636293 |
| 525 | MspFrag145717 | 20 | 55399273 | 55399609 |
| 526 | MspFrag145731 | 20 | 55533586 | 55533993 |
| 527 | MspFrag145848 | 20 | 56850090 | 56850439 |
| 528 | MspFrag145928 | 20 | 57131598 | 57132025 |
| 529 | MspFrag146021 | 20 | 59404205 | 59404898 |
| 530 | MspFrag146035 | 20 | 59903253 | 59903692 |
| 531 | MspFrag146294 | 20 | 60809849 | 60810182 |
| 532 | MspFrag146425 | 20 | 61188038 | 61188341 |
| 533 | MspFrag146427 | 20 | 61189329 | 61189632 |
| 534 | MspFrag146564 | 20 | 61463569 | 61463852 |
| 535 | MspFrag146589 | 20 | 61523181 | 61523518 |
| 536 | MspFrag147018 | 20 | 62158835 | 62159160 |
| 537 | MspFrag147620 | 21 | 33327565 | 33327930 |

(continued)

| SEQ ID NO: | Frag ID | Chromosome | Start | End |
|---|---|---|---|---|
| 538 | MspFrag147887 | 21 | 36990800 | 36991207 |
| 539 | MspFrag147896 | 21 | 36992311 | 36992534 |
| 540 | MspFrag148458 | 21 | 43964947 | 43965429 |
| 541 | MspFrag148624 | 21 | 44930972 | 44931714 |
| 542 | MspFrag148771 | 21 | 45568987 | 45569301 |
| 543 | MspFrag148921 | 21 | 46119009 | 46119510 |
| 544 | MspFrag149461 | 22 | 17536199 | 17536687 |
| 545 | MspFrag149605 | 22 | 18168920 | 18169266 |
| 546 | MspFrag149782 | 22 | 19034057 | 19034356 |
| 547 | MspFrag149784 | 22 | 19035655 | 19035873 |
| 548 | MspFrag149785 | 22 | 19035874 | 19036170 |
| 549 | MspFrag149787 | 22 | 19036333 | 19036659 |
| 550 | MspFrag149788 | 22 | 19036660 | 19037337 |
| 551 | MspFrag149790 | 22 | 19038177 | 19038476 |
| 552 | MspFrag149791 | 22 | 19038477 | 19039097 |
| 553 | MspFrag149792 | 22 | 19039098 | 19039826 |
| 554 | MspFrag149794 | 22 | 19039962 | 19040676 |
| 555 | MspFrag149824 | 22 | 19109258 | 19109530 |
| 556 | MspFrag150393 | 22 | 24071950 | 24072354 |
| 557 | MspFrag150632 | 22 | 28031149 | 28031471 |
| 558 | MspFrag151442 | 22 | 37421867 | 37422481 |
| 559 | MspFrag151528 | 22 | 37962171 | 37962758 |
| 560 | MspFrag151564 | 22 | 38109182 | 38109628 |
| 561 | MspFrag152094 | 22 | 41917375 | 41918092 |
| 562 | MspFrag152213 | 22 | 43445922 | 43446102 |
| 563 | MspFrag152321 | 22 | 44582503 | 44582872 |
| 564 | MspFrag152480 | 22 | 45091310 | 45091573 |
| 565 | MspFrag152489 | 22 | 45194587 | 45195050 |
| 566 | MspFrag152494 | 22 | 45250387 | 45250713 |
| 567 | MspFrag152496 | 22 | 45250831 | 45251397 |
| 568 | MspFrag152632 | 22 | 47145509 | 47145882 |
| 569 | MspFragl52655 | 22 | 47247350 | 47247678 |
| 570 | MspFrag152681 | 22 | 47331247 | 47331652 |
| 571 | MspFrag152714 | 22 | 47818757 | 47819111 |
| 572 | MspFrag152716 | 22 | 47821576 | 47822084 |
| 573 | MspFrag152736 | 22 | 48119202 | 48119610 |
| 574 | MspFrag152748 | 22 | 48288961 | 48289335 |
| 575 | MspFrag153027 | 22 | 48991342 | 48991874 |

(continued)

| SEQ ID NO: | Frag ID | Chromosome | Start | End |
|---|---|---|---|---|
| 576 | MspFrag153087 | 22 | 49023037 | 49023473 |
| 577 | MspFrag153362 | 23 | 106714 | 106947 |
| 578 | MspFrag153363 | 23 | 106948 | 107207 |
| 579 | MspFrag153364 | 23 | 107208 | 107441 |
| 580 | MspFrag153365 | 23 | 107442 | 107957 |
| 581 | MspFrag153563 | 23 | 407042 | 407560 |
| 582 | MspFrag154875 | 23 | 39303900 | 39304278 |
| 583 | MspFrag155418 | 23 | 47418801 | 47419138 |
| 584 | MspFrag155823 | 23 | 52912797 | 52913213 |
| 585 | MspFrag156275 | 23 | 71242026 | 71242406 |
| 586 | MspFrag156306 | 23 | 72006660 | 72007155 |
| 587 | MspFrag156308 | 23 | 72081592 | 72082087 |
| 588 | MspFrag156440 | 23 | 82569986 | 82570585 |
| 589 | MspFrag156491 | 23 | 90495771 | 90495990 |
| 590 | MspFrag156922 | 23 | 114782761 | 114783003 |
| 591 | MspFrag157076 | 23 | 117741123 | 117741602 |
| 592 | MspFrag157770 | 23 | 135838695 | 135839395 |
| 593 | MspFrag158624 | 23 | 154810057 | 154810810 |
| 594 | MspFrag158646 | 24 | 106714 | 106947 |
| 595 | MspFrag158647 | 24 | 106948 | 107207 |
| 596 | MspFrag158648 | 24 | 107208 | 107441 |
| 597 | MspFrag158649 | 24 | 107442 | 107957 |
| 598 | MspFrag158845 | 24 | 407042 | 407560 |
| 599 | MspFrag158867 | 24 | 554703 | 554798 |
| 600 | MspFrag158958 | 24 | 1628781 | 1629129 |

In an embodiment a method 10 is provided, according to Fig. 1. Said method 10 comprises selecting 100 a feature subset comprising at least one post from the methylation classification list according to SEQ ID NO. 1 to SEQ ID NO. 600.

Selecting 100 a feature subset may be performed based on hierarchical clustering with Pearson correlation and complete linkage to characterize the fitness of each feature subset, given a dataset with methylation characterization for of each sample ($s_i$, i=1..M) in a form of a vector $m_i$ ofN values, where $m_{i,j}$ provides the methylation status for the i-th sample and the j-th probe. Typically, some statistical analysis of the measured signal will produce a set of probes (features) to be input to the hierarchical clustering method above.

The feature subset selection 100 uses a Genetic Algorithm (GA), which repetitively evaluate feature subsets based on a *fitness function* that in some way characterizes some property of the feature subset. In an embodiment, hierarchical clustering with Pearson correlation and complete linkage is used as the fitness function to assess how good a feature subset is.

The following example is used to illustrate the principle.

Fig. 2 show a dataset 20 of measurements, in this case 5 samples, which are displayed as 1 to 5 are characterized with 8 features, which are displayed as letters A to H. Figs. 3 and 4 show two feature subsets, generated from the measurements dataset by selecting rows (features) from the dataset. Fig. 3 shows a first feature subset 30 with the 5 samples, which are displayed as 1 to 5, but only four of the features. Fig. 4 shows a second subset 40 with the 5 samples, which are displayed as 1 to 5, but only six of the features.

Next, clustering may be performed. Fig. 5 show clusters, or dendrograms, based on the datasets from Figs. 2 to 4, when subjected to hierarchical clustering with Pearson correlation and complete linkage. Fig. 5A shows a first cluster 51 based on the total dataset 20. Fig. 5B shows a second cluster 52 based on the first feature subset 30 and Fig. 5C shows a third cluster 53 based on the second feature subset 40.

After having clustered the datasets, a ranking of all clustering results is performed. In one embodiment, a cluster analysis method is used for the ranking. For example, it is possible to characterize and rank individual clusters based on their validity, for example in terms of cluster cohesion or separation. This may be done in one of multiple ways well known to a person skilled in the art. Thus, it is possible to rank two or more feature subsets based on the quality of the clusters they generate when used to cluster the samples.

In another embodiment, some property of the samples (e.g. cancer subtype based on pathology) is used for ranking. From this property, the same or related subtypes are grouped together. For example, if the five samples from Figs. 2 to 4 have the following subtype labels associated with them {1=X, 2=X, 3=Y, 4=Y, 5=X} respectively, this would then produce the following label groupings for the three clusters shown in Fig. 5: A: {XXY, YX}; B: {XY, YXX}; C: {XXX, YY}. In this case, the second subset 40, represented by Fig. 5C, is clearly better compared to the first feature subset 30 or the clustering based on the entire dataset 20, since it correctly cluster the subtypes together.

In an embodiment, two clustering outputs $D_1$ and $D_2$, are compared based on the clusters.. First, N ($C_1$, $C_2$, ... $C_N$) clusters are obtained based on the dendrogram, produced by the clustering. Then, a property is computed based on the clusters, such as the popular method of silhouette width- SIL($C_i$). Now a single-number characterization of a clustering is obtained by the formula:

$$\text{AVGSIL(D)} = (\text{SUM[i=1..N] SIL}(C_i))/\text{N}$$

By comparing AVGSIL($D_1$) and AVGSIL($D_2$), it maybe determined which clustering is preferable.In another embodiment, build a data structure G is built in form of a matrix with dimensions N x L, where L is the number of distinct labels available for the samples. With labels {X. Y}, L = 2, or for labels {normal, aggressive cancer, non-aggressive cancer} L = 3. Then for each cluster i (i=1..N) L values are obtained in the following manner for each element $g_{ij}$ from G:

$g_{ij}$ = count(sample in cluster i and has label j)

Now, it is possible to compute uniformity of each cluster $C_i$:

$$\text{UNIFORMITY}(C_i) = \text{max(counts in row i in G)/sum(counts in row i in G)}$$

Finally, the clustering is characterized with:

$$\text{AVGUNIFORMITY(D)} = \text{SUM[i=1..N] (UNIFORMITY}(C_i))/\text{N}$$

as a single-number characterization of a clustering. By comparing AVGUNIFORMITY ($D_1$) and AVGUNIFORMITY ($D_2$) it may be determined which clustering is preferable.

Iterative repetition of this selection process gradually refines the quality of the clustering of the feature subsets discovered by the GA. After a number of repetitions, all evaluated features subsets can be further filtered based on their performance during the GA execution. In one embodiment, feature subsets are sorted by the average clustering performance in stratification of the clinical samples. In another embodiment, feature subsets, in addition to the average performance, are filtered based on their persistent re-evaluation. In other words, feature subsets that are repeatedly selected for further evaluation are preferred to feature subsets that are dropped from consideration only after a few iterations. The final output of a GA feature subset selection is to run multiple instances with different initial conditions, and merge the filtered feature subsets from each of these instances. Feature subsets from one such evaluation are listed in Table 3A. Furthermore, a cumulative characterization of a collection of GA runs can be obtained and used to generate feature subsets that aggregate the feature subsets in single set of subsets. In one embodiment, the appearance of each feature in feature subsets is counted and a total histogram is obtained giving the degree of utilization of each of the 600 features. Based on this information and for example in one embodiment the frequencies of the pairwise occurrences of the 600 features are used to build feature subsets that summarize the GA run in a single set of subsets, a so called trend pattern. Table 3B provides such feature subset of lengths 45 and 60.

Examples of feature subsets are provided in Tables 2, 3A and 3B. Thus, in an embodiment, the feature subset comprises the CpG dinucleotides according to one of the selections listed in Table 2.

*Table 2.* Feature subsets. Each subset comprise a selection of sequences indicated by numbers corresponding to the FragID:s in table 1.

| Selection number: | FragID:s |
|---|---|
| 1 | 152494, 110545, 1212, 55649, 102005, 129193, 86866, 89848, 1601, 153363, 158647, 1311, 128850, 19926, 123622, 149824, 72674, 150393, 10496, 17697, 95107, 85656, 65670, 55275, 149782, 124610, 124844, 49687, 14334, 757, 157076, 79207, 11782, 120745, 127220, 114108, 22036, 11474, 52434, 136153, 110848, 90376, 145015, 80728, 99113, 158958, 110494, 47510, 26073, 71105, 20024, 10537, 145717, 146294, 1534, 50717, 24273, 143733, 71090, 92849, 111358, 57442, 80168, 61099, 80989, 22213, 141818, 71700 |
| 2 | 152494, 1650, 102005, 14197, 21537, 110668, 158646, 13583, 73586, 38815, 19926, 114107, 103295, 80645, 149824, 127886, 115442, 151564, 113247, 38281, 126936, 121549, 74598, 65670, 55275, 80954, 1241, 118491, 142017, 1377, 105085, 120745, 3535, 36661, 87210, 110848, 138677, 145015, 143616, 8778, 26073, 25164, 9703, 145717, 72461, 1339, 122371, 133709, 27379, 56289, 17091, 153087, 5525, 146564, 57442, 80112, 28326, 113989, 157770, 147896, 98985, 121727, 73907, 9029 |
| 3 | 152494, 110545, 55649, 133765, 114140, 129193, 5071, 86866, 99554, 72675, 45501, 52027, 1173, 19926, 153364, 103295, 123622, 149824, 5104, 151564, 118551, 98223, 14203, 147018, 65670, 4389, 105101, 147620, 149788, 55218, 118491, 118129, 152681, 64725, 39543, 87210, 38910, 80728, 153563, 71121, 71105, 152094, 50717, 87160, 71090, 33136, 76797, 78440, 26333, 145587, 63043, 50444, 5980, 9937, 7359, 158867, 141818 |
| 4 | 110545, 86939, 55649, 102005, 152632, 129193, 86866, 103518, 153363, 158647, 145928, 7228, 67459, 19926, 10427, 4823, 149824, 14609, 149605, 47435, 92237, 152489, 85089, 98223, 108348, 65670, 105101, 118491, 149792, 757, 10623, 118129, 27685, 99472, 36661, 87210, 90376, 138677, 152716, 158624, 149787, 148624, 60779, 71105, 152094, 123955, 50717, 73062, 42953, 80169, 42441, 78440, 119665, 113989, 10916, 118998, 145587, 102061,151528 |
| 5 | 152494, 110545, 55649, 102005, 25023, 158649, 130916, 114218, 74424, 80975, 73586, 1173, 114107, 32667, 103295, 126928, 115442, 127254, 134481, 147018, 121549, 110579, 65670, 14202, 147620, 96587, 149788, 14254, 757, 121238, 1377, 120745, 120286, 87210, 38910, 25187, 90376, 149787, 55475, 99113, 8778, 99150, 71121, 92533, 71105, 9703, 82920, 149785, 14451, 122371, 1534, 29324, 10916, 145587, 63043, 87698, 27677, 156491, 20225 |
| 6 | 152494, 110545, 80343, 55649, 1650, 114140, 102005, 129193, 144651, 99554, 158647, 149824, 115442, 71104, 52792, 113247, 126936, 52897, 85656, 65670, 68271, 55275, 147620, 96587, 38714, 130315, 757, 121238, 5190, 116223, 148458, 87210, 110848, 90376, 145015, 8778, 31913, 26073, 99150, 149790, 122729, 92520, 71105, 2123, 15066, 152094, 72461, 130161, 73062, 94051, 5525, 4820, 1391, 108016, 157770, 46277, 134630, 7153, 158867,9029 |
| 7 | 110545, 114140, 102005, 25023, 130916, 129193, 99554, 65671, 153363, 158646, 128850, 13583, 7228, 19926, 158648, 45007, 149824, 47435, 92237, 152496, 138648, 116804, 65670, 4389, 147620, 140214, 14231, 99472, 148458, 1249, 87210, 26133, 152716, 93471, 115251, 71121, 25164, 71216, 133709, 123786, 25517, 94051, 36595, 5525, 80169, 108016, 103793, 146564, 54796, 156440, 35700, 2643, 143864, 115870, 11354, 71700 |
| 8 | 110545, 86939, 55649, 1650, 129193, 99554, 62044, 152321, 72675, 120416, 128414, 60291, 152655, 80645, 149824, 72674, 127886, 56402, 132985, 95107, 152496, 117255, 138648, 134481, 147018, 121549, 65670, 55275, 4389, 124610, 20895, 66071, 136002, 1377, 118129, 127220, 36661, 11474, 145015, 39760, 48491, 99113, 94345, 125612, 47510, 31913, 122729, 71105, 27268, 82920, 149785, 154875, 1534, 123955, 133709, 50717, 142439, 71090, 80989, 72750, 46277, 14656, 121727, 113614, 27495, 88140 |

(continued)

| Selection number: | FragID:s |
|---|---|
| 9 | 152494, 110545, 1211, 55649, 152714, 129193, 114087, 152321, 153363, 80854, 128414, 13583, 45501, 63267, 60291, 80645, 9601, 4823, 14921, 115442, 151564, 132985, 47435, 92237, 95107, 152496, 114207, 65670, 55275, 4389, 66146, 38491, 149788, 114206, 118132, 757, 71581, 99668, 136002, 76422, 123180, 148458, 87210, 136153, 110848, 137207, 45409, 7116, 60779, 1324, 131108, 138910, 15478, 138344, 149785, 60445, 68970, 42953, 71090, 80169, 59067, 80112, 131234, 10916, 118998, 63043, 87698, 156491, 113614 |
| 10 | 152494, 55649, 158649, 33381, 129193, 38485, 86866, 1601, 153363, 158646, 72675, 128850, 13583, 4109, 38815, 63267, 19926, 103295, 79123, 4823, 80726, 115442, 25715, 71104, 92237, 152496, 134481, 1359, 65670, 55275, 77777, 114219, 118132, 149792, 757, 27685, 71089, 120745, 3535, 36661, 52666, 148458, 56504, 87210, 110848, 39760, 152716, 94345, 47510, 87185, 156306, 71105, 89865, 54424, 95724, 153087, 42953, 71090, 57442, 76797, 70538, 156440, 113989, 13394, 46277, 14656, 20225, 9029, 89183 |
| 11 | 152494, 110545, 12301, 14289, 61152, 1650, 129193, 99554, 153362, 72675, 120416, 149794, 13583, 19926, 32667, 103295, 150393, 92237, 45338, 95107, 96587, 149788, 66071, 14254, 757, 37395, 99668, 14231, 118129, 152681, 155418, 36661, 146589, 148458, 1249, 55611, 110848, 71074, 88982, 32624, 47510, 31913, 26073, 71121, 71105, 145717, 72461, 15478, 118488, 153027, 154875, 133709, 144856, 60445, 73062, 5525, 152213, 92849,80168,63043,90137,56922 |
| 12 | 152494, 110545, 114218, 129193, 86495, 86866, 99554, 45501, 38815, 19926, 158648, 103295, 60291, 10427, 149824, 115442, 151564, 152496, 98223, 147018, 65670, 77777, 55218, 118491, 118132, 33338, 142017, 54824, 55941, 36661, 145238, 87210, 138677, 39760, 45409, 123890, 99150, 71121, 25164, 1324, 71105, 82920, 1534, 123955, 133709, 24273, 60445, 94051, 71090, 80169, 108016, 70538, 78440, 39539, 131234, 134630, 50444, 87698,143864,90137,64684,45650 |
| 13 | 152494, 110545, 55649, 1650, 102005, 158649, 129193, 86495, 86866, 128414, 128850, 146035, 1173, 19926, 153364, 4823, 149824, 14609, 72674, 56402, 118551, 45338, 65670, 114220, 61161, 118491, 130315, 18856, 118129, 148458, 87210, 110848, 134826, 145015, 93471, 48491, 80728, 125612, 46110, 110793, 99150, 71121, 96210, 10393, 2123, 15066, 152094, 27268, 28887, 1339, 133709, 111802, 76797, 42441, 145731, 26333, 147896, 63043,87698,11354,73907,27495 |
| 14 | 114205, 129193, 86866, 99554, 152321, 52027, 80645, 72674, 76619, 151564, 71104, 113247, 47435, 95107, 126936, 136763, 147018, 84490, 65670, 55275, 105101, 20895, 757, 99668, 50853, 27685, 148458, 56504, 110848, 145015, 144226, 89408, 99113, 158958, 125612, 144360, 7116, 26073, 99150, 96210, 71105, 124831, 152094, 71216, 1339, 14451, 88395, 142439, 71090, 92849, 103793, 57442, 119665, 88411, 46277, 10916, 134630, 11354,90137,27495 |
| 15 | 110545, 102005, 129193, 158646, 153362, 73586, 27115, 114138, 127886, 56402, 5104, 115442, 150632, 151564, 71104, 152496, 53338, 114207, 134481, 116804, 65670, 55275, 118132, 130315, 96227, 71581, 118129, 79207, 155418, 123180, 114108, 52666, 1249, 84518, 64725, 87210, 136153, 135257, 145015, 156308, 48491, 152480, 45409, 88982, 26073, 71121, 152094, 40505, 149461, 54424, 28887, 14451, 123955, 56289, 83839, 1391, 108016, 39539, 119665, 88411, 9278, 102061, 27677, 115870, 14656, 56922 |
| 16 | 152494, 110545, 86939, 55649, 102005, 25023, 128737, 129193, 14197, 99554, 152321, 153362, 72675, 13583, 39470, 61003, 103295, 79123, 80726, 118551, 114139, 147620, 96587, 55218, 38714, 8273, 757, 54400, 1823, 15771, 46721, 157076, 71120, 3535, 52666, 11474, 148458, 87210, 57206, 152480, 55475, 89408, 99113, 148624, 7116, 8778, 110793, 47510, 26073, 76120, 25164, 71105, 124831, 127669, 9928, 27268, 154875, 144856, 60445, 88395, 94051, 36595, 71090, 111358, 76797, 50444, 27677, 23738, 76467, 71700 |

(continued)

| Selection number: | FragID:s |
| --- | --- |
| 17 | 110545, 114140, 102005, 129193, 99554, 152321, 128850, 5455, 124390, 149824, 80726, 126928, 56402, 151564, 17697, 47435, 152496, 38417, 147018, 116804, 84490, 65670, 4389, 118491, 757, 99668, 15771, 46721, 118129, 79207, 105085, 127220, 36661, 22036, 148458, 64725, 52146, 87210, 136153, 145015, 31913, 26073, 71105, 15066, 145717, 20134, 130161, 14451, 50717, 17091, 60445, 87160, 33136, 54796, 57442, 76797, 59067, 61099, 20706, 28326, 72750, 76801, 82859, 105873, 27677, 113614, 9029 |
| 18 | 152494, 110545, 55649, 153365, 129193, 21537, 86866, 99554, 72675, 120581, 52027, 19926, 103295, 114138, 1340, 151564, 128857, 132985, 118551, 95107, 152748, 98223, 14203, 65670, 149788, 55218, 118491, 118132, 142017, 118129, 11782, 27685, 99472, 36661, 87210, 38910, 55611, 135107, 135257, 149787, 48491, 80728, 7116, 110793, 99150, 71105, 9928, 40858, 58680, 1534, 133709, 60445, 94051, 5525, 71090, 70538, 80112, 2643, 9937,98985,64684 |

In an embodiment, the feature subset comprises the CpG dinucleotides according to one of the selections listed in Table 3A.

*Table 3A.* Feature subsets. Each subset comprise a selection of sequences indicated by numbers corresponding to the FragID:s in table 1.

| Selection number: | FragID:s |
| --- | --- |
| 1 | 145469, 158845, 1211, 110545, 133397, 99554, 114107, 151442, 99150, 6914, 14609, 74424, 130315, 152714, 115251, 25023, 96210, 117255, 147887, 124390, 135107, 152716, 14457, 149605, 134595, 158958, 86939, 158624, 20895, 56289, 150632, 54400, 114205, 99310, 120416, 123890, 115870 |
| 2 | 145469, 158845, 1211, 110545, 133397, 99554, 114107, 151442, 99150, 6914, 14609, 74424, 130315, 152714, 115251, 25023, 96210, 117255, 147887, 124390, 135107, 152716, 14457, 149605, 134595, 158958, 86939, 158624, 20895, 56289, 150632, 54400, 114205, 99310, 120416, 123890, 115870 |
| 3 | 145469, 158845, 1211, 110545, 133397, 99554, 114107, 151442, 99150, 6914, 14609, 74424, 130315, 152714, 115251, 25023, 96210, 117255, 147887, 118998, 135107, 152748, 14457, 133709, 149605, 1321, 110848, 134595, 158958, 86939, 158624, 20895, 56289, 150632, 54400, 47196, 114205, 99310, 123890, 115870 |
| 4 | 145469, 158845, 1211, 110545, 133397, 99554, 114107, 151442, 99150, 6914, 14609, 74424, 130315, 152714, 115251, 25023, 96210, 117255, 147887, 110848, 135107, 152748, 14457, 149605, 134595, 158958, 86939, 158624, 20895, 56289, 150632, 54400, 47196, 114205, 99310, 120416, 123890, 115870 |
| 5 | 145469, 158845, 1211, 110545, 133397, 99554, 114107, 151442, 99150, 6914, 14609, 74424, 130315, 152714, 115251, 25023, 96210, 117255, 147887, 123955, 135107, 47196, 14457, 149605, 134595, 158958, 86939, 158624, 20895, 56289, 150632, 54400, 114205, 99310,120416,123890,115870 |
| 6 | 145469, 158845, 1211, 110545, 133397, 99554, 114107, 151442, 99150, 6914, 14609, 74424, 130315, 152714, 115251, 25023, 96210, 117255, 147887, 118998, 135107, 47196, 14457, 133709, 149605, 1321, 110848, 134595, 158958, 86939, 158624, 20895, 56289, 150632, 54400, 114205, 99310, 123890, 115870 |
| 7 | 145469, 158845, 1211, 110545, 133397, 99554, 114107, 151442, 99150, 6914, 14609, 74424, 130315, 152714, 115251, 25023, 96210, 117255, 147887, 124390, 135107, 47196, 14457, 149605, 134595, 158958, 86939, 158624, 20895, 56289, 150632, 54400, 114205, 99310, 120416, 123890, 115870 |

(continued)

| Selection number: | FragID:s |
|---|---|
| 8 | 145469, 158845, 1211, 110545, 133397, 99554, 114107, 151442, 99150, 6914, 14609, 74424, 130315, 152714, 115251, 25023, 96210, 117255, 147887, 124390, 135107, 47196, 14457, 149605, 134595, 158958, 86939, 158624, 20895, 56289, 150632, 54400, 114205, 99310, 120416, 123890, 115870 |

In an embodiment, the feature subset comprises the CpG dinucleotides according to one of the selections listed in Table 3B.

*Table 3B.* Feature subsets. Each subset comprise a selection of sequences indicated by numbers corresponding to the FragID:s in table 1.

| Selection number: | FragID:s |
|---|---|
| 1 | 145469, 158845, 1211, 110545, 133397, 99554, 114107, 151442, 99150, 6914, 14609, 74424, 130315, 152714, 117255, 96210, 149605, 146589, 77777, 115251, 25023, 120416, 124390, 147887, 123955, 79123, 152716, 134495, 118998, 133709, 91076, 14457, 110848, 54400, 158624, 134595, 1321, 80728, 146294, 136763, 158958, 115870, 20895, 56289, 59067, 104383, 114205, 130161, 152748, 123890, 142557, 86866, 26333, 80726 |
| 2 | 145469, 158845, 1211, 38910, 133397, 99554, 114107, 151442, 99150, 6914, 14609, 74424, 130315, 152714, 117255, 96210, 149605, 146589, 77777, 115251, 114220, 120416, 124390, 147887, 123955, 79123, 47196, 134495, 118998, 133709, 91076, 14457, 110848, 54400, 158624, 134595, 1321, 80728, 146294, 136763, 158958, 115870, 20895, 56289, 59067, 104383, 114205, 130161, 152748, 123890, 142557, 86866, 135107, 26333, 80726 |
| 3 | 145469, 158845, 1211, 110545, 133397, 99554, 114107, 151442, 99150, 6914, 14609, 74424, 130315, 152714, 117255, 114220, 149605, 146589, 77777, 115251, 25023, 120416, 124390, 147887, 123955, 79123, 47196, 134495, 118998, 133709, 91076, 14457, 110848, 54400, 158624, 134595, 1321, 80728, 146294, 136763, 158958, 115870, 20895, 56289, 59067, 104383, 114205, 130161, 152748, 123890, 142557, 86866, 26333, 80726 |
| 4 | 145469, 158845, 1211, 110545, 133397, 99554, 114107, 151442, 99150, 6914, 14609, 74424, 130315, 152714, 117255, 96210, 149605, 146589, 77777, 115251, 114220, 120416, 124390, 147887, 123955, 79123, 47196, 134495, 118998, 133709, 91076, 14457, 110848, 54400, 158624, 134595, 1321, 80728, 146294, 136763, 158958, 115870, 20895, 56289, 59067, 104383, 114205, 130161, 152748, 123890, 142557, 86866, 135107, 26333, 80726 |
| 5 | 145469, 158845, 1211, 38910, 133397, 99554, 114107, 151442, 99150, 6914, 14609, 74424, 130315, 152714, 117255, 96210, 149605, 146589, 77777, 115251, 114220, 120416, 124390, 147887, 123955, 79123, 47196, 134495, 118998, 133709, 91076, 14457, 110848, 54400, 158624, 134595, 1321, 80728, 146294, 136763, 158958, 115870, 20895, 56289, 5190, 104383, 114205, 130161, 152748, 123890, 142557, 86866, 135107, 26333, 80726 |
| 6 | 145469, 158845, 1211, 110545, 133397, 99554, 114107, 151442, 99150, 6914, 14609, 74424, 130315, 152714, 117255, 96210, 149605, 146589, 77777, 115251, 114220, 120416, 124390, 147887, 123955, 79123, 47196, 134495, 118998, 133709, 91076, 14457, 110848, 54400, 158624, 134595, 1321, 80728, 146294, 136763, 158958, 115870, 20895, 56289, 5190, 104383, 114205, 130161, 152748, 123890, 142557, 86866, 135107, 26333, 80726 |
| 7 | 145469, 158845, 1211, 38910, 133397, 99554, 114107, 151442, 99150, 6914, 14609, 74424, 130315, 152714, 117255, 114220, 149605, 146589, 77777, 115251, 135107, 120416, 124390, 147887, 123955, 79123, 152716, 134495, 118998, 133709, 91076, 14457, 110848, 54400, 158624, 134595, 1321, 80728, 146294, 136763, 158958, 115870, 20895, 56289, 59067, 104383, 114205, 130161, 152748, 123890, 142557, 86866, 26333, 80726 |

(continued)

| Selection number: | FragID:s |
|---|---|
| 8 | 145469, 158845, 1211, 110545, 133397, 99554, 114107, 151442, 99150, 6914, 14609, 74424, 130315, 152714, 117255, 114220, 149605, 146589, 77777, 115251, 135107, 120416, 124390, 147887, 123955, 79123, 47196, 134495, 118998, 133709, 91076, 14457, 110848, 54400, 158624, 134595, 1321, 80728, 146294, 136763, 158958, 115870, 20895, 56289, 5190, 104383, 114205, 130161, 152748, 123890, 142557, 86866, 26333, 80726 |
| 9 | 145469, 158845, 1211, 110545, 133397, 99554, 114107, 151442, 99150, 6914, 14609, 74424, 130315, 152714, 117255, 114220, 149605, 146589, 77777, 115251, 25023, 120416, 124390, 147887, 123955, 79123, 47196, 134495, 118998, 133709, 91076, 14457, 110848, 54400, 158624, 134595, 1321, 80728, 146294, 136763, 158958, 25517, 20895, 56289, 59067, 104383, 114205, 130161,152748,123890,142557,86866,26333,80726 |
| 10 | 145469, 158845, 1211, 110545, 133397, 99554, 114107, 151442, 99150, 6914, 14609, 74424, 130315, 152714, 117255, 96210, 149605, 146589, 77777, 115251, 135107, 120416, 124390, 147887, 123955, 79123, 47196, 134495, 118998, 133709, 91076, 14457, 110848, 54400, 158624, 134595, 1321, 80728, 146294, 136763, 158958, 25517, 20895, 56289, 5190, 104383, 114205, 130161, 152748, 123890, 142557, 86866, 26333, 80726 |

In an embodiment the method 10 comprises determining 120 the methylation status of more than one CpG dinucleotides in a sequence selected from the group of sequences corresponding to the marker panel, resulting in a methylation classification list. There are numerous methods for determining 120 the methylation status of a DNA molecule of a subject, corresponding to the feature subset. The DNA maybe obtained by any method for purifying DNA known to a person skilled in the art. In an embodiment the methylation status is determined 110 by means of one or more of the methods selected form the group of, bisulfite sequencing, pyrosequencing, methylation-sensitive single-strand conformation analysis (MS-SSCA), high resolution melting analysis (HRM), methylation-sensitive single nucleotide primer extension (MS-SnuPE), base-specific cleavage/MALDI-TOF, methylation-specific PCR (MSP), microarray-based methods, msp I cleavage.

In an embodiment, the method 10 also comprises statistically analyzing 120 the methylation classification list, thus obtaining a category of the breast cancer of the subject. This may be done by jointly clustering the subject methylation data and the samples from the clinical study. The resulting clustering is then split in N groups (e.g. by cutting the clustering dendrogram into N sub-trees). The sub-tree containing the subject is evaluated for the categories of breast cancer present in the study samples and the subject sample is assigned the category of the majority samples in the sub-tree.

In an embodiment, the method 10 further comprises classifying (130) the subject as belonging to one of the five major subtypes of breast cancers.

In an embodiment according to Fig. 6, a computer program product 60 is provided. The computer program product 60 is stored on a computer-readable medium, which comprises a first 61, second 62, third 63 and forth 64 code segments arranged, when run by an apparatus having computer-processing properties, for performing all of the method steps defined in some embodiments.

In an embodiment according to Fig. 7, a device 70 for supporting a clinician is provided. Said device comprising means for selecting 700 a feature subset comprising at least one post from the methylation classification list according to SEQ ID NO. 1 to SEQ ID NO. 600. Furthermore, the device 70 comprises means for determining 710 the methylation status of more than one CpG dinucleotides in DNA of a subject, corresponding to the feature subset. Furthermore, the device 70 comprises means for statistically analyzing 720 the methylation classification list, thus obtaining a category of the breast cancer of the subject. Furthermore, the device 70 comprises means for classifying 730 the subject as belonging to one of the five major subtypes of breast cancers. Said means 700, 710, 720, 730 maybe operatively connected to each other.

The invention may be implemented in any suitable form including hardware, software, firmware or any combination of these. However, preferably, the invention is implemented as computer software running on one or more data processors and/or digital signal processors. The elements and components of an embodiment of the invention may be physically, functionally and logically implemented in any suitable way. Indeed, the functionality may be implemented in a single unit, in a plurality of units or as part of other functional units. As such, the invention may be implemented in a single unit, or may be physically and functionally distributed between different units and processors.

Although the present invention has been described above with reference to specific embodiments, it is not intended to be limited to the specific form set forth herein. Rather, the invention is limited only by the accompanying claims and, other embodiments than the specific above are equally possible within the scope of these appended claims.

In the claims, the term "comprises/comprising" does not exclude the presence of other elements or steps. Furthermore, although individually listed, a plurality of means, elements or method steps may be implemented by e.g. a single unit or processor. Additionally, although individual features may be included in different claims, these may possibly advantageously be combined, and the inclusion in different claims does not imply that a combination of features is not feasible and/or advantageous. In addition, singular references do not exclude a plurality. The terms "a", "an", "first", "second" etc do not preclude a plurality. Reference signs in the claims are provided merely as a clarifying example and shall not be construed as limiting the scope of the claims in any way.

LIST OF REFERENCE SIGNS:

| | |
|---|---|
| 10 | A method |
| 100 | A selecting step |
| 110 | A determining step |
| 120 | An analyzing step |
| 130 | A classifying step |
| 20 | A dataset |
| 30 | A first feature subset |
| 40 | A second feature subset |
| 51 | A first cluster |
| 52 | A second cluster |
| 53 | A third cluster |
| 60 | A computer program product |
| 61 | A first code segment |
| 62 | A second code segment |
| 63 | A third code segment |
| 64 | A fourth code segment |
| 70 | A device |
| 700 | Selecing means |
| 710 | Determining means |
| 720 | Analyzing means |
| 730 | Classifying means |
| 1 to 5 | Sample numbers |

### Claims

**1.** Method (10) for the analysis of breast cancer disorders, comprising determining the genomic methylation status of more than one CpG dinucleotides in a sequence selected from the group of sequences consisting of SEQ ID NO. 1 to SEQ ID NO. 600, wherein the analysis is categorization of breast cancer in a subject, and wherein the following steps are performed,

a. selecting (100) a feature subset comprising at least one post from the methylation classification list according to the following list of the FragID:s/SEQ IDs in table 1:

| | | | | | | | | | | |
|---|---|---|---|---|---|---|---|---|---|---|
| Frag ID No | 145469 | 158845 744241 | 1211 30315 | 133397 | 99554 | 114107 | 151442 | 99150 | 6914 | 14609 |
| | 152714 | 117255 7912 | 149605 134495 | 146589 | 77777 | 115251 | 120416 | 124390 | 147887 | 123955 |
| | 118998 | 133709 146294 | 91076 136763 | 14457 | 110848 | 54400 | 158624 | 134595 | 1321 | 80728 |
| | 158958 | 20895 26333 | 56289 80726 | 104383 | 114205 | 130161 | 152748 | 123890 | 142557 | 86866 |
| SEQ ID No | 522 | 598 467 | 4 | 475 | 364 398 | 558 | 360 | 44 | 91 | 278 |
| | 571 | 415 481 | 545 | 535 | 289 410 | 426 | 445 | 538 | 444 | 291 |
| | 423 | 476 490 | 338 | 89 | 389 203 | 593 | 482 | 10 | 301 | 531 |
| | 600 | 111 300 | 214 | 374 | 403 465 | 574 | 443 | 504 | 316 | 129 |

b. determining (110) the methylation status of more than one CpG dinucleotides in DNA of a subject, corresponding to the feature subset;
c. statistically analyzing (120) the methylation classification list, thus obtaining a category of the breast cancer of the subject, and
d. classifying (130) the subject as belonging to one of the five major subtypes of breast cancers, namely luminal A, luminal B, basal, ERBB2 overexpressing, or normal-like,

**2.** Method according to claim 1, wherein the methylation status is determined (110) by means of one or more of the methods selected form the group of,

a. bisulfite sequencing
b. pyrosequencing
c. methylation-sensitive single-strand conformation analysis(MS-SSCA)
d. high resolution melting analysis (HRM)
e. methylation-sensitive single nucleotide primer extension (MS-SnuPE)
f. base-specific cleavage/MALDI-TOF
g. methylation-specific PCR (MSP)
h. microarray-based methods and
i. msp I cleavage.

**3.** A computer program product (60) stored on a computer-readable medium comprising software code adapted to perform the steps of the method according to claim 1-2when executed on a data-processing apparatus.

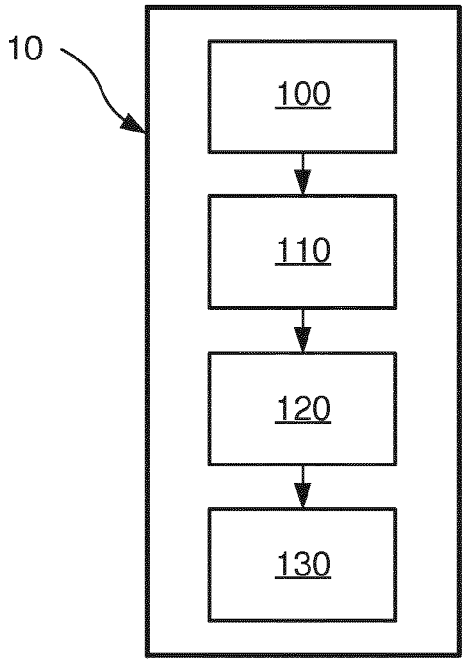
10
100
110
120
130

FIG. 1

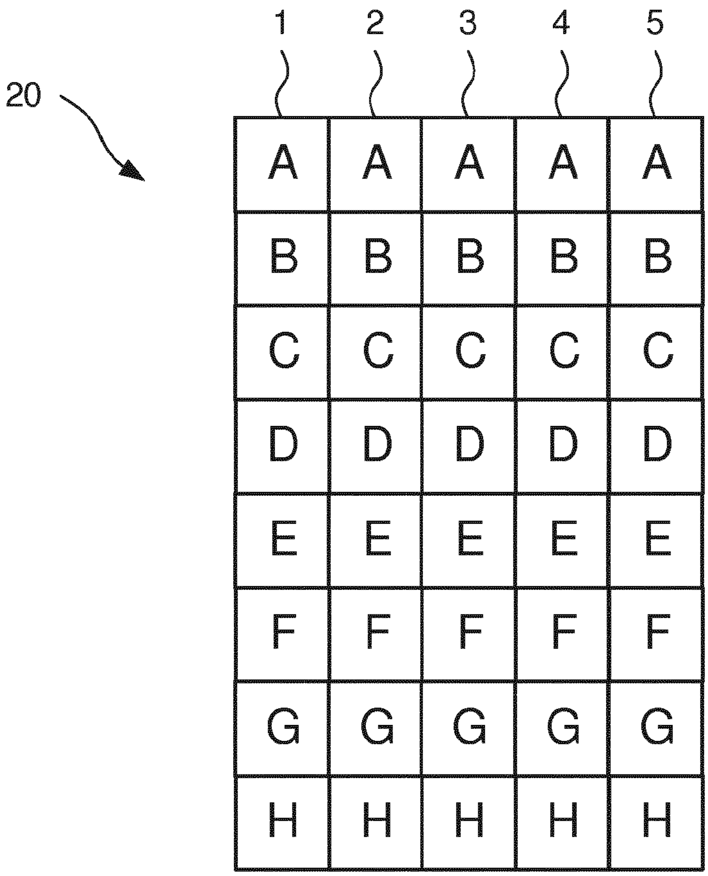
1
2
3
4
5
20
A A A A A
B B B B B
C C C C C
D D D D D
E E E E E
F F F F F
G G G G G
H H H H H

FIG. 2

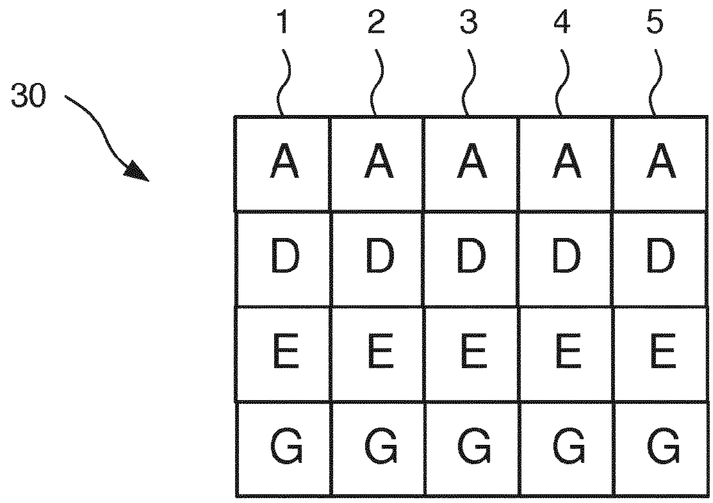
30
1
2
3
4
5
A A A A A
D D D D D
E E E E E
G G G G G

FIG. 3

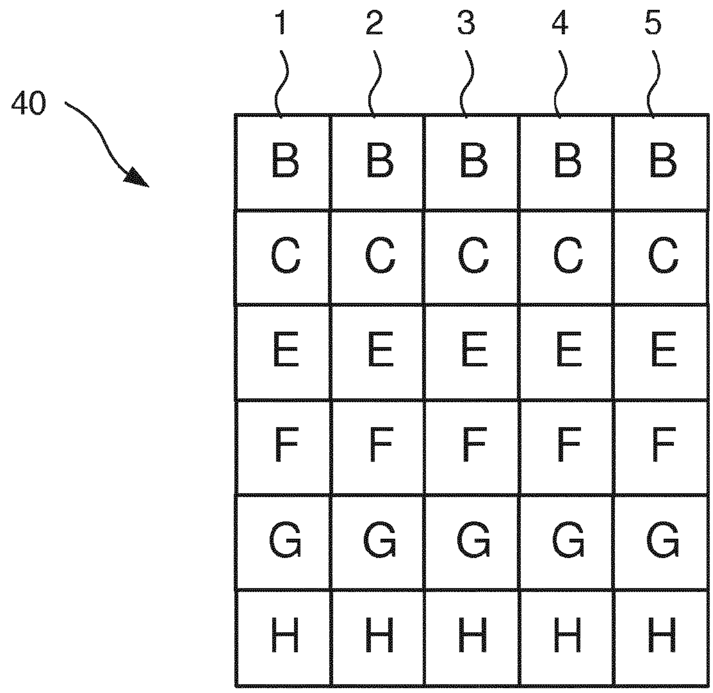
40
1
2
3
4
5
B B B B B
C C C C C
E E E E E
F F F F F
G G G G G
H H H H H

FIG. 4

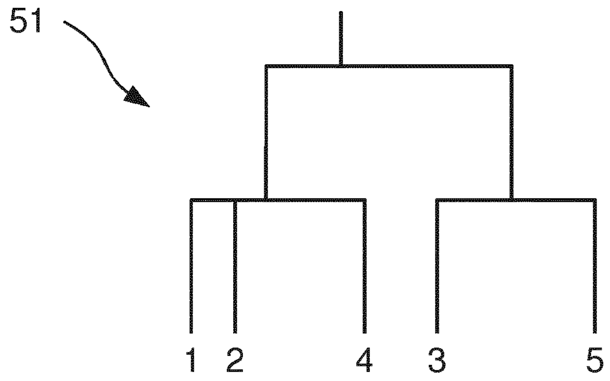


FIG. 5A

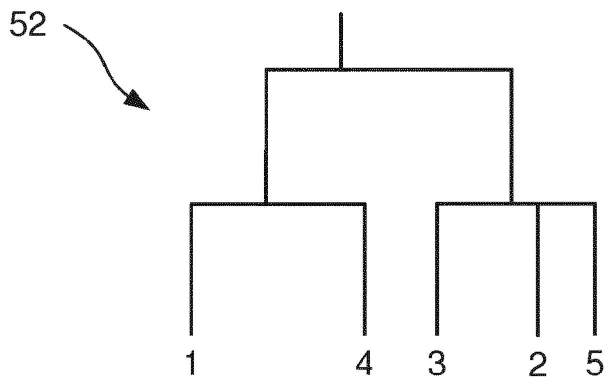


FIG. 5B

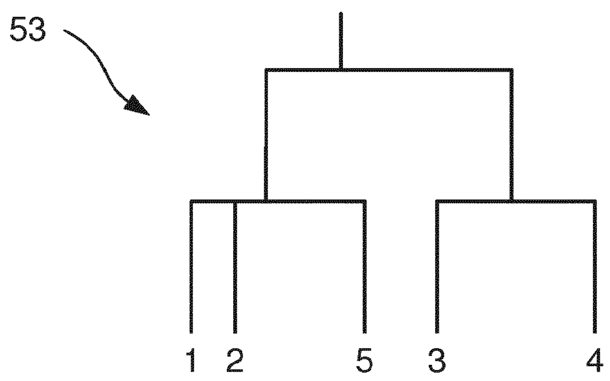


FIG. 5C

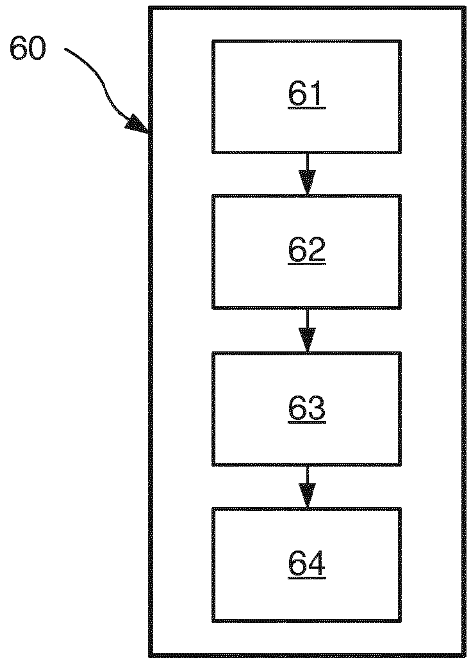


FIG. 6

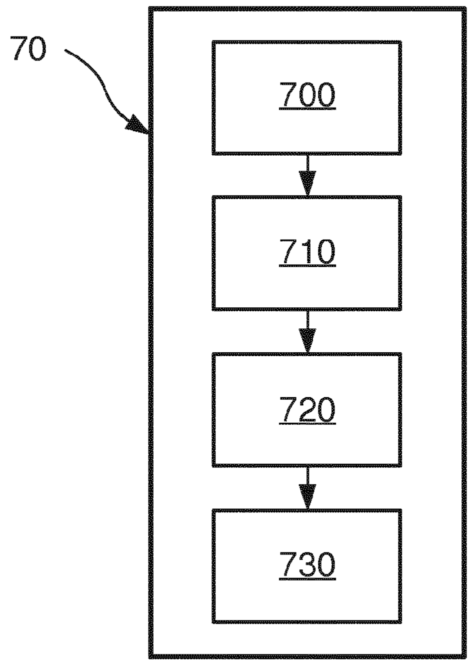


FIG. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20050021240 A1